(19) Europäisches Patentamt
European Patent Office
Office européen des brevets

(11) EP 4 660 182 A1

(12) # EUROPEAN PATENT APPLICATION
published in accordance with Art. 153(4) EPC

(43) Date of publication:
**10.12.2025 Bulletin 2025/50**

(21) Application number: **24749438.8**

(22) Date of filing: **26.01.2024**

(51) International Patent Classification (IPC):
***C07C 233/64*** *(2006.01)* ***C07C 243/38*** *(2006.01)*
***C07C 243/16*** *(2006.01)* ***C07C 15/00*** *(2006.01)*
***C07D 213/00*** *(2006.01)* ***C07D 401/00*** *(2006.01)*
***A61K 31/166*** *(2006.01)* ***A61P 25/02*** *(2006.01)*
***A61P 25/04*** *(2006.01)* ***A61P 29/00*** *(2006.01)*

(52) Cooperative Patent Classification (CPC):
**A61K 31/166; A61P 25/02; A61P 25/04; A61P 29/00; C07C 15/00; C07C 233/64; C07C 243/16; C07C 243/38; C07D 213/00; C07D 401/00**

(86) International application number:
**PCT/BR2024/050025**

(87) International publication number:
**WO 2024/159285 (08.08.2024 Gazette 2024/32)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **30.01.2023 US 202363482209 P**

(71) Applicants:
- **Eurofarma Laboratórios S.A.**
  **06696-000 São Paulo - SP (BR)**
- **Universidade Federal Do Rio De Janeiro - UFRJ**
  **21941-853 Rio de Janeiro -RJ (BR)**

(72) Inventors:
- **BARREIRO, Gabriela**
  **SP 04514-032 São Paulo (BR)**
- **SANT'ANA, Danilo Pereira De**
  **SP 06709-320 São Paulo (BR)**
- **MONTEIRO, Júlia Lammoglia**
  **SP 06704175 São Paulo (BR)**
- **GAMBA, Luis Eduardo Reina**
  **SP 06404-326 São Paulo (BR)**
- **FRAGA, Carlos Alberto Manssour**
  **(deceased) (BR)**
- **BARREIRO, Eliezer Jesus De Lacerda**
  **(deceased) (BR)**
- **LIMA, Lídia Moreira**
  **RJ 21931-220 Rio de Janeiro (BR)**

(74) Representative: **Engelhard, Markus**
**Boehmert & Boehmert**
**Anwaltspartnerschaft mbB**
**Pettenkoferstrasse 22**
**80336 München (DE)**

(54) **NAV1.7- AND/OR NAV1.8-INHIBITING ARYL PYRIDINE COMPOUNDS, PROCESSES FOR THE PREPARATION THEREOF, COMPOSITIONS, USES, METHODS FOR TREATMENT USING SAME, AND KITS**

(57) The present invention relates to aryl pyridine blocking compounds of Nav 1.7 and/or Nav 1.8. More specifically, the present invention is related to aryl pyridines comprising Formula (I), in which the substituents $R_1$ to $R_{10}$ are selected independently of the groups defined in the specification, as well as their preparation processes, compositions comprising at least one of these compounds, uses, treatment methods to treat or prevent pain-related pathologies and kits. The present invention belongs to the fields of medicinal chemistry, organic synthesis, as well as to the treatment of pain-related diseases.

EP 4 660 182 A1

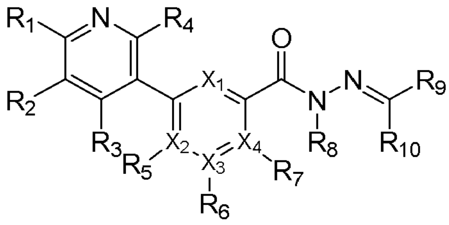

Formula (I)

## Description

## FIELD OF INVENTION

The present invention refers to aryl pyridine blocking compounds of Nav 1.7 and/or Nav 1.8, processes for preparing same, compositions containing these, uses, kits and treatment methods to treat or prevent pain-related pathologies. The present invention belongs to the fields of medicinal chemistry, organic synthesis, as well as to the treatment of pain-related diseases.

## BACKGROUND OF INVENTION

Physiological pain is an important protective mechanism designed to alert the body to real or potential injuries that can put the integrity thereof at risk. Broadly speaking, physiological pain can be classified into nociceptive pain and inflammatory pain. Nociceptive pain is characterized by having a high activation threshold, which remains until the stimulus that generated it is eliminated. Inflammatory pain, which arises as a response to tissue damage, is characterized by having a low activation threshold and is a consequence of the activity of molecular mediators of the inflammatory process in sensitizing nociceptors (Schaible. Langenbecks Arch. Surg. 2004, 389, 237). When these nociceptive processes remain in the absence of noxious stimuli or in response to non noxious stimuli, the protective and repair role of pain loses its functionality, configuring a maladaptive picture of neural plasticity and, as a consequence, a pathological state of chronic pain. Among the syndromes included in this classification, neuropathic pain has a high prevalence and impact today (Smith. Pain. 2020, 161, 1:S127; Cavalli. Int. J. Immunopathol. Pharmacol. 2019, 33:2058738419838383; Bouhassira. Rev Neurol (Paris). 2019, 175(1-2):16; Scholz. Nature Neurosci., 2002, 5,1062; Costigan. Annu. Rev. Neurosci., 2009, 32, 1).

Neuropathic pain is defined by the International Association for the Study of Pain (IASP) as pain initiated or caused by a primary dysfunction or injury in the central and/or peripheral nervous system (Dworkin. Clin. J. Pain, 2002, 18(6), 343). Central neuropathic pain comes from spinal cord injuries or central nervous system diseases such as multiple sclerosis or Parkinson's disease (Ducreux. Brain, 2006, 129, 963). Peripheral neuropathic pain, on the other hand, can be caused by trauma, metabolic disorders, chemical neurotoxicity, infection, or tumor invasion, among others. Among the most common syndromes of neuropathic pain are chemotherapy-induced neuropathic pain, complex regional pain, neuropathy related to viral infection, neuropathy secondary to tumor infiltration, diabetic neuropathy, phantom limb pain, postherpetic neuralgia, trigeminal neuralgia, and postsurgical neuralgia (Pak. Curr. Pain Headache Rep., 2018, 22(2), 9).

Currently, there is no specific treatment for the control of pathologies related to neuropathic pain, however, the first-line alternative consists of the use of opioid analgesics, and - as adjuvants - local anesthetics, anticonvulsants and antidepressants. However, the adverse effects and low efficacy drastically limit the use of these agents in the control of various pain-related pathologies (Kushnarev. Expert Opin. Investig. Drugs, 2020, 29(3), 259; Emery. Expert Opin. Ther. Targets, 2016, 20(8), 975).

Voltage-gated sodium channels (Nav) play a key role in the transmission of pain-related stimuli. These channels are activated in response to membrane depolarization, allowing the generation and propagation of action potentials in neurons (and other electrically excitable cells), by controlling the flow of sodium ions through the membranes. Structurally, voltage-gated sodium channels are heteromeric transmembrane proteins consisting of one $\alpha$ subunit and two $\beta$ auxiliary subunits. The $\alpha$ subunit is organized into four homologous domains (I-IV), each with six transmembrane segments (S1-S6). The S4 segment of each domain is characterized by presenting a conserved region of arginine residues, which act as sensors of the intra- and extracellular electrical environment of the neuron. This mechanism makes it possible to transform changes in the cellular electric field into specific conformational changes that, in turn, regulate the activation, deactivation and inactivation of voltage-gated sodium channels (Catterall. Nat. Chem. Biol., 2020, 16, 1314; Wisedchaisri. Cell., 2019, 178(4), 993; Clairfeuille. Science, 2019, 363, 1302).

In mammals, nine subunits $\alpha$ (Nav 1.1 - Nav 1.9) and four auxiliary subunits $\beta$ ($\beta$1-$\beta$4) have been identified. The $\alpha$ subunits can also be classified according to their susceptibility to blocking by tetrodotoxin (TTX), being classified as sensitive to tetrodotoxin (Nav 1.1, Nav 1.2, Nav 1.3, Nav 1.4, Nav 1.6 and Nav 1.7) or resistant to tetrodotoxin (Nav 1.5, Nav 1.8 and Nav 1.9) (Lera-Ruiz. J. Med. Chem., 2015, 58(18), 7093; Bagal. J. Med. Chem., 2013, 56(3), 593). Each of these subunits $\alpha$ has a different profile of expression and function, so that some of them are essential for the proper functioning of organs such as the heart and/or brain. Thus, the non-selective blockage of these channels is related to several types of adverse effects, such as migraine, epilepsy, paralysis and muscle and cardiac syndromes, among others (Bagal. J. Med. Chem., 2013, 56(3), 593; Bagal. Channels, 2015, 9(6), 360).

Broadly speaking, sodium channels are distributed mainly in the central and peripheral nervous system, in neurons and glia. Nav channels 1.1, 1.2, and 1.3 are primarily expressed in the brain. The Nav 1.4 and Nav 1.5 channels are found primarily in skeletal and cardiac muscles, respectively. Nav 1.6 channels are expressed in the central and peripheral nervous systems, while Nav 1.9 channels are selectively expressed in C-type nociceptive fibers in the dorsal

root ganglion. On the other hand, the Nav 1.7 and Nav 1.8 channels are mainly found in the peripheral nervous system and are directly related to the processes of pain transmission (Law. Drug Discovery Today, 2019, 24(7), 1389; Bagal. Channels (Austin), 2015, 9(6), 360; Lera-Ruiz. J. Med. Chem., 2015, 58(18), 7093).

Nav 1.7 sodium channels are expressed broadly in the olfactory epithelium, sympathetic ganglion, and dorsal root ganglion, predominantly in nociceptive fibers C and A$\delta$. A large amount of evidence supports the important role of Nav 1.7 sodium channels in pain transmission processes. For example, gain-of-function related mutations in the gene (SCN9A), which encodes sodium channel Nav 1.7, are associated with extreme pain disorders such as congenital pain insensitivity, paroxysmal extreme pain disorder, and primary erythromelalgia. On the other hand, mutations related to loss of gene function (SCN9A) are related to congenital insensitivity to pain in individuals who, in general terms, are free of motor or cognitive impairment (Vetter. Pharmacology & Therapeutics, 2017, 172, 73; Ahuja. Science, 2015, 350(6267), 1491; Kingwell. Nat. Rev. Drug Discov., 2019, 18, 321; Safina. J. Med. Chem., 2021, 64, 2953; Luo. J. Med. Chem., 2019, 62, 831; Bankar. Cell Reports, 2018, 24, 3133).

Nav 1.8 sodium channels are most expressed in the peripheral nervous system, widely (but not exclusively) in C-type nociceptive fibers in the dorsal root ganglion. Recent evidence including elevated expression levels of Nav 1.8 in chronic pain states, data with Nav 1.8 *knockout* animals, and analgesic activity of Nav 1.8-specific desensitizing oligodeoxynucleotides, among others (Brown. Bioorg. Med. Chem., 2019, 27(1), 230; Payne. Br. J. Pharmacol., 2015, 172(10), 2654; Bagal. Med. Chem. Lett. 2015, 6(6) 650; Kort. J. Med. Chem. 2008, 51, 407; Zhang. Neuropharmacology, 2010, 59, 201 and 207), support the role of the sodium channel Nav 1.8 in the development and process of pain-related pathologies (Kingwell. Nat. Rev. Drug Discov., 2019, 18, 321; Law. Drug Discovery Today, 2019, 24(7), 1389; Bagal. Channels (Austin), 2015, 9(6), 360; Lera-Ruiz. J. Med. Chem., 2015, 58(18), 7093).

Thus, the voltage-gated sodium channels Nav 1.7 and Nav 1.8 are considered promising therapeutic targets for the treatment of neuropathic pain-related dysfunctions (Kornecook. J. Pharmacol. Exp. Ther., 2017, 362, 146; Kingwell. Nat. Rev. Drug Discov., 2019, 18, 321; Bagal. Channels (Austin), 2015, 9(6), 360; Lera-Ruiz. J. Med. Chem., 2015, 58(18), 7093; Deuis. Neuropharmacology, 2017, 127, 87 and 108; Kushnarev. Expert Opin. Investig. Drugs, 2020, 29(3), 259; McKerrall. Bioorganic & Medicinal Chemistry Lett., 2018, 28, 3141; Emery. Expert Opin. Ther. Targets, 2016, 20(8), 975; Bagal. Bioorganic & Medicinal Chemistry Lett., 2014, 24, 3690).

A large number of compounds have been described in the literature for their ability to act as blockers of Nav 1.7 and 1.8 sodium channels, however, they present a great structural diversity, a fact that does not allow the establishment of a common pharmacophoric group.

The patent literature contains several examples of compounds that act as sodium channel blockers. In particular, Nav 1.7 selective sodium channel blockers are described in US10550080, US9765029, and US10000475. Additionally, some documents describe selective blockers of Nav 1.8 sodium channels such as WO2020261114, WO2020092667, US9163042, WO2014120808, WO2014120815, WO2018213426, WO2019014352, WO2015006280, and US7928107. These documents reveal compounds with different structures from the present invention.

In addition, there are patent documents that describe dual Nav blockers 1.7 and 1.8, including WO2018235851, US8629149, JP2017001991 that reveal, respectively, pyridyl amines, oxopiperazine derivatives and benzoxazolons. However, all these documents reveal compounds with structures and physicochemical characteristics different from the present invention.

In this context, it is advantageous to develop new alternatives of compounds that can act as Nav 1.7 and/or Nav 1.8 blockers that have adequate pharmacological action and, preferably, provide mitigated adverse effects. Therefore, the present invention refers to aryl pyridines as an alternative and/or complement to the treatment of pain-related diseases.

## SUMMARY OF THE INVENTION

The present invention discloses pyridine aryl with blocking activity of Nav 1.7 and/or 1.8 channels against pain-related pathologies, as well as related compositions, uses, kits, treatment methods and preparation processes.

The present invention refers to compound(s) of Formula (I):

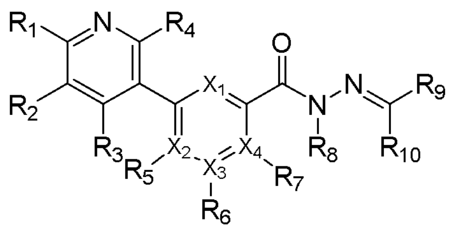

Formula (I)

or a pharmaceutically acceptable salt, hydrate, solvate and isomer thereof, wherein:

- $X_1$, $X_2$, $X_3$, and $X_4$ are independently selected from the group consisting of carbon, CH, or nitrogen,

wherein:

- when at least one of the substituents $X_2$, $X_3$ and $X_4$ is N or CH, at least one of $R_5$, $R_6$ and/or $R_7$, respectively, is null, and
- when two of the substituents $X_1$, $X_2$, $X_3$ and $X_4$ are nitrogen, the other substituents must be carbon;
- $R_1$, $R_2$, $R_3$, and $R_4$ are independently selected from the group consisting of hydrogen, halogen, hydroxy, hydroxy (alkyl $C_2$-$C_4$ linear or branched), alkyl $C_1$-$C_6$ linear or branched, alkoxy $C_1$-$C_6$ linear or branched, cycloalkoxy $C_3$-$C_7$, haloalkoxy $C_1$-$C_6$, haloalkyl $C_1$-$C_6$ hydroxy, hydroxyalkoxy $C_1$-$C_6$ linear or branched, difluoromethyl, trifluoromethyl, azetidinyloxy, azetidinylalkoxy $C_1$-$C_3$, N-alkyl ($C_1$-$C_3$ linear) - azetidinyloxy, N-alkyl ($C_1$-$C_3$ linear) azetidinyalkoxy $C_1$-$C_3$, carboxyl, carboxyalkyl ester $C_1$-$C_3$, imidazole, nitrile, sulfinyl, alkylsulfinyl $C_1$-$C_6$, sulfonamide, alkylsulfonylamide $C_1$-$C_6$, *N,N*-(dialkyl)sulfonylamide $C_1$-$C_6$, sulfonyl, alkylsulfonyl $C_1$-$C_6$, amino (alkyl $C_2$-$C_4$ linear or branched) alkoxy, ($C_1$-$C_6$ alkylamino) alkoxy $C_1$-$C_6$, aminoalkoxy $C_1$-$C_6$, (-S-alkyl $C_1$-$C_6$)alkoxy $C_1$-$C_6$, carbamoyl, *N*-alkylcarbamoyl $C_1$-$C_6$, *N,N*-dialkylcarbamoyl $C_1$-$C_6$, $C_1$-$C_2$ alkoxy containing one or more isotopically enriched atoms such as hydrogen isotope $^2H$ (also represented as 'D' of deuterium), but not limited to the same;
- $R_5$ and $R_7$ are independently selected from the group consisting of hydrogen, halogen, $C_1$-$C_6$ linear or branched alkyl, amine, hydroxy, carbonyl, $C_1$-$C_6$ linear or branched alkoxy, haloalkyl $C_1$-$C_6$; where when $R_5$ is carbonyl, the ring of $X_2$ will have only two unsaturations and $X_2$ will be carbon;
- $R_6$ is selected from hydrogen, halogen, or alkyl $C_1$-$C_2$;
- $R_8$ and $R_{10}$ are independently selected from hydrogen and alkyl $C_1$-$C_6$ linear or branched;
- $R_9$ is selected from the group consisting of aryl, pyridinyl or substituted pyridinyl, pyridone, cycloalkyl $C_3$-$C_7$ saturated or unsaturated, cycloalkyl $C_3$-$C_7$ saturated or unsaturated, alkyl substituted $C_1$-$C_5$, cycloalkyl $C_3$-$C_7$ saturated or unsaturated, halosubstituted or $R_{11}$ or $R_{17}$;
- $R_{11}$ is:

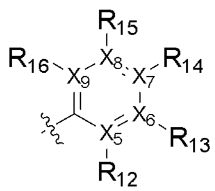

wherein:

- $X_5$, $X_6$, $X_7$, $X_8$, $X_9$ are independently selected from the group consisting of carbon, CH, or nitrogen, where:
    - when at least one of the substituents $X_5$, $X_6$, $X_7$, $X_8$ and $X_9$ is N or CH, at least one of $R_{12}$, $R_{13}$, $R_{14}$, $R_{15}$ and/or $R_{16}$, respectively, is null;
    - $R_{12}$, $R_{13}$, $R_{14}$, $R_{15}$ , and $R_{16}$ are independently selected from the group consisting of hydrogen, halogen, hydroxy, hydroxy (alkyl $C_2$-$C_4$ linear or branched), alkyl $C_1$-$C_4$ linear or branched, $C_1$-$C_4$ linear or branched alkoxy, $C_3$-$C_7$ cycloalkoxy, haloalkoxy, carboxyl, carboxyalkyl ester $C_1$-$C_3$, amine, *N*-alkyl $C_1$-$C_4$-amine, *N,N*-dialkyl $C_1$-$C_4$-amine, amino(alkyl $C_2$-$C_4$ linear or branched), sulfonamide, alkylsulfonylamide $C_1$-$C_6$, *N,N*-(dialkyl) sulfonylamide $C_1$-$C_6$, sulfonyl, alkyl sulfonyl $C_1$-$C_6$, sulfinyl, alkylsulfinyl $C_1$-$C_6$, azetidinyloxy, azetidinylalkoxy $C_1$-$C_3$, alkoxy $C_1$-$C_2$ linear or branched, hydroxyazetidinyl, oxo-dihydropyridinyl, oxo-dihydropyridinylalkoxy $C_1$-$C_4$ linear or branched, $C_1$-$C_6$ alkoxy containing one or more isotopically enriched atoms such as hydrogen isotope $^2H$, but not limited to the same;
    - $R_{17}$ is selected from the group consisting of:

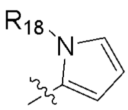
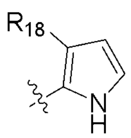
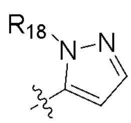
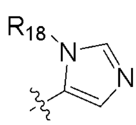
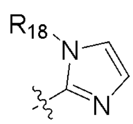

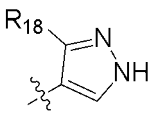
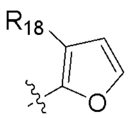
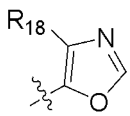
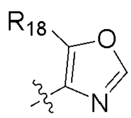
,

wherein:

- $R_{18}$ is independently selected from the group consisting of hydrogen, halogen, hydroxy, hydroxy (alkyl $C_2$-$C_4$ linear or branched), alkyl $C_1$-$C_4$ linear or branched, alkoxy $C_1$-$C_4$ linear or branched, $C_3$-$C_7$ cycloalkoxy, haloalkoxy, nitrile, carboxyl, carboxyalkyl ester $C_1$-$C_3$, amine, *N*-alkylamine, *N,N*-dialkylamine, amino(alkyl $C_2$-$C_4$ linear or branched), sulfonamide, alkylsulfonylamide $C_1$-$C_6$, *N,N*-(dialkyl) sulfonylamide $C_1$-$C_6$, sulfonyl, alkylsulfonyl $C_1$-$C_6$, sulfinyl, alkylsulfinyl $C_1$-$C_6$, azetidinyloxy, azetidinylalkoxy $C_1$-$C_3$, alkoxy $C_1$-$C_2$ linear or branched, hydroxyazetidinyl, alkoxy $C_1$-$C_6$ containing one or more isotopically enriched atoms, such as hydrogen isotopes $^2H$, but not limited to the same.

Additionally, some compounds described in this invention may exist as tautomers, so the individual tautomers, as well as their mixtures, are included in Formula I compounds.

In addition, the present invention further refers to compositions comprising one or more compound(s) of Formula (I) or a salt, hydrate, solvate and pharmaceutically acceptable isomer thereof; and one or more pharmaceutically acceptable excipients.

In addition, kits in accordance with this invention may comprise such compositions and application devices, which may include ampoules, syringes and others. Alternatively, the kits according to this invention comprise more than one compound of Formula (I) arranged in one or more dosage forms, including without limitation, tablets, accompanied by administration instructions.

The present invention further refers to methods of treatment, prevention, relief, suppression and/or control of diseases related to neuropathic pain. Uses of Formula (I) compound(s) to prepare a drug for the treatment of pathologies related to neuropathic pain are also taught. Finally, the present invention teaches processes for obtaining compound(s) of Formula (I).

## DETAILED DESCRIPTION OF THE INVENTION

The present invention presents, in a first embodiment, the compound of Formula (I):

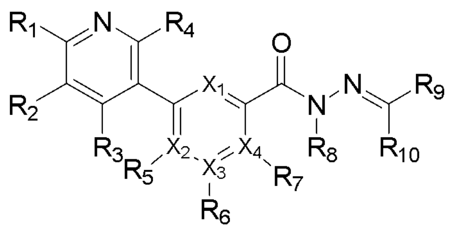

Formula (I)

or a pharmaceutically acceptable salt, hydrate, solvate and isomer thereof, wherein:

- $X_1$, $X_2$, $X_3$, and $X_4$ are independently selected from the group consisting of carbon, CH, or nitrogen, where:
  - when at least one of the substituents $X_2$, $X_3$ and $X_4$ is N or CH, at least one of $R_5$, $R_6$ and/or $R_7$, respectively, is null, and;
  - when two of the substituents $X_1$, $X_2$, $X_3$ and $X_4$ are nitrogen, the remaining substituents must be carbon;
  - $R_1$, $R_2$, $R_3$, and $R_4$ are independently selected from the group consisting of hydrogen, halogen, hydroxy, hydroxy (alkyl $C_2$-$C_4$ linear or branched), alkyl $C_1$-$C_6$ linear or branched, alkoxy $C_1$-$C_6$ linear or branched, cycloalkoxy $C_3$-$C_7$, haloalkoxy $C_1$-$C_6$, haloalkyl $C_1$-$C_6$ hydroxy, hydroxyalkoxy $C_1$-$C_6$ linear or branched, difluoromethyl, trifluoromethyl, azetidinyloxy, azetidinylalkoxy $C_1$-$C_3$, *N*-alkyl ($C_1$-$C_3$ linear) - azetidiniloxy, *N*-alkyl($C_1$-$C_3$ linear) azetidinylalkoxy $C_1$-$C_3$, carboxyl, carboxyalkyl ester $C_1$-$C_3$, imidazole, nitrile, sulfinyl, alkylsulfinyl $C_1$-$C_6$, sulfonamide, alkylsulfonylamide $C_1$-$C_6$, *N,N*-(dialkyl)sulfonylamide $C_1$-$C_6$, sulfonyl, alkylsulfonyl $C_1$-$C_6$, amino(alkyl $C_2$-$C_4$ linear or branched) alkoxy, ($C_1$-$C_6$ alkylamino) alkoxy $C_1$-$C_6$, aminoalkoxy $C_1$-$C_6$, (-S-alkyl $C_1$-$C_6$) alkoxy $C_1$-$C_6$, carbamoyl, *N*-alkylcarbamoyl $C_1$-$C_6$, *N,N*-dialquilcarbamoyl $C_1$-$C_6$, alkoxy $C_1$-$C_2$ containing one or more isotopically enriched atoms such as hydrogen isotope $^2H$, but not limited to the same;
  - $R_5$ and $R_7$ are independently selected from the group consisting of hydrogen, halogen, $C_1$-$C_6$ linear or branched alkyl, amine, hydroxy, carbonyl, $C_1$-$C_6$ linear or branched alkoxy, haloalkyl $C_1$-$C_6$; where when $R_5$ is carbonyl, the ring of $X_2$ will have only two unsaturations and $X_2$ will be carbon;
  - $R_6$ is selected from hydrogen, halogen, or alkyl $C_1$-$C_2$;
  - $R_8$ and $R_{10}$ are independently selected from hydrogen and alkyl $C_1$-$C_6$ linear or branched;

- $R_9$ is selected from the group consisting of aryl, pyridinyl or substituted pyridinyl, pyridone, cycloalkyl $C_3$-$C_7$ saturated or unsaturated, cycloalkyl $C_3$-$C_7$ saturated or unsaturated, substituted alkyl $C_1$-$C_5$, cycloalkyl $C_3$-$C_7$ saturated or unsaturated, halosubstituted or $R_{11}$ or $R_{17}$;
- $R_{11}$ is:

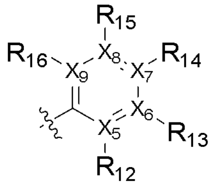

wherein:

- $X_5$, $X_6$, $X_7$, $X_8$, $X_9$ are independently selected from the group consisting of carbon, CH, or nitrogen, wherein:

  - when at least one of the substituents $X_5$, $X_6$, $X_7$, $X_8$ and $X_9$ is N or CH, at least one of $R_{12}$, $R_{13}$, $R_{14}$, $R_{15}$ and/or $R_{16}$, respectively, is null;
  - $R_{12}$, $R_{13}$, $R_{14}$, $R_{15}$ , and $R_{16}$ are independently selected from the group consisting of hydrogen, halogen, hydroxy, hydroxy (alkyl $C_2$-$C_4$ linear or branched), alkyl $C_1$-$C_4$ linear or branched, $C_1$-$C_4$ linear or branched alkoxy, $C_3$-$C_7$ cycloalkoxy, haloalkoxy, carboxyl, carboxyalkyl ester $C_1$-$C_3$, amine, *N*-alkyl $C_1$-$C_4$-amine, *N,N*-dialkyl $C_1$-$C_4$-amine, amino(alkyl $C_2$-$C_4$ linear or branched), sulfonamide, alkylsulfonylamide $C_1$-$C_6$, *N,N*-(dialkyl) sulfonylamide $C_1$-$C_6$, sulfonyl, alkyl sulfonyl $C_1$-$C_6$, sulfinyl, alkylsulfinyl $C_1$-$C_6$, azetidinyloxy, azetidinylalkoxy $C_1$-$C_3$, alkoxy $C_1$-$C_2$ linear or branched, hydroxyazetidinyl, oxo-dihydropyridinyl, oxo-dihydropyridinylalkoxy $C_1$-$C_4$ linear or branched, $C_1$-$C_6$ alkoxy containing one or more isotopically enriched atoms such as hydrogen isotope $^2H$, but not limited to same;
  - $R_{17}$ is selected from the group consisting of:

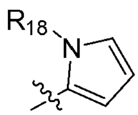 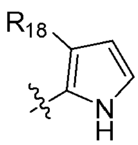 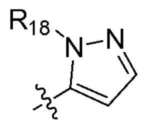 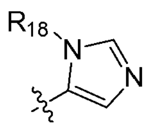 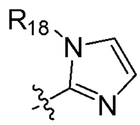

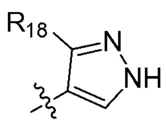 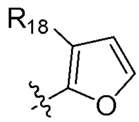 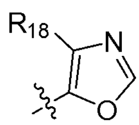 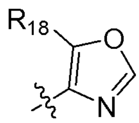 ,

wherein:

  - $R_{18}$ is independently selected from the group consisting of hydrogen, halogen, hydroxy, hydroxy (alkyl $C_2$-$C_4$ linear or branched), alkyl $C_1$-$C_4$ linear or branched, alkoxy $C_1$-$C_4$ linear or branched, cycloalkoxy $C_3$-$C_7$, haloalkoxy $C_1$-$C_6$,, nitrile, carboxyl, carboxyalkyl ester $C_1$-$C_3$, amine, *N*-alkylamine, *N,N*-dialkylamine, amino(alkyl $C_2$-$C_4$ linear or branched), sulfonamide, alkylsulfonylamide $C_1$-$C_6$, *N,N*-(dialkyl) sulfonylamide $C_1$-$C_6$, sulfonyl, alkylsulfonyl $C_1$-$C_6$, sulfinyl, alkylsulfinyl $C_1$-$C_6$, azetidinyloxy, azetidinylalkoxy $C_1$-$C_3$, alkoxy $C_1$-$C_2$ linear or branched, hydroxyazetidinyl, alkoxy $C_1$-$C_6$ containing one or more isotopically enriched atoms, such as hydrogen isotopes $^2H$, but not limited to the same.

As described herein, the compounds provided by Markush Formula I of the present invention comprise multiple variable groups (R, X, etc.). As a person skilled in the art will recognize, the group combinations contemplated by this invention are those combinations that result in the formation of stable or chemically viable compounds.

The term "stable" in this context refers to compounds that are not substantially altered when subjected to conditions that allow their production, detection, and preferably their recovery, purification, and use for one or more of the purposes disclosed herein.

In some embodiments, a stable compound or chemically viable compound is one that is not substantially altered

when maintained at a temperature of 40 °C or less, in the absence of moisture or other chemically reactive conditions, for at least one week.

Additionally, the chemical structures drawn herein are intended to be understood as they would be understood by a person skilled in the art. For example, with respect to Formulas III, IV, X, a person skilled in the art would understand that $X_2$ and $X_3$ are connected by a simple bond, even though the bonds between these groups can be hidden by the labels of the atoms in the chemical structures.

In an embodiment, $X_1$, $X_2$, $X_3$, and $X_4$ are independently selected from the group consisting of carbon and nitrogen, in which when at least one of the substituents $X_2$, $X_3$, and $X_4$ is nitrogen or CH, at least one of $R_5$, $R_6$ and/or $R_7$, respectively, is null, and when two of the substituents $X_1$, $X_2$, $X_3$ and $X_4$ are nitrogen, the remaining substituents must be carbon; $R_1$ is selected from the group consisting of hydroxy, linear or branched $C_1$-$C_6$alkoxy, cycloalkoxy $C_3$-$C_7$, haloalkoxy $C_1$-$C_{6,}$ hydroxyalkoxy C{$_1$-$C_6$ linear or branched, azetidiniloxy, azetidinylalkoxy $C_1$-$C_3$, *N*-alkyl($C_1$-$C_3$ linear) -azetidiniloxy, N-alkyl ($C_1$-$C_3$ linear) azetidinylalkoxy $C_1$-$C_3$, carboxyl, imidazole, nitrile, alkylsulfinyl $C_1$-$C_6$, sulfonamide, alkylsulfonamide $C_1$-$C_6$, aminoalkoxy $C_1$-$C_6$, alkoxy $C_1$-$C_2$ containing but not limited to one or more isotopically enriched atoms such as hydrogen isotope $^2$H; $R_2$ is selected from the group consisting of hydrogen, hydroxy, and imidazole; $R_3$ is selected from the group consisting of hydrogen, hydroxy, alkyl $C_1$-$C_6$ linear and alkoxy $C_1$-$C_6$ linear; $R_4$ is selected from the group consisting of hydrogen, hydroxy, alkyl $C_1$-$C_6$ linear and alkoxy linear $C_1$-$C_6$; $R_5$ is selected from the group consisting of hydrogen, amine, hydroxy, carbonyl, or null when $X_2$ is nitrogen; where when $R_5$ is carbonyl, the ring of $X_2$ will have only two unsaturations and $X_2$ will be carbon; $R_6$ is selected from the group consisting of hydrogen and alkyl $C_1$-$C_6$ linear; $R_7$ is selected from the group consisting of hydrogen, amine, and hydroxy; $R_8$ is selected from the group consisting of hydrogen and alkyl $C_1$-$C_6$ linear; $R_{10}$ is hydrogen; $R_9$ is selected from the group consisting of $R_{11}$ or $R_{17}$; $R_{11}$ is

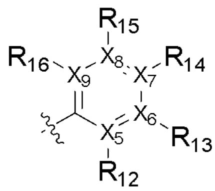

where, $R_{12}$ is hydrogen; $R_{13}$ is selected from the group consisting of hydrogen, hydroxy, hydroxy(alkyl $C_2$-$C_4$ linear or branched), alkoxy $C_1$-$C_4$ linear or branched, cycloalkoxy $C_3$-$C_7$,*N*-alkyl $C_1$-$C_4$-amine,*N,N*-dialkyl $C_1$-$C_4$-amine, sulfonamide, sulfinyl, azetidinyloxy, hydroxyazetidinyl, and alkoxy $C_1$-$C_6$ containing one or more isotopically enriched atoms, such as hydrogen isotope $^2$H, but not limited to the same; $R_{14}$ is selected from the group consisting of hydrogen and halogen; $R_{15}$ is selected from the group consisting of hydrogen, hydroxy, hydroxy (alkyl $C_2$-$C_4$ linear or branched), and *N*-alkyl $C_1$-$C_4$-amine; $R_{16}$ is selected from the group consisting of hydrogen, halogen, hydroxy, and alkyl $C_2$-$C_4$ linear or branched; $R_{17}$ is selected from the group consisting of:

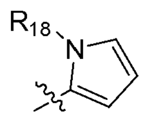 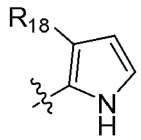 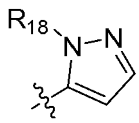 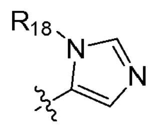 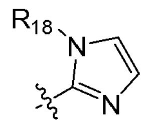

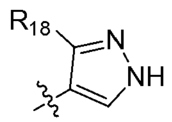 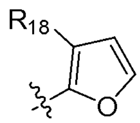 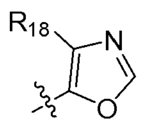 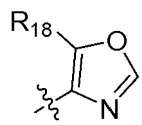 ,

where $R_{18}$ is selected from the group consisting of linear or branched $C_1$-$C_4$ alkyl, linear or branched $C_1$-$C_4$alkoxy, and nitrile.

In a preferred embodiment, $R_1$ is selected from the group consisting of hydroxy, methoxy, ethoxy, propoxy, isopropoxy, butoxy, sec-butoxy, tert-butoxy, cyclopropoxy, cyclobutoxy, cyclopenthoxy, fluoromethoxy, difluoromethoxy, trifluoromethoxy, chloromethoxy, dichloromethoxy, trichloromethoxy, 2-fluoroethoxy, 2,2-difluoroethoxy, 2,2,2-trifluoroethoxy, 2-chloroethoxy, 2,2-dichloroethoxy, 2,2,2-trichloroethoxy, 1-hydroxyethoxy, 2-hydroxyethoxy 1-hydroxypropoxy, 2-hydroxypropoxy, 3-hydroxypropoxy, azetidin-3-yloxy, azetidine-2-yloxy, azetidine-1-yloxy, azetidinylmethoxy, azetidinylethoxy, azetidinylpropoxy, methylazidiniloxy, ethylacetylidiniloxy, methylazetidinylmethoxy, methylazetidinylethoxy, methylazetidinylpropoxy, ethylazetidinylmethoxy, ethylazetidinylpropoxy, propylazetidinylmethoxy, propylazetidinylmethoxy, propylazetidinylpropoxy, carboxyl, imidazole, nitrile, methylsulfinyl, ethylsulfinyl, propylsulfinyl, sulfonamide, methylsulfonamide, ethylsulfonamide, propylsulfonamide, aminomethoxy, 1-aminoethoxy, 2-aminoethoxy, 1-aminopropoxy, 2-aminopropoxy, 3-aminopropoxy, methoxy-$d_1$, methoxy-$d_2$, methoxy-$d_3$, ethoxy-1-$d_1$, ethoxy-2-$d_1$, ethoxy-1,1-$d_2$, ethoxy-1,2-$d_2$, ethoxy-2,2-$d_2$, ethoxy-1,1,1-$d_3$, ethoxy-1,1,2-$d_3$, ethoxy-1,2,2-$d_3$, ethoxy-2,2,2-$d_3$, ethoxy-1,1,2,2-$d_4$, ethoxy-1,2,2,2-$d_4$, ethoxy-$d_5$. Preferably, $R_1$ is selected from the group consisting of hydroxy, ethoxy, cyclopropoxy, difluoromethoxy, trifluoromethoxy, 2-hydroxyethoxy, 2-hydroxypropoxy, azetidine-3-yloxy, azetidine-3-ylmethoxy, azetidine-3-ylethoxy, 1-methylazetidine-3-yloxy, 1-methylazetidine-3-ylmethoxy, 1-methylazetidine-3-ylethoxy, carboxyl, imidazole, nitrile, methylsulfinyl, ethylsulfinyl, sulfonamide, methylsulfonamide, 2-aminoethoxy, 2-aminopropoxy, ethoxy-1,1-$d_2$, ethoxy-2,2,2-$d_3$ and ethoxy-$d_5$; where $R_1$ is imidazole when combined with $R_2$.

In a preferred embodiment, $R_2$ is selected from the group consisting of hydrogen, hydroxy, and imidazole, where $R_2$ is imidazole when combined with $R_1$.

In a preferred embodiment, $R_3$ is selected from the group consisting of hydrogen, hydroxy, methyl, ethyl, propyl, isopropyl, butyl, *sec*-butyl, *tert*-butyl, methoxy, ethoxy, propoxy, isopropoxy, butoxy, *sec*-butoxy, and *tert*-butoxy. Preferably, $R_3$ is selected from the group consisting of hydrogen, hydroxy, methyl, and methoxy.

In a preferred embodiment, $R_4$ is selected from the group consisting of hydrogen, hydroxy, methyl, ethyl, propyl, isopropyl, butyl, *sec*-butyl, *tert*-butyl, methoxy, ethoxy, propoxy, isopropoxy, butoxy, *sec*-butoxy, and *tert*-butoxy. Preferably, $R_4$ is selected from the group consisting of hydrogen, hydroxy, methyl, and methoxy.

In a preferred embodiment, $R_5$ is selected from the group consisting of hydrogen, amine, hydroxy, carbonyl, or null when $X_2$ is nitrogen; where when $R_5$ is carbonyl, the ring of $X_2$ will have only two unsaturations and $X_2$ will be carbon.

In a preferred embodiment, $R_6$ is selected from the group consisting of hydrogen, methyl, ethyl, propyl, isopropyl, butyl, *sec*-butyl, and *tert*-butyl. Preferably, $R_6$ is selected from the group consisting of hydrogen and methyl.

In a preferred embodiment, $R_7$ is selected from the group consisting of hydrogen, amine, and hydroxy.

In a preferred embodiment, $R_8$ is selected from the group consisting of hydrogen, methyl, ethyl, propyl, isopropyl, butyl, *sec*-butyl, and *tert*-butyl. Preferably, $R_8$ is selected from the group consisting of hydrogen and methyl.

In a preferred completion, $R_9$ is selected from the group consisting of $R_{11}$ and $R_{17}$.

In a preferred embodiment, $R_{10}$ is hydrogen.

In a preferred embodiment, $R_{11}$ is

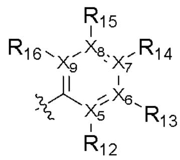

.

In a preferred embodiment, $R_{12}$ is hydrogen.

In a preferred embodiment, $R_{13}$ is selected from the group consisting of hydrogen, hydroxy, 1-hydroxyethyl, 2-hydroxyethyl, 1-hydroxypropyl, 2-hydroxypropyl, 3-hydroxypropyl, 1-hydroxybutyl, 2-hydroxybutyl, 3-hydroxybutyl, 4-hydroxybutyl, methoxy, ethoxy, propoxy, isopropoxy, butoxy, *sec*-butoxy, *tert*-butoxy, cyclopropoxy, cyclobutoxy, cyclopentoxy, aminomethyl, 1-aminoethyl, 2-aminoethyl, 1-aminopropyl, 2-aminopropyl, 3- aminopropyl, dimethylamino, diethylamino, dipropylamino, sulfonamide, sulfinyl, azetidinyl, methylazetidiniloxy, ethylazetidinyloxy, 2-hydroxyazetidinyl, 3-hydroxyazetidinyl, methoxy-$d_1$, methoxy-$d_2$, methoxy-$d_3$, ethoxy-1-$d_1$, ethoxy-2-$d_1$, ethoxy-1, 1-$d_2$, ethoxy-1,2-$d_2$, ethoxy-2,2-$d_2$, ethoxy-1,1,1-$d_3$, ethoxy-1,1,2-$d_3$, ethoxy-1,2,2-$d_3$, ethoxy-2,2,2-$d_3$, ethoxy-1,1,2,2-$d_4$, ethoxy-1,2,2,2-$d_4$, and ethoxy-$d_5$. Preferably, $R_{13}$ is selected from the group consisting of hydrogen, hydroxy, 1-hydroxyethyl, methoxy, ethoxy, isopropoxy, cyclopropoxy, cyclobutoxy, aminomethyl, 1-aminoethyl, dimethylamino, sulfonamide, methylsulfinyl, azetidinyl, azetidinyl, methylazidinyloxy, 3-hydroxyazetidinyl, and methoxy-d3.

In a preferred embodiment, $R_{14}$ is selected from the group consisting of hydrogen, fluorine, chlorine, and bromine. Preferably, $R_{14}$ is selected from the group consisting of hydrogen and fluorine.

In a preferred embodiment, $R_{15}$ is selected from the group consisting of hydrogen, hydroxy, 1-hydroxyethyl, 2-hydroxyethyl, 1-hydroxypropyl, 2-hydroxypropyl, 3-hydroxypropyl, 1-hydroxybutyl, 2-hydroxybutyl, 3-hydroxybutyl, 4-hydroxybutyl, aminomethyl, 1-aminoethyl, 2-aminoethyl, 1-aminopropyl, 2-aminopropyl, and 3-aminopropyl. Preferably, $R_{15}$ is selected from the group consisting of hydrogen, hydroxy, 1-hydroxyethyl, and 1-aminoethyl.

In a preferred embodiment, $R_{16}$ is selected from the group consisting of hydrogen, fluorine, chlorine, bromine, hydroxy, methyl, ethyl, propyl, isopropyl, butyl, *sec*-butyl, and *tert*-butyl. Preferably, $R_{16}$ is selected from the group consisting of hydrogen, fluorine, hydroxy, and methyl.

In a preferred embodiment, $R_{17}$ is selected from the group consisting of:

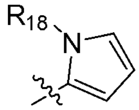 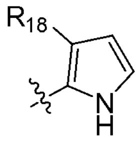 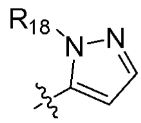 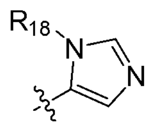 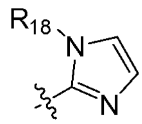

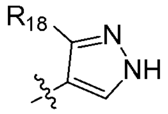 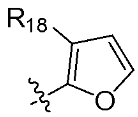 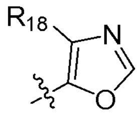 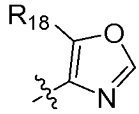 .

In a preferred embodiment, $R_{18}$ is selected from the group consisting of methyl, ethyl, propyl, isopropyl, butyl, *sec*-butyl, *tert*-butyl, methoxy, ethoxy, propoxy, isopropoxy, butoxy, *sec*-butoxy, *tert*-butoxy, and nitrile. Preferably, $R_{18}$ is selected from the group consisting of methyl, ethyl, isopropyl, methoxy, and nitrile.

In a preferred embodiment, the Formula (I) compound(s) is selected from the group(s) consisting of:

(*E*)-6-(6-ethoxypyridine-3-yl)-*N'*-(2-fluoro-5-methoxybenzylidene)pyrazine-2-carbohydrazide (Compound 1);
(*E*)-6-(6-ethoxypyridine-3-yl)-*N'*-(4-fluoro-3-methoxybenzylidene)pyrazine-2-carbohydrazide (Compound 2);
(*E*)-*N'*-(2,4-difluoro-5-methoxybenzylidene)-6-(6-ethoxypyridine-3-yl)pyrazine-2-carbohydrazide (Compound 3);
(*E*)-*N'*-(5-ethoxy-2-fluorobenzylidene)-6-(6-ethoxypyridine-3-yl)pyrazine-2-carbohydrazide (Compound 4);
(*E*)-6-(6-ethoxypyridine-3-yl)-*N'*-(2-fluoro-5-isopropoxybenzylidene)pyrazine-2-carbohydrazide (Compound 5);
(*E*)-*N'*-(5-cyclopropoxy-2-fluorobenzylidene)-6-(6-ethoxypyridine-3-yl)pyrazine-2-carbohydrazide (Compound 6);
(*E*)-6-(6-ethoxypyridine-3-yl)-*N'*-((2-fluoro-5-methoxypyridine-3-yl)methylene)pyrazine-2-carbohydrazide (Compound 7);
(*E*)-6-(6-ethoxypyridine-3-yl)-*N'*-((5-fluoro-2-methoxypyridine-4-yl)methylene)pyrazine-2-carbohydrazide (Compound 8);
(*E*)-6-(6-ethoxypyridine-3-yl)-*N'*-((4-methoxypyridine-2-yl)methylene)pyrazine-2-carbohydrazide (Compound 9);
(*E*)-3-(2-(6-(6-(6-ethoxypyridine-3-yl)pyrazine-2-carbonyl)hydrazinylidene) methyl)-4-fluorobenzoic acid (Compound 10);
(*E*)-N'-(5-(dimethylamino)-2-fluorobenzylidene)-6-(6-ethoxypyridine-3-yl)pyrazine-2-carbohydrazide (Compound 11);
(*E*)-*N'*-((5-(dimethylamino)-2-fluoropyridine-3-yl)methylene)-6-(6-ethoxypyridine-3-yl)pyrazine-2-carbohydrazide (Compound 12);
(*E*)-*N'*-(5-(aminomethyl)-2-fluorobenzylidene)-6-(6-ethoxypyridine-3-yl)pyrazine-2-carbohydrazide (Compound 13);
(*E*)-3-((2-(6-(6-ethoxypyridine-3-yl)pyrazine-2-carbonyl)hydrazinylidene)methyl)-4-fluorobenzenesulfonamide (Compound 14);
(*E*)-6-(6-ethoxypyridine-3-yl)-*N'*-((3-fluoro-6-methoxypyridine-2-yl)methylene)pyrazine-2-carbohydrazide (Compound 15);
(*E*)-6-(6-ethoxypyridine-3-yl)-*N'*-(2-fluoro-5-(methylsulfinyl)benzylidene)pyrazine-2-carbohydrazide (Compound 16);
(*E*)-6-(6-ethoxypyridine-3-yl)-*N'*-(5-methoxy-2-methylbenzylidene)pyrazine-2-carbohydrazide (Compound 17);
(*E*)-6-(6-ethoxypyridine-3-yl)-*N'*-((5-methoxypyridine-3-yl)methylene)pyrazine-2-carbohydrazide (Compound 18);
(*E*)-6-(6-ethoxypyridine-3-yl)-*N'*-(2-hydroxy-5-methoxybenzylidene)pyrazine-2-carbohydrazide (Compound 19);
(*E*)-*N'*-(5-(azetidine-3-yloxy)-2-fluorobenzylidene)-6-(6-ethoxypyridine-3-yl)pyrazine-2-carbohydrazide (Compound 20);
(*E*)-*N'*-(5-cyclobutoxy-2-fluorobenzylidene)-6-(6-ethoxypyridine-3-yl)pyrazine-2-carbohydrazide (Compound 21);
(*E*)-6-(6-ethoxypyridine-3-yl)-*N'*-(3-(1-hydroxyethyl)benzylidene)pyrazine-2-carbohydrazide (Compound 22);
(*E*)-6-(6-ethoxypyridine-3-yl)-*N'*-(2-fluoro-5-(1-hydroxyethyl)benzylidene)pyrazine-2-carbohydrazide (Compound 23);
(*E*)-6-(6-ethoxypyridine-3-yl)-*N'*-((5-(1-hydroxyethyl)pyridine-3-yl)methylene)pyrazine-2-carbohydrazide (Compound 24);
(*E*)-6-(6-ethoxypyridine-3-yl)-*N'*-((2-fluoro-5-(1-hydroxyethyl)pyridine-3-yl)methylene) pyrazine-2-carbohydrazide (Compound 25);
(*E*)-6-(6-ethoxypyridine-3-yl)-*N'*-(3-(1-hydroxyethyl)-5-methoxybenzylidene)pyrazine-2-carbohydrazide (Compound 26);
(*E*)-6-(6-ethoxypyridine-3-yl)-*N'*-((5-methoxy-2-methylpyridine-3-yl)methylene)pyrazine-2-carbohydrazide (Compound 27);

(*E*)-6-(6-ethoxypyridine-3-yl)-*N'*-((2-fluoro-5-hydroxypyridine-3-yl)methylene)pyrazine-2-carbohydrazide (Compound 28);
(*E*)-*N'*-(3-(1-aminoethyl)-5-methoxybenzylidene)-6-(6-ethoxypyridine-3-yl)pyrazine-2-carbohydrazide (Compound 29);
(*E*)-*N'*-(5-(1-aminoethyl)-2-fluorobenzylidene)-6-(6-ethoxypyridine-3-yl)pyrazine-2-carbohydrazide (Compound 30);
(*E*)-6-(6-ethoxypyridine-3-yl)-*N'*-((4-(1-hydroxyethyl)pyridine-2-yl)methylene)pyrazine-2-carbohydrazide (Compound 31);
(*E*)-6-(6-ethoxypyridine-3-yl)-*N'*-((2-fluoro-5-(3-hydroxyazetidin-1-yl)pyridine-3-yl) methylene)pyrazine-2-carbohydrazide (Compound 32);
(*E*)-*N'*-((5-(azetidin-1-yl)-2-fluoropyridine-3-yl)methylene)-6-(6-ethoxypyridine-3-yl) pyrazine-2-carbohydrazide (Compound 33);
(*E*)-*N'*-((5-(azetidin-3-yloxy)-2-fluoropyridine-3-yl)methylene)-6-(6-ethoxypyridine-3-yl) pyrazine-2-carbohydrazide (Compound 34);
(*E*)-6-(6-ethoxypyridine-3-yl)-*N'*-((2-fluoro-5-((1-methylazetidine-3-yl)oxy)pyridine-3-yl) methylene)pyrazine-2-carbohydrazide (Compound 35);
(*E*)-*N'*-((5-cyclobutoxy-2-fluoropyridine-3-yl)methylene)-6-(6-ethoxypyridine-3-yl)pyrazine-2-carbohydrazide (Compound 36);
(*E*)-6-(6-ethoxypyridine-3-yl)-*N'*-(2-fluoro-5-(3-hydroxyazetidine-1-yl)benzylidene) pyrazine-2-carbohydrazide (Compound 37);
(*E*)-*N'*-(5-(azetidin-1-yl)-2-fluorobenzylidene)-6-(6-ethoxypyridine-3-yl)pyrazine-2-carbohydrazide (Compound 38);
(*E*)-6-(6-ethoxypyridine-3-yl)-*N'*-(2-fluoro-5-((1-methylazetidine-3-yl)oxy)benzylidene) pyrazine-2-carbohydrazide (Compound 39);
(*E*)-6-(6-ethoxypyridine-3-yl)-*N'*-((6-hydroxypyridine-2-yl)methylene)pyrazine-2-carbohydrazide (Compound 40);
(*E*)-6-(6-ethoxypyridine-3-yl)-*N'*-((6-hydroxy-4-methoxypyridine-2-yl)methylene) pyrazine-2-carbohydrazide (Compound 41);
(*E*)-6-(6-ethoxypyridine-3-yl)-*N'*-((2-hydroxy-5-methoxypyridine-3-yl)methylene) pyrazine-2-carbohydrazide (Compound 42);
(*E*)-6-(6-ethoxypyridine-3-yl)-*N'*-((2-hydroxy-6-methoxypyridine-4-yl)methylene) pyrazine-2-carbohydrazide (Compound 43);
(*E*)-6-(6-ethoxypyridine-3-yl)-*N'*-(2-fluoro-3-hydroxy-5-methoxybenzylidene) pyrazine-2-carbohydrazide (Compound 44);
(*E*)-6-(6-ethoxypyridine-3-yl)-*N'*-(2-hydroxy-5-methoxybenzylidene)pyrazine-2-carbohydrazide (Compound 45);
(*E*)-6-(6-ethoxypyridine-3-yl)-*N'*-(2-fluoro-3-(1-hydroxyethyl)-5-methoxybenzylidene) pyrazine-2-carbohydrazide (Compound 46);
(*E*)-6-(6-ethoxypyridine-3-yl)-*N'*-(2-fluoro-3-(1-hydroxyethyl)benzylidene)pyrazine-2-carbohydrazide (Compound 47);
(*E*)-6-(6-ethoxypyridine-3-yl)-*N'*-((6-(1-hydroxyethyl)-4-methoxypyridine-2-yl)methylene) pyrazine-2-carbohydrazide (Compound 48);
(*E*)-6-(6-ethoxypyridine-3-yl)-*N'*-((6-(1-hydroxyethyl)pyridine-2-yl)methylene)pyrazine-2-carbohydrazide (Compound 49);
(*E*)-6-(6-ethoxypyridine-3-yl)-*N'*-((1-methyl-1*H*-pyrrole-2-yl)methylene)pyrazine-2-carbohydrazide (Compound 50);
(*E*)-6-(6-ethoxypyridine-3-yl)-*N'*-((1-methyl-1*H*-pyrazole-5-yl)methylene)pyrazine-2-carbohydrazide (Compound 51);
(*E*)-6-(6-ethoxypyridine-3-yl)-*N'*-((1-ethyl-1*H*-pyrazole-5-yl)methylene)pyrazine-2-carbohydrazide (Compound 52);
(*E*)-6-(6-ethoxypyridine-3-yl)-*N'*-((1-isopropyl-1*H*-imidazole-5-yl)methylene)pyrazine-2-carbohydrazide (Compound 53);
(*E*)-6-(6-ethoxypyridine-3-yl)-*N'*-((1-isopropyl-1*H*-pyrazole-5-yl)methylene)pyrazine-2-carbohydrazide (Compound 54);
(*E*)-6-(6-ethoxypyridine-3-yl)-*N'*-((3-methoxyfuran-2-yl)methylene)pyrazine-2-carbohydrazide (Compound 55);
(*E*)-6-(6-ethoxypyridine-3-yl)-*N'*-((3-methyl-1*H*-pyrrole-2-yl)methylene)pyrazine-2-carbohydrazide (Compound 56);
(*E*)-6-(6-ethoxypyridine-3-yl)-*N'*-((3-methyl-1*H*-pyrazole-4-yl)methylene)pyrazine-2-carbohydrazide (Compound 57);
(*E*)-6-(6-ethoxypyridine-3-yl)-*N'*-((1-methyl-1*H*-imidazole-5-yl)methylene)pyrazine-2-carbohydrazide (Compound 58);
(*E*)-*N'*-((3-cyano-1*H*-pyrrole-2-yl)methylene)-6-(6-ethoxypyridine-3-yl)pyrazine-2-carbohydrazide (Compound 59);
(*E*)-6-(6-ethoxypyridine-3-yl)-*N'*-((1-ethyl-1*H*-imidazole-2-yl)methylene)pyrazine-2-carbohydrazide (Compound 60);

(*E*)-6-(6-ethoxypyridine-3-yl)-*N*'-((4-methyloxazole-5-yl)methylene)pyrazine-2-carbohydrazide (Compound 61);
(*E*)-6-(6-ethoxypyridine-3-yl)-*N*'-((5-methyloxazole-4-yl)methylene)pyrazine-2-carbohydrazide (Compound 62);
(*E*)-5-amino-6-(6-ethoxypyridine-3-yl)-*N'*-(2-fluoro-5-methoxybenzylidene) pyrazine-2-carbohydrazide (Compound 63);
(*E*)-3-amino-6-(6-ethoxypyridine-3-yl)-*N'*-(2-fluoro-5-methoxybenzylidene) pyrazine-2-carbohydrazide (Compound 64);
(*E*)-6-(6-ethoxypyridine-3-yl)-*N'*-((2-fluoro-5-methoxypyridine-3-yl)methylene)-3-hydroxypyrazine-2-carbohydrazide (Compound 65);
(*E*)-6-(6-ethoxypyridine-3-yl)-N'-(2-fluoro-5-methoxybenzylidene)-5-hydroxypyrazine-2-carbohydrazide (Compound 66);
(*E*)-6-(6-ethoxypyridine-3-yl)-*N'*-((2-fluoro-5-methoxypyridine-3-yl)methylene)-5-hydroxypyrazine-2-carbohydrazide (Compound 67);
(*E*)-6-(6-ethoxypyridine-3-yl)-*N'*-(2-fluoro-5-methoxybenzylidene)-3-hydroxypyrazine-2-carbohydrazide (Compound 68);
(*E*)-6-(6-ethoxypyridine-3-yl)-5-hydroxy-*N'*-((1-methyl-1*H*-pyrrole-2-yl)methylene)pyrazine-2-carbohydrazide (Compound 69);
(*E*)-6-(6-ethoxypyridine-3-yl)-3-hydroxy-*N'*-((1-methyl-1*H*-pyrrole-2-yl)methylene)pyrazine-2-carbohydrazide (Compound 70);
(*E*) -6'-ethoxy-*N'*-((2-fluoro-5-methoxypyridine-3-yl)methylene)-[2,3'-bipyridine]-6-carbohydrazide (Compound 71);
(*E*)-2-(6-ethoxypyridine-3-yl)-*N'*-((2-fluoro-5-methoxypyridine-3-yl)methylene) pyrimidine-4-carbohydrazide (Compound 72);
(*E*)-4-(6-ethoxypyridine-3-yl)-*N'*-((2-fluoro-5-methoxypyridine-3-yl)methylene) pyrimidine-2-carbohydrazide (Compound 73);
(*E*)-6'-ethoxy-*N'*-((2-fluoro-5-methoxypyridine-3-yl)methylene)-[3,3'-bipyridine]-5-carbohydrazide (Compound 74);
(*E*)-6-(6-ethoxypyridine-3-yl)-*N'*-((2-fluoro-5-methoxypyridine-3-yl)methylene)-4-methyl-5-oxo-4,5-dihydropyrazine-2-carbohydrazide (Compound 75);
(*E*)-6-(6-ethoxypyridine-3-yl)-*N'*-(2-fluoro-5-methoxybenzylidene)-4-methyl-5-oxo-4,5-dihydropyrazine-2-carbohydrazide (Compound 76);
(*E*)-6-(6-cyclopropoxypyridine-3-yl)-*N*'-(2-fluoro-5-methoxybenzylidene)pyrazine-2-carbohydrazide (Compound 77);
(*E*)-6-(6-cyclopropoxypyridine-3-yl)-*N*'-(2,4-difluoro-5-methoxybenzylidene) pyrazine-2-carbohydrazide (Compound 78);
(*E*)-6-(6-cyclopropoxypyridine-3-yl)-*N*'-(2-fluoro-5-isopropoxybenzylidene) pyrazine-2-carbohydrazide (Compound 79);
(*E*)-6-(6-cyclopropoxypyridine-3-yl)-*N*'-(5-ethoxy-2-fluorobenzylidene)pyrazine-2-carbohydrazide (Compound 80);
(*E*)-6-(6-cyclopropoxypyridine-3-yl)-*N*'-(4-fluoro-3-methoxybenzylidene)pyrazine-2-carbohydrazide (Compound 81);
(*E*)-N'-(5-cyclopropoxy-2-fluorobenzylidene)-6-(6-cyclopropoxypyridine-3-yl)pyrazine-2-carbohydrazide (Compound 82);
(*E*)-*N'*-(2-fluoro-5-methoxybenzylidene)-6-(6-(trifluoromethoxy)pyridine-3-yl)pyrazine-2-carbohydrazide (Compound 83);
(*E*)-*N'*-(pyridine-2-ylmethylene)-6-(6-(trifluoromethoxy)pyridine-3-yl)pyrazine-2-carbohydrazide (Compound 84);
(*E*)-*N'*-((4-methoxypyridine-2-yl)methylene)-6-(6-(trifluoromethoxy)pyridine-3-yl)pyrazine-2-carbohydrazide (Compound 85);
(*E*)-*N'*-(pyridine-3-ylmethylene)-6-(6-(trifluoromethoxy)pyridine-3-yl)pyrazine-2-carbohydrazide (Compound 86);
(*E*)-*N'*-(pyridine-4-ylmethylene)-6-(6-(trifluoromethoxy)pyridine-3-yl)pyrazine-2-carbohydrazide (Compound 87);
(*E*)-*N'*-(2-methylbenzylidene)-6-(6-(trifluoromethoxy)pyridine-3-yl)pyrazine-2-carbohydrazide (Compound 88);
(*E*)-*N'*-(2-fluorobenzylidene)-6-(6-(trifluoromethoxy)pyridine-3-yl)pyrazine-2-carbohydrazide (Compound 89);
(*E*)-*N'*-(3-methoxybenzylidene)-6-(6-(trifluoromethoxy)pyridine-3-yl)pyrazine-2-carbohydrazide (Compound 90);
(*E*)-*N'*-(5-ethoxy-2-fluorobenzylidene)-6-(6-(trifluoromethoxy)pyridine-3-yl)pyrazine-2-carbohydrazide (Compound 91);
(*E*)-*N*'-(2-fluoro-5-isopropoxybenzylidene)-6-(6-(trifluoromethoxy)pyridine-3-yl)pyrazine-2-carbohydrazide (Compound 92);
(*E*)-*N'*-(5-(dimethylamino)-2-fluorobenzylidene)-6-(6-(trifluoromethoxy)pyridine-3-yl) pyrazine-2-carbohydrazide (Compound 93);
(*E*)-*N'*-(5-cyclopropoxy-2-fluorobenzylidene)-6-(6-(trifluoromethoxy)pyridine-3-yl)pyrazine-2-carbohydrazide (Compound 94);
(*E*)-*N*'-((2-fluoro-5-methoxypyridine-3-yl)methylene)-6-(6-(trifluoromethoxy)pyridine-3-yl)pyrazine-2-carbohydrazide (Compound 95);
(*E*)-*N*'-((5-fluoro-2-methoxypyridine-4-yl)methylene)-6- (6-(trifluoromethoxy)pyridine-3-yl)pyrazine-2-carbohydrazide (Compound 96);
(*E*)-*N'*-((3-fluoro-6-methoxypyridine-2-yl)methylene)-6-(6-(trifluoromethoxy)pyridine-3-yl)pyrazine-2-carbohydrazide (Compound 97);
(*E*)-*N'*-(2-fluoro-5-methoxybenzylidene)-5-hydroxy-6-(6-(trifluoromethoxy)pyridine-3-yl)pyrazine-2-carbohydrazide (Compound 98);
(*E*)-*N'*-(2-fluoro-5-methoxybenzylidene)-3-hydroxy-6-(6-(trifluoromethoxy)pyridine-3-yl)pyrazine-2-carbohydrazide (Compound 99);
(*E*)-6-(6-(difluoromethoxy)pyridine-3-yl)-*N'*-(2-fluoro-5-methoxybenzylidene) pyrazine-2-carbohydrazide (Compound 100);
(*E*)-6-(6-(difluoromethoxy)pyridine-3-yl)-*N'*-((2-fluoro-5-methoxypyridine-3-yl)methylene)pyrazine-2-carbohydrazide (Compound 101);
(*E*)-6-(6-(difluoromethoxy)pyridine-3-yl)-*N'*-(5-methoxy-2-methylbenzylidene) pyrazine-2-carbohydrazide (Compound 102);
(*E*)-6-(6-(difluoromethoxy)pyridine-3-yl)-*N'*-((5-methoxy-2-methylpyridine-3-yl)methylene)pyrazine-2-carbohydrazide (Compound 103);
(*E*)-6-(6-(difluoromethoxy)pyridine-3-yl)-*N*'-(5-(dimethylamino)-2-fluorobenzylidene) pyrazine-2-carbohydrazide (Compound 104);
(*E*)-6-(6-(difluoromethoxy)pyridine-3-yl)-*N'*-((5-(dimethylamino)-2-fluoropyridine-3-yl)methylene)pyrazine-2-carbohydrazide (Compound 105);
(*E*)-6-(6-(difluoromethoxy)pyridine-3-yl)-*N'*-((5-fluoro-2-methoxypyridine-4-yl)methylene)pyrazine-2-carbohydrazide (Compound 106);
(*E*)-6-(6-(difluoromethoxy)pyridine-3-yl)-*N'*-((5-methoxypyridine-3-yl)methylene)pyrazine-2-carbohydrazide (Compound 107);
(*E*)-6-(6-(difluoromethoxy)pyridine-3-yl)-*N'*-(2-hydroxy-5-methoxybenzylidene) pyrazine-2-carbohydrazide (Compound 108);
(*E*)-6-(6-(difluoromethoxy)pyridine-3-yl)-*N'*-(3-(1-hydroxyethyl)benzylidene) pyrazine-2-carbohydrazide (Compound 109);
(*E*)-6-(6-(difluoromethoxy)pyridine-3-yl)-*N'*-(2-fluoro-5-(1-hydroxyethyl)benzylidene)pyrazine-2-carbohydrazide (Compound 110);
(*E*)-6-(6-(difluoromethoxy)pyridine-3-yl)-*N'*-((5-(1-hydroxyethyl)pyridine-3-yl)methylene)pyrazine-2-carbohydrazide (Compound 111);
(*E*)-6-(6-(difluoromethoxy)pyridine-3-yl)-*N'*-((2-fluoro-5-(1-hydroxyethyl)pyridine-3-yl)methylene)pyrazine-2-carbohydrazide (Compound 112);
(*E*)-6-(6-(difluoromethoxy)pyridine-3-yl)-*N'*-((2-fluoro-5-hydroxypyridine-3-yl)methylene)pyrazine-2-carbohydrazide (Compound 113);
(*E*)-6-(6-(difluoromethoxy)pyridine-3-yl)-*N'*-((4-(1-hydroxyethyl)pyridine-2-yl)methylene)pyrazine-2-carbohydrazide (Compound 114);
(*E*)-6-(6-(difluoromethoxy)pyridine-3-yl)-*N'*-((6-hydroxypyridine-2-yl)methylene) pyrazine-2-carbohydrazide (Compound 115);
(*E*)-6-(6-(difluoromethoxy)pyridine-3-yl)-*N'*-((6-hydroxy-4-methoxypyridine-2-yl)methylene)pyrazine-2-carbohydrazide (Compound 116);
(*E*)-6-(6-(difluoromethoxy)pyridine-3-yl)-*N'*-((2-hydroxy-5-methoxypyridine-3-yl)methylene)pyrazine-2-carbohydrazide (Compound 117);
(*E*)-6-(6-(difluoromethoxy)pyridine-3-yl)-*N'*-((2-hydroxy-6-methoxypyridine-4-yl)methylene)pyrazine-2-carbohydrazide (Compound 118);
(*E*)-6-(6-(difluoromethoxy)pyridine-3-yl)-*N'*-(2-fluoro-3-hydroxy-5-methoxybenzylidene)pyrazine-2-carbohydrazide (Compound 119);
(*E*)-6-(6-(difluoromethoxy)pyridine-3-yl)-*N'*-(2-hydroxy-5-methoxybenzylidene)pyrazine-2-carbohydrazide (Compound 120);
(*E*)-6-(6-(difluoromethoxy)pyridine-3-yl)-*N'*-(2-fluoro-3-(1-hydroxyethyl)-5-methoxybenzylidene)pyrazine-2-carbohydrazide (Compound 121);
(*E*)-6-(6-(difluoromethoxy)pyridine-3-yl)-*N'*-(2-fluoro-3-(1-hydroxyethyl)benzylidene)pyrazine-2-carbohydrazide (Compound 122);
(*E*)-6-(6-(difluoromethoxy)pyridine-3-yl)-*N'*-((6-(1-hydroxyethyl)-4-methoxypyridine-2-yl)methylene)pyrazine-2-carbohydrazide (Compound 123);
(*E*)-6-(6-(difluoromethoxy)pyridine-3-yl)-*N'*-((6-(1-hydroxyethyl)pyridine-2-yl)methylene) pyrazine-2-carbohydrazide (Compound 124);
(*E*)-6'-(difluoromethoxy)-*N'*-((2-fluoro-5-methoxypyridine-3-yl)methylene)-[2,3'-bipyridine]-6-carbohydrazide (Compound 125);
(*E*)-2-(6-(difluoromethoxy)pyridine-3-yl)-*N'*-((2-fluoro-5-methoxypyridine-3-yl)methylene) pyrimidine-4-carbohydrazide (Compound 126);
(*E*)-4-(6-(difluoromethoxy)pyridine-3-yl)-*N'*-((2-fluoro-5-methoxypyridine-3-yl)methylene)pyrimidine-2-carbohydrazide (Compound 127);
(*E*)-6'-(difluoromethoxy)-*N'*-((2-fluoro-5-methoxypyridine-3-yl)methylene)-[3,3'-bipyridine]-5-carbohydrazide (Compound 128);
(*E*)-5-(6-(2-(2-(2-fluoro-5-methoxybenzylidene)hydrazine-1-carbonyl)pyrazine-2-yl)picolinic acid (Compound 129);
(*E*)-6-(6-cyanopyridine-3-yl)-*N'*-(2-fluoro-5-methoxybenzylidene)pyrazine-2-carbohydrazide (Compound 130);
(*E*)-*N'*-(2-fluoro-5-methoxybenzylidene)-6-(6-(methylsulfinyl)pyridine-3-yl)pyrazine-2-carbohydrazide (Compound 131);
(*E*)-6-(6-(ethylsulfinyl)pyridine-3-yl)-*N'*-(2-fluoro-5-methoxybenzylidene)pyrazine-2-carbohydrazide (Compound 132);
(*E*)-5-(6-(2-(2-fluoro-5-methoxybenzylidene)hydrazine-1-carbonyl)pyrazine-2-yl)pyridine-2-sulfonamide (Compound 133);
(*E*)-5-(6-(2-(2-fluoro-5-methoxybenzylidene)hydrazine-1-carbonyl)pyrazine-2-yl)-*N*-methylpyridine-2-sulfonamide (Compound 134);
(*E*)-6-(6-(2-aminoethoxy)pyridine-3-yl)-*N'*-(2-fluoro-5-methoxybenzylidene) pyrazine-2-carbohydrazide (Compound 135);
(*E*)-6-(6-(2-aminopropoxy)pyridine-3-yl)-*N'*-(2-fluoro-5-methoxybenzylidene) pyrazine-2-carbohydrazide (Compound 136);
(*E*)-*N'*-(2-fluoro-5-methoxybenzylidene)-6-(6-(2-hydroxyethoxy)pyridine-3-yl)pyrazine-2-carbohydrazide (Compound 137);
(*E*)-*N'*-(2-fluoro-5-methoxybenzylidene)-6-(6-(2-hydroxypropoxy)pyridine-3-yl) pyrazine-2-carbohydrazide (Compound 138);
(*E*)-6-(6-(azetidin-3-yloxy)pyridine-3-yl)-*N'*-(2-fluoro-5-methoxybenzylidene) pyrazine-2-carbohydrazide (Compound 139);
(*E*)-6-(6-(azetidin-3-ylmethoxy)pyridine-3-yl)-*N'*-(2-fluoro-5-methoxybenzylidene) pyrazine-2-carbohydrazide (Compound 140);
(*E*)-6-(6-(2-(azetidin-3-yl)ethoxy)pyridine-3-yl)-*N'*-(2-fluoro-5-methoxybenzylidene)pyrazine-2-carbohydrazide (Compound 141);
(*E*)-6-(6-(azetidin-3-yloxy)pyridine-3-yl)-*N'*-((2-fluoro-5-methoxypyridine-3-yl)methylene)pyrazine-2-carbohydrazide (Compound 142);
(*E*)-6-(6-(azetidin-3-ylmethoxy)pyridine-3-yl)-*N'*-((2-fluoro-5-methoxypyridine-3-yl)methylene)pyrazine-2-carbohydrazide (Compound 143);
(*E*)-6-(6-(2-(azetidine-3-yl)ethoxy)pyridine-3-yl)-*N'*-((2-fluoro-5-methoxypyridine-3-yl)methylene)pyrazine-2-carbohydrazide (Compound 144);
(*E*)-*N'*-(2-fluoro-5-methoxybenzylidene)-6-(6-((1-methylazetidine-3-yl)oxy)pyridine-3-yl)pyrazine-2-carbohydrazide (Compound 145);
(*E*)-*N'*-(2-fluoro-5-methoxybenzylidene)-6-(6-((1-methylazetidine-3-yl)methoxy)pyridine-3-yl)pyrazine-2-carbohydrazide (Compound 146);
(*E*)-*N'*-(2-fluoro-5-methoxybenzylidene)-6-(6-(2-(1-methylazetidine-3-yl)ethoxy)pyridine-3-yl)pyrazine-2-carbohydrazide (Compound 147);
(*E*)-*N'*-((2-fluoro-5-methoxypyridine-3-yl)methylene)-6-(6-((1-methylazetidine-3-yl)oxy)pyridine-3-yl)pyrazine-2-carbohydrazide (Compound 148);
(*E*)-*N'*-((2-fluoro-5-methoxypyridine-3-yl)methylene)-6-(6-((1-methylazetidine-3-yl)methoxy)pyridine-3-yl)pyrazine-2-carbohydrazide (Compound 149);
(*E*)-*N'*-((2-fluoro-5-methoxypyridine-3-yl)methylene)-6-(6-(2-(1-methylazetidine-3-yl)ethoxy)pyridine-3-yl)pyrazine-2-carbohydrazide (Compound 150);
(*E*)-6-(6-ethoxy-4-methylpyridine-3-yl)-*N'*-((2-fluoro-5-methoxypyridine-3-yl)methylene)pyrazine-2-carbohydrazide (Compound 151);
(*E*)-6-(6-ethoxy-2-methylpyridine-3-yl)-*N'*-((2-fluoro-5-methoxypyridine-3-yl)methylene)pyrazine-2-carbohydrazide (Compound 152);
(*E*)-6-(6-ethoxy-4-methoxypyridine-3-yl)-*N'*-((2-fluoro-5-methoxypyridine-3-yl)methylene)pyrazine-2-carbohydrazide (Compound 153);
(*E*)-6-(6-ethoxy-2-methoxypyridine-3-yl)-*N'*-((2-fluoro-5-methoxypyridine-3-yl)methylene)pyrazine-2-carbohydrazide (Compound 154);
(*E*)-6-(6-ethoxy-2-hydroxypyridine-3-yl)-*N'*-((2-fluoro-5-hydroxypyridine-3-yl)methylene)pyrazine-2-carbohydrazide (Compound 155);
(*E*)-6-(6-ethoxy-2-hydroxypyridine-3-yl)-*N'*-((2-fluoro-5-methoxypyridine-3-yl)methylene)pyrazine-2-carbohydrazide (Compound 156);
(*E*)-6-(6-ethoxy-5-hydroxypyridine-3-yl)-*N'*-((2-fluoro-5-methoxypyridine-3-yl)methylene) pyrazine-2-carbohydrazide (Compound 157);
(*E*)-6-(6-ethoxy-4-hydroxypyridine-3-yl)-*N'*-((2-fluoro-5-methoxypyridine-3-yl)methylene)pyrazine-2-carbohydrazide (Compound 158);
(*E*)-6-(6-(difluoromethoxy)-2-methylpyridine-3-yl)-*N'*-((2-fluoro-5-methoxypyridine-3-yl)methylene)pyrazine-2-carbohydrazide (Compound 159);
(*E*)-6-(6-(difluoromethoxy)-4-methylpyridine-3-yl)-*N'*-((2-fluoro-5-methoxypyridine-3-yl)methylene)pyrazine-2-carbohydrazide (Compound 160);
(*E*)-6-(6-(difluoromethoxy)-2-methoxypyridine-3-yl)-*N'*-((2-fluoro-5-methoxypyridine-3-yl)methylene)pyrazine-2-carbohydrazide (Compound 161);
(*E*)-6-(6-(difluoromethoxy)-4-methoxypyridine-3-yl)-*N'*-((2-fluoro-5-methoxypyridine-3-yl)methylene)pyrazine-2-carbohydrazide (Compound 162);
(*E*)-6-(6-(difluoromethoxy)-2-hydroxypyridine-3-yl)-*N'*-((2-fluoro-5-hydroxypyridine-3-yl)methylene)pyrazine-2-carbohydrazide (Compound 163);
(*E*)-6-(6-(difluoromethoxy)-2-hydroxypyridine-3-yl)-*N'*-((2-fluoro-5-methoxypyridine-3-yl)methylene)pyrazine-2-carbohydrazide (Compound 164);
(*E*)-6-(6-(difluoromethoxy)-4-hydroxypyridine-3-yl)-*N'*-((2-fluoro-5-methoxypyridine-3-yl)methylene)pyrazine-2-carbohydrazide (Compound 165);
(*E*)-N'-(2-fluoro-5-methoxybenzylidene)-6-(1*H*-imidazo[4,5-b]pyridine-6-yl) pyrazine-2-carbohydrazide (Compound 166);
(*E*)-*N'*-((2-fluoro-5-methoxypyridine-3-yl)methylene)-6-(6-hydroxypyridine-3-yl) pyrazine-2-carbohydrazide(Compound 167);
(*E*)-6-(6-(ethoxy-2,2,2-$d_3$)pyridine-3-yl)-*N'*-((2-fluoro-5-methoxypyridine-3-yl)methylene)pyrazine-2-carbohydrazide (Compound 168);
(*E*)-6-(6-(ethoxy-1,1-$d_2$)pyridine-3-yl)-*N'*-((2-fluoro-5-methoxypyridine-3-yl)methylene)pyrazine-2-carbohydrazide (Compound 169);
(*E*)-6-(6-(ethoxy-$d_5$)pyridine-3-yl)-*N'*-((2-fluoro-5-methoxypyridine-3-yl)methylene) pyrazine-2-carbohydrazide (Compound 170);
(*E*)-6-(6-ethoxypyridine-3-yl)-*N'*-((2-fluoro-5-(methoxy-$d_3$)pyridine-3-yl)methylene) pyrazine-2-carbohydrazide (Compound 171);
(*E*)-6-(6-(ethoxy-1,1-$d_2$)pyridine-3-yl)-*N'*-((2-fluoro-5-(methoxy-$d_3$)pyridine-3-yl) methylene)pyrazine-2-carbohydrazide (Compound 172);
(*E*)-6-(6-(ethoxy-2,2,2-$d_3$)pyridine-3-yl)-*N'*-((2-fluoro-5-(methoxy-$d_3$)pyridine-3-yl)methylene)pyrazine-2-carbohydrazide (Compound 173);
(*E*)-6-(6-(ethoxy-$d_5$)pyridine-3-yl)-*N'*-((2-fluoro-5-(methoxy-$d_3$)pyridine-3-yl) methylene)pyrazine-2-carbohydrazide (Compound 174);
(*E*)-6-(6-ethoxypyridine-3-yl)-*N'*-((2-fluoro-5-methoxypyridine-3-yl)methylene)-*N*-methylpyrazine-2-carbohydrazide (Compound 175); and
(*E*)-6-(6-(difluoromethoxy)pyridine-3-yl)-*N'*-((2-fluoro-5-methoxypyridine-3-yl)methylene)-*N*-methylpyrazine-2-carbohydrazide (Compound 176).

In an implementation of the first embodiment, the Formula (I) compound(s) are voltage-gated sodium channel blockers Nav 1.7 and/or Nav 1.8. In a preferred embodiment, the Formula (I) compound(s) are dual voltage-gated sodium channel blockers Nav 1.7 and Nav 1.8.

In an implementation of the first embodiment, the compound(s) of Formula (I) can be acidic in nature and, consequently, pharmaceutically acceptable salts can be obtained by the addition of organic or inorganic bases. Nonlimiting examples of organic bases that can be used are methylamine, trimethylamine, among others. Among the inorganic bases, sodium hydroxide, potassium hydroxide, magnesium hydroxide, ammonium hydroxide, among others, can be mentioned.

In an implementation of the first embodiment, the compound(s) of Formula (I) may have a basic nature and, consequently, pharmaceutically acceptable salts may be obtained by the addition of organic or inorganic acids. Nonlimiting examples of organic acids that can be used are fumaric, maleic, benzoic, lactic acids, among others. Among the inorganic acids, there can be mentioned hydrochloric acid, phosphoric acid, hydrobromic acid, sulfuric acid, nitric acid,

among others.

In an implementation of the first embodiment, the compound(s) of Formula (I) can be obtained in the form of crystals, which can be optionally presented as pharmaceutically acceptable solvates, in which the solvent is incorporated in stoichiometric proportions or not into the crystal lattice. In further embodiments, the crystallization solvent is water, resulting in pharmaceutically acceptable hydrates.

Finally, in an implementation of the first embodiment, the Formula (I) compound(s) may present more than one isomer, including without limitation, spatial isomerism, such as geometric and optical isomerism.

**DEFINITIONS:**

In order to clarify or elucidate the terms used in this invention, the following definitions are presented, whereby the scope is not limited thereto.

The term "halogen" refers to the elements of the 7A family of the periodic table, which are: fluorine (F), chlorine (Cl), bromine (Br), iodine (I), astatine (At) and tennessine (Ts).

The term "hydroxy(alkyl $C_2$-$C_4$ linear or branched)" refers to groups such as 1-hydroxyethyl, 2-hydroxyethyl, 1-hydroxypropyl, 2-hydroxypropyl, 3-hydroxypropyl, 1-hydroxyisopropyl, 2-hydroxyisopropyl, 1-hydroxybutyl, 2-hydroxybutyl, 3-hydroxybutyl, 4-hydroxybutyl, hydroxy-*sec*-butyl and hydroxy-*tert*-butyl.

The term "linear or branched $C_1$-$C_6$ alkyl refers to saturated hydrocarbon groups of linear or branched chain, such as, for example, methyl, ethyl, propyl, isopropyl, isobutyl, butyl, *sec*- butyl and *tert*-butyl, but not being limited to same.

The term "linear or branched $C_1$-$C_6$ alkoxy" refers to alkyl groups attached to an oxygen radical, such as, for example, methoxy, ethoxy, propoxy, isopropoxy, butoxy, *sec*-butoxy, and *tert*-butoxy, but not being limited to the same.

The term "cycloalkoxy $C_3$-$C_7$" refers to the alkyl cycle groups attached to an oxygen radical, such as, for example, cyclopropoxy, cyclobutoxy, cyclopentoxy, and cyclohexyl, but not being limited to the same.

The term "haloalkoxy $C_1$-$C_6$ refers to the alkyl groups attached to an oxygen radical and at least one halogen, such as chloromethoxy, dichloromethoxy, trichloromethoxy, fluoromethoxy, difluoromethoxy, and trifluoromethoxy, but not being limited to the same.

The term "haloalkyl $C_1$-$C_6$ hydroxy" refers to the alkyl groups attached to a hydroxy and at least one halogen, e.g., hydroxyfluoroethane.

The term "hydroxyalkoxy $C_1$-$C_6$ linear or branched" refers to the "linear or branched" $C_1$-$C_6$ alkoxy groups attached to a hydroxy, e.g., hydroxymethoxy, hydroxyethoxy, hydroxypropoxy, and hydroxybutoxy.

The term "azetidinylalkoxy $C_1$-$C_3$" refers to the azetidinyl groups attached to an "alkoxy $C_1$-$C_3$" group, e.g., azetidinylmethoxy, azetidinylethoxy, azetidinylpropoxy, and azetidinylbutoxy.

The term "*N*-alkyl($C_1$-$C_3$ linear)-azetidinyloxy" refers to azetidinyloxy groups with a $C_1$-$C_3$ alkyl linear bound in the nitrogen of the azetidinyloxy ring, e.g., 1-methylazetidinyloxy, 1-ethylazetidinyloxy, and 1-propylazetidinyloxy.

The term "*N*-alkyl($C_1$-$C_3$ linear) azetidinylalkoxy $C_1$-$C_3$" refers to azetidinyloxy groups with a $C_1$-$C_3$ linear alkyl linked in the nitrogen of the azetidinyl ring, which is also bound with an alkoxy portion $C_1$-$C_3$, e.g., 1-methylazetidinylmethoxy, 1-ethylacetidylylethoxy, and 1-propylazetidinylpropoxy.

The term "$C_1$-$C_6$ alkylsulfinyl" refers to the sulfinyl groups ($R_2S$=*O*) linked to the $C_1$-$C_6$ alkyl group, e.g., methylsulfinyl, ethylsulfinyl, propylsulfinyl, isopropylsulfinyl, butylsulfinyl, *sec*-butylsulfinyl, and *tert*-butylsulfinyl.

The term "sulfonamide" refers to the amide groups of sulfonic acids, with the general formula given by - $SO_2NH_2$.

The term "$C_1$-$C_6$" refers to sulfonamide groups with a $C_1$-$C_6$ alkyl bonded in sulfur, e.g., methylsulfonamide, 1-ethylsulfonamide, 2-ethylsulfonamide, 1-propylsulfonamide, 2-propylsulfonamide, 3-propylsulfonamide, 1-butylsulfonamide, 2-butylsulfonamide, 3-butylsulfonamide, and 4-butylsulfonamide.

The term "*N,N*-(dialkyl)sulfonylamide $C_1$-$C_6$" refers to the sulfonamide groups with two alkyl $C_1$-$C_6$ nitrogen-bound, with a general formula given by -$SO_2NRR'$, e.g., dimethylsulfonylamide, diethylsulfononylamide, and methylethyl sulfonylamide.

The term "sulfonyl" refers to the groups represented by the general formula R-S(=O)$_2$-R', where there are two double bonds between sulfur and oxygen.

The term " alkylsulfonyl $C_1$-$C_6$" refers to the sulfonyl groups associated with a $C_1$-$C_6$ alkyl.

The term "($C_1$-$C_6$ alkylamino)alkoxy $C_1$-$C_6$" refers to amino groups with an alkyl $C_1$-$C_6$ and an alkoxy $C_1$-$C_6$ bound therein.

The term "aminoalkoxy $C_1$-$C_6$" refers to the amino groups linked with an alkoxy $C_1$-$C_6$, e.g., 1-aminoethoxy, 2-aminoethoxy, 1-aminopropoxy, 2-aminopropoxy, and 3-aminopropoxy.

The term "(-S-alkyl $C_1$-$C_6$) alkoxy $C_1$-$C_6$" refers to the "-S-alkyl $C_1$-$C_6$ groups linked with an alkoxy $C_1$-C , e.g., -S-methylmethoxy, -S-ethylmethoxy, -S-propylmethoxy, - S-butylmethoxy, -S-pentylmethoxy, -S-isopropylmethoxy, -S-isobutylmethoxy, -S- *tert*-butylmethoxy, -S-*sec*-butylmethoxy, but not limited thereto.

The term "N-alkylcarbamoyl $C_1$-$C_6$" and the term "*N,N*-dialkylcarbamoyl $C_1$-$C_6$" refer to groups such as mono- and di-alkylates *N*-methylcarbamoyl, *N*-ethylcarbamoyl, *N*-propylcarbamoyl, *N,N*-dimethylcarbamoyl, *N,N*-diethylcarbamoyl, *N,N*-propylcarbamoyl, but not limited thereto.

Terms not explained herein should be interpreted in their broadest sense for those skilled in the art.

In a second embodiment, the present invention presents a composition comprising a therapeutically effective amount of compound(s) of Formula (I) of the present invention or a pharmaceutically acceptable salt, hydrate, solvate, and isomer thereof; and one or more pharmaceutically acceptable excipients.

Pharmaceutically acceptable excipients are any substance, other than the active pharmaceutical ingredient, that has been evaluated for its safety and that is intentionally added to the dosage form. Such excipients are selected according to the pharmaceutical dosage form of interest, the route of administration thereof, physicochemical compatibility with the active ingredient, and the effect on efficacy.

In addition, these excipients are widely known in the state of the art and are classified according to their function, including without limitation diluents, binders, disintegrants or disaggregators, lubricants, suspending agents, thickeners, solvents, surfactants, sliders, anti-caking agents or flow agents, glazing agents, plasticizers, sweeteners, isotonicity agents, dyes and pigments, preservatives, antioxidants, modifying agents or pH control, complexing agents, chelating agents, flavorings, viscosity modifying agents, opacifiers, permeation promoters, among others.

In an implementation of the second embodiment, the pharmaceutical compositions of the present invention can be administered by several routes including oral, sublingual, nasal, parenteral, injectable, submuscular, topical, transdermal, ocular, rectal, but not limited to these.

In a third embodiment, the present invention presents the use of compound(s) of Formula (I) or a salt, hydrate, solvate and pharmaceutically acceptable isomer thereof to prepare a drug for treating pathologies related to neuropathic pain.

In an implementation of the third embodiment, said pathologies are selected from the group consisting of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia and postsurgical neuralgia.

In a fourth embodiment, the present invention presents a method of treatment, prevention, relief, suppression and/or control of pathologies related to neuropathic pain comprising the administration of an effective amount of Formula (I) compound(s) or a salt, hydrate, solvate and pharmaceutically acceptable isomer thereof.

In an implementation of the fourth embodiment, the method is for the treatment, prevention, relief, suppression and/or control of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia and postsurgical neuralgia.

In another implementation of the fourth embodiment, the administration of at least one Formula (I) compound is selected from the group comprising oral, sublingual, nasal, parenteral, injectable, submuscular, topical, transdermal, ocular and rectal routes.

In a fifth embodiment, processes for obtaining Formula (I) compound(s) are presented, comprising the following steps:

(a) formation of the Formula III intermediate:

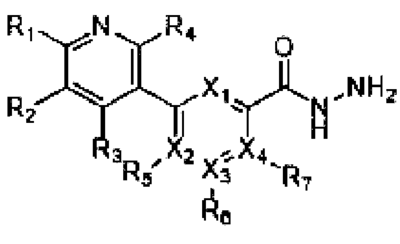

Formula III

from the hydrazinolysis reaction of a Formula IV intermediate:

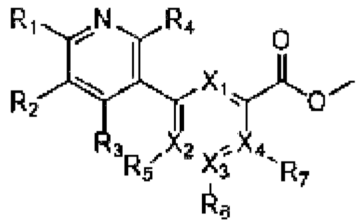

**Formula IV**

(b) obtaining a Formula I compound wherein $R_8$ is hydrogen;

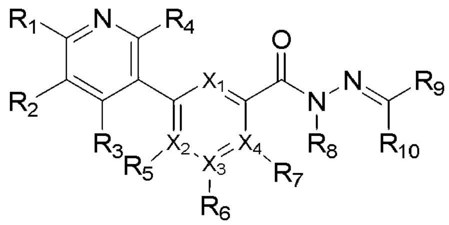

Formula (I)

from the condensation of Formula II intermediates:

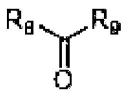

**Formula II**

and Formula III with or without the presence of a catalyst and a suitable solvent, wherein:

- $X_1$, $X_2$, $X_3$, and $X_4$ are independently selected from the group consisting of carbon, CH, or nitrogen, where:
    - when at least one of the substituents $X_2$, $X_3$ and $X_4$ is N or CH, at least one of $R_5$, $R_6$ and/or $R_7$, respectively, is null; and
    - when two of the substituents $X_1$, $X_2$, $X_3$ and $X_4$ are nitrogen, the remaining substituents must be carbon;
    - $R_1$, $R_2$, $R_3$, and $R_4$ are independently selected from the group consisting of hydrogen, halogen, hydroxy, hydroxy (alkyl $C_2$-$C_4$ linear or branched), alkyl $C_1$-$C_6$ linear or branched, alkoxy $C_1$-$C_6$ linear or branched, cycloalkoxy $C_3$-$C_7$, haloalkoxy $C_1$-$C_6$, haloalkyl $C_1$-$C_6$ hydroxy, hydroxyalkoxy $C_1$-$C_6$ linear or branched, difluoromethyl, trifluoromethyl, azetidinyloxy, azetidinylalkoxy $C_1$-$C_3$, *N*-alkyl ($C_1$-$C_3$ linear) - azetidinyloxy, *N*-alkyl($C_1$-$C_3$ linear)azetidinylalkoxy $C_1$-$C_3$, carboxyl, carboxyalkyl ester $C_1$-$C_3$, imidazole, nitrile, sulfinyl, alkylsulfinyl $C_1$-$C_6$, sulfonamide, alkylsulfonylamide $C_1$-$C_6$, *N,N*-(dialkyl)sulfonylamide $C_1$-$C_6$, sulfonyl, alkylsulfonyl $C_1$-$C_6$, amino (alkyl $C_2$-$C_4$ linear or branched) alkoxy, ($C_1$-$C_6$ alkyl amino) alkoxy $C_1$-$C_6$, (-S-alkyl $C_1$-$C_6$) alkoxy $C_1$-$C_6$, carbamoyl, *N*-alkylcarbamoyl $C_1$-$C_6$, *N,N*-dialkylcarbamoyl $C_1$-$C_6$, alkoxy $C_1$-$C_2$ containing one or more isotopically enriched atoms such as hydrogen isotopes $^2$H, but not limited to the same;
    - $R_5$ and $R_7$ are independently selected from the group consisting of hydrogen, halogen, $C_1$-$C_6$ linear or branched alkyl, amine, hydroxy, carbonyl, $C_1$-$C_6$ linear or branched alkoxy, haloalkyl $C_1$-$C_6$; where when $R_5$ is carbonyl, the ring of $X_2$ will have only two unsaturations and $X_2$ will be carbon;
    - $R_6$ is selected from hydrogen, halogen, or alkyl $C_1$-$C_2$;
    - $R_8$ and $R_{10}$ are independently selected from hydrogen and alkyl $C_1$-$C_6$ linear or branched;
    - $R_9$ is selected from the group consisting of aryl, pyridinyl or substituted pyridinyl, pyridone, cycloalkyl $C_3$-$C_7$ saturated or unsaturated, cycloalkyl $C_3$-$C_7$ saturated or unsaturated alkylsubstituted $C_1$-$C_5$, cycloalkyl $C_3$-$C_7$ saturated or unsaturated, halosubstituted or $R_{11}$ or $R_{17}$;
    - $R_{11}$ is:

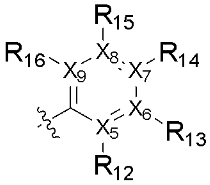

wherein:

- $X_5$, $X_6$, $X_7$, $X_8$, $X_9$ are independently selected from the group consisting of carbon, CH, or nitrogen, wherein:
  - when at least one of the substituents $X_5$, $X_6$, $X_7$, $X_8$ and $X_9$ is N or CH, at least one of $R_{12}$, $R_{13}$, $R_{14}$, $R_{15}$ and/or $R_{16}$, respectively, is null;
  - $R_{12}$, $R_{13}$, $R_{14}$, $R_{15}$ , and $R_{16}$ are independently selected from the group consisting of hydrogen, halogen, hydroxy, hydroxy (alkyl $C_2$-$C_4$ linear or branched), alkyl $C_1$-$C_4$ linear or branched, $C_1$-$C_4$ linear or branched alkoxy, $C_3$-$C_7$ cycloalkoxy, haloalkoxy, carboxyloxy, carboxyalkyl ester $C_1$-$C_3$, amine, *N*-alkylamine, *N, N*-dialkylamine, amino(alkyl $C_2$-$C_4$ linear or branched), sulfonamide, alkylsulfonylamide $C_1$-$C_6$, *N,N*-(dialkyl) sulfonylamide $C_1$-$C_6$, sulfonyl, alkylsulfonyl $C_1$-$C_6$, sulfinyl, alkylsulfinyl $C_1$-$C_6$, azetidininyloxy, azetidinylalkoxy $C_1$-$C_3$, alkoxy $C_1$-$C_2$ linear or branched, hydroxyazetidinyl, oxo-dihydropyridinyl, oxo-dihydropyridinylalkoxy $C_1$-$C_4$ linear or branched, alkoxy $C_1$-$C_6$ containing one or more isotopically enriched atoms such as hydrogen isotope $^2$H, but not limited to the same;
  - $R_{17}$ is selected from the group consisting of:

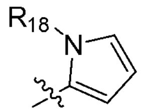
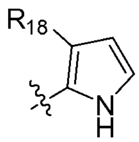
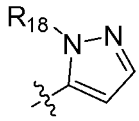
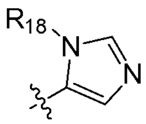
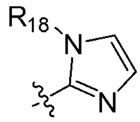

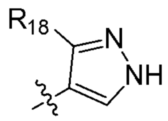
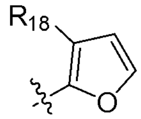
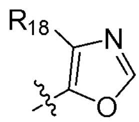
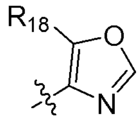
,

wherein:

- $R_{18}$ is independently selected from the group consisting of hydrogen, halogen, hydroxy, hydroxy (alkyl $C_2$-$C_4$ linear or branched), alkyl $C_1$-$C_4$ linear or branched, alkoxy $C_1$-$C_4$ linear or branched, $C_3$-$C_7$ cycloalkoxy, haloalkoxy, nitrile, carboxyl, carboxyalkyl ester $C_1$-$C_3$, amine, *N*-alkylamine, *N, N*-dialkylamine, amino(alkyl $C_2$-$C_4$ linear or branched), sulfonamide, alkylsulfonylamide $C_1$-$C_6$, *N,N*-(dialkyl)sulfonylamide $C_1$-$C_6$, sulfonyl, alkylsulfonyl $C_1$-$C_6$, sulfinyl, alkylsulfinyl $C_1$-$C_6$, azetidinyloxy, azetidinylalkoxy $C_1$-$C_3$, alkoxy $C_1$-$C_2$ linear or branched, hydroxyazetidinyl, alkoxy $C_1$-$C_6$ containing one or more isotopically enriched atoms, such as hydrogen isotopes $^2$H, but not limited to the same.

In an implementation of the fifth embodiment, the process additionally comprises a step (c) of formation of compound(s) of Formula (I) in which $R_7$ is linear or branched $C_1$-$C_6$ alkyl from the nucleophilic substitution reaction of a compound obtained in step (b), with linear or branched $C_1$-$C_6$ alkyl halides in the presence of inorganic base and polar aprotic solvents.

In an implementation of the fifth embodiment, the catalyst of step (b) is selected from concentrated hydrochloric acid, acetic acid, trifluoroacetic acid, formic acid, or combinations of these, and the solvent is selected from dimethylformamide, alcohols, or combinations thereof.

In another implementation of the fifth embodiment, said inorganic basis of step (c) is selected from $K_2CO_3$ or NaH.

In a sixth embodiment, the present invention presents kits comprising a pharmaceutical composition of the present invention and an application device.

In an implementation of the sixth embodiment, the kits of the present invention may comprise such compositions and application devices, which may include ampoules, syringes and others. Alternatively, the kits of this invention comprise more than one compound of Formula (I) arranged in one or more dosage forms, including without limitation, tablets, accompanied by administration instructions.

The compound(s) of Formula (I) of this invention have been prepared from the synthetic route described in General Scheme 1. However, those skilled in the art will readily notice that additional detailing and/or modifications to the arrangement of one or more steps can be accomplished without departing from the processes taught herein. Such variations can be, without limitation, combinations of solvents and catalysts, including stereoselective ones, protective groups, among others.

In the following general scheme 1, the formation of the Formula II, III and IV intermediates is described, in addition to the formation of the Formula (I) compound(s), where Formula Ia corresponds to compounds in which $R_7$ is hydrogen and Formula Ib corresponds to compounds in which $R_7$ is an alkyl group $C_1$-$C_6$ (represented by R in the scheme), from these intermediates.

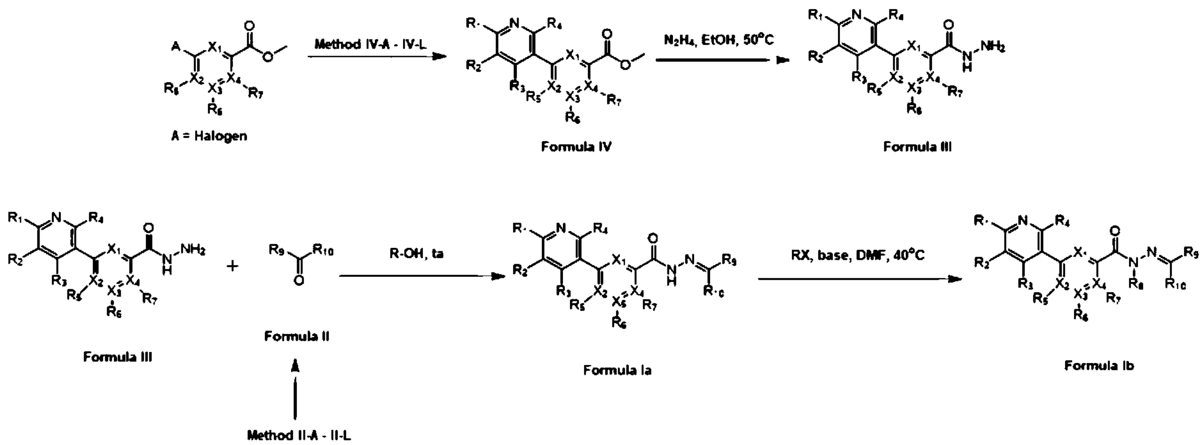


GENERAL SCHEME 1

**FORMULA IV INTERMEDIATES:**

Following General Scheme 1, the methods from IV-A to IV-L for the formation of formula IV intermediates are presented, however these methods are not limiting.

**METHOD IV-A**

*Intermediates IV-1 to IV-30:*

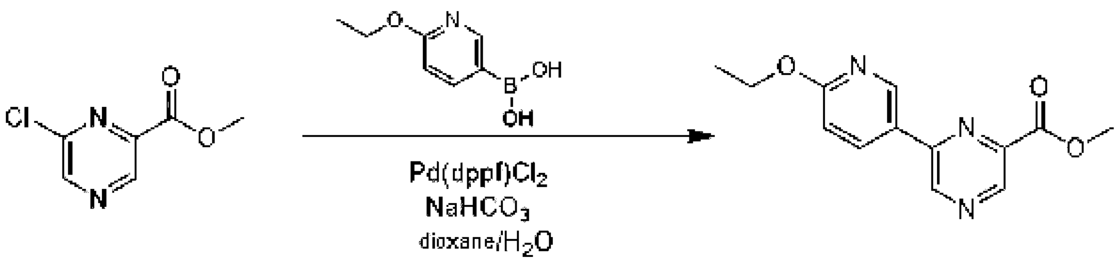


In a round-bottomed flask containing methyl 6-chloropyrazine-2-carboxylate (11.6 mmol) in dioxane (50.0 mL) and $H_2O$ (10.0 mL), boronic acid 6-(ethoxy-pyridine-3-yl), $NaHCO_3$ (17.4 mmol), and $Pd(dppf)Cl_2$ (579 μmol) were added under $N_2$ atmosphere. The reaction mixture was maintained under agitation at 70 °C for 12 hours. After full consumption of the starting reagent (monitored by CCD), the mixture was concentrated under reduced pressure to remove the solvent. The residue obtained was diluted with $H_2O$ (100 mL) and extracted with AcOEt (100 mL x 3). The organic phase was concentrated under reduced pressure and purified by column chromatography (petroleum ether/AcOEt:50/10/1). 3.00 g (99.9% yield) of the corresponding ester were obtained in the form of a white solid.

**TABLE 1. INTERMEDIATES OBTAINED FROM THE CORRESPONDING REAGENTS FOLLOWING METHOD IV-A.**

| Intermediate | Structure | Intermediate | Structure |
|---|---|---|---|
| IV-1 | 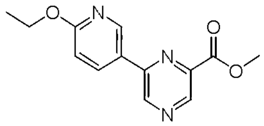 | IV-16 | 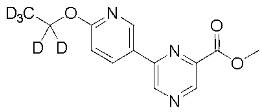 |
| IV-2 | 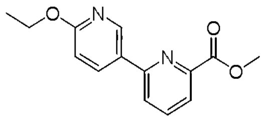 | IV-17 | 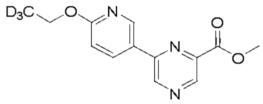 |
| IV-3 | 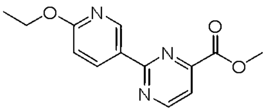 | IV-18 | 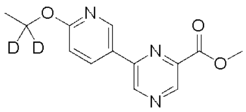 |
| IV-4 | 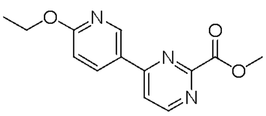 | IV-19 | 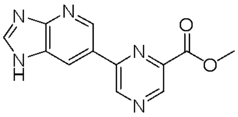 |
| IV-5 | 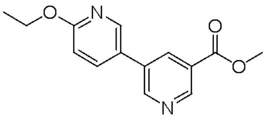 | IV-20 | 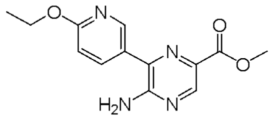 |
| IV-6 | 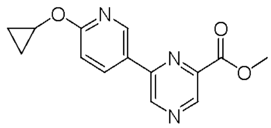 | IV-21 | 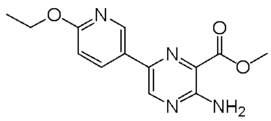 |
| IV-7 | 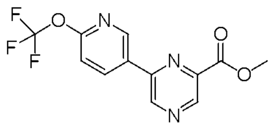 | IV-22 | 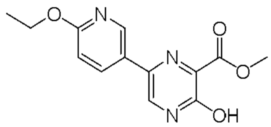 |
| IV-8 | 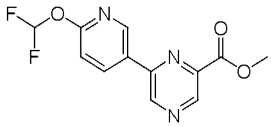 | IV-23 | 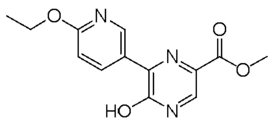 |
| IV-9 | 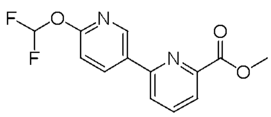 | IV-24 | 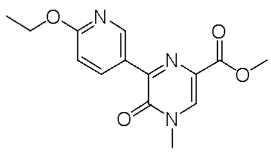 |
| IV-10 | 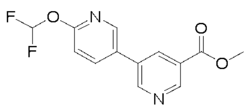 | IV-25 | 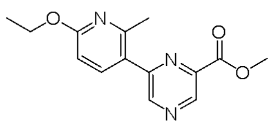 |
| IV-11 | 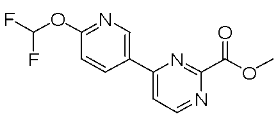 | IV-26 | 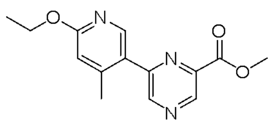 |

(continued)

| Intermediate | Structure | Intermediate | Structure |
|---|---|---|---|
| IV-12 | 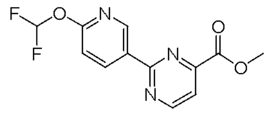 | IV-27 | 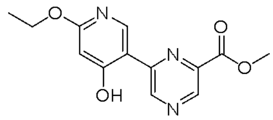 |
| IV-13 | 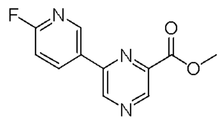 | IV-28 | 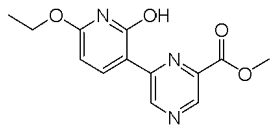 |
| IV-14 | 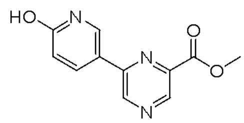 | IV-29 | 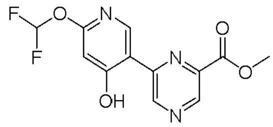 |
| IV-15 | 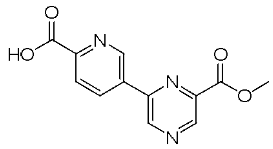 | IV-30 | 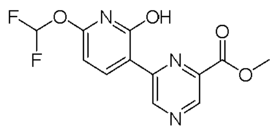 |

**METHOD IV-B**

*IV-31 Intermediate:*

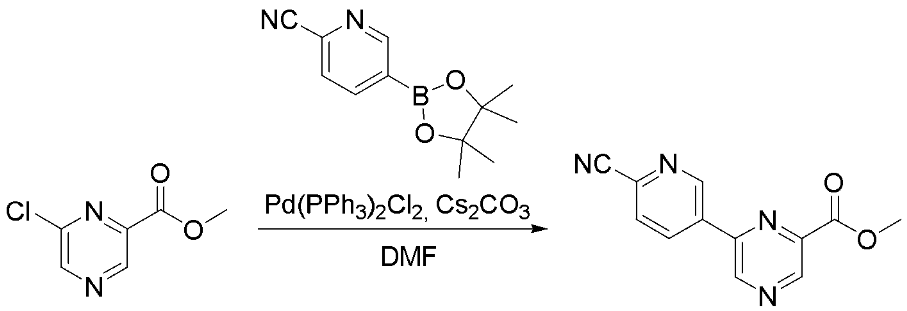


In a round-bottomed flask containing methyl 6-chloropyrazine-2-carboxylate (7.53 mmol) in DMF (20 mL) under agitation, cesium carbonate (15.07 mmol) and 5-(4,4,5,5-tetramethyl-1,3,2-dioxaborolan-2-yl)picolinonitrile (9.04 mmol) were added. The mixture was de-gassed and purged with $N_2$ for 5 min. Subsequently, bis(triphenylphosphine)palladium(II) chloride (0.226 mmol) was added. Next, the mixture was maintained at 55 °C for 16 hours. After total consumption of the starting reagent (monitored by CCD), the mixture was cooled to room temperature, diluted with AcOEt (50 mL) and filtered with celite. The filtrate was washed with water, dried under anhydrous $Na_2SO_4$, filtered, concentrated under reduced pressure and purified by column chromatography. 1.0 g (55.3% yield) of desired ester were obtained in the form of a white solid.

**METHOD IV-C**

Step IV-C-i:

*Precursors IV-32i - IV-33i:*

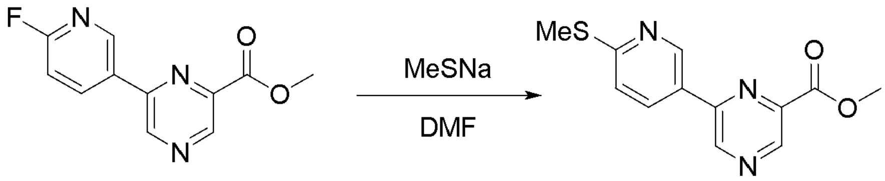


In a round-bottomed flask containing methyl 6-(6-fluoropyridine-3-yl)pyrazine-2-carboxylate (2.14 mmol) in DMF (10.0 mL), sodium thiomethoxide (2.14 mmol) was added at 25 °C. The reaction mixture remained under agitation at 25 °C for 2 hours. After total consumption of the starting reagent (monitored by CCD), the mixture was diluted with $H_2O$ and extracted with AcOEt. The organic phase was dried on anhydrous $Na_2SO_4$, filtered, concentrated under reduced pressure and purified by column chromatography. 500 mg (89.5% yield) of the desired ester were obtained in the form of a white solid.

**TABLE 2. INTERMEDIATES OBTAINED FROM THE CORRESPONDING REAGENTS FOLLOWING STEP IV-C-i.**

| Intermediate | Structure |
|---|---|
| IV-32i | 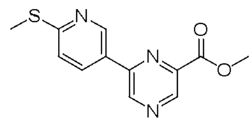 |
| IV-33i | 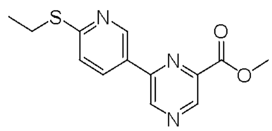 |

Step IV-C-ii:

*Intermediates IV-32 - IV-33:*

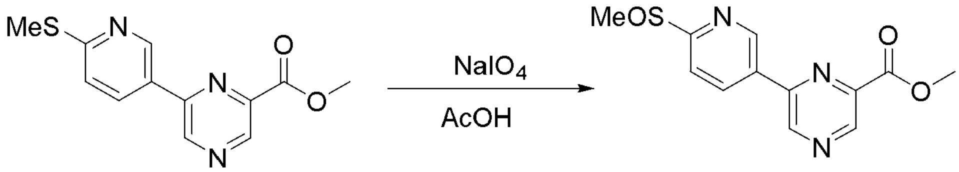


In a round-bottomed flask containing methyl 6-(6-(methylthio)pyridine-3-yl)pyrazine-2-carboxylate (1.91 mmol) in AcOH (5.0 mL), sodium periodate (7.65 mmol) was added at 0 °C. The reaction mixture remained under agitation at 25 °C for 2 hours. After total consumption of the starting reagent (monitored by CCD), the mixture was diluted with $H_2O$ and extracted with AcOEt. The organic phase was dried on anhydrous $Na_2SO_4$, filtered and concentrated under reduced pressure. 370 mg (69.8% yield) of methyl 6-(6-(methylsulfinyl)pyridine-3-yl)pyrazine-2-carboxylate were obtained in the form of a white solid, which was sent to the next step without further purification.

**TABLE 3. INTERMEDIATES OBTAINED FROM THE CORRESPONDING REAGENTS FOLLOWING STEP IV-C-ii.**

| Intermediate | Structure |
|---|---|
| IV-32 | 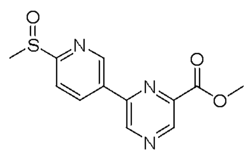 |

(continued)

| Intermediate | Structure |
|---|---|
| IV-33 | 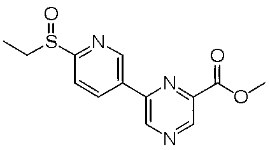 |

**METHOD IV-D**

Step IV-D-i:

*Precursor IV-34i:*

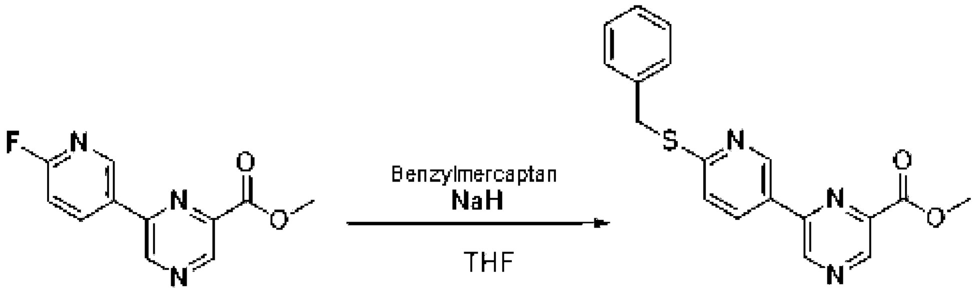


In a round-bottomed flask containing NaH (9.43 mmol) in DMF (20 mL) under agitation, benzylmercaptan (8.58 mmol) was added at 0 °C. The mixture was kept under agitation at 0 °C for 15 minutes. Next, methyl 6-(6-fluoropyridine-3-yl)pyrazine-2-carboxylate (8.58 mmol) was added and the mixture was kept at 0 °C for another 1 hour. After the end of the reaction (monitored by CCD), the mixture was diluted with saturated solution of $NH_4Cl$ (10 mL) and extracted with AcOEt. The organic phase was dried on anhydrous $Na_2SO_4$, filtered and concentrated under reduced pressure. The residue was adsorbed by silica gel (60-120 mesh) and purified by column chromatography (hexane/AcOEt:100/100/0). 2.0 g (67.9% yield) of the desired ester were obtained in the form of a white solid.

Step IV-D-ii:

*Precursor IV-34ii:*

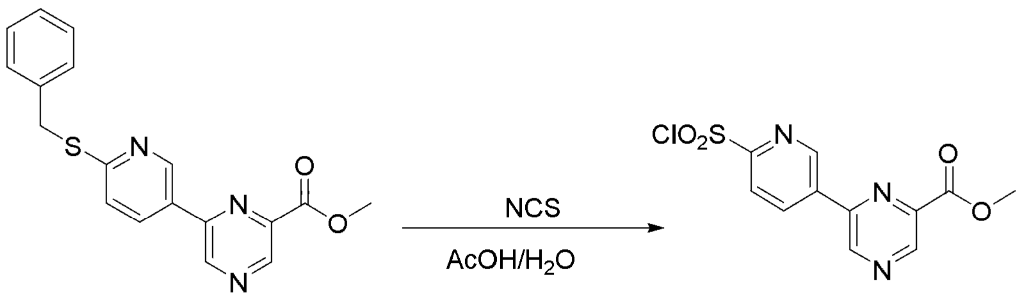


In a round-bottomed flask containing methyl 6-(6-(benzylthio)pyridine-3-yl)pyrazine-2-carboxylate (2.96 mmol) in AcOH/$H_2O$ (10 mL) under agitation, NCS (14.82 mmol) was added at 25 °C. The mixture was kept under agitation at 25 °C for 16 hours. After total consumption of the starting reagent (monitored by CCD), the mixture was extracted with AcOEt. The organic phase was washed with saturated solution of $NaHCO_3$, dried on $Na_2SO_4$ anhydrous, filtered and concentrated under reduced pressure. 500 mg (53.8% yield) of the desired product were obtained in the form of a white solid, which was sent to the next step without further purification.

Step IV-D-iii:

IV-34 Intermediate:

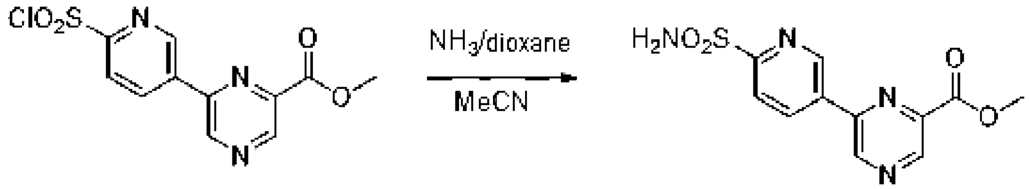


In a round-bottomed flask containing methyl 6-(6-(chlorosulfonyl)pyridine-3-yl)pyrazine-2-carboxylate (1.59 mmol) in MeCN (5 mL), ammonia was added to dioxane (7.97 mmol). The mixture was kept under agitation at room temperature for 16 hours. After total consumption of the starting reagent (monitored by CCD), the mixture was concentrated under reduced pressure. 300 mg (13.83% yield) of the desired product were obtained in the form of a yellow solid, which was sent to the next step without further purification.

**METHOD IV-E**

IV-35 Intermediate:

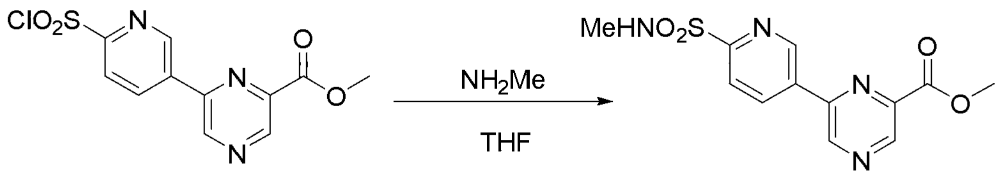


In a round-bottomed flask containing methyl 6-(6-(chlorosulfonyl)pyridine-3-yl)pyrazine-2-carboxylate (1.59 mmol) in MeCN (5.0 mL), methylamine in THF (7.97 mmol) at 0 °C was added. The reaction mixture remained under agitation at 80 °C for 30 minutes. After total consumption of the starting reagent (monitored by CCD), the mixture was concentrated under reduced pressure. 300 mg (61.2% yield) of the desired product was obtained in the form of a white solid, which was sent to the next step without further purification.

**METHOD IV-F**

Intermediates IV-36 to IV-38:

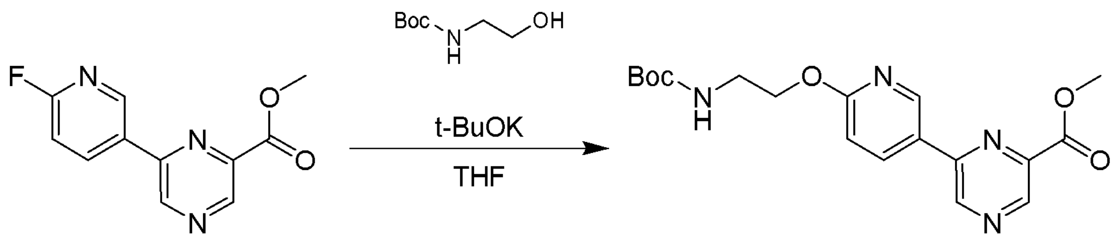


In a round-bottomed flask containing methyl 6-(6-fluoropyridine-3-yl)pyrazino-2-carboxylate (8.58 mmol) and N-Boc ethanolamine (10.3 mmol) in THF (20 Ml), t-BuONa (12.8 mmol) was added. The reaction mixture remained at 25 °C for 12 hours. After total consumption of the starting reagent (monitored by LC-MS), the mixture was diluted with $H_2O$ (50 mL) and extracted with AcOEt (50 mL x 3). The organic phase was concentrated under reduced pressure and purified by preparative HPLC ([$H_2O(NH_4HCO_3)$ / MeCN] 35.0% → 65.0% B). 800 mg (24.90% yield) of the ester of interest was obtained in the form of a white solid.

**TABLE 4. INTERMEDIATES OBTAINED FROM THE CORRESPONDING REAGENTS FOLLOWING THE IV-F METHOD.**

| Intermediate | Structure |
|---|---|
| IV-35 | 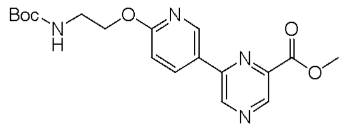 |
| IV-36 | 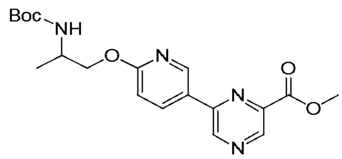 |
| IV-37 | 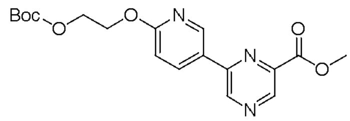 |
| IV-38 | 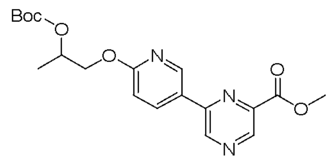 |

**METHOD IV-G**

Step IV-G-i:

*Precursors IV-39i* to *IV-44i:*

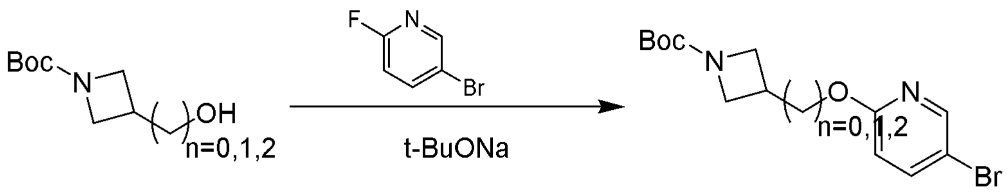


In a round-bottomed flask containing the respective starting azetidine (115.47 mmol) in THF (200 mL), 5-bromo-2-fluoropyridine (127.01 mmol) and t-BuONa (138.56 mmol) were added. The reaction mixture remained at 25 °C for 12 hours. After total consumption of the starting reagent (monitored by LC-MS), the mixture was diluted with $H_2O$ (240 mL) and extracted with AcOEt (80 mL x 3). The organic phase was washed with $H_2O$, dried under $Na_2SO_4$ and concentrated under reduced pressure. 38.0 g (99.9% yield) of the product of interest were obtained.

**TABLE 5. PRECURSORS OBTAINED FROM THE CORRESPONDING REAGENTS FOLLOWING *STEP IV-G-i.***

| Precursor | Structure |
|---|---|
| IV-39i | 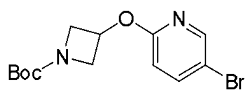 |
| IV-40i | 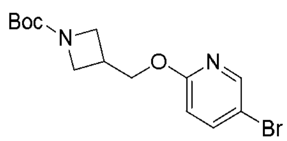 |

(continued)

| Precursor | Structure |
|---|---|
| IV-41i | 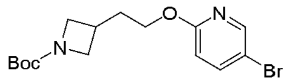 |
| IV-42i | 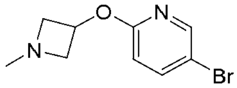 |
| IV-43i | 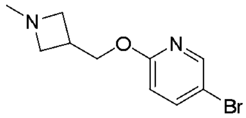 |
| IV-44i | 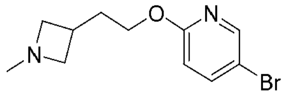 |

Step IV-G-ii:

*Precursors IV-39ii to IV-44ii:*

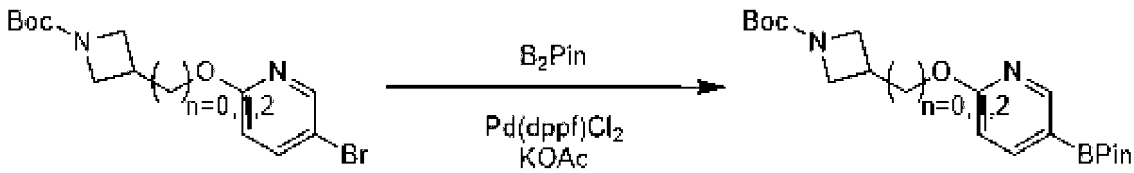


In a round-bottomed flask, a mixture of the respective starting halide (15.19 mmol), $Pd(dppf)Cl_2$ (0.76 mmol), 4,4,5,5-tetramethyl-2-(4,4,5,5-tetramethyl-1,3,2-dioxaborolan-2-yl)-1,3,2-dioxaborolan (22.8 mmol) and EtOK (22.8 mmol) in dioxane (50.0 mL) was de-gassed and purged with $N_2$ 3 times. The mixture was maintained in agitation under $N_2$ atmosphere at 90 °C for 12 hours. After total consumption of the starting reagent (monitored by LC-MS), the mixture was diluted with $H_2O$ (300 mL) and extracted with AcOEt (100 mL x 3). The organic phase was washed with $H_2O$, dried under $Na_2SO_4$ and concentrated under pressure. 11.0 g of the product of interest were obtained, which was sent to the next step without further purification.

**TABLE 6. PRECURSORS OBTAINED FROM THE CORRESPONDING REAGENTS FOLLOWING *STEP IV-G-ii.***

| Precursor | Structure |
|---|---|
| IV-39ii | 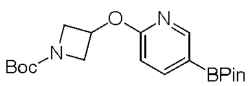 |
| IV-40ii | 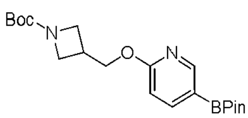 |
| IV-41ii | 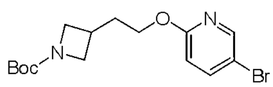 |
| IV-42ii | 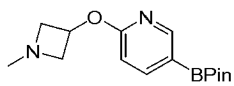 |

(continued)

| Precursor | Structure |
|---|---|
| IV-43ii | 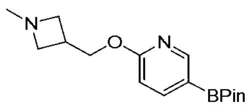 |
| IV-44ii | 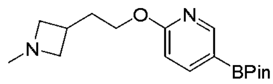 |

Step IV-G-iii:

*Intermediates IV-39 to IV-44:*

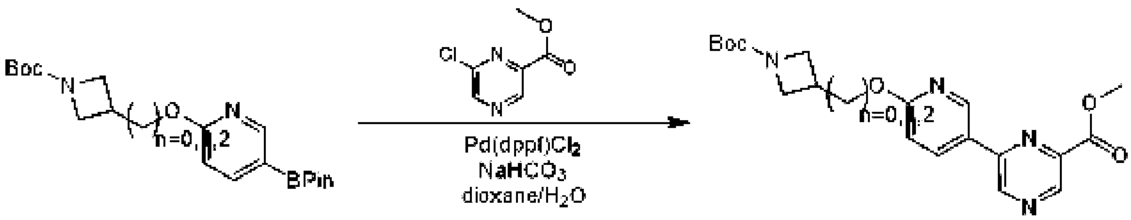


In a round-bottomed flask, a mixture of the respective pinacol ester (15.15 mmol), methyl 6-chloropyrazine-2-carboxylate (16.7 mmol), Pd(dppf)$Cl_2$ (0.76 mmol), $NaHCO_3$ (30.36 mmol) in dioxane (50 mL), and $H_2O$ (10 mL) was degassed and purged with $N_2$ 3 times. The mixture was maintained in agitation under $N_2$ atmosphere at 60 °C for 12 hours. After total consumption of the starting reagent (monitored by LC-MS), the mixture was diluted with $H_2O$ (450 mL) and extracted with AcOEt (150 mL × 3). The organic phase was washed with $H_2O$, dried under $Na_2SO_4$ and concentrated under pressure. The residue was purified by column chromatography (petroleum ether/AcOEt:100/10/1). 1.30 g of the product of interest were obtained.

**TABLE 7. INTERMEDIATES OBTAINED FROM THE CORRESPONDING REAGENTS FOLLOWING *STEP IV-G-iii.***

| Intermediate | Structure |
|---|---|
| IV-39 | 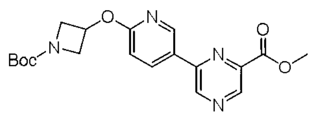 |
| IV-40 | 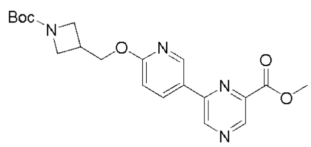 |
| IV-41 | 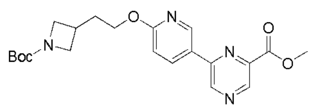 |
| IV-42 | 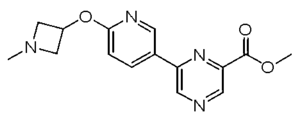 |

(continued)

| Intermediate | Structure |
|---|---|
| IV-43 | 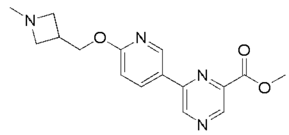 |
| IV-44 | 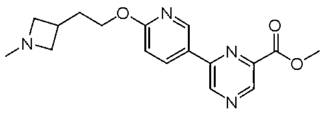 |

**METHOD IV-H**

Step IV-H-i:

*Precursor IV-45i:*

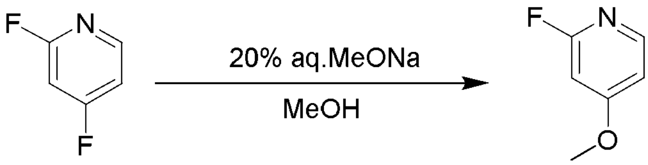


In a round-bottomed flask containing 2,4-difluoropyridine (86.9 mmol) in MeOH (60.0 mL), sodium methoxide (95.5 mmol) was added at 0 °C. The reaction mixture remained at 25 °C for 16 hours under an atmosphere of $N_2$. After total consumption of the starting reagent (monitored by CCD), the mixture was diluted with $H_2O$ (50.0 mL) and extracted with AcOEt (50.0 mL x 2). The organic phase was washed with saturated sodium chloride solution (*brine*) (40.0 mL) and concentrated under reduced pressure. 8.30 g (75.1% yield) of the product of interest were obtained in the form of a colorless oil, which was sent to the next step without further purification.

*Step IV-H-ii:*

*Precursors IV-45ii and IV-46ii:*

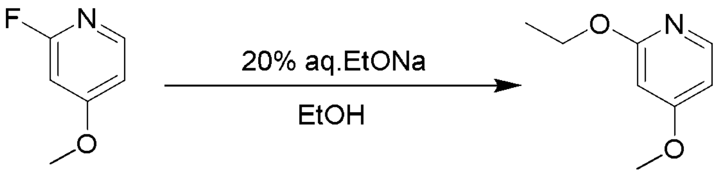


In a round-bottomed flask containing 2-fluoro-4-methoxypyridine (65.2 mmol) in EtOH (20.0 mL), sodium acetate (326 mmol) was added. The reaction mixture remained at 50 °C for 16 hours under an atmosphere of $N_2$. After total consumption of the starting reagent (monitored by CCD), the mixture was diluted with a citric acid solution (10%, 50.0 mL) and extracted with AcOEt (40.0 mL x 2). The organic phase was concentrated under reduced pressure. 7.20 g (71.9% yield) of the product of interest were obtained in the form of a colorless oil, which was sent to the next step without further purification.

**TABLE 8. PRECURSORS OBTAINED FROM THE CORRESPONDING REAGENTS FOLLOWING *STEP IV-H-ii.***

| Precursor | Structure |
|---|---|
| IV-45ii | 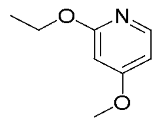 |
| IV-46ii | 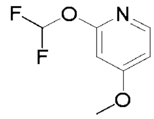 |

<u>Step IV-H-iii</u>

*Precursors IV-45-iii and IV-46iii:*

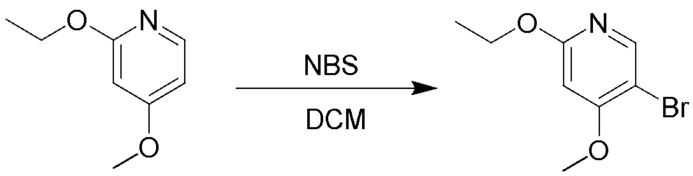


In a round-bottomed flask containing 2-ethoxy-4-methoxypyridine (47.0 mmol) in DCM (45.0 mL), NBS (56.4 mmol) was added at 0 °C. The reaction mixture remained at 25 °C for 16 hours. After total consumption of the starting reagent (monitored by CCD), the mixture was diluted in $H_2O$ (30.0 mL) and extracted with AcOEt (15.0 mL x 2). The organic phase was concentrated under reduced pressure and purified by column chromatography (petroleum ether/AcOEt:20/11/1). 4.30 g (39.4% yield) of the product of interest were obtained in the form of a yellow oil.

**TABLE 9. PRECURSORS OBTAINED FROM THE CORRESPONDING REACTANTS FOLLOWING *STEP IV-H-iii.***

| Precursor | Structure |
|---|---|
| IV-45iii | 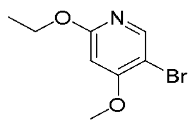 |
| IV-46iii | 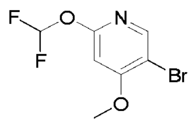 |

<u>Step IV-H-iv:</u>

*Precursors IV-45iv and IV-46iv:*

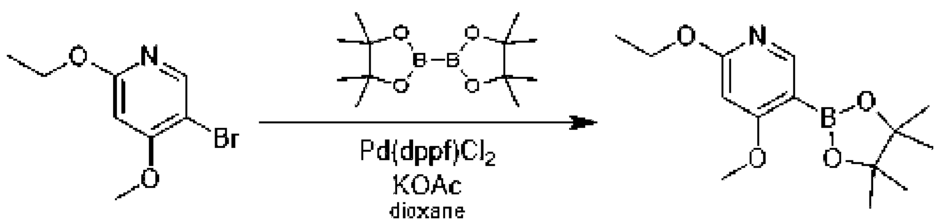

In a round-bottomed flask containing 5-bromo-2-ethoxy-4-methoxypyridine (18.1 mmol) in dioxane (25.2 mL), 4,4,5,5-tetramethyl-2-(4,4,5,5-tetramethyl-1,3,2-dioxaborolan-2-yl)-1,3,2-dioxaborolan (21.7 mmol), Pd(dppf)$Cl_2$ (1.81 mmol), and potassium acetate (54.2 mmol) were added. The reaction mixture remained at 90 °C for 16 hours. After total consumption of the starting reagent (monitored by CCD), the mixture was diluted with $H_2O$ (20.0 mL) and extracted with AcOEt (15.0 mL x 2). The organic phase was concentrated under reduced pressure. 6.00 g (92.5% yield) of the product of interest were obtained in the form of a yellow oil, which was sent to the next step without further purification.

**TABLE 10. PRECURSORS OBTAINED FROM THE CORRESPONDING REAGENTS FOLLOWING *STEP IV-H-iv.***

| Precursor | Structure |
|---|---|
| IV-45iii | 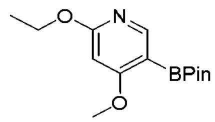 |
| IV-46iii | 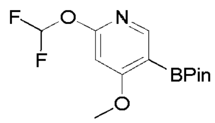 |

Step IV-H-v:

IV-45 and IV-46 Intermediates:

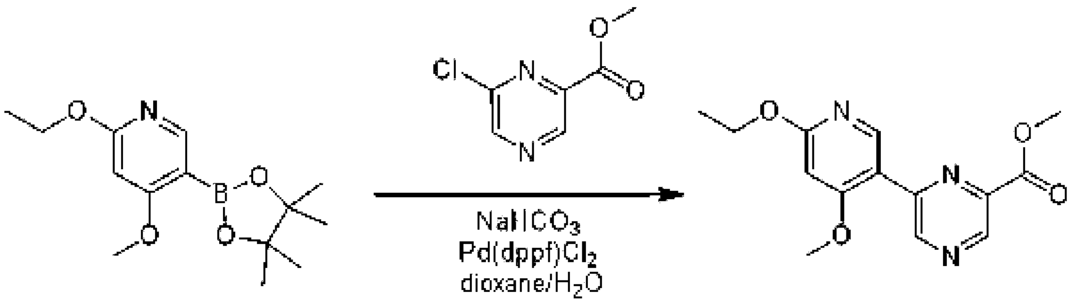


In a round-bottomed flask, a mixture of 2-ethoxy-4-methoxy-5-(4,4,5,5-tetramethyl-1,3,2-dioxaborolan-2-yl) pyridine (63.7 mmol), methyl 6-chloropyrazine-2-carboxylate (57.9 mmol), $NaHCO_3$ (86.9 mmol), and Pd(dppf)$Cl_2$ (2.90 mmol) in dioxane (100 mL) and $H_2O$ (100 mL) was de-gassed and purged with $N_2$ 3 times. The mixture was maintained in agitation under an atmosphere of $N_2$ at 70 °C for 16 hours. After total consumption of the starting reagent (monitored by LC-MS), the mixture was diluted with $H_2O$ (100 mL) and extracted with AcOEt (100 mL x 3). The organic phase was concentrated under reduced pressure and purified by chromatography column (petroleum ether/AcOEt:8/11/1). 11.0 g (81.4% yield) of the ether of interest were obtained in the form of a white solid.

**TABLE 11. INTERMEDIATES OBTAINED FROM THE CORRESPONDING REAGENTS FOLLOWING *STEP IV-H-v.***

| Intermediate | Structure |
|---|---|
| IV-45 | 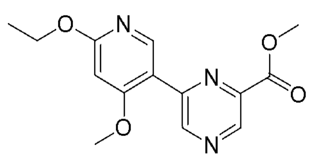 |
| IV-46 | 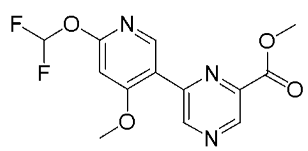 |

**METHOD IV-I**

Step IV-I-i:

*Precursors IV-47i and IV-48i:*

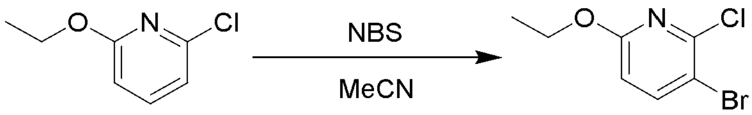


In a round-bottomed flask containing 2-chloro-6-ethoxypyridine (47.0 mmol) in MeCN (30.0 mL), NBS (24.4 mmol) was added. The reaction mixture remained at 80 °C for 16 hours. After total consumption of the starting reagent (monitored by CCD), the mixture was diluted in $H_2O$ (20.0 mL) and extracted with AcOEt (15.0 mL x 2). The organic phase was concentrated under reduced pressure and purified by column chromatography (petroleum ether/AcOEt:10/11/1). 5.20 g (99.0% yield) of the product of interest were obtained in the form of a yellow oil.

**TABLE 12. PRECURSORS OBTAINED FROM THE CORRESPONDING REAGENTS FOLLOWING *STEP IV-I-i.***

| Precursor | Structure |
|---|---|
| *IV-47i* | 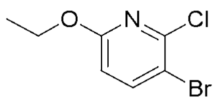 |
| *IV-48i* | 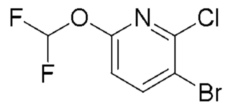 |

Step IV-I-ii:

Precursors *IV-47ii and IV-48ii:*

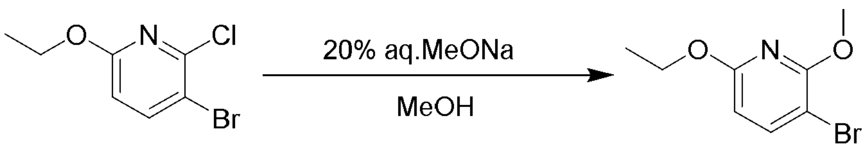


In a round-bottomed flask containing 3-bromo-2-chloro-6-ethoxypyridine (21.9 mmol) in MeOH (20.0 mL), sodium methoxide (109 mmol) was added under $N_2$ atmosphere. The reaction mixture remained at 70 °C for 16 hours. After total consumption of the starting reagent (monitored by CCD), the mixture was diluted with a citric acid solution (10.0%, 40.0 mL) and extracted with AcOEt (20.0 mL x 3). The organic phase was washed with saturated sodium chloride solution *(brine)* (40.0 mL) and concentrated under reduced pressure and purified by preparative HPLC ( [$H_2O$ (TFA) /MeCN] 55. 0% → 85.0% B). 5.00 g (97.9% yield) of the product of interest were obtained in the form of a colorless oil.

**TABLE 13. PRECURSORS OBTAINED FROM THE CORRESPONDING REAGENTS FOLLOWING *STEP IV-I-ii.***

| Precursor | Structure |
|---|---|
| *IV-47ii* | 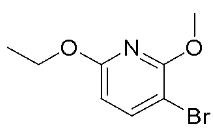 |

(continued)

| Precursor | Structure |
|---|---|
| *IV-48ii* | 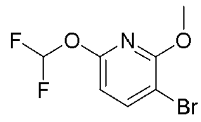 |

Step IV-I-iii:

Precursors *IV-47iii and IV-48iii:*

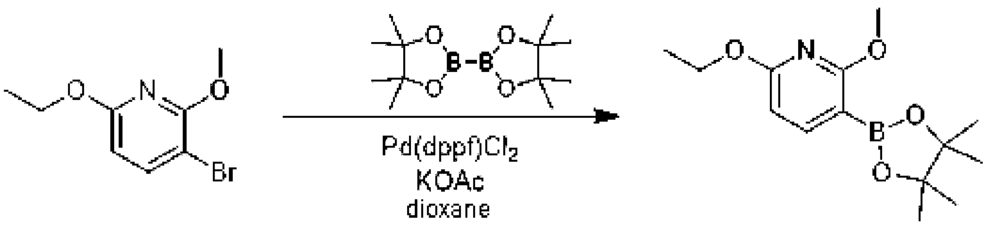


In a round-bottomed flask containing 3-bromo-6-ethoxy-2-methoxypyridine (21.5 mmol) in dioxane (25.0 mL), 4,4,5,5-tetramethyl-2-(4,4,5,5-tetramethyl-1,3,2-dioxaborolan-2-yl)-1,3,2-dioxaborolan (21.5 mmol), potassium acetate (64.6 mmol), and Pd(dppf)$Cl_2$ (2.15 mmol) was added under $N_2$ atmosphere. The reaction mixture remained at 90 °C for 16 hours. After total consumption of the starting reagent (monitored by CCD), the mixture was diluted with $H_2O$ (20.0 mL) and extracted with AcOEt (15.0 mL x 2). The organic phase was concentrated under reduced pressure. 8.06 g (67.0% yield) of the product of interest were obtained in the form of a black oil, which was sent to the next step without further purification.

**TABLE 14. PRECURSORS OBTAINED FROM THE CORRESPONDING REAGENTS FOLLOWING *STEP IV-I-iii.***

| Precursor | Structure |
|---|---|
| *IV-47iii* | 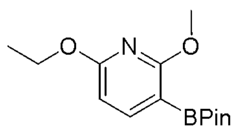 |
| *IV-48iii* | 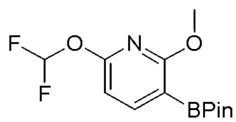 |

Step IV-I-iv:

Intermediates *IV-47 and IV-48:*

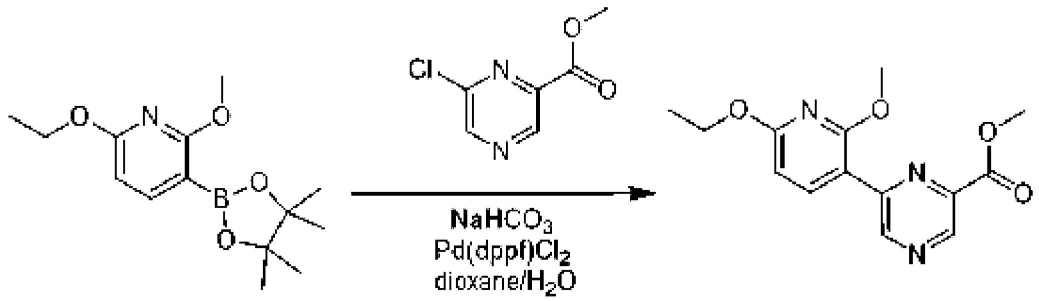


In a round-bottomed flask, a mixture of 6-ethoxy-2-methoxy-3-(4,4,5,5-tetramethyl-1,3,2-dioxaborolan-2-yl) pyridine (63.7 mmol), methyl 6-chloropyrazine-2-carboxylate (57.9 mmol), $NaHCO_3$ (86.9 mmol), and Pd(dppf)$Cl_2$ (2.90 mmol) in dioxane (100 mL) and $H_2O$ (100 mL) was de-gassed and purged with $N_2$3 times. The mixture was maintained in agitation under an atmosphere of $N_2$ at 70 °C for 16 hours. After total consumption of the starting reagent (monitored by LC-MS), the mixture was diluted with $H_2O$ (100 mL) and extracted with AcOEt (100 mL x 3). The organic phase was concentrated under reduced pressure and purified by chromatography column (petroleum ether/AcOEt:8/11/1). 11.0 g (81.4% yield) of the ether of interest were obtained in the form of a white solid.

**TABLE 15. INTERMEDIATES OBTAINED FROM THE CORRESPONDING REAGENTS FOLLOWING *STEP IV-I-iv.***

| Intermediate | Structure |
|---|---|
| *IV-47* | 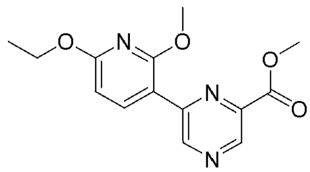 |
| *IV-48* | 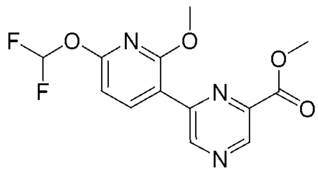 |

**METHOD IV-J**

Step IV-J-i:

*Precursor IV-49i:*

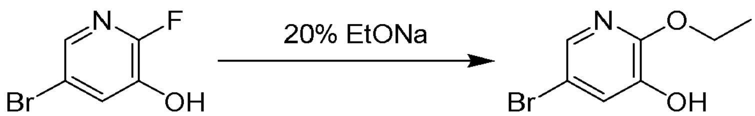


In a round-bottomed flask containing 5-bromo-2-fluoropyridine-3-ol (36.4 mmol) in EtOH (100.0 mL), sodium ethoxide (182 mmol) was added under $N_2$ atmosphere. The reaction mixture remained at 85 °C for 16 hours. After total consumption of the starting reagent (monitored by LC-MS), the pH of the mixture was adjusted to 5 by adding citric acid. Subsequently, the residue was diluted with a citric acid solution (50.0 mL) and extracted with AcOEt (50.0 mL x 3). The organic phase was concentrated under reduced pressure. 7.28 g (90.4% yield) of the product of interest were obtained in the form of a yellow solid, which was sent to the next step without further purification.

Step IV-J-ii:

*Precursor IV-49ii:*

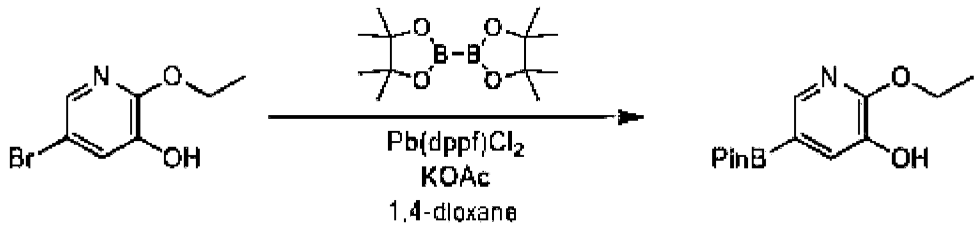


In a round-bottomed flask containing 5-bromo-2-ethoxypyridine-3-ol (18.3 mmol), 4,4,5,5-tetramethyl-2-(4,4,5,5-tetramethyl-1,3,2-dioxaborolan-2-yl)-1,3,2-dioxaborolan (22.0 mmol) and potassium acetate (55.0 mmol) in 1,4-dioxane (80.0 mL), was added, and Pd(dppf)$Cl_2$ (1.83 mmol) under $N_2$ atmosphere. The reaction mixture remained at 90 °C for 16 hours. After total consumption of the starting reagent (monitored by LC-MS), the mixture was diluted with $H_2O$ (80.0 mL) and extracted with AcOEt (80.0 mL x 3). The organic phase was washed with saturated sodium chloride solution *(brine)* (160.0 mL), dried under $Na_2SO_4$, filtered and concentrated under reduced pressure. 3.21 g (63.1% yield) of the product of interest were obtained in the form of a colorless oil, which was sent to the next step without further purification.

<u>Step IV-J-ii:</u>

*IV-49 Intermediate:*

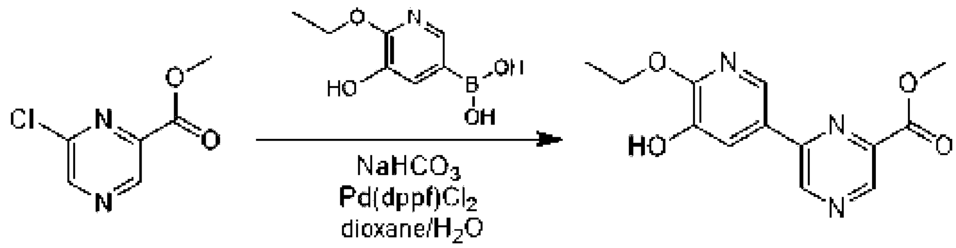


In a round-bottomed flask containing methyl 6-chloropyrazine-2-carboxylate (908 μmol) in MeOH (5.00 mL), (6-ethoxy-5-hydroxypyridine-3-yl)boronic acid (1.09 mmol) was added. The reaction mixture remained at 60 °C for 16 hours. After total consumption of the starting reagent (monitored by CCD), the mixture was filtered, washed with MeOH (15.0 mL) and dried under vacuum. The residue obtained was placed under agitation with MeOH (5.00 mL) at 20 °C for 30 minutes. 270 mg (70.3% yield) of the ester of interest was obtained in the form of a gray solid.

**METHOD IV-K**

<u>Step IV-K-i:</u>

*Precursor IV-50i:*

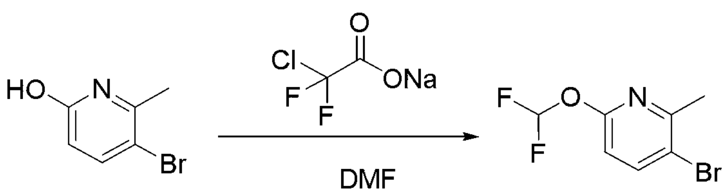


In a round-bottomed flask containing 5-bromo-6-methylpyridine-2-ol (5.32 mmol) in DMF (10 mL) under

agitation, sodium 2-chloro-2,2-difluoroacetate (10.64 mmol) was added at 25 °C. The mixture was heated to 80 °C and maintained under agitation for 12 hours. After total consumption of the starting reagent (monitored by CCD), the mixture was diluted with $H_2O$ (100 mL) and $NaHCO_3$ solution (50 mL). The aqueous phase was extracted with AcOEt (250 mL) and washed with saturated NaCl solution *(brine)* (50 mL). The organic phase was concentrated under reduced pressure and purified by column chromatography (100% hexane). 300 mg (23% yield) of the product of interest was obtained in the form of a colorless liquid.

Step IV-K-ii:

*Precursor IV-50ii:*

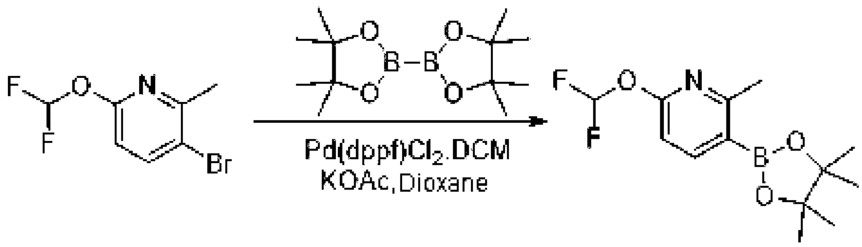


In a round-bottomed flask containing 3-bromo-6-(difluoromethoxy)-2-methylpyridine (1.68 mmol) in 1,4-dioxane (5.0 mL) under agitation, 4,4',4',5,5,5',5'-octamethyl-2,2'-bi(1,3,2-dioxaborole) (2.52 mmol) and potassium acetate (5.04 mmol) at 25 °C were added. The mixture was purged with $N_2$ for 10 minutes, and then Pd(dppf)$Cl_2$ (0.084 mmol) was added. Subsequently, the reaction mixture was kept under agitation at 70 °C for 4 hours. After total consumption of the starting reagent, the mixture was diluted with $H_2O$ (10.0 mL) and extracted with AcOEt (10.0 mL x 3). The organic phase was washed with saturated sodium chloride solution *(brine)* (5.0 mL x 1), dried under $Na_2SO_4$, filtered and concentrated under reduced pressure. The residue obtained was purified by column chromatography (100% hexane). 350 mg (39% yield) of the product of interest were obtained in the form of a yellow solid.

Step IV-K-ii:

IV-50 Intermediate:

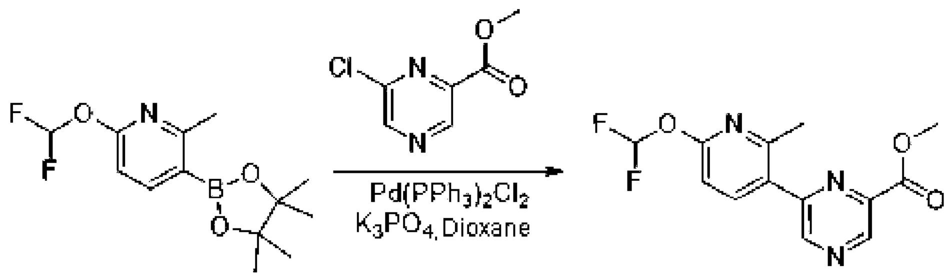


In a round-bottomed flask containing 6-(difluoromethoxy)-2-methyl-3-(4,4,5,5-tetramethyl-1,3,2-dioxaborolan-2-yl) e (1,043 mmol) in 1,4-dioxane (5.0 mL) and $H_2O$ (1,250 mL) under agitation, methyl 6-chloropyrazine-2-carboxylate (0.869 mmol) and trypotassic phosphate (1,738 mmol) were added at 25 °C. The mixture was purged with $N_2$ for 30 minutes, and then bis(triphenylphosphine)palladium(II) chloride (0.043 mmol) was added. Subsequently, the reaction mixture was kept under agitation at 60 °C for 4 hours. After total consumption of the starting reagent, the mixture was filtered with celite, washed with $H_2O$ (200 mL) and extracted with AcOEt (300 mL). The organic phase was washed with saturated sodium chloride solution *(brine)* (100 mL) and concentrated under reduced pressure. The residue obtained was purified by column chromatography (hexane/AcOEt:75/25). 160 mg (58% yield) of ester of interest was obtained in the form of a colorless liquid.

**METHOD IV-L**

Step IV-L-i:

*Precursor IV-51i:*

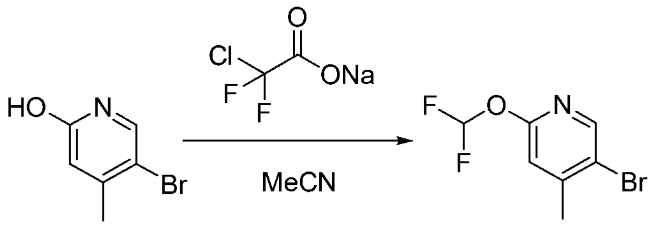


In a round-bottomed flask containing 5-bromo-4-methylpyridine-2-ol (2.66 mmol) in DMF (5 mL) and $H_2O$ (1 mL) under agitation, sodium 2-chloro-2,2-difluoroacetate (5.32 mmol) was added at 25 °C. The mixture was heated to 100 °C and kept under agitation for 12 hours. After total consumption of the starting reagent (monitored by CCD), the mixture was diluted with $H_2O$ (100 mL) and $NaHCO_3$ solution (50 mL). The aqueous phase was extracted with AcOEt (250 mL) and washed with saturated NaCl solution *(brine)* (50 mL). The organic phase was concentrated under reduced pressure and purified by column chromatography (100% hexane). 200 mg (31% yield) of the product of interest was obtained in the form of a colorless liquid.

Step IV-L-ii:

*Precursor IV-51ii:*

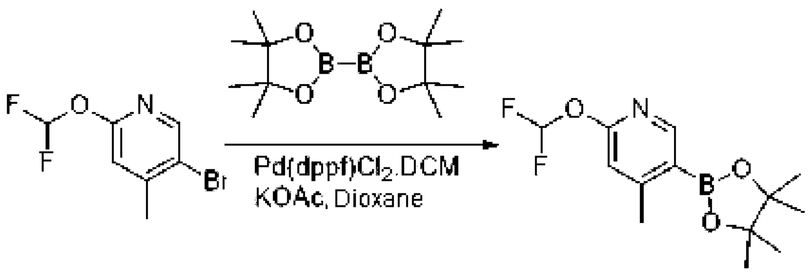


In a round-bottomed flask containing 5-bromo-2-(difluoromethoxy)-4-methylpyridine (2.10 mmol) in 1,4-dioxane (5.0 mL) under agitation, 4,4',4',5,5,5',5'-octamethyl-2,2'-bi(1,3,2-dioxaborole) (4.20 mmol) and potassium acetate (6.30 mmol) at 25 °C were added. The mixture was purged with $N_2$ for 30 minutes, and then Pd(dppf)$Cl_2$ (0.105 mmol) was added. Subsequently, the reaction mixture was kept under agitation at 80 °C for 4 hours. After total consumption of the starting reagent, the mixture was filtered using celite and synthesized funnel. The filtrate was concentrated under reduced pressure and purified by column chromatography (100% hexane). 1.07 g of the mixture of the product of interest were obtained in the form of a colorless liquid.

Step IV-L-iii:

IV-50 Intermediate:

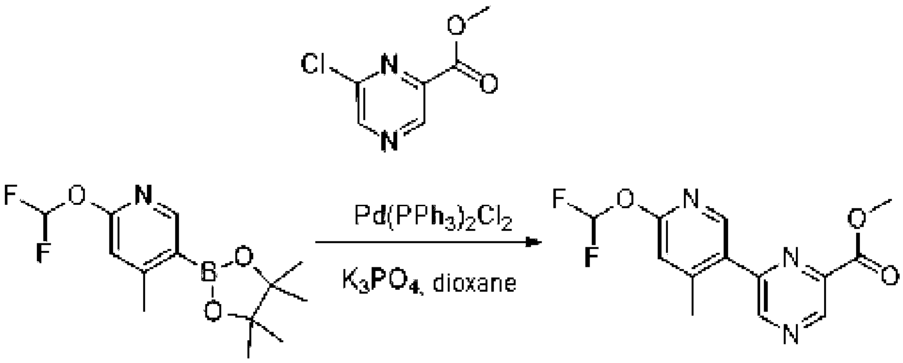


In a round-bottomed flask containing 2-(difluoromethoxy)-4-methyl-5-(4,4,5,5-tetramethyl-1,3,2-dioxaborolan-2-yl)pyridine (2.64 mmol) in 1,4-dioxane (13 mL) and $H_2O$ (3.25 mL) under agitation, methyl 6-chloropyrazine-2-carboxylate (2.202 mmol) and trypotassic phosphate (4.40 mmol) were added at 25 °C. The mixture was purged with $N_2$ for 30 minutes, and then bis(triphenylphosphine)palladium(II) chloride (0.11 mmol) was added. Subsequently, the reaction mixture was kept under agitation at 70 °C for 4 hours. After total consumption of the starting reagent, the mixture was filtered with celite, washed with $H_2O$ (200 mL) and extracted with AcOEt (300 mL). The organic phase was washed with saturated sodium chloride solution *(brine)* (100 mL) and concentrated under reduced pressure. The residue obtained was purified by column chromatography (hexane/AcOEt:90/10). 350 mg (53% yield) of the product of interest were obtained.

**OBTAINING THE FORMULA III INTERMEDIATES:**

Following General Scheme 1, the formula III intermediates are presented.

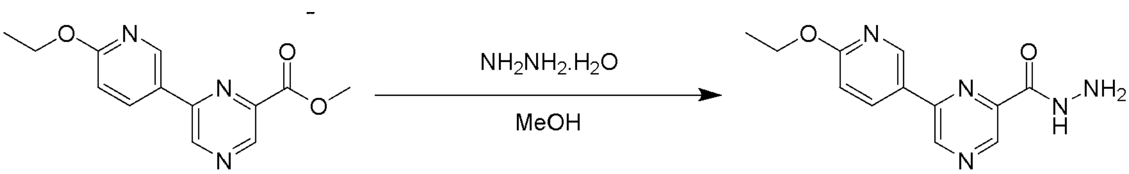


In a round-bottomed flask containing methyl 6-(6-ethoxypyridine-3-yl)pyrazine-2-carboxylate (11.6 mmol) in MeOH (30.0 mL), $N_2H_4 \cdot H_2O$ (17.4 mmol) was added. The reaction mixture was kept under agitation at 60 °C for 3 hours. After total consumption of the starting reagent (monitored by CCD), the mixture was filtered and concentrated. 2.10 g (70.0% yield) of the hydrazide of interest was obtained in the form of a white solid, which was sent to the next step without further purification (Table 16).

**TABLE 16. INTERMEDIATES OBTAINED FROM THE CORRESPONDING REAGENTS FOLLOWING THE METHOD DESCRIBED IN OBTAINING THE COMPOUNDS OF FORMULA III.**

| Intermediate | Structure | Intermediate | Structure |
|---|---|---|---|
| III-1 | 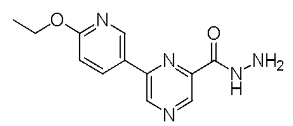 | III-11 | 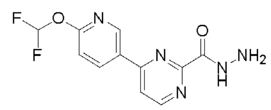 |
| III-2 | 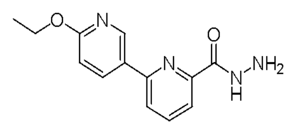 | III-12 | 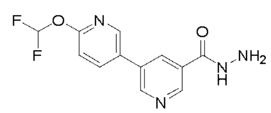 |
| III-3 | 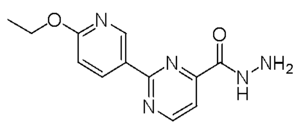 | III-13 | 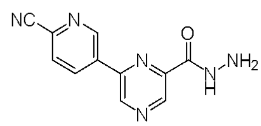 |

(continued)

| Intermediate | Structure | Intermediate | Structure |
|---|---|---|---|
| III-4 | 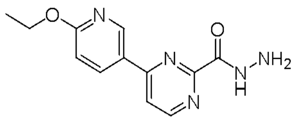 | III-14 | 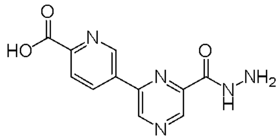 |
| III-5 | 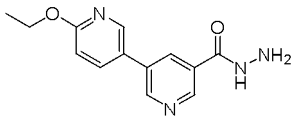 | III-15 | 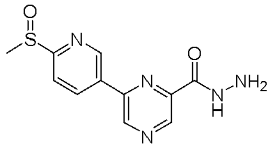 |
| III-6 | 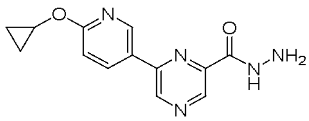 | III-16 | 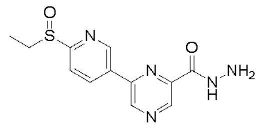 |
| III-7 | 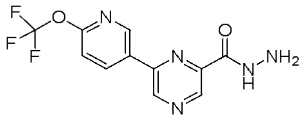 | III-17 | 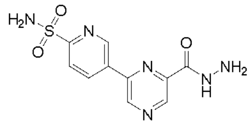 |
| III-8 | 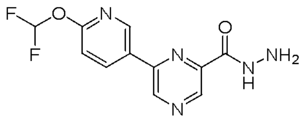 | III-18 | 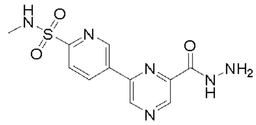 |
| III-9 | 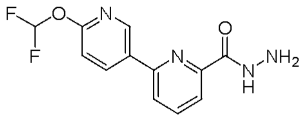 | III-19 | 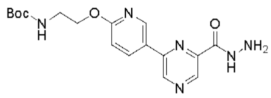 |
| III-10 | 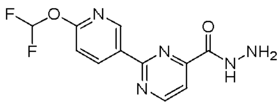 | III-20 | 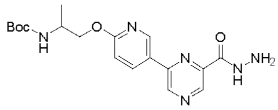 |
| III-21 | 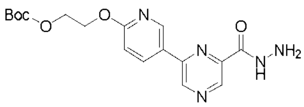 | III-31 | 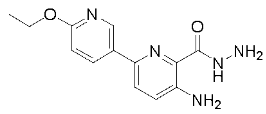 |
| III-22 | 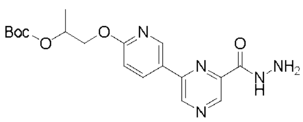 | III-32 | 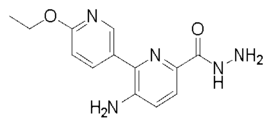 |
| III-23 | 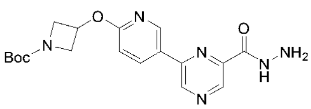 | III-33 | 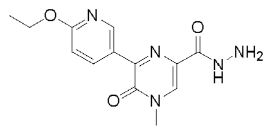 |
| III-24 | 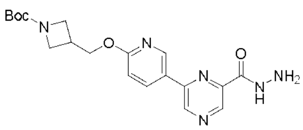 | III-34 | 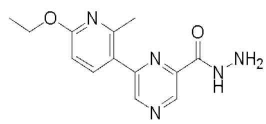 |

(continued)

| Intermediate | Structure | Intermediate | Structure |
|---|---|---|---|
| III-25 | 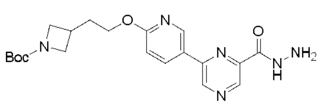 | III-35 | 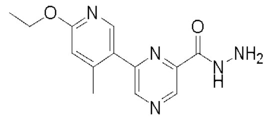 |
| III-26 | 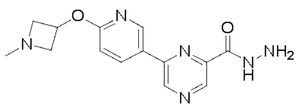 | III-36 | 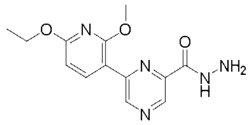 |
| III-27 | 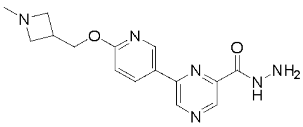 | III-37 | 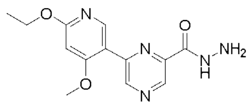 |
| III-28 | 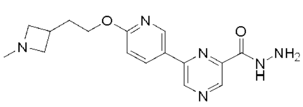 | III-38 | 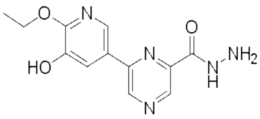 |
| III-29 | 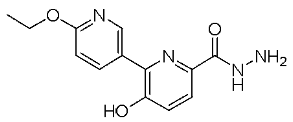 | III-39 | 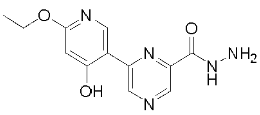 |
| III-30 | 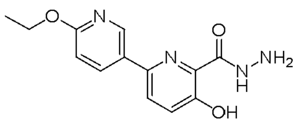 | III-40 | 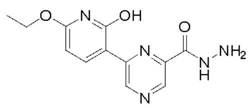 |
| III-41 | 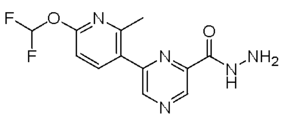 | III-47 | 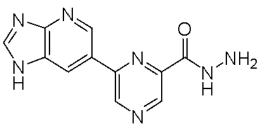 |
| III-42 | 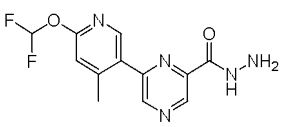 | III-48 | 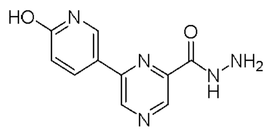 |
| III-43 | 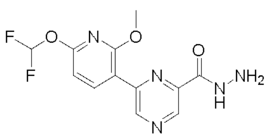 | III-49 | 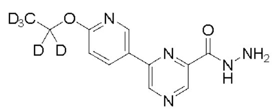 |
| III-44 | 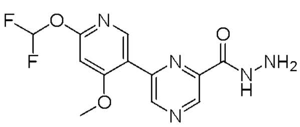 | III-50 | 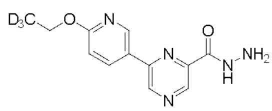 |
| III-45 | 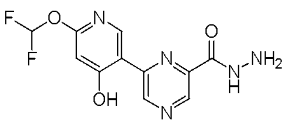 | III-51 | 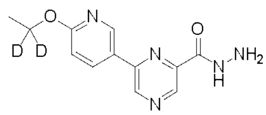 |

(continued)

| Intermediate | Structure | Intermediate | Structure |
|---|---|---|---|
| III-46 | 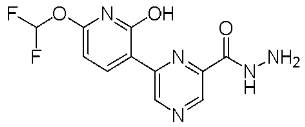 | | |

## OBTAINING FORMULA II COMPOUNDS

Following General Scheme 1, the example methods II-A to II-X for the formation of formula II intermediates are presented without limitations below.

## METHOD II-A

*Intermediate II-1:*

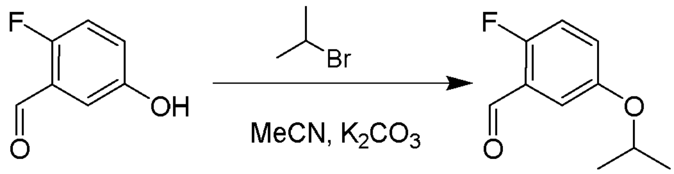


In a round-bottomed flask containing 2-fluoro-5-hydroxybenzaldehyde (14.2 mmol) and MeCN (10.0 mL), 2-bromopropane (28.5 mmol, 2.68 mL) and $K_2CO_3$ (28.5 mmol) were added. Subsequently, the reaction mixture was kept under agitation at 80 °C for 12 hours. After total consumption of the starting reagent (monitored by LC-MS), the reaction mixture was filtered and concentrated under reduced pressure. 1.90 g of the aldehyde of interest were obtained in the form of a yellow solid, which was used for the next step without further purification.

**TABLE 17. INTERMEDIATES OBTAINED FROM THE CORRESPONDING REAGENTS FOLLOWING METHOD II.**

| Intermediate | Structure |
|---|---|
| II-1 | 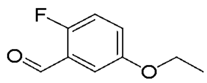 |
| II-2 | 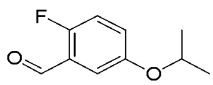 |
| II-3 | 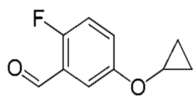 |

**METHOD** II-B

<u>Step II-B-i:</u>

*Precursor II-4i:*

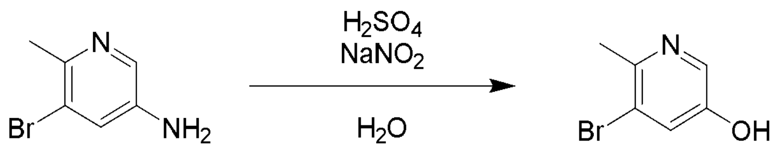


In a round-bottomed flask containing 5-bromo-6-methylpyridine-3-amine (133 mmol), $H_2SO_4$ (668 mmol) in $H_2O$ (250 mL), a solution of $NaNO_2$ (147 mmol) was added in $H_2O$ (50.0 mL) at 0 °C for 30 minutes. Subsequently, the reaction mixture was kept under agitation at 25 °C for 16 hours. After total consumption of the starting reagent (monitored by CCD), the pH of the reaction mixture was adjusted to 7.0 by adding $Na_2CO_3$ at room temperature. The mixture was extracted with AcOEt (15.0 mL x 3). Subsequently, the organic phase was washed with saturated sodium chloride solution *(brine)* (200 mL), dried on anhydrous sodium sulfate, filtered, concentrated under reduced pressure and purified by column chromatography (petroleum ether/AcOEt:10/11/1). 13.5 g (53.7% yield) of the product of interest were obtained in the form of a yellow solid.

Step II-B-ii:

*Precursor II-4ii:*

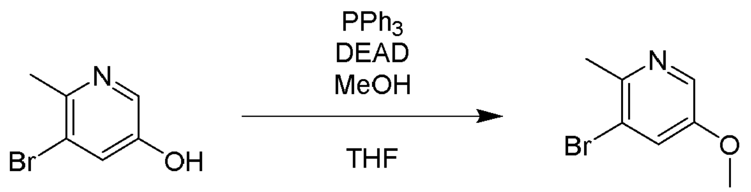


In a round-bottomed flask containing 5-bromo-6-methylpyridine-3-ol (31.9 mmol) in THF (60.0 mL), $PPh_3$ (47.8 mmol), MeOH (63.8 mmol), and DEAD (47.8 mmol) were added at 0 °C. Subsequently, the reaction mixture was kept under agitation at 25 °C for 16 hours. After total consumption of the starting reagent (monitored by CCD), the reaction mixture was diluted in $H_2O$ (40.0 mL) and extracted with AcOEt (20.0 mL x 2). Subsequently, the organic phase was concentrated under reduced pressure and purified by column chromatography (petroleum ether/AcOEt:10/11/1). 3.20 g (49.6% yield) of the product of interest were obtained in the form of a rose oil.

Step II-B-iii:

*Precursor II-4iii:*

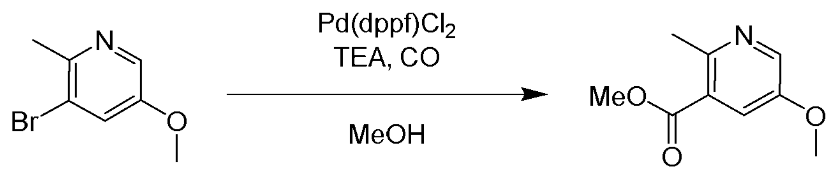


In a round-bottomed flask containing 3-bromo-5-methoxy-2-methylpyridine (31.9 mmol) in MeOH (20.0 mL), $Pd(dppf)Cl_2$ (989 μmol) and TEA (19.8 mmol) were added. Subsequently, the reaction mixture was de-gassed and purged with CO repeatedly. The mixture was kept in agitation under CO atmosphere (50 Psi) at 70 °C for 16 hours. After total consumption of the starting reagent (monitored by CCD), the reaction mixture was diluted in $H_2O$ (15.0 mL) and extracted with AcOEt (8.00 mL x 2). Subsequently, the organic phase was concentrated under reduced pressure and purified by column chromatography (petroleum ether/AcOEt:10/11/1). 1.10 g (61.3% yield) of the product of interest were obtained in the form of a colorless oil.

Step II-B-iv:

*Precursor II-4iv:*

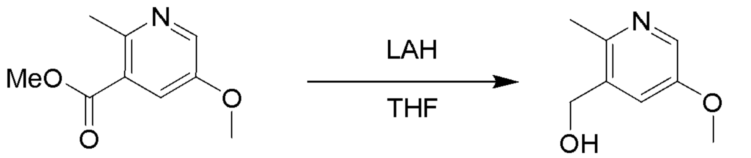


In a round-bottomed flask containing methyl 5-methoxy-2-methylnicotinate (6.07 mmol) in THF (11.0 mL), $LiAlH_4$ (12.1 mmol) was added at 0 °C. Subsequently, the reaction mixture was kept in agitation at 25 °C for 2 hours. After total consumption of the starting reagent (monitored by CCD), $Na_2SO_4 \cdot 10H_2O$ (500 mg) at 0 °C. Next, the mixture was filtered and concentrated under reduced pressure. 900 mg (96.7% yield) of the product of interest was obtained in the form of a colorless oil, which was used for the next step without further purification.

Step II-B-v:

*Intermediate II-4:*

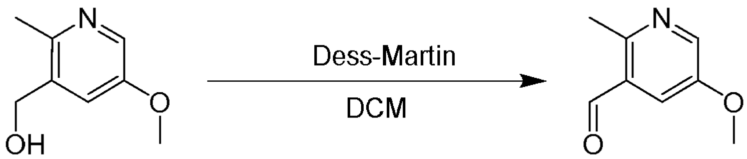


In a round-bottomed flask containing (5-methoxy-2-methylpyridine-3-yl)methanol (5.88 mmol) in DCM (4.20 mL), 1,1-diacetoxy-3-oxo-1,2-benziodoxol-1-yla acetate (8.81 mmol) was added at 0 °C. Subsequently, the reaction mixture was kept in agitation at 25 °C for 2 hours. After total consumption of the starting reagent (monitored by CCD), a solution of $Na_2CO_3$ (10.0%), at room temperature, up to pH = 8, was added to the reaction mixture. Next, the mixture was filtered, extracted with DCM (2.00 mL x 2) and concentrated under reduced pressure. 650 mg (73.1% yield) of the desired aldehyde was obtained in the form of a colorless oil, which was used for the next step without further purification.

**METHOD II-C**

Step II-C-i:

*Precursor II-5i:*

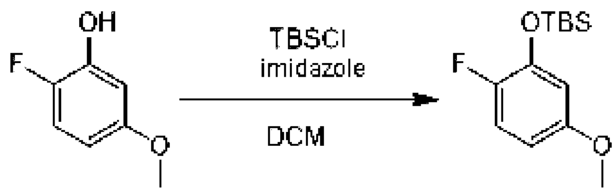


In a round-bottomed flask containing 2-fluoro-5-methoxyphenol (35.2 mmol) and imidazole (52.8 mmol) in DCM (30.0 mL), TBSCl (52.8 mmol) was added. Subsequently, the reaction mixture was kept in agitation at 20 °C for 16 hours. After total consumption of the starting reagent (monitored by CCD and LC-MS), the reaction mixture was diluted with $H_2O$ (30.0 mL) and extracted with DCM (30.0 mL x 3). Subsequently, the organic phase was washed with saturated sodium chloride solution (60.0 mL), dried on anhydrous sodium sulfate, filtered, concentrated under reduced pressure. The resulting product was purified by column chromatography (petroleum ether/AcOEt:10/11/1). 7.88 g (86.1% yield) of the product of interest were obtained in the form of a colorless oil.

Step II-C-ii:

*Precursor II-5ii:*

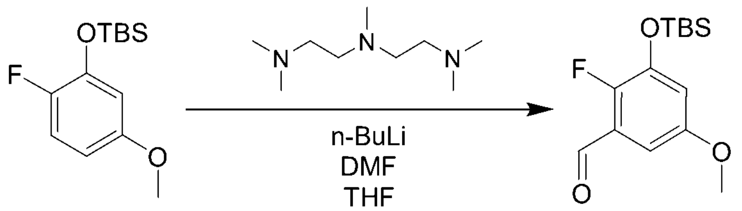


In a round-bottomed flask containing tert-butyl (2-fluoro-5-methoxyphenoxy) dimethylsilane (7.80 mmol) and N'-[2-(dimethylamino)ethyl]-N,N,N'-trimethyl-ethane-1,2-diamine (11.7 mmol) in THF (10.0 mL), n-BuLi (2.50 M, 12.5 mL) was added at -70 °C under $N_2$ atmosphere. Subsequently, the reaction mixture was kept in agitation at -70 °C for 3 hours. Next, DMF (15.6 mmol) in THF (2.00 mL) was added and the mixture was kept in agitation at 10 °C for 1 hour. After total consumption of the starting reagent (monitored by CCD), the reaction mixture was diluted with $H_2O$ (20.0 mL) at 0 °C and extracted with AcOEt (20.0 mL x 3). The organic phase was washed with saturated sodium chloride solution *(brine)* (60.0 mL), dried on anhydrous sodium sulfate, filtered, concentrated under reduced pressure. 2.19 g (70.2% yield) of the product of interest were obtained in the form of a colorless oil, which was used in the next step without further purification.

**METHOD II-D**

Step II-D-i:

*Precursor II-6i:*

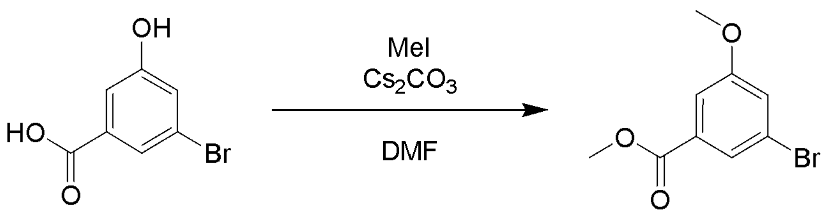


In a round-bottomed flask containing 3-bromo-5-hydroxybenzoic acid (92.2 mmol) in DMF (200 mL), $Cs_2CO_3$ (184 mmol) and methyl iodide (230 mmol) at 0 °C were added. Subsequently, the reaction mixture was kept in agitation at 25 °C for 16 hours under $N_2$ atmosphere. After total consumption of the starting reagent (monitored by LC-MS), the reaction mixture was slowly diluted with $H_2O$ (300 mL) and extracted with AcOEt (150 mL x 3). The organic phase was washed with saturated sodium chloride solution *(brine)* (200 mL), dried on anhydrous sodium sulfate, filtered, concentrated under reduced pressure and purified by column chromatography (petroleum ether/AcOEt:2/1). 20.5 g (90.8% yield) of the product of interest were obtained in the form of a yellow oil.

Step II-D-ii:

*Precursor II-6ii:*

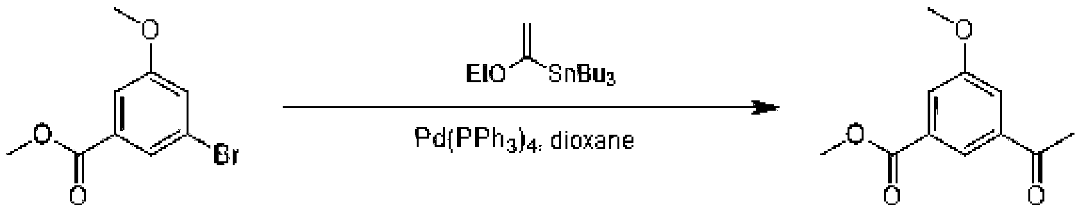


In a round-bottomed flask containing methyl 3-bromo-5-methoxybenzoate (65.3 mmol) in dioxane (160 mL), tributyl(1-ethoxyvinyl)tin (196 mmol) and $Pd(PPh_3)_4$ (32.6 mmol) were added at 25 °C. Subsequently, the reaction mixture was maintained in agitation at 100 °C for 16 hours under $N_2$ atmosphere. After total consumption of the starting reagent (monitored by LC-MS), the reaction mixture was cooled to 25 °C. Next, an HCl solution (2 M, 80.0 mL) was added. The mixture was kept in agitation at 45 °C for 0.5 hours, concentrated and diluted in DCM (200 mL). Subsequently, the pH of the mixture was adjusted to 4.0 with saturated NaHCO solutions. The organic phase was washed with saturated sodium chloride solution *(brine)* (300 mL), dried on anhydrous sodium sulfate, filtered, concentrated under reduced pressure and purified by column chromatography (petroleum ether/AcOEt:3/1). 9.40 g (69.2% yield) of the product of interest were obtained in the form of a yellow solid.

Step II-D-iii:

*Precursor II-6iii:*

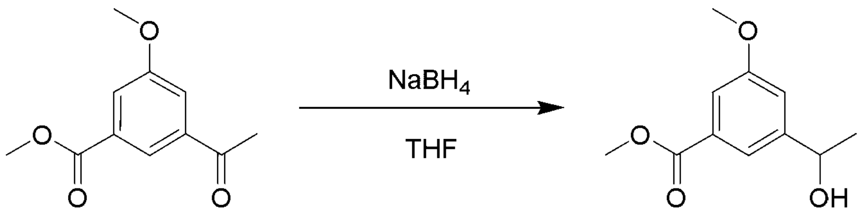


In a round-bottomed flask containing methyl 3-acetyl-5-methoxybenzoate (24.0 mmol) in THF (200 mL), $NaBH_4$ (36.0 mmol) at 0 °C was added. Subsequently, the reaction mixture was kept in agitation at 25 °C for 4 hours under $N_2$ atmosphere. Monitoring by CCD and LC-MS showed that the starting reagent was not completely consumed and a main peak with the desired mass was detected. A solution of $NH_4Cl$ (20.0 mL) was added and the reaction mixture was extracted with AcOEt (300 mL x 3). The organic phase was washed with saturated sodium chloride solution *(brine)* (300 mL), dried on anhydrous sodium sulfate, filtered, concentrated under reduced pressure and purified by column chromatography (petroleum ether/AcOEt:3/11/1). 2.00 g of the product of interest were obtained in the form of a white solid.

Step II-D-iv:

*Precursor II-6iv:*

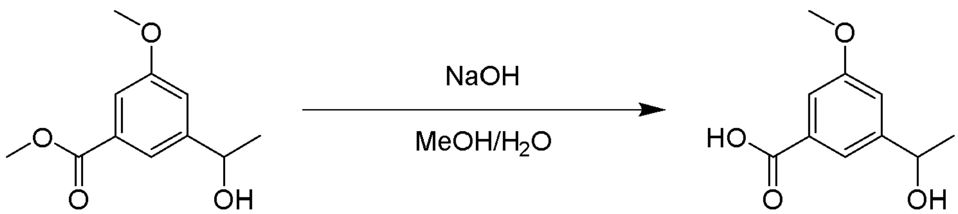


In a round-bottomed flask containing 3-(1-hydroxyethyl)-5-methyl methoxybenzoate (12.8 mmol) in MeOH (20.0 mL), NaOH (15.4 mmol) was added in $H_2O$ (20.0 mL) at 25 °C. Subsequently, the reaction mixture was kept in agitation at 25 °C for 16 hours under $N_2$ atmosphere. After total consumption of the starting reagent (monitored by LC-MS and CCD), the reaction mixture was concentrated, dissolved in DCM (5.00 mL) and the pH was adjusted to 2.0 with HCl solution (2 M). The aqueous phase was extracted with DCM (30.0 mL x 3). Subsequently, the organic phase was washed with saturated

sodium chloride solution *(brine)* (50.0 mL), dried on anhydrous sodium sulfate, filtered, concentrated under reduced pressure. 3.00 g of the product of interest was obtained in the form of a yellow oil, which was used without further purification.

Step II-D-v:

*Precursor II-6v:*

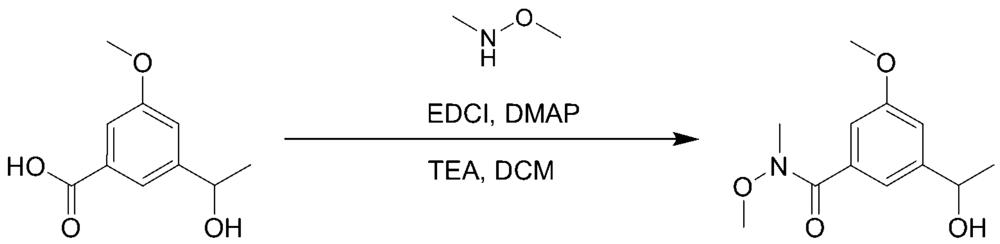


In a round-bottomed flask containing 3-(1-hydroxyethyl)-5-methoxybenzoic acid (15.3 mmol) in DCM (30.0 mL), N,O-dimethylhydroxylamine (16.8 mmol), DMAP (7.65 mmol), TEA (22.9 mmol), and EDCI (22.9 mmol) was added at 25 °C. Subsequently, the reaction mixture was kept in agitation at 25 °C for 16 hours under $N_2$ atmosphere. After total consumption of the starting reagent (monitored by LC-MS and CCD), the reaction mixture was diluted in $H_2O$ (40.0 mL) and extracted with DCM (40.0 mL x 3). The organic phase was washed with saturated sodium chloride solution *(brine)* (50.0 mL), dried on anhydrous sodium sulfate, filtered, concentrated under reduced pressure and purified by column chromatography (DCM/MeOH: 10/1). 1.00 g of the product of interest were obtained in the form of a yellow oil.

Step II-D-vi:

*Intermediate II-6:*

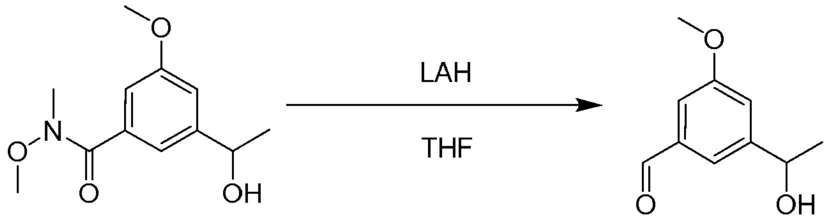


In a round-bottomed flask containing 3-(1-hydroxyethyl)-N,5-dimethoxy-N-methylbenzamide (12.5 mmol) in THF (30 mL), $LiAlH_4$ (2.5 M, 7.52 mL) was added at 0 °C. Subsequently, the reaction mixture was kept in agitation at 25 °C for 16 hours under $N_2$ atmosphere. After total consumption of the starting reagent (monitored by CCD), it was added to the $NH_4Cl$ (100 mL) reaction mixture and extracted with DCM (100 mL x 3). The organic phase was washed with saturated sodium chloride solution *(brine)* (100.0 mL), dried on anhydrous sodium sulfate, filtered and concentrated under reduced pressure. 1.68 g of the product of interest were obtained in the form of a yellow oil, which was used for the next step without further purification.

**METHOD II-E**

Step II-E-i:

*Precursor II-7i:*

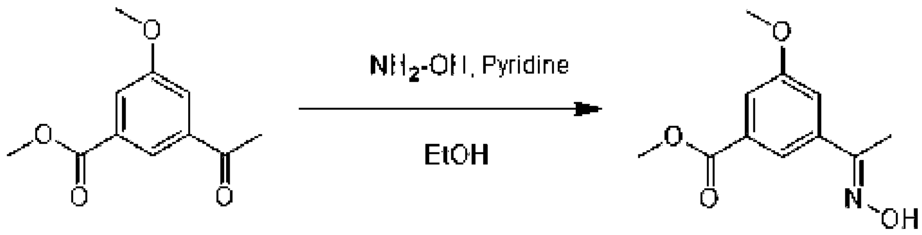


In a round-bottomed flask containing methyl 3-acetyl-5-methoxybenzoate (19.2 mmol) in EtOH (40 mL), pyridine (38.4 mmol) and $NH_2OH.HCl$ (9.61 mmol) at 25 °C. were added. Subsequently, the reaction mixture was kept in agitation at 80 °C for 16 hours under $N_2$ atmosphere. After total consumption of the starting reagent (monitored by LC-MS), the reaction mixture was concentrated under reduced pressure and purified by column chromatography (petroleum ether/-AcOEt:2/1). 2.50 g (58.3% yield) of the product of interest were obtained in the form of a yellow solid.

Step II-E-ii:

*Precursor II-7ii:*

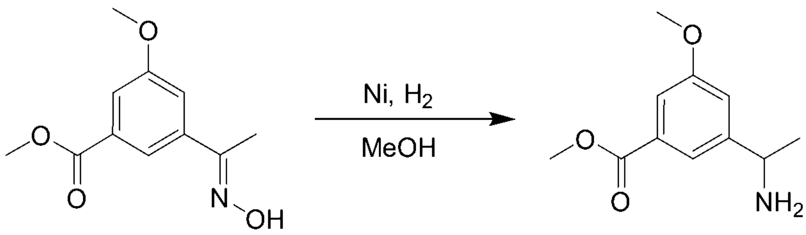


In a round-bottomed flask containing 3-(1-(hydroxymine)ethyl)-5-methyl methoxybenzoate (19.3 mmol) in MeOH (43.0 mL), Raney-Ni (50.2 mmol) was added. Subsequently, the reaction mixture was maintained in agitation at 25 °C for 1 hour under an atmosphere of $H_2$ (19.3 mmol). After full consumption of the starting reagent (monitored by LC-MS). The mixture was filtered and concentrated under reduced pressure. 3.40 g of the product of interest were obtained in the form of a white oil.

Step II-E-iii:

*Precursor II-7iii:*

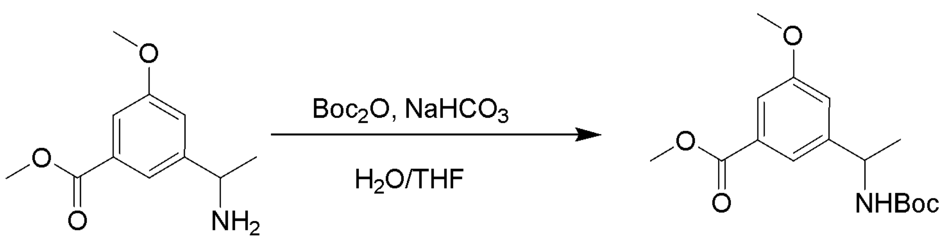


In a round-bottomed flask containing 3-(1-aminoethyl)-5-methoxybenzoate (15.3 mmol) in THF (15.0 mL) and $H_2O$ (15.0 mL), $NaHCO_3$ (30.6 mmol) and $Boc_2O$ (22.9 mmol) at 25 °C was added. Subsequently, the reaction mixture was kept in agitation at 25 °C for 16 hours under $N_2$ atmosphere. After total consumption of the starting reagent (monitored by CCD), the mixture was diluted in $H_2O$ (20.0 mL) and extracted with AcOEt (30.0 mL x 3). The organic phase was washed with saturated sodium chloride solution *(brine)* (30.0 mL), dried on anhydrous sodium sulfate, filtered, concentrated under reduced pressure. 5.00 g of the product of interest were obtained in the form of a white solid, which was used in the next step without further purification.

Step II-E-iv:

*Precursor II-7iv:*

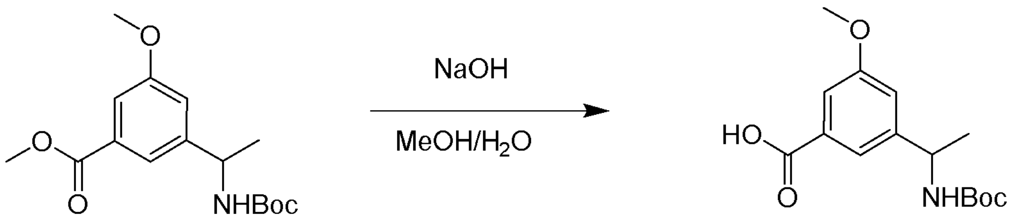


In a round-bottomed flask containing 3-(1-((tert-butoxycarbonyl)amino)ethyl)-5-methyl methoxybenzoate (19.4 mmol) in MeOH (30.0 mL), NaOH (58.2 mmol) was added in $H_2O$ (30.0 mL) at 25 °C. Subsequently, the reaction mixture was kept in agitation at 25 °C for 16 hours under $N_2$ atmosphere. After total consumption of the starting reagent (monitored by CCD), the reaction mixture was concentrated, and the pH was adjusted to 2.0 with HCl solution (2 M). The aqueous phase was extracted with DCM (30 mL x 3). Subsequently, the organic phase was washed with saturated sodium chloride solution *(brine)* (30 mL x 3), dried on anhydrous sodium sulfate, filtered, concentrated under reduced pressure. 4.40 g of the product of interest were obtained in the form of a white solid, which was used without further purification.

Step II-E-v:

*Precursor II-7v:*

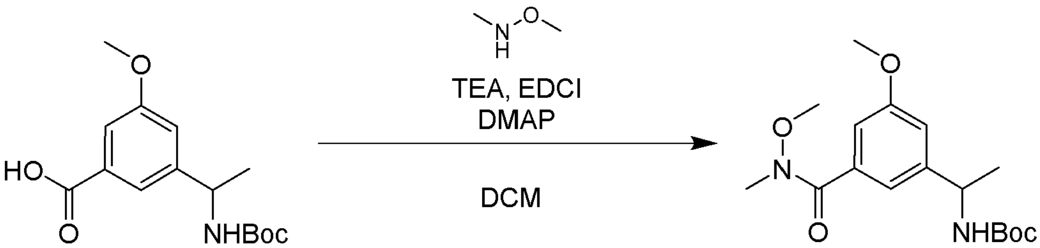


In a round-bottomed flask containing 3-(1-((tert-butoxycarbonyl)amino)ethyl)-5-methoxybenzoic acid (8.47 mmol) in DCM (25.0 mL), N,O-dimethylhydroxylamine (5.83 mmol), DMAP (12.7 mmol), TEA (25.4 mmol), and EDCI (12.7 mmol) was added at 25 °C. Subsequently, the reaction mixture was kept in agitation at 25 °C for 16 hours under $N_2$ atmosphere. After total consumption of the starting reagent (monitored by LC-MS), the reaction mixture was diluted in $H_2O$ and extracted with DCM (30 mL x 2). The organic phase was washed with saturated sodium chloride solution *(brine)* (50 mL x 3), dried on anhydrous sodium sulfate, filtered and concentrated under reduced pressure. 2.60 g of the product of interest were obtained in the form of a white solid.

Step II-E-vi:

*Intermediate II-7:*

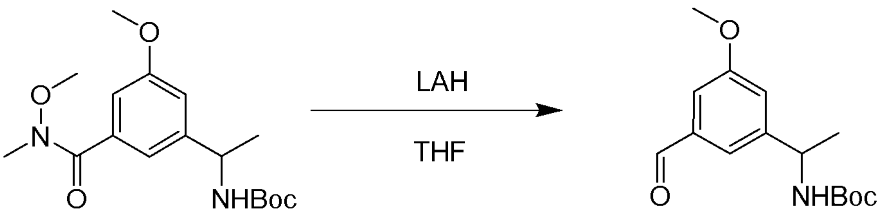


In a round-bottomed flask containing tert-butyl (1-(3-methoxy-5-(methoxy(methyl)carbamoyl)phenyl)ethyl)carbamate (29.6 mmol) in THF (100 mL), $LiAlH_4$ (2.5 M, 17.7 mL) at 0 °C was added. Subsequently, the reaction mixture was

kept in agitation at 25 °C for 16 hours under $N_2$ atmosphere. After total consumption of the starting reagent (monitored by CCD), it was added to the $NH_4Cl$ (100 mL) reaction mixture and extracted with DCM (100 mL x 3). The organic phase was washed with saturated sodium chloride solution *(brine)* (100 mL), dried on anhydrous sodium sulfate, filtered and concentrated under reduced pressure. 9.50 g of the product were obtained in the form of a yellow oil, which was used for the next step without further purification.

**METHOD II-F**

Step II-F-i:

*Precursor II-8i:*

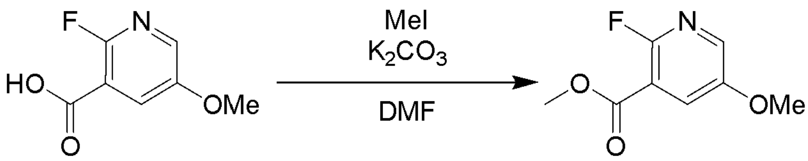


In a round-bottomed flask containing 2-fluoro-5-methoxyicotinic acid (64.3 mmol) and $K_2CO_3$ (96.4 mmol) in DMF (50.0 mL), methyl iodide (129 mmol) was added. Subsequently, the reaction mixture was kept in agitation at 30 °C for 16 hours. After total consumption of the starting reagent (monitored by LC-MS), the reaction mixture was diluted with $H_2O$ (200 mL) and extracted with AcOEt (50.0 mL x 3). The organic phase was washed with saturated sodium chloride solution *(brine)* (100 mL x 2), dried on anhydrous sodium sulfate, filtered, concentrated under reduced pressure. 10.0 g (84.0% yield) of the product of interest were obtained in the form of a brown solid, which was used for the next step without further purification.

Step II-F-ii:

*Precursor II-8ii:*

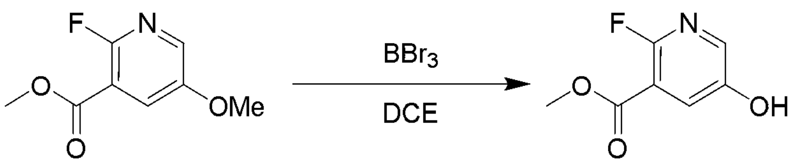


In a round-bottomed flask containing methyl 2-fluoro-5-methoxynicotinate (91.8 mmol) in DCE (400 mL), $BBr_3$ (230 mmol) was added at 0 °C. Subsequently, the reaction mixture was kept in agitation at 80 °C for 4 hours. After total consumption of the starting reagent (monitored by LC-MS), the reaction mixture was cooled to 20 °C, diluted with $H_2O$ (200 mL), and extracted with DCM/MeOH: 5/1 (100 mL x 5). The organic phase was washed with saturated sodium chloride solution (*brine*) (100 mL), dried on anhydrous sodium sulfate, filtered and concentrated under reduced pressure. 11.0 g (70.0% yield) of the product of interest were obtained in the form of a brown solid, which was used for the next step without further purification.

Step II-F-iii:

*Precursor II-8iii:*

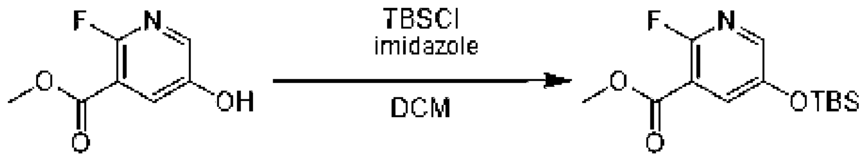


In a round-bottomed flask containing methyl 2-fluoro-5-hydroxynicotinate (40.9 mmol) and imidazole (81.8 mmol) in DCM (50.0 mL), TBSCl (45.0 mmol) was added at 0 °C. Subsequently, the reaction mixture was kept in agitation at 20 °C for 16 hours. After total consumption of the starting reagent (monitored by CCD), the reaction mixture was diluted with $H_2O$ (50.0 mL) and extracted with DCM (50.0 mL x 3). Subsequently, the organic phase was concentrated under reduced pressure and purified by column chromatography (petroleum ether/AcOEt:50/10/1). 6.50 g (55.7% yield) of the product of interest were obtained in the form of a colorless oil.

Step II-F-iv:

*Precursor II-8iv:*

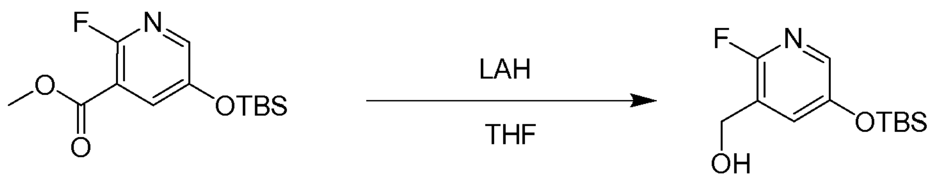


In a round-bottomed flask containing methyl 5-((tert-butyldimethyl)oxy)-2-fluoronicotinate (22.8 mmol) in THF (50.0 mL), $LiAlH_4$ (45.6 mmol) was added at 0 °C. Subsequently, the reaction mixture was kept in agitation at 20 °C for 1 hour. After full consumption of the starting reagent (monitored by CCD), $Na_2SO_4 \cdot 10H_2O$ (3.00 g) was added to the reactional mixture. Next, the mixture was filtered and extracted with AcOEt (10.0 mL x 3). The organic phase was concentrated under reduced pressure. 4.00 g (68.2% yield) of the product of interest were obtained in the form of a yellow oil, which was used for the next step without further purification.

Step II-F-v:

*Precursor II-8v:*

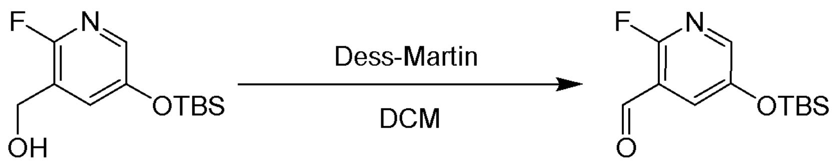


In a round-bottomed flask containing (5-((tert-butyldimethylsilyl)oxy)-2-fluoropyridin-3-yl)methanol (15.5 mmol) in DCM (30.0 mL), 1,1-diacetoxy-3-oxo-1,2-benziodoxol-1-yla acetate (23.3 mmol) was added at 0 °C. Subsequently, the reaction mixture was kept in agitation at 20 °C for 16 hours. After total consumption of the starting reagent (monitored by CCD and LC-MS), a solution of $NaHCO_3$ up to pH = 8 was added to the reaction mixture. The mixture was then filtered, diluted in $H_2O$ (50.0 mL), extracted with DCM (50.0 mL x 3), concentrated under reduced pressure, and purified by column chromatography (petroleum ether/AcOEt: 10/10/1). 3.20 g (80.6% yield) of the product of interest were obtained in the form of a yellow oil.

Step II-F-vi:

*Intermediate II-8:*

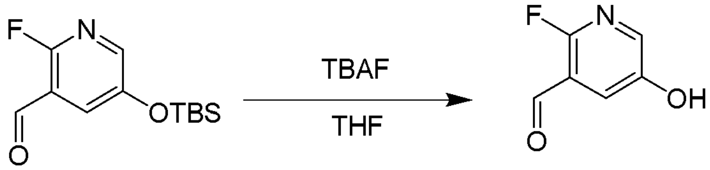


In a round-bottomed flask containing 5-((tert-butyldimethyl)oxy)-2-fluoronicotinaldehyde (12.5 mmol) in THF (15.0 mL), TBAF (1 M, 18.8 mL) was added. Subsequently, the reaction mixture was kept in agitation at 20 °C for 1 hour. After total consumption of the starting reagent (monitored by CCD and LC-MS), the reaction mixture was diluted with $H_2O$ (10.0 mL) and extracted with AcOEt (10.0 mL x 3). The organic phase was washed with saturated sodium chloride solution *(brine)* (10.0 mL), dried on anhydrous sodium sulfate, filtered, concentrated under reduced pressure and purified by column chromatography (petroleum ether/AcOEt:10/10/1). 1.50 g (84.8% yield) of the product of interest were obtained in the form of a white solid.

**METHOD II-G**

Step II-G-i:

*Precursor II-9i:*

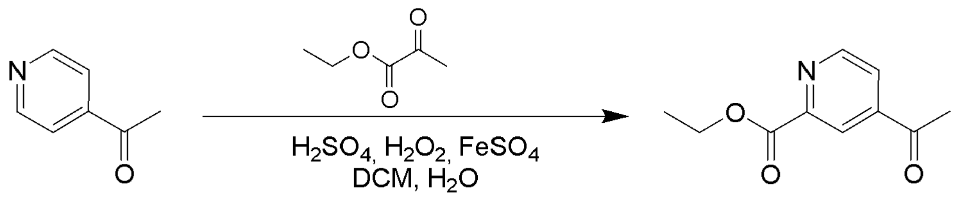


In a round-bottomed flask containing ethyl 2-oxopropanoate (371 mmol) $H_2O_2$ (30.0%, 353 mmol) was added dropwise under agitation at 0 °C. The resulting solution was slowly added to the mixture containing 1-(pyridine-4-yl) ethan-1-one (123 mmol), $H_2SO_4$ (123 mmol), and $FeSO_4.7H_2O$ (123 mmol) in DCM (375 mL) and $H_2O$ (25.0 mL) at 25 °C. Subsequently, the reaction mixture was kept in agitation at 25 °C for 2 hours. After total consumption of the starting reagent (monitored by LC-MS), the organic phase was separated, and the aqueous phase was extracted with DCM (100 mL x 5). Subsequently, the organic phases were combined and washed with a solution of $Na_2SO_3$ (5%), $H_2O$ and dried. The mixture from the combination of two reactions was purified by HPLC in reverse phase (neutral condition). 15.0 g (30.4% yield) of the product of interest were obtained in the form of a white solid.

Step II-G-ii:

*Precursor II-9ii:*

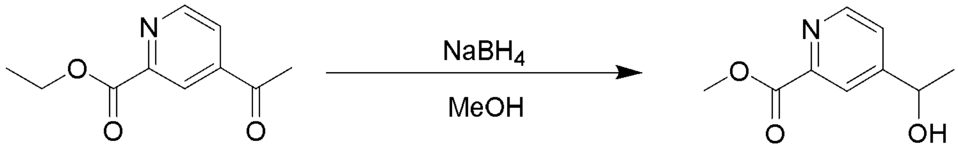


In a round-bottomed flask containing 4-ethyl acetylpicolinate (15.5 mmol) in MeOH (10.0 mL), $NaBH_4$ (18.6 mmol) was added at 0 °C. Subsequently, the reaction mixture was kept in agitation at 25 °C for 2 hours. After total consumption of the starting reagent (monitored by LC-MS), the reaction mixture from the combination of four reactions was diluted with a saturated solution of $NH_4Cl$ (30.0 mL) and extracted with AcOEt. The organic phase was washed with saturated sodium chloride solution *(brine),* dried on anhydrous sodium sulfate, filtered, concentrated under reduced pressure. 5.20 g of the product of interest were obtained in the form of a colorless oil, which was used for the next step

without further purification.

Step II-G-ii:

*Intermediate II-9:*

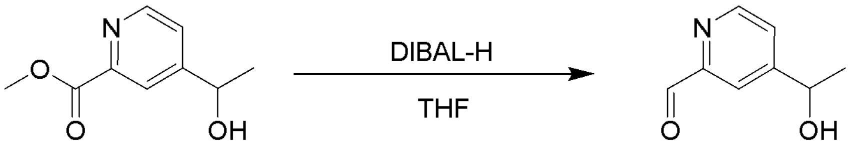


In a round-bottomed flask containing 4-(1-hydroxyethyl)methyl picolinate (27.60 mmol) in THF (20.0 mL), DIBAL-H (1.00 M, 82.7 mL) was added at -78 °C. Subsequently, the reaction mixture was kept in agitation at -78 °C for 3 hours. After total consumption of the starting reagent (monitored by CCD), the reaction mixture was diluted with a solution of $NH_4Cl$ (100 mL), filtered and dried on anhydrous sodium sulfate, filtered, concentrated under reduced pressure, and purified by column chromatography (petroleum ether/AcOEt: 100/11/1). 1.70 g of the product of interest were obtained in the form of a colorless oil.

**METHOD II-H**

Step II-H-i:

*Precursor II-10i:*

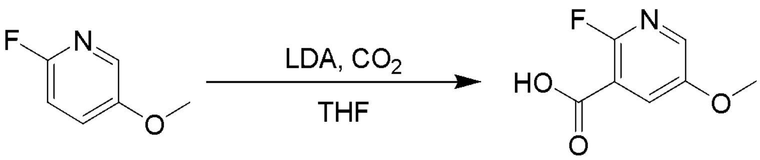


In a round-bottomed flask containing 2-fluoro-5-methoxypyridine (275 mmol) in THF (350 mL), a solution of LDA (2 M, 275 mL) was added dropwise at -60 °C under nitrogen atmosphere. The reaction mixture remained at -60 °C for 0.5 hours. After total consumption of the starting reagent (monitored by LC-MS), an HCl solution (2N, 200 mL) at 0 °C was added. Subsequently, the reaction mixture was extracted with AcOEt (200 mL x 3). The organic phase was washed with saturated sodium chloride solution *(brine)* (100 mL), dried on anhydrous sodium sulfate, filtered and concentrated under reduced pressure. 30.0 g (63.6% yield) of the product of interest was obtained in the form of a yellow solid, which was used for the next step without further purification.

Step II-H-ii:

*Precursor II-10ii:*

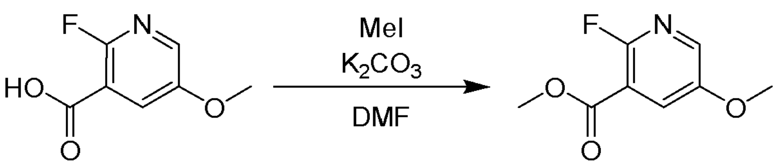


In a round-bottomed flask containing 2-fluoro-5-methoxynicotinic acid (151 mmol) in DMF (156 mL), methyl iodide (227 mmol) and $K_2CO_3$ (227 mmol) were added. Subsequently, the reaction mixture was kept in agitation at 25 °C for 16 hours. After total consumption of the starting reagent (monitored by CCF), the reaction mixture was diluted with $H_2O$ (700 mL) and extracted with AcOEt (300 mL x 2). The organic phase was concentrated and under reduced pressure. 20.0 g

(62.7% yield) of the product of interest were obtained in the form of a yellow solid, which was used for the next step without further purification.

Step II-H-iii:

*Precursor II-10iii:*

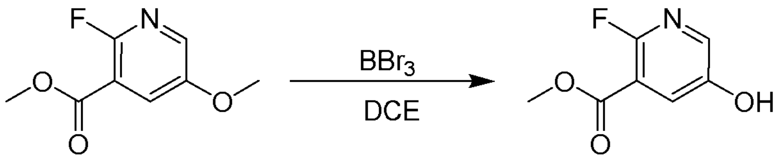


In a round-bottomed flask containing methyl 2-fluoro-5-methoxynicotinate (91.8 mmol) in DCE (400 mL), $BBr_3$ (230 mmol) was added at 0 °C. Subsequently, the reaction mixture was kept in agitation at 70 °C for 16 hours. After total consumption of the starting reagent (monitored by LC-MS), the reaction mixture was cooled to 20 °C, diluted with $H_2O$ (200 mL), and extracted with DCM/MeOH: 5/1 (100 mL x 5). The organic phase was washed with saturated sodium chloride solution *(brine) (100* mL), dried on anhydrous sodium sulfate, filtered and concentrated under reduced pressure. 11.0 g (70.0% yield) of the product of interest were obtained in the form of a brown solid, which was used for the next step without further purification.

Step II-H-iv:

*Precursor II-10iv:*

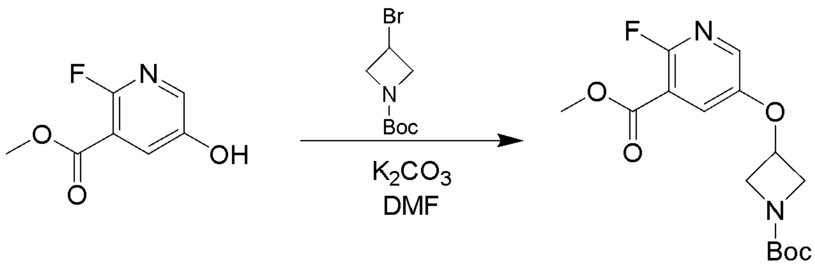


In a round-bottomed flask containing methyl 2-fluoro-5-hydroxynicotinate (14.0 mmol) in DMF (16.8 mL), tert-butyl 3-bromoazetidine-1-carboxylate (16.8 mmol) and $K_2CO_3$ (21.0 mmol) were added under $N_2$ atmosphere. Subsequently, the reaction mixture was kept in agitation at 80 °C for 16 hours. After total consumption of the starting reagent (monitored by CCD), the reaction mixture was diluted with $H_2O$ (20.0 mL) and extracted with AcOEt (15.0 mL x 2). The organic phase was concentrated under reduced pressure. The resulting product was purified by column chromatography (petroleum ether/AcOEt:20/11/1). 1.00 g (21.8% yield) of the product of interest was obtained in the form of a yellow solid.

Step II-H-v:

*Precursor II-10v:*

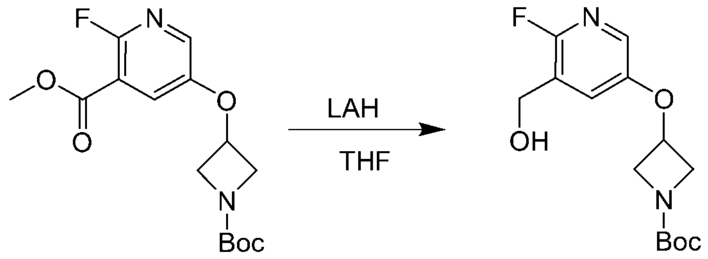


In a round-bottomed flask containing 5-((1-(tert-butoxycarbonyl)azetidine-3-yl)oxy)-2-methyl fluoronicotinate (3.06 mmol) in THF (10.0 mL), $LiAlH_4$ (4.60 mmol) was added at 0 °C. Subsequently, the reaction mixture was kept in agitation at 20 °C for 2 hours under $N_2$ atmosphere. After total consumption of the starting reagent (monitored by CCD), $Na_2SO_4 \cdot 10H_2O$ (600 mg) were added at 0 °C. Next, the mixture was filtered and concentrated under reduced pressure. 630 mg (68.92% yield) of the product of interest was obtained in the form of a colorless oil, which was used for the next step without further purification.

Step II-H-vi

Intermediate II-10:

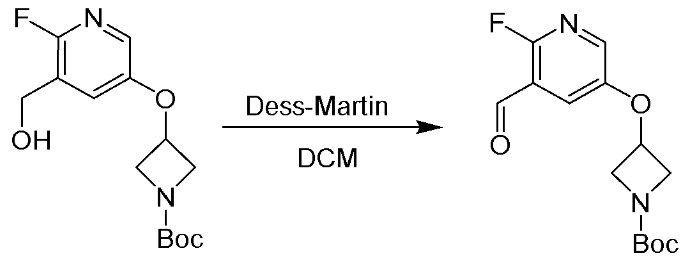


In a round-bottomed flask containing tert-butyl 3-((6-fluoro-5-(hydroxymethyl)pyridine-3-yl)oxy)azetidine-1-carboxylate (2.11 mmol) in DCM (6.30 mL), 1,1-diacetoxy-3-oxo-1,2-benziodoxol-1-yl acetate (3.17 mmol) was added at 0 °C. Subsequently, the reaction mixture was kept under agitation at 20 °C for 2 hours. After total consumption of the starting reagent (monitored by CCD), a solution of $Na_2CO_3$ (10%), at room temperature, up to pH = 8, was added to the reaction mixture. Next, the mixture was filtered, extracted with DCM (5.00 mL x 2) and concentrated under reduced pressure. 300 mg (43.0% yield) of the product of interest were obtained in the form of a black oil, which was used for the next step without further purification.

**METHOD II-I**

Step II-I-i

*Precursor II-11i:*

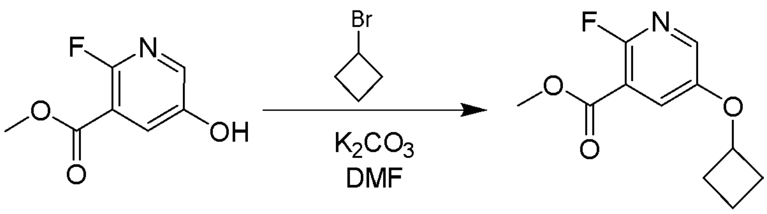


In a round-bottomed flask containing methyl 2-fluoro-5-hydroxynicotinate (5.84 mmol) and DMF (6.00 mL), bromocyclobutane (7.01 mmol) and $K_2CO_3$ (8.77 mmol) were added. Subsequently, the reaction mixture was kept under agitation at 80 °C for 16 hours. After total consumption of the starting reagent (monitored by CCD), the reaction mixture was diluted with $H_2O$ (30.0 mL) and extracted with AcOEt (20.0 mL x 2). Subsequently, the organic phase was concentrated

and purified by column chromatography (petroleum ether/AcOEt:20/11/1). 680 mg (51.6% yield) of the product of interest were obtained in the form of a yellow oil.

Step II-I-ii:

*Precursor II-11ii:*

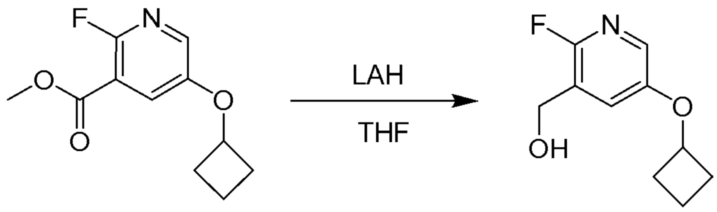


In a round-bottomed flask containing methyl 5-cyclobutoxy-2-fluoronicotinate (3.02 mmol) in THF (6.8 mL), $LiAlH_4$ (4.53 mmol) was added at 0 °C. Subsequently, the reaction mixture was kept in agitation at 20 °C for 2 hours under $N_2$ atmosphere. After total consumption of the starting reagent (monitored by CCD), $Na_2SO_4 \cdot 10H_2O$ (500 mg) at 0 °C. Next, the mixture was filtered and concentrated under reduced pressure. 460 mg (77.2% yield) of the product of interest were obtained in the form of a colorless oil, which was used in the next step without further purification.

Step II-I-ii:

*Intermediate II-11:*

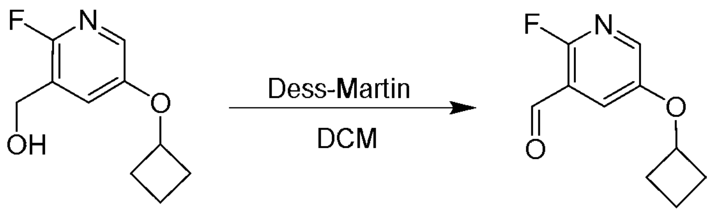


In a round-bottomed flask containing (5-cyclobutoxy-2-fluoropyridine-3-yl)methanol (2.33 mmol) in DCM (4.60 mL), 1,1-diacetoxy-3-oxo-1,2-benziodoxol-1-yla acetate (3.50 mmol) was added at 0 °C. Subsequently, the reaction mixture was kept in agitation at 20 °C for 2 hours. After total consumption of the starting reagent (monitored by CCD), a solution of $Na_2CO_3$ (10%), at room temperature, up to pH = 8, was added to the reaction mixture. Next, the mixture was filtered, extracted with DCM (6.00 mL x 2) and concentrated under reduced pressure. 400 mg (87.8% yield) of the product of interest were obtained in the form of a yellow oil, which was used for the next step without further purification.

**METHOD II-J**

Step II-J-i:

*Precursor II-12i:*

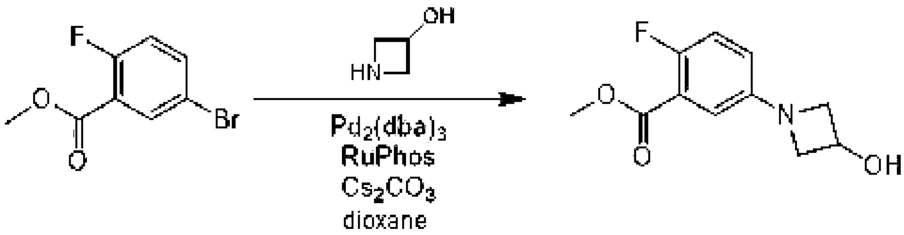


In a round-bottomed flask containing methyl 5-bromo-2-fluorobenzoate (21.4 mmol) in dioxane (50.0 mL), azetidine-3-ol hydrochloride (25.7 mmol), RuPhos (2.15 mmol), $Cs_2CO_3$ (64.3 mmol) and $Pd_2$ (dba) $_3$ (2.15 mmol) was added under $N_2$ atmosphere. Subsequently, the reaction mixture was kept in agitation at 80 °C for 16 hours. After total consumption of the starting reagent (monitored by CCD), the reaction mixture was diluted with $H_2O$ (40.0 mL) and extracted with AcOEt (30.0 mL x 2). Subsequently, the organic phase was concentrated under reduced pressure and purified by column chromatography (petroleum ether/AcOEt:20/11/1). 1.80 g (37.2% yield) of the product of interest were obtained in the form of a white solid.

Step II-J-ii:

*Precursor II-12ii:*

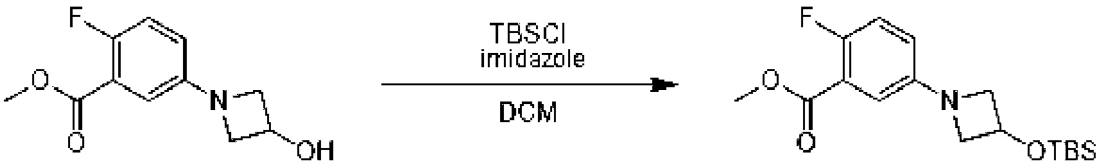


In a round-bottomed flask containing 2-fluoro-5-(3-hydroxyazetidine-1-yl)methyl benzoate (4.44 mmol) in DCM (10.0 mL), TBSCl (6.66 mmol) and imidazole (6.66 mmol) at 0 °C was added. Subsequently, the reaction mixture was kept in agitation at 20 °C for 16 hours. After total consumption of the starting reagent (monitored by LC-MS), the reaction mixture was diluted with $H_2O$ (10.0 mL) and extracted with DCM (6.00 mL x 2). Subsequently, the organic phase was concentrated under reduced pressure. 1.40 g (92.8% yield) of the product of interest were obtained in the form of a yellow oil.

Step II-J-iii:

*Precursor II-12iii:*

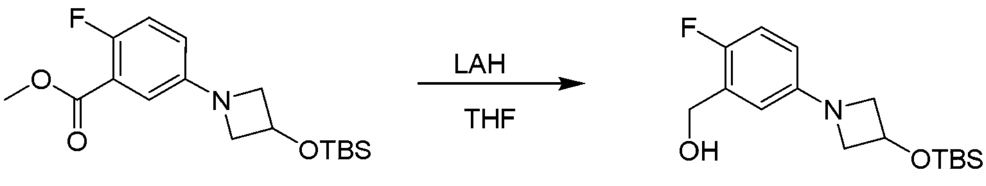


In a round-bottomed flask containing 5-(3-((tert-butyldimethylsilyl)oxy)azetidine-1-yl)-2-methyl fluorobenzoate (4.12 mmol) in THF (14.0 mL), $LiAlH_4$ (6.19 mmol) was added at 0 °C. Subsequently, the reaction mixture was kept in agitation at 20 °C for 2 hours under $N_2$ atmosphere. After total consumption of the starting reagent (monitored by LC-MS), $Na_2SO_4 \cdot 10H_2O$ (600 mg) was added at 0 °C. Next, the mixture was filtered and concentrated under reduced pressure. 800 mg (62.2% yield) of the product of interest were obtained in the form of a colorless oil, which was used for the next step without further purification.

Step II-J-iv:

*Intermediate II-12:*

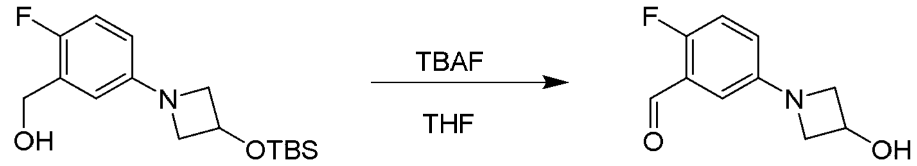


In a round-bottomed flask containing (5-(3-((tert-butyldimethylsilyl)oxy)azetidin-1-yl)-2-fluorofenyl)methanol (2.26 mmol) in THF (4.90 mL), TBAF (1.00 M, 3.39 mL) was added at 0 °C. Subsequently, the reaction mixture was kept in agitation at 20 °C for 2 hours. After total consumption of the starting reagent (monitored by CCD), the reaction mixture was diluted with $H_2O$ (10.0 mL) and extracted with AcOEt (6.00 mL x 2). Subsequently, the organic phase was concentrated under reduced pressure and purified by column chromatography (petroleum ether/AcOEt:20/10/1). 400 mg (90.5% yield) of the product of interest were obtained in the form of a yellow oil.

**METHOD II-K**

Step II-K-i:

*Precursor II-13i:*

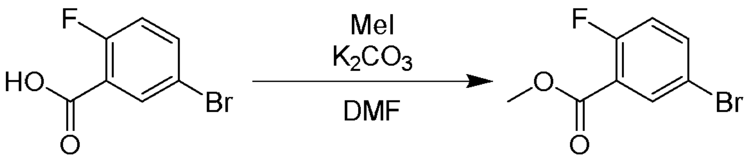


In a round-bottomed flask containing 5-bromo-2-fluorobenzoic acid (31.9 mmol) in DMF (49.0 mL), methyl iodide (47.9 mmol) and $K_2CO_3$ (47.9 mmol) were added. Subsequently, the reaction mixture was kept in agitation at 50 °C for 16 hours. After total consumption of the starting reagent (monitored by LC-MS), the reaction mixture was diluted with $H_2O$ (200 mL) and extracted with AcOEt (150 mL x 2). The organic phase was concentrated and under reduced pressure. 7.30 g (98.0% yield) of the product of interest were obtained in the form of a yellow oil, which was used for the next step without further purification.

Step II-K-ii:

*Precursor II-13ii:*

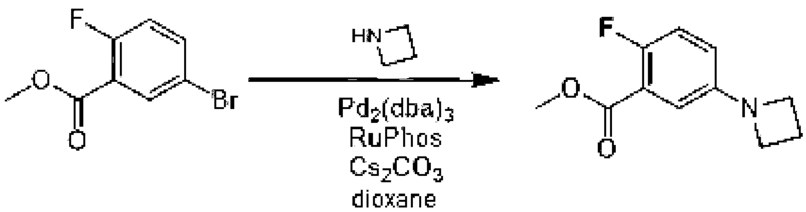


In a round-bottomed flask containing methyl 5-bromo-2-fluorobenzoate (8.58 mmol) in dioxane (20.0 mL), azetidine-3-ol hydrochloride (14.3 mmol), RuPhos (858 μmol), $Cs_2CO_3$ (25.7 mmol), and $Pd_2(dba)_3$ (858.2 μmol) was added under $N_2$ atmosphere. Subsequently, the reaction mixture was kept in agitation at 70 °C for 16 hours. After total consumption of the starting reagent (monitored by CCD), the reaction mixture was diluted with $H_2O$ (20.0 mL) and extracted with AcOEt (8.00 mL x 2). Subsequently, the organic phase was concentrated under reduced pressure and purified by column chromatography (petroleum ether/AcOEt:20/11/1). 650 mg (36.2% yield) of the product of interest were obtained in the form of a white solid.

Step II-K-iii:

*Precursor II-13iii:*

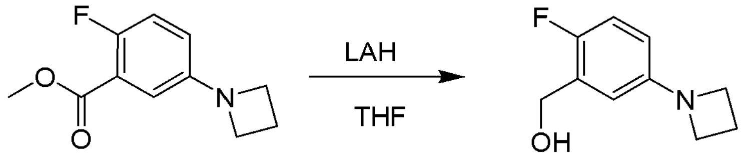


In a round-bottomed flask containing methyl 5-(azetidine-1-yl)-2-fluorobenzoate (4.45 mmol) in THF (6.51 mL), $LiAlH_4$ (6.67 mmol) at 0 °C was added. Subsequently, the reaction mixture was kept in agitation at 25 °C for 2 hours under $N_2$ atmosphere. After total consumption of the starting reagent (monitored by CCD), $Na_2SO_4 \cdot 10H_2O$ (600 mg) were added at 0 °C. Next, the mixture was filtered and concentrated under reduced pressure. 720 mg (89.3% yield) of the product of interest were obtained in the form of a colorless oil, which was used for the next step without further purification.

Step II-K-iv:

*Intermediate II-13iv:*

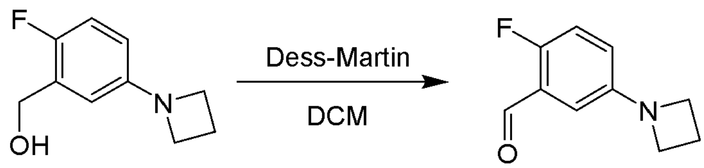


In a round-bottomed flask containing (5-(azetidine-1-yl)-2-fluorophenyl)methanol (3.42 mmol) in DCM (6.20 mL), 1,1-diacetoxy-3-oxo-1,2-benziodoxol-1-yla acetate (5.13 mmol) was added. Subsequently, the reaction mixture was kept in agitation at 20 °C for 2 hours. After total consumption of the starting reagent (monitored by CCD), a solution of $Na_2CO_3$ (10%), at room temperature, up to pH = 8, was added to the reaction mixture. Next, the mixture was filtered, extracted with DCM (2.00 mL x 2) and concentrated under reduced pressure. 450 mg (73.4% yield) of the product of interest were obtained in the form of a black oil, which was used for the next step without further purification.

**METHOD II-L**

Step II-L-i:

*Precursor II-14i:*

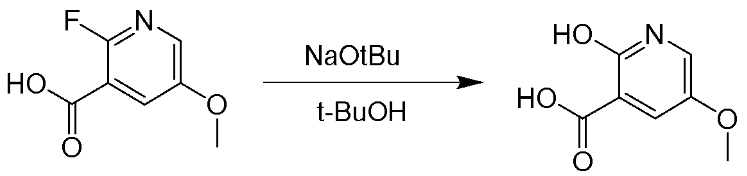


In a round-bottomed flask containing 2-fluoro-5-methoxyicotinic acid (17.5 mmol) in t-BuOH (100 mL), NaOtBu (87.7 mmol) was added. Subsequently, the reaction mixture was kept in agitation at 80 °C for 16 hours. After total consumption of the starting reagent (monitored by LC-MS), the pH of the reaction mixture was adjusted to 5.0 by adding a citric acid solution. Then, the mixture was extracted with AcOEt (50.0 mL x 3). The organic phase washed with saturated sodium chloride solution *(brine),* dried on anhydrous sodium sulfate, filtered and concentrated under reduced pressure. 2.80 g (94.4% yield) of the product of interest were obtained in the form of a brown solid.

Step II-L-ii:

*Precursor II-14ii:*

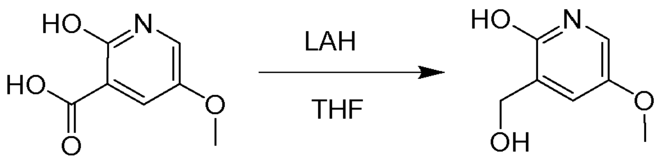


In a round-bottomed flask containing 2-hydroxy-5-methoxyicotinic acid (16.5 mmol) in THF (15.0 mL), $LiAlH_4$ (24.8 mmol) was added at 0 °C. Subsequently, the reaction mixture was kept in agitation at 20 °C for 1 hour. After full consumption of the starting reagent (monitored by LC-MS), $Na_2SO_4.10H_2O$ 2.00 g) was added to the reaction mixture. Next, the mixture was filtered and extracted with AcOEt (10.0 mL x 3). The organic phase was concentrated under reduced pressure. 1.80 g (70.0% yield) of the product of interest were obtained in the form of a yellow oil, which was used for the next step without further purification.

Step II-L-iii:

*Intermediate II-14iii:*

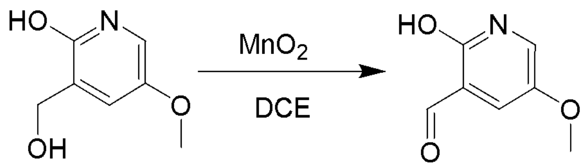


In a round-bottomed flask containing 3-(hydroxymethyl)-5-methoxypyridine-2-ol (11.6 mmol) in DCE (20.0 mL), $MnO_2$ (116 mmol) was added. Subsequently, the reaction mixture was kept in agitation at 80 °C for 16 hours. After total consumption of the starting reagent (monitored by LC-MS), the reaction mixture was filtered and purified by preparative HPLC ([$H_2O(NH_4HCO_3)$/MeCN] 41% → 71% B). 1.60 g (90.0% yield) of the product of interest were obtained in the form of a white solid.

**EXAMPLES**

The following examples, described in detail, serve to illustrate the embodiments of this invention without, however, having any limitation character to the scope of protection thereof.

The General Formula I compound(s) of this invention were obtained from the condensation of Formula III intermediates with commercial aldehydes or with the Formula II intermediates previously described, synthesized according to the various methodologies previously described and schematized in General Scheme 1, but not limited thereto.

**EXAMPLE 1. (*E*)-6-(6-ETHOXYPYRIDINE-3-IL)-*N*'-(2-FLUORO-5-METHOXYBENZYLIDENE)PYRAZINE-2-CARBOHYDRAZIDE**

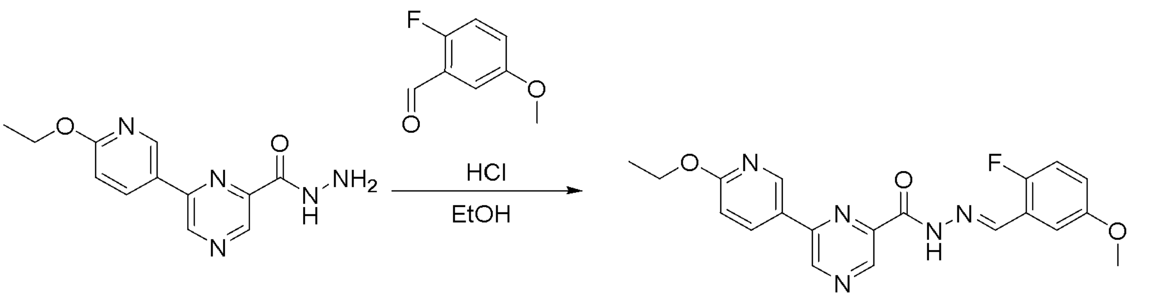

In a round-bottomed flask, 6-(6-ethoxypyridine-3-yl)pyrazine-2-carbohydrazide (2.08 mmol) was added to an aqueous solution of HCl (20.81 mmol). The reaction mixture was kept under agitation for 16 hours. Upon conclusion of the reaction, the mixture was concentrated under reduced pressure. The solid obtained was washed with hexane (20 mL x 3) and $H_2O$ (20 mL x 3), to obtain 540 mg (64.8% yield) of the product of interest 1 in the form of a white solid.

Compounds 2 to 174, as shown in table 18, are obtained by the procedure described for example 1 (compound 1), changing the corresponding intermediates II and III.

**TABLE 18. COMPOUNDS OBTAINED FROM THE CORRESPONDING REACTANTS FOLLOWING THE I-A METHOD.**

| Compound Nº | Structure | $^1$H-RMN / MS(m/z) [M+H]$^+$ |
|---|---|---|
| 1 | 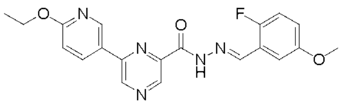 (*E*)-6-(6-ethoxypyridine-3-yl)-*N'*-(2-fluoro-5-methoxybenzylidene)pyrazine -2-carbohydrazide | $^1$H-NMR, 400 MHz, DMSO-$d_6$ $\delta$ = 12.24 (s, 1H), 9.54 (s, 1H), 9.29 (d, J = 2.5 Hz, 1H), 9.18 (s, 1H), 8.98 (s, 1H), 8.77 (dd, J = 2.4, 8.8 Hz, 1H), 7.44 (dd, J = 3.2, 5.6 Hz, 1H), 7.29 (t, J = 9.5 Hz, 1H), 7.14 - 7.07 (m, 1H), 7.03 (d, J = 8.7 Hz, 1H), 4.43 (q, J = 7.0 Hz, 2H), 3.83 (s, 3H), 1.38 (t, J = 7.0 Hz, 3H). [M+H]$^+$: 236.1. |
| 2 | 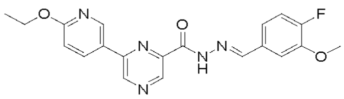 (*E*)-6-(6-ethoxypyridine-3-yl)-*N'*-(4-fluoro-3-methoxybenzylidene)pyrazine -2-carbohydrazide | $^1$H-NMR, 400 MHz, DMSO-$d_6$ $\delta$ = 12.08 (s, 1H), 9.54 (s, 1H), 9.28 (d, J = 2.3 Hz, 1H), 9.18 (s, 1H), 8.77 (dd, J = 2.5, 8.7 Hz, 1H), 8.72 (s, 1H), 7.57 (d, J = 7.7 Hz, 1H), 7.42 - 7.32 (m, 2H), 7.02 (d, J = 8.7 Hz, 1H), 4.44 (q, J = 7.1 Hz, 2H), 3.94 (s, 3H), 1.39 (t, J = 7.0 Hz, 3H). [M+H]$^+$= 396.1. |
| 3 | 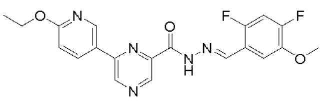 (*E*)-*N'*-(2,4-difluoro-5-methoxybenzylidene)-6-(6-ethoxypyridine-3-yl)pyrazine-2-carbohydrazide | $^1$H-NMR, 400 MHz, DMSO-$d_6$ $\delta$ = 12.22 (s, 1H), 9.54 (s, 1H), 9.29 (d, J = 2.1 Hz, 1H), 9.18 (s, 1H), 8.96 (s, 1H), 8.77 (dd, J = 2.5, 8.7 Hz, 1H), 7.60 (dd, J = 6.7, 9.5 Hz, 1H), 7.49 (t, J = 10.7 Hz, 1H), 7.03 (d, J = 8.8 Hz, 1H), 4.43 (q, J = 7.0 Hz, 2H), 3.94 (s, 3H), 1.38 (t, J = 7.0 Hz, 3H). [M+H]$^+$: 414.0. |
| 4 | 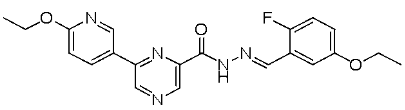 (*E*)-*N'*-(5-ethoxy-2-fluorobenzylidene)-6-(6-ethoxypyridine-3-yl) pyrazine-2-carbohydrazide | $^1$H-NMR, 400 MHz, DMSO-$d_6$ $\delta$ = 12.21 (s, 1H), 9.53 (s, 1H), 9.28 (d, J = 2.3 Hz, 1H), 9.17 (s, 1H), 8.96 (s, 1H), 8.76 (dd, J = 2.5, 8.8 Hz, 1H), 7.42 (dd, J = 3.2, 5.6 Hz, 1H), 7.27 (t, J = 9.6 Hz, 1H), 7.11 - 6.99 (m, 2H), 4.43 (q, J = 7.0 Hz, 2H), 4.08 (q, J = 7.0 Hz, 2H), 1.37 (q, J = 7.1 Hz, 6H). [M+H]$^+$: 410.2. |

(continued)

| Compound No | Structure | $^1$H-RMN / MS(m/z) $[M+H]^+$ |
|---|---|---|
| 5 | 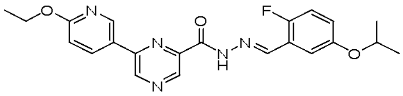 (*E*)-6-(6-ethoxypyridine-3-yl)-*N'*-(2-fluoro-5-isopropoxybenzylidene)pyraz ine-2-carbohydrazide | $^1$H-NMR, 400 MHz, DMSO-$d_6$ $\delta$ = 12.21 (s, 1H), 9.53 (s, 1H), 9.28 (d, J = 2.0 Hz, 1H), 9.17 (s, 1H), 8.95 (s, 1H), 8.76 (dd, J = 2.4, 8.8 Hz, 1H), 7.42 (dd, J = 3.4, 5.9 Hz, 1H), 7.28 - 7.22 (m, 1H), 7.08 (td, J = 3.8, 8.6 Hz, 1H), 7.01 (d, J = 8.8 Hz, 1H), 4.61 (td, J = 5.9, 12.1 Hz, 1H), 4.42 (q, J = 7.2 Hz, 2H), 1.38 (t, J = 7.1 Hz, 3H), 1.30 (d, J = 5.9 Hz, 6H). $[M+H]^+$: 424.2. |
| 6 | 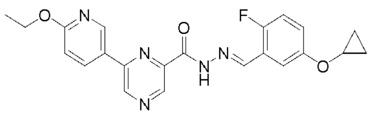 (*E*)-*N'*-(5-cyclopropoxy-2-fluorobenzylidene)-6-(6-ethoxypyridine-3-yl)pyrazine-2-carbohydrazide | $^1$H-NMR, 400 MHz, DMSO-$d_6$ $\delta$ = 12.22 (s, 1H), 9.53 (s, 1H), 9.28 (d, J = 2.0 Hz, 1H), 9.17 (s, 1H), 8.99 - 8.94 (m, 1H), 8.76 (dd, J = 2.4, 8.8 Hz, 1H), 7.61 (dd, J = 3.2, 5.6 Hz, 1H), 7.32 - 7.24 (m, 1H), 7.19 - 7.11 (m, 1H), 7.02 (d, J = 8.8 Hz, 1H), 4.42 (q, J = 6.8 Hz, 2H), 3.93 (td, J = 2.9, 5.9 Hz, 1H), 1.38 (t, J = 7.1 Hz, 3H), 0.88 - 0.66 (m, 4H). $[M+H]^+$: 422.2. |
| 7 | 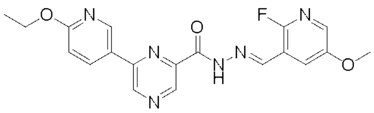 (*E*)-6-(6-ethoxypyridine-3-yl)-*N'*-((2-fluoro-5-methoxypyridine-3-yl)methylene)pyrazine-2-carbohydrazide | $^1$H-NMR, 400 MHz, DMSO-$d_6$ $\delta$ = 12.34 (s, 1H), 9.54 (s, 1H), 9.28 (d, J = 2.1 Hz, 1H), 9.18 (s, 1H), 8.89 (s, 1H), 8.76 (dd, J = 2.4, 8.8 Hz, 1H), 8.03 (br d, J = 1.5 Hz, 1H), 7.90 (dd, J = 3.1, 7.6 Hz, 1H), 7.02 (d, J = 8.8 Hz, 1H), 4.43 (q, J = 7.0 Hz, 2H), 3.92 (s, 3H), 2.82 - 2.58 (m, 4H), 1.38 (t, J = 7.1 Hz, 3H). $[M+H]^+$: 397.1. |
| 8 | 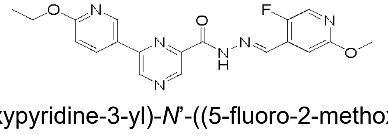 (*E*)-6-(6-ethoxypyridine-3-yl)-*N'*-((5-fluoro-2-methoxypyridine-4-yl)methylene)pyrazine-2-carbohydrazide | $^1$H-NMR, 400 MHz, DMSO-$d_6$ $\delta$ = 2.42 (s, 1H), 9.55 (s, 1H), 9.28 (d, J = 2.00 Hz, 1H), 9.19 (s, 1H), 8.92 (s, 1H), 8.76 (dd, J = 2.40, 8.80 Hz, 1H), 8.32 (d, J = 1.60 Hz, 1H), 7.21 (d, J = 4.40 Hz, 1H), 7.02 (d, J = 8.80 Hz, 1H), 4.43 (q, J = 7.20 Hz, 1H), 3.89 (s, 3H), 1.38 (t, J = 7.20 Hz, 3H). $[M+H]^+$: 397.2. |

(continued)

| Compound No | Structure | $^1$H-RMN / MS(m/z) [M+H]$^+$ |
|---|---|---|
| 9 | 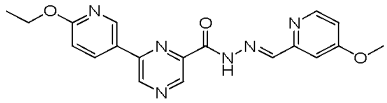<br>(*E*)-6-(6-ethoxypyridine-3-yl)-*N*'-((4-methoxypyridine-2-yl)methylene)pyrazine-2-carbohydrazide | $^1$H-NMR, 400 MHz, DMSO-$d_6$ $\delta$ = 12.27 (s, 1H), 9.54 (s, 1H), 9.29 (d, J = 2.00 Hz, 1H), 9.18 (s, 1H), 8.78-8.75 (m, 2H), 8.48 (d, J = 6.00 Hz, 1H), 7.51 (d, J = 2.40 Hz, 1H), 7.07-7.03 (m, 1H), 7.03-7.01 (m, 1H), 4.43 (q, J = 7.20 Hz, 2H), 3.93 (s, 3H), 1.38 (t, J = 6.80 Hz, 3H).<br>[M+H]$^+$: 379.2. |
| 10 | 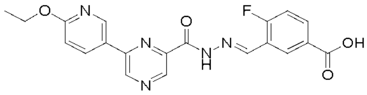<br>(*E*)-3-((2-(6-(6-ethoxypyridine-3-yl)pyrazine-2-carbonyl)hydrazinoylidene)m ethyl)-4-fluorobenzoic acid | $^1$H-NMR, 400 MHz, DMSO-$d_6$ $\delta$ = 12.30 (s, 1H), 9.54 (s, 1H), 9.29 (d, J = 2.00 Hz, 1H), 9.19 (s, 1H), 9.02 (s, 1H), 8.76 (dd, J = 2.40, 8.80 Hz, 1H), 8.60 (dd, J = 2.40, 7.00 Hz, 1H), 8.09-8.05 (m, 1H), 7.47 (dd, J = 8.80, 10.00 Hz, 1H), 7.02 (d, J = 8.40 Hz, 1H), 4.43 (q, J = 7.20 Hz, 2H), 1.38 (t, J = 7.20 Hz, 3H).<br>[M+H]$^+$: 410.5. |
| 11 | 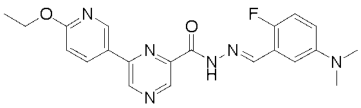<br>(*E*)-*N*'-(5-(dimethylamino)-2-fluorobenzylidene)-6-(6-ethoxypyridine-3-yl)pyrazine-2-carbohydrazide | $^1$H-NMR, 400 MHz, DMSO-$d_6$ $\delta$ = 12.17 (s, 1H), 9.54 (s, 1H), 9.29 (d, J = 2.00 Hz, 1H), 9.17 (s, 1H), 8.97 (s, 1H), 8.78 (dd, J = 2.80, 8.60 Hz, 1H), 7.21 to 7.15 (m, 2H), 7.03 (d, J = 8.80 Hz, 1H), 6.93 (dd, J = 5.20, 8.20 Hz, 1H), 4.43 (q, J = 7.20 Hz, 2H), 2.94 (s, 6H), 1.38 (t, J = 7.20 Hz, 3H).<br>[M+H]$^+$: 409.1. |
| 12 | 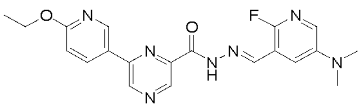<br>(*E*)-*N*'-((5-(dimethylamino)-2-fluoropyridine-3-yl)methylene)-6-(6-ethoxypyridine-3-yl)pyrazine-2-carbohydrazide | $^1$H-NMR, 400 MHz, DMSO-$d_6$ $\delta$ = 12.28 (s, 1H), 9.54 (s, 1H), 9.29 (s, 1H), 9.18 (s, 1H), 8.89 (s, 1H), 8.78-8.76 (m, 1H), 7.79 (d, J = 2.00 Hz, 1H), 7.65-7.63 (m, 1H), 7.04-7.02 (m, 1H), 4.43 (q, J = 6.80 Hz, 2H), 2.99 (s, 6H), 1.38 (t, J = 6.80 Hz, 3H).<br>[M+H]$^+$: 410.2. |

(continued)

| Compound No | Structure | $^1$H-RMN / MS(m/z) $[M+H]^+$ |
|---|---|---|
| 13 | 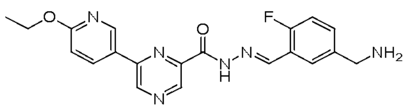 (*E*)-*N'*-(5-(aminomethyl)-2-fluorobenzylidene)-6-(6-ethoxypyridine-3-yl)pyrazine-2-carbohydrazide | $^1$H-NMR, 400 MHz, DMSO-$d_6$ $\delta$ = 9.54 (s, 1H), 9.29 (d, J = 2.00 Hz, 1H), 9.19 (s, 1H), 9.00 (s, 1H), 8.78 (d, J = 2.80 Hz, 1H), 8.32 (m, 1H), 8.06-7.56 (m, 1H), 7.56-7.53 (m, 1H), 7.33 (dd, J = 8.80, 9.00 Hz, 1H), 7.03 (dd, J = 0.40, 8.80 Hz, 1H), 4.43 (q, J = 6.80 Hz, 2H), 1.38 (t, J = 6.80 Hz, 3H). $[M+H]^+$: 395.2 |
| 14 | 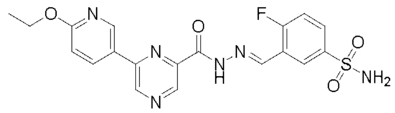 (*E*)-3-((2-(6-(6-ethoxypyridine-3-yl)pyrazine-2-carbonyl)hydrazinoylidene)m ethyl)-4-fluorobenzenesulfonamide | $^1$H-NMR, 400 MHz, DMSO-$d_6$ $\delta$ = 12.34 (s, 1H), 9.55 (s, 1H), 9.29 (d, J = 2.00 Hz, 1H), 9.20 (s, 1H), 9.04 (s, 1H), 8.77 (dd, J = 2.80, 8.60 Hz, 1H), 8.50 (dd, J = 2.40, 6.80 Hz, 1H), 7.96-7.92 (m, 1H), 7.60-7.55 (m, 3H), 7.03 (dd, J = 0.40, 8.80 Hz, 1H), 4.43 (q, J = 7.20 Hz, 2H), 1.38 (t, J = 6.80 Hz, 3H). [M-2]: 443.0. |
| 15 | 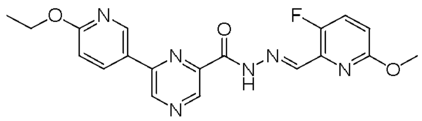 (*E*)-6-(6-ethoxypyridine-3-yl)-*N'*-((3-fluoro-6-methoxypyridine-2-yl)methylene)pyrazine-2-carbohydrazide | $^1$H-NMR, 400 MHz, DMSO-$d_6$ $\delta$ = 12.24 (s, 1H), 9.55 (s, 1H), 9.30 (d, J = 2.00 Hz, 1H), 9.18 (s, 1H), 8.92 (s, 1H), 8.79 (dd, J = 2.40, 8.80 Hz, 1H), 7.81 (t, J = 9.20 Hz, 1H), 7.03-7.01 (m, 1H), 7.00-6.97 (m, 1H), 4.43 (q, J = 6.80 Hz, 2H), 3.92 (s, 3H), 1.38 (t, J = 6.80 Hz, 3H). $[M+H]^+$: 397.1. |
| 16 | 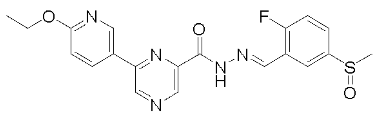 (*E*)-6-(6-ethoxypyridine-3-yl)-*N'*-(2-fluoro-5-(methylsulfinyl)benzylidene )pyrazine-2-carbohydrazide | $^1$H-NMR, 400 MHz, DMSO-$d_6$ $\delta$ = 12.32 (s, 1H), 9.55 (s, 1H), 9.29-9.29 (m, 1H), 9.19 (d, J = 3.20 Hz, 1H), 9.05 (s, 1H), 8.77 (dd, J = 2.80, 8.60 Hz, 1H), 8.33 (dd, J = 2.00, 6.60 Hz, 1H), 7.84-7.81 (m, 1H), 7.58 (dd, J = 8.80, 10.40 Hz, 1H), 7.04 (d, J = 0.40 Hz, 1H), 4.43 (q, J = 7.20 Hz, 2H), 2.81 (s, 3H), 1.38 (t, J = 6.80 Hz, 3H). [M+2]: 377.0. |

(continued)

| Compound No | Structure | $^1$H-RMN / MS(m/z) [M+H]$^+$ |
|---|---|---|
| 17 | 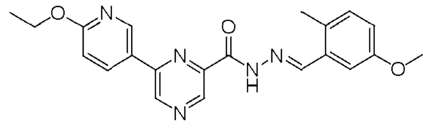<br>(*E*)-6-(6-ethoxypyridine-3-yl)-*N'*-(5-methoxy-2-methylbenzylidene)pyrazine-2-carbohydrazide | $^1$H-NMR, 400 MHz, DMSO-$d_6$ $\delta$ = 12.08 (s, 1H), 9.53 (s, 1H), 9.28 (d, J = 2.4 Hz, 1H), 9.17 (s, 1H), 8.97 (s, 1H), 8.75 (dd, J = 2.4, 8.7 Hz, 1H), 7.43 (d, J = 2.7 Hz, 1H), 7.21 (d, J = 8.3 Hz, 1H), 7.03 (d, J = 8.7 Hz, 1H), 6.96 (dd, J = 2.7, 8.5 Hz, 1H), 4.43 (q, J = 7.0 Hz, 2H), 3.79 (s, 3H), 2.43 (s, 3H), 1.38 (t, J = 7.0 Hz, 3H).<br>[M+H]$^+$: 392.1. |
| 18 | 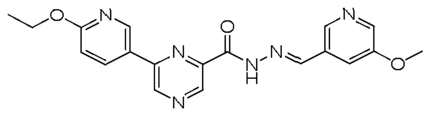<br>(*E*)-6-(6-ethoxypyridine-3-yl)-*N'*-((5-methoxypyridine-3-yl)methylene)pyrazine-2-carbohydrazide | $^1$H-NMR, 400 MHz, DMSO-$d_6$ $\delta$ = 12.23 (s, 1H), 9.54 (s, 1H), 9.29 (d, J = 2.3 Hz, 1H), 9.18 (s, 1H), 8.84 - 8.72 (m, 2H), 8.50 (d, J = 1.4 Hz, 1H), 8.39 (d, J = 2.9 Hz, 1H), 7.71 (dd, J = 1.7, 2.6 Hz, 1H), 7.02 (d, J = 8.7 Hz, 1H), 4.43 (q, J = 7.0 Hz, 2H), 3.93 (s, 3H), 1.38 (t, J = 7.0 Hz, 3H).<br>[M+H]$^+$: 379.1 |
| 19 | 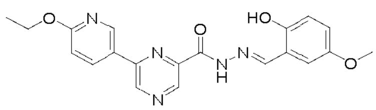<br>(*E*)-6-(6-ethoxypyridine-3-yl)-*N'*-(2-hydroxy-5-methoxybenzylidene)pyrazine -2-carbohydrazide | $^1$H-NMR, 400 MHz, DMSO-$d_6$ $\delta$ = 1.38 (t, J=7.07 Hz, 3 H) 3.75 (s, 3 H) 4.43 (q, J=7.00 Hz, 2 H) 6.81 - 7.08 (m, 3 H) 7.17 (d, J=3.00 Hz, 1 H) 8.77 (dd, J=8.76, 2.50 Hz, 1 H) 8.93 (s, 1 H) 9.17 (s, 1 H) 9.28 (d, J=2.38 Hz, 1 H) 9.53 (s, 1 H) 10.56 (s, 1 H) 12.27 (s, 1 H) .<br>[M+H]$^+$: 394.4. |
| 20 | 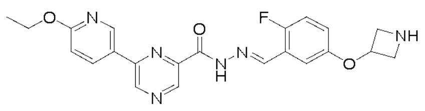<br>(*E*)-*N'*-(5-(azetidine-3-yloxy)-2-fluorobenzylidene)-6-(6-ethoxypyridine-3-yl)pyrazine-2-carbohydrazide | $^1$H-NMR, 400 MHz, DMSO-$d_6$ $\delta$ = 1.37 (t, J=7.00 Hz, 3 H) 3.13 - 3.19 (m, 1 H) 3.53 (br d, J=7.00 Hz, 2 H) 3.78 (br t, J=6.44 Hz, 2 H) 4.42 (q, J=7.00 Hz, 2 H) 4.98 - 5.07 (m, 1 H) 6.94 - 7.04 (m, 2 H) 7.23 - 7.33 (m, 2 H) 8.76 (dd, J=8.76, 2.50 Hz, 1 H) 8.94 (s, 1 H) 9.17 (s, 1 H) 9.28 (d, J=2.25 Hz, 1 H) 9.53 (s, 1 H) 12.06 - 12.41 (m, 1 H).<br>[M+H]$^+$: 437.1. |

(continued)

| Compound No | Structure | $^1$H-RMN / MS(m/z) $[M+H]^+$ |
|---|---|---|
| 21 | 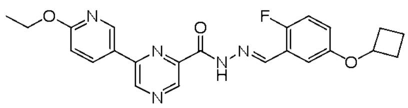 (*E*)-*N*'-(5-cyclobutoxy-2-fluorobenzylidene)-6-(6-ethoxypyridine3-yl)pyrazine-2-carbohydrazide | $^1$H-NMR, 400 MHz, DMSO-$d_6$ $\delta$ = 1.37 (t, J=7.07 Hz, 3 H) 1.61 - 1.88 (m, 2 H) 2.01 - 2.16 (m, 2 H) 2.38 - 2.44 (m, 2 H) 4.42 (q, J=7.00 Hz, 2 H) 4.66 - 4.78 (m, 1 H) 6.98 - 7.05 (m, 2 H) 7.25 (t, J=9.57 Hz, 1 H) 7.34 (dd, J=5.63, 3.13 Hz, 1 H) 8.77 (dd, J=8.76, 2.50 Hz, 1 H) 8.95 (s, 1 H) 9.17 (s, 1 H) 9.28 (d, J=2.13 Hz, 1 H) 9.54 (s, 1 H) 12.23 (s, 1 H).<br>$[M+H]^+$: 436.1. |
| 22 | 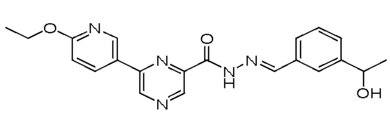 (*E*)-6-(6-ethoxypyridine-3-yl)-*N'*-(3-(1-hydroxyethyl)benzylidene)py razine-2-carbohydrazide | $^1$H-NMR, 400 MHz, DMSO-$d_6$ $\delta$ = 12.05 (1H, s), 9.53 (1H, s), 9.29 (1H, d, J = 0.9 Hz), 9.18 (1H, s), 8.79-8.73 (2H, m), 7.83 (1H, s), 7.60 (1H, s), 7.45 (2H, d, J = 4.0 Hz), 7.02 (1H, d, J = 8.8 Hz), 5.30 (1H, d, J = 4.0 Hz), 4.83-4.78 (1H, m), 4.43 (2H, q, J = 6.9 Hz), 1.39-1.35 (6H, m).<br>$[M+H]^+$: 392.6. |
| 23 | 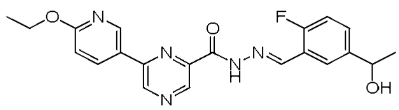 (*E*)-6-(6-ethoxypyridine-3-yl)-*N*'-(2-fluoro-5-(1-hydroxyethyl)benzylidene)py razine-2-carbohydrazide | $^1$H-NMR, 400 MHz, DMSO-$d_6$ $\delta$ = 12.20 (1H, s), 9.54 (1H, s), 9.29 (1H, d, J = 2.1 Hz), 9.18 (1H, s), 9.00 (1H, s), 8.77 (1H, dd, J = 2.5, 8.8 Hz), 8.01 (1H, dd, J = 2.0, 7.0 Hz), 7.49-7.44 (1H, m), 7.27 (1H, dd, J = 8.6, 10.4 Hz), 7.02 (1H, d, J = 8.8 Hz), 5.36 (1H, d, J = 4.1 Hz), 4.83 to 4.77 (1H, m), 4.43 (2H, q, J = 7.1 Hz), 1.40 to 1.33 (6H, m).<br>$[M+H]^+$: 410.3. |
| 24 | 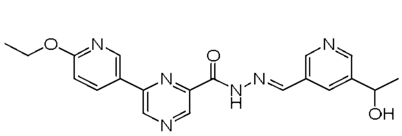 (*E*)-6-(6-ethoxypyridine-3-yl)-*N*'-((5-(1-hydroxyethyl)pyridine-3-yl)methylene)pyrazine-2-carbohydrazide | $^1$H-NMR, 400 MHz, DMSO-$d_6$ $\delta$ = 12.21 (1H, s), 9.54 (1H, s), 9.28 (1H, d, J = 2.1 Hz), 9.18 (1H, s), 8.80 (1H, s), 8.76 (1H, dd, J = 2.5, 8.7 Hz), 8.73 (1H, d, J = 2.0 Hz), 8.62 (1H, d, J = 2.0 Hz), 8.20 (1H, t, J = 2.0 Hz), 7.02 (1H, d, J = 8.8 Hz), 5.48 (1H, d, J = 4.5 Hz), 4.92-4.85 (1H, m), 4.43 (2H, q, J = 7.0 Hz), 1.42-1.35 (6H, m).<br>$[M+H]^+$: 393.6. |

(continued)

| Compound No | Structure | $^1$H-RMN / MS(m/z) [M+H]$^+$ |
|---|---|---|
| 25 | 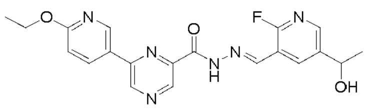 (*E*)-6-(6-ethoxypyridine-3-yl)-*N*'-((2-fluoro-5-(1-hydroxyethyl)pyridine-3-yl)methylene)pyrazine-2-carbohydrazide | $^1$H-NMR, 400 MHz, DMSO-$d_6$ $\delta$ = 12.33 (1H, s), 9.55 (1H, s), 9.29 (1H, d, J = 2.0 Hz), 9.19 (1H, s), 8.93 (1H, s), 8.77 (1H, dd, J = 2.6, 8.7 Hz), 8.46 (1H, dd, J = 2.3, 9.3 Hz), 8.28 (1H, d, J = 1.9 Hz), 7.03 (1H, d, J = 8.6 Hz), 5.56 (1H, d, J = 4.4 Hz), 4.95-4.87 (1H, m), 4.43 (2H, q, J = 7.0 Hz), 1.43 to 1.36 (6H, m). [M+H]$^+$: 411.6. |
| 26 | 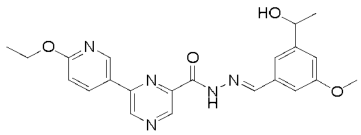 (*E*)-6-(6-ethoxypyridine-3-yl)-*N*'-(3-(1-hydroxyethyl)-5-methoxybenzylidene)pyrazine -2-carbohydrazide | $^1$H-NMR, 400 MHz, DMSO-$d_6$ $\delta$ = 1.44 - 1.55 (m, 6 H) 3.89 (s, 3 H) 4.48 (q, J = 7.2 Hz, 2 H) 4.82 - 5.08 (m, 1 H) 6.92 (d, J =8.8 Hz, 1 H) 7.05 (s, 1 H) 7.31 (s, 1 H) 7.37 - 7.41 (m, 1 H) 8.20 - 8.29 (m, 1 H) 8.40 (s, 1 H) 8.88 (d, J = 2.4 Hz, 1 H) 9.13 - 9.26 (m, 1 H) 9.42 (s, 1 H). [M+H]$^+$: 268.3. |
| 27 | 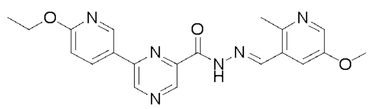 (*E*)-6-(6-ethoxypyridine-3-yl)-*N*'-((5-methoxy-2-methylpyridine-3-yl)methylene)pyrazine-2-carbohydrazide | $^1$H-NMR, 400 MHz, DMSO-$d_6$ $\delta$ = 12.20 (s, 1H), 9.54 (s, 1H), 9.28 (d, J = 2.0 Hz, 1H), 9.18 (s, 1H), 8.98 (s, 1H), 8.74 (dd, J = 2.6, 8.7 Hz, 1H), 8.26 (d, J = 3.0 Hz, 1H), 7.72 (d, J = 3.0 Hz, 1H), 7.03 (d, J = 8.8 Hz, 1H), 4.42 (q, J = 7.0 Hz, 2H), 3.88 (s, 3H), 2.61 (s, 3H), 1.37 (t, J = 7.1 Hz, 3H). [M+H]$^+$: 393.4. |
| 28 | 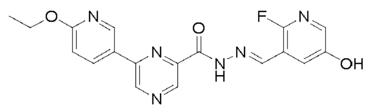 (*E*)-6-(6-ethoxypyridine-3-yl)-*N'*-(((2-fluoro-5-hydroxypyridine-3-yl)methylene)pyrazine-2-carbohydrazide | $^1$H-NMR, 400 MHz, DMSO-$d_6$ $\delta$ = 12.31 (br s, 1H) , 10.27 (br s, 1H) , 9.54 (s, 1H) , 9.28 (d, J = 2.3 Hz, 1H) , 9.18 (s, 1H) , 8.84 (s, 1H) , 8.76 (dd, J = 2.5, 8.6 Hz, 1H) , 7. 91 - 7.76 (m, 2H) , 7.02 (d, J = 8.8 Hz, 1H) , 4.43 (q, J = 7.0 Hz, 2H) , 1.38 (t, J = 7.1 Hz, 3H). [M+H]$^+$: 383.4. |

(continued)

| Compound Nº | Structure | $^1$H-RMN / MS(m/z) [M+H]$^+$ |
|---|---|---|
| 29 | 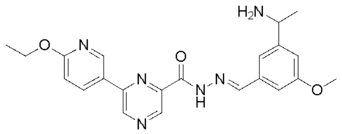 (*E*)-*N*'-(3-(1-aminoethyl)-5-methoxybenzylidene)-6-(6-ethoxy-pyridine-3-yl)pyrazine-2-carbohydrazide | $^1$H-NMR, 400 MHz, DMSO-$d_6$ $\delta$ = 1.38 (t, J = 7.2 Hz, 3 H) 1.53 (d, J = 6.8 Hz, 3 H) 3.86 (s, 3 H) 4.43 (q, J = 7.2 Hz, 2 H) 4.47 - 4.54 (m, 1 H) 7.02 (d, J = 8.8 Hz, 1 H) 7.20 (s, 1 H) 7.29 (s, 1 H) 7.53 (s, 1 H) 8.16 - 8.39 (m, 3 H) 8.73 (s, 1 H) 8.74 - 8.79 (m, 1 H) 9.18 (s, 1 H) 9.28 (d, J =2.4 Hz, 1 H) 9.55 (s, 1 H) 12.13 (s, 1 H) [M+H]$^+$: 409.7. |
| 30 | 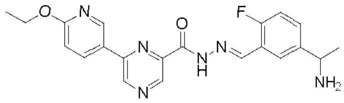 (*E*)-*N*'-(5-(1-aminoethyl)-2-fluorobenzylidene)-6-(6-ethoxypyridine-3-yl)pyrazine-2-carbohydrazide | |
| 31 | 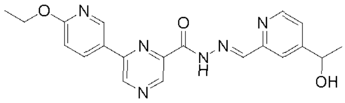 (*E*)-6-(6-ethoxypyridine-3-yl)-*N*'-((4-(1-hydroxyethyl)pyridine-2-yl)methylene)pyrazine-2-carbohydrazide | $^1$H-NMR, 400 MHz, DMSO-$d_6$ $\delta$ = 12.18 - 12.36 (m, 1H), 9.53 (s, 1H), 9.29 (d, J = 2.4 Hz, 1H), 9.18 (s, 1H), 8.82 - 8.68 (m, 2H), 8.57 (d, J = 5.1 Hz, 1H), 8.07 (s, 1H), 7.40 (dd, J = 1.3, 5.1 Hz, 1H), 7.01 (d, J = 8.8 Hz, 1H), 5.55 (d, J = 4.4 Hz, 1H), 4.88 - 4.76 (m, 1H), 4.42 (q, J = 7.1 Hz, 2H), 1.34 - 1.40 (m, 6H). [M+H]$^+$: 393.4. |
| 32 | 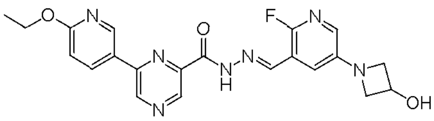 (*E*)-6-(6-ethoxypyridine-3-yl)-N'-((2-fluoro-5-(3-hydroxyazetidine-1-yl)pyridine-3-yl)methylene)pyrazine-2-carbohydrazide | |
| 33 | 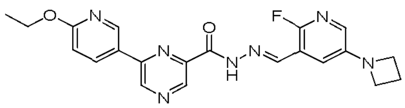 (*E*)-N'-((5-(azetidine-1-yl)-2-fluoropyridine-3-yl)methylene)-6-(6-ethoxypyridine-3-yl)pyrazine-2-carbohydrazide | |
| 34 | 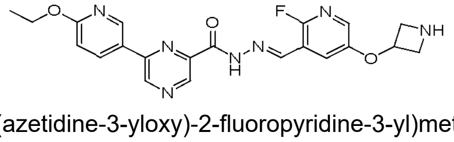 (*E*)-*N*'-((5-(azetidine-3-yloxy)-2-fluoropyridine-3-yl)methylene)-6-(6-ethoxypyridine-3-yl)pyrazine-2-carbohydrazide | $^1$H-NMR, 400 MHz, DMSO-$d_6$ $\delta$ = 12.37 (s, 1H), 9.55 (s, 1H), 9.28 (d, J = 2.4 Hz, 1H), 9.18 (s, 2H), 8.90 - 8.86 (m, 1H), 8.82 - 8.70 (m, 2H), 8.00 (dd, J = 1.6, 2.9 Hz, 1H), 7.87 (dd, J = 3.0, 7.6 Hz, 1H), 7.03 (d, J = 8.8 Hz, 1H), 5.32 - 5.25 (m, 1H), 4.51 - 4.39 (m, 4H), 4.14 - 4.05 (m, 2H), 1.38 (t, J = 7.0 Hz, 3H). [M+H]$^+$: 438.4. |

(continued)

| Compound No | Structure | $^1$H-RMN / MS(m/z) [M+H]$^+$ |
|---|---|---|
| 35 | 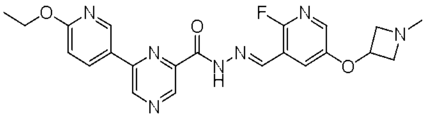 (*E*)-6-(6-ethoxypyridine-3-yl)-*N*'-((2-fluoro-5-((1-methylazetidine-3-yl)oxy)pyridine-3-yl)methylene) pyrazine-2-carbohydrazide | |
| 36 | 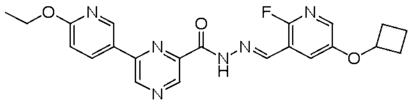 (*E*)-*N*'-((5-cyclobutoxy-2-fluoropyridine-3-yl)methylene)-6-(6-ethoxypyridine-3-yl)pyrazine-2-carbohydrazide | $^1$H-NMR, 400 MHz, DMSO-d$_6$ $\delta$ = 12.34 (s, 1H), 9.54 (s, 1H), 9.28 (d, J = 2.4 Hz, 1H), 9.18 (s, 1H), 8.87 (s, 1H), 8.76 (dd, J = 2.5, 8.6 Hz, 1H), 7.94 (dd, J = 1.6, 2.9 Hz, 1H), 7.80 (dd, J = 3.1, 7.7 Hz, 1H), 7.02 (d, J = 8.8 Hz, 1H), 4.85 (quin, J = 7.1 Hz, 1H), 4.42 (q, J = 7.1 Hz, 2H), 2.46 - 2.42 (m, 2H), 2.17 - 2.03 (m, 2H), 1.89 - 1.64 (m, 2H), 1.37 (t, J = 7.1 Hz, 3H). [M+H]$^+$: 437.4 |
| 37 | 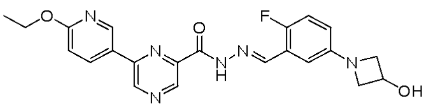 (*E*)-6-(6-ethoxypyridine-3-yl)-*N*'-(2-fluoro-5-(3-hydroxyzaetidine-1-yl)benzylidene)pyrazine-2-carbohydrazide | $^1$H-NMR, 400 MHz, DMSO-d$_6$ $\delta$ = 12.16 (s, 1H), 9.53 (s, 1H), 9.28 (d, J = 2.5 Hz, 1H), 9.16 (s, 1H), 8.94 (s, 1H), 8.76 (dd, J = 2.5, 8.8 Hz, 1H), 7.15 (dd, J = 9.0, 10.1 Hz, 1H), 7.01 (d, J = 8.8 Hz, 1H), 6.90 (dd, J = 2.9, 5.8 Hz, 1H), 6.59 (td, J = 3.8, 8.7 Hz, 1H), 5.64 (d, J = 6.6 Hz, 1H), 4.61 - 4.53 (m, 1H), 4.42 (q, J = 7.0 Hz, 2H), 4.12 (t, J = 7.0 Hz, 2H), 3.53 (dd, J = 5.1, 7.5 Hz, 2H), 1.37 (t, J = 7.1 Hz, 3H). [M+H]$^+$: 437.4 |
| 38 | 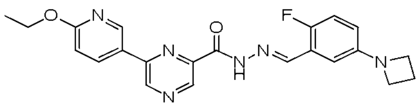 (*E*)-*N*'-(5-(azetidine-1-yl)-2-fluorobenzylidene)-6-(6-ethoxypyridine-3-yl)pyrazine-2-carbohydrazide | $^1$H-NMR, 400 MHz, DMSO-d$_6$ $\delta$ = 12.16 (s, 1H), 9.53 (s, 1H), 9.28 (d, J = 2.4 Hz, 1H), 9.16 (s, 1H), 8.94 (s, 1H), 8.76 (dd, J = 2.5, 8.8 Hz, 1H), 7.15 (dd, J = 9.0, 10.3 Hz, 1H), 7.01 (d, J = 8.8 Hz, 1H), 6.88 (dd, J = 2.9, 5.8 Hz, 1H), 6.59 - 6.53 (m, 1H), 4.42 (q, J = 7.0 Hz, 2H), 3.84 (t, J = 7.2 Hz, 4H), 2.36 - 2.28 (m, 2H), 1.37 (t, J = 7.1 Hz, 3H). [M+H]$^+$: 421.1. |

(continued)

| Compound No | Structure | $^1$H-RMN / MS(m/z) [M+H]$^+$ |
|---|---|---|
| 39 | 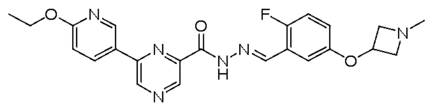 (*E*)-6-(6-ethoxypyridine-3-yl)-*N'*-(2-fluoro-5-((1-methylazetidine-3-yl)oxy)benzylidene)pyrazine -2-carbohydrazide | $^1$H-NMR, 400 MHz, methanol-$d_4$ $\delta$ = 9.36 (s, 1H), 9.26 (s, 1H), 9.07 (d, J = 2.4 Hz, 1H), 8.84 (s, 1H), 8.64 (dd, J = 2.5, 8.8 Hz, 1H), 7.64 (br s, 1H), 7.25 - 7.16 (m, 1H), 7.13 - 7.05 (m, 1H), 6.96 (d, J = 8.8 Hz, 1H), 5.31 - 5.11 (m, 1H), 5.02 - 4.89 (m, 2H), 4.60 - 4.39 (m, 4H), 4.17 (br d, J = 7.0 Hz, 1H), 3.07 (br d, J = 11.9 Hz, 3H), 1.43 (t, J = 7.0 Hz, 3H). [M+H]$^+$: 451.4. |
| 40 | 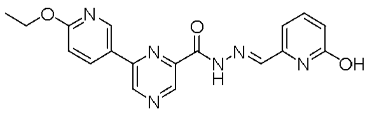 (*E*)-6-(6-ethoxypyridine-3-yl)-*N'*-((6-hydroxypyridine-2-yl)methylene)pyrazine-2-carbohydrazide | $^1$H-NMR, 400 MHz, DMSO-$d_6$ $\delta$ = 1.38 (t, J=7.07 Hz, 3 H) 4.43 (q, J=7.00 Hz, 2 H) 6.44 - 7.15 (m, 3 H) 7.54 - 7.62 (m, 1 H) 8.46 - 8.53 (m, 1 H) 8.68 - 8.78 (m, 1 H) 9.14 - 9.21 (m, 1 H) 9.27 (d, J=2.13 Hz, 1 H) 9.54 (s, 1 H) 10.93 - 11.29 (m, 1 H) 12.24 - 12.53 (m, 1 H). [M+H]$^+$: 387.4. |
| 41 | 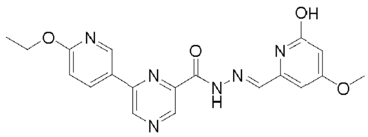 (*E*)-6-(6-ethoxypyridine-3-yl)-*N'*-((6-hydroxy-4-methoxypyridine-2-yl)methylene)pyrazine-2-carbohydrazide | |
| 42 | 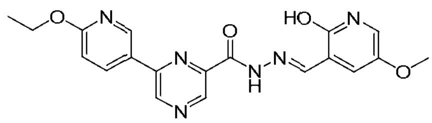 (*E*)-6-(6-ethoxypyridine-3-yl)-*N'*-((2-hydroxy-5-methoxypyridine-3-yl)methylene)pyrazine-2-carbohydrazide | $^1$H-NMR, 400 MHz, DMSO-$d_6$ $\delta$ = 12,19(s,1H) , 11,98 - 11.63 (m, 1H), 9.52 (s, 1H), 9.27 (d, *J* = 2.0 Hz, 1H), 9.15 (s, 1H), 8.88 (s, 1H), 8.80 (dd, J = 2.5, 8.8 Hz, 1H), 7.81 (d, J = 3.4 Hz, 1H), 7.29 (br s, 1H), 7.09 - 6.96 (m, 1H), 4.42 (q, J = 7.1 Hz, 2H), 3.73 (s, 3H), 1.38 (t, J = 7.1 Hz, 3H). [M+H]$^+$: 395.3. |
| 43 | 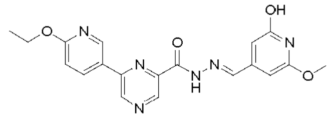 (*E*)-6-(6-ethoxypyridine-3-yl)-*N'*-( (2-hydroxy-6-methoxypyridine-4-yl)methylene)pyrazine-2-carbohydrazide | $^1$H-NMR, 400 MHz, DMSO-$d_6$ $\delta$ = 12.22 (s, 1H) , 11.01 - 10.68 (m, 1H) , 9.54 (s, 1H) , 9.28 (d, J = 2.5 Hz, 1H) , 9.18 (s, 1H) , 8.76 (dd, J = 2.5, 8.8 Hz, 1H) , 8.60 (s, 1H) , 7.02 (d, J = 8.6 Hz, 1H) , 6.67 - 6.35 (m, 2H) , 4.43 (q, J = 7.0 Hz, 2H) , 3.85 (s, 3H) , 1.38 (t, J = 7.0 Hz, 3H). [M+H]$^+$: 395.0. |

(continued)

| Compound No | Structure | $^1$H-RMN / MS(m/z) [M+H]$^+$ |
|---|---|---|
| 44 | 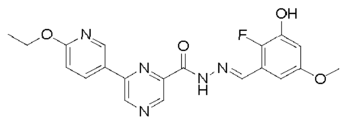 (*E*)-6-(6-ethoxypyridine-3-yl)-*N'*-(2-fluoro-3-hydroxy-5-methoxy-benzylidene)pyrazine -2-carbohydrazide | $^1$H-NMR, 400 MHz, DMSO-$d_6$ $\delta$ = 1.37 (t, J=7.07 Hz, 3 H) 3.76 (s, 3 H) 4.42 (d, J=7.00 Hz, 2 H) 6.61 (dd, J=7.38, 3.13 Hz, 1 H) 6.86 (dd, J=4.31, 3.19 Hz, 1 H) 7.01 (d, J=8.75 Hz, 1 H) 8.76 (dd, J=8.76, 2.50 Hz, 1 H) 8.96 (s, 1 H) 9.16 (s, 1 H) 9.28 (d, J=2.00 Hz, 1 H) 9.53 (s, 1 H) 10.07 - 10.35 (m, 1 H) 12.18 (s, 1 H). [M+H]$^+$: 412.3. |
| 45 | 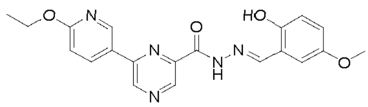 (*E*)-6-(6-ethoxypyridine-3-yl)-*N'*-(2-hydroxy-5-methoxybenzyli-dene)pyrazine -2-carbohydrazide | $^1$H-NMR, 400 MHz, DMSO-$d_6$ $\delta$ = 1.38 (t, J=7.07 Hz, 3 H) 3.75 (s, 3 H) 4.43 (q, J=7.00 Hz, 2 H) 6.81 - 7.08 (m, 3 H) 7.17 (d, J=3.00 Hz, 1 H) 8.77 (dd, J=8.76, 2.50 Hz, 1 H) 8.93 (s, 1 H) 9.17 (s, 1 H) 9.28 (d, J=2.38 Hz, 1 H) 9.53 (s, 1 H) 10.56 (s, 1 H) 12.27 (s, 1 H). [M+H]$^+$: 394.4. |
| 46 | 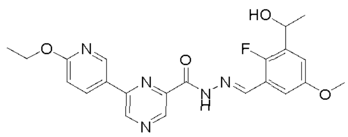 (*E*)-6-(6-ethoxypyridine-3-yl)-*N'*-(2-fluoro-3-(1-hydroxyethyl)-5-methoxybenzylidene)pyrazine-2-car-bohydrazide | |
| 47 | 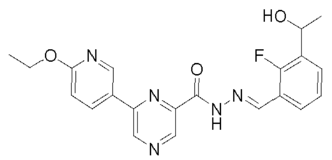 (*E*)-6-(6-ethoxypyridine-3-yl)-*N'*-(2-fluoro-3-(1-hydroxyethyl)benzylidene)pyrazine-2-carbohydrazide | |
| 48 | 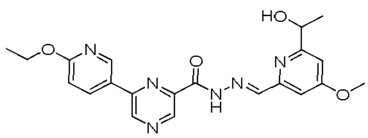 (*E*)-6-(6-ethoxypyridine-3-yl)-*N'*-((6-(1-hydroxyethyl)-4-methoxypyridine-2-yl)methylene)pyrazine-2-carbohydrazide | |
| 49 | 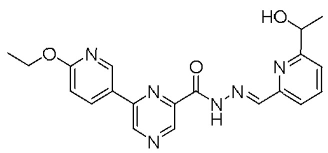 (*E*)-6-(6-ethoxypyridine-3-yl)-*N'*-((6-(1-hydroxyethyl)pyridine-2-yl)methylene)pyrazine-2-carbohy-drazide | |

(continued)

| Compound No | Structure | $^1$H-RMN / MS(m/z) $[M+H]^+$ |
|---|---|---|
| 50 | 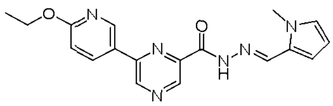 (*E*)-6-(6-ethoxypyridine-3-yl)-*N'*-((1-methyl-1*H*-pyrrole-2-yl) methylene)pyrazine-2-carbohydrazide | $^1$H-NMR, 400 MHz, DMSO-$d_6$ $\delta$ = 11.76 (s, 1H), 9.50 (s, 1H), 9.26 (d, J = 2.5 Hz, 1H), 9.13 (s, 1H), 8.74 (dd, J = 2.5, 8.6 Hz, 1H), 8.64 (s, 1H), 7.06 - 6.94 (m, 2H), 6.57 (dd, J = 1.7, 3.8 Hz, 1H), 6.14 (dd, J = 2.6, 3.6 Hz, 1H), 4.42 (q, J = 7.1 Hz, 2H), 3.91 (s, 3H), 1.37 (t, J = 7.1 Hz, 3H) $[M+H]^+$: 351.1. |
| 51 | 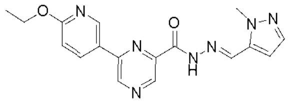 (*E*)-6-(6-ethoxypyridine-3-yl)-*N'*-((1-methyl-1*H*-pyrazole-5-yl) methylene)pyrazine-2-carbohydrazide | $^1$H-NMR, 400 MHz, DMSO-$d_6$ $\delta$ = 12.11 (s, 1H), 9.53 (s, 1H), 9.28 (d, J = 2.3 Hz, 1H), 9.17 (s, 1H), 8.83 (s, 1H), 8.74 (dd, J = 2.5, 8.7 Hz, 1H), 7.52 (d, J = 2.0 Hz, 1H), 7.02 (d, J = 8.7 Hz, 1H), 6.72 (d, J = 1.9 Hz, 1H), 4.42 (q, J = 7.1 Hz, 2H), 4.09 (s, 3H), 1.37 (t, J = 7.0 Hz, 3H). $[M+H]^+$: 352.1. |
| 52 | 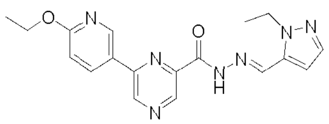 (*E*)-6-(6-etnoxypyriaine-3-yl)-*N'*-((1-ethyl-1*H*-pyrazole-5-yl) methylene)pyrazine-2-carbohydrazide | $^1$H-NMR, 400 MHz, DMSO-$d_6$ $\delta$ = 12.10 (s, 1H), 9.54 (s, 1H), 9.28 (d, J = 2.2 Hz, 1H), 9.17 (s, 1H), 8.83 (s, 1H), 8.74 (dd, J = 2.5, 8.7 Hz, 1H), 7.54 (d, J = 2.0 Hz, 1H), 7.02 (d, J = 8.8 Hz, 1H), 6.71 (d, J = 2.0 Hz, 1H), 4.55 - 4.37 (m, 4H), 1.38 (dt, J = 4.5, 7.1 Hz, 6H). $[M+H]^+$: 366.0. |
| 53 | 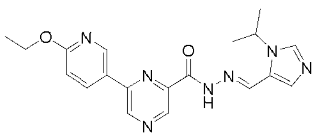 (*E*)-6-(6-ethoxypyridine-3-yl)-*N'*-((1-isopropyl-1*H*-imidazole-5-yl) methylene)pyrazine-2-carbohydrazide | $^1$H-NMR, 400 MHz, DMSO-$d_6$ $\delta$ = 11.92 (s, 1H), 9.57 - 9.43 (m, 1H), 9.26 (d, J = 2.3 Hz, 1H), 9.14 (s, 1H), 8.83 - 8.65 (m, 2H), 8.06 (s, 1H), 7.42 (s, 1H), 7.01 (d, J = 8.7 Hz, 1H), 5.20 (spt, J = 6.7 Hz, 1H), 4.52 - 4.32 (m, 2H), 1.50 (d, J = 6.7 Hz, 6H), 1.42 - 1.29 (m, 3H). $[M+H]^+$: 380.0. |
| 54 | 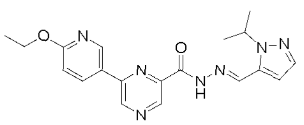 (*E*)-6-(6-ethoxypyridine-3-yl)-*N'*-((1-isopropyl-1*H*-pyrazole-5-yl) methylene)pyrazine-2-carbohydrazide | $^1$H-NMR, 400 MHz, DMSO-$d_6$ $\delta$ = 12.06 (s, 1H), 9.53 (s, 1H), 9.27 (d, J = 2.2 Hz, 1H), 9.17 (s, 1H), 8.87 (s, 1H), 8.74 (dd, J = 2.6, 8.8 Hz, 1H), 7.56 (d, J = 1.8 Hz, 1H), 7.02 (d, J = 8.8 Hz, 1H), 6.69 (d, J = 1.8 Hz, 1H), 5.19 (spt, J = 6.5 Hz, 1H), 4.43 (q, J = 7.1 Hz, 2H), 1.46 (d, J = 6.5 Hz, 6H), 1.38 (t, J = 7.0 Hz, 3H). $[M+H]^+$: 380.1. |

(continued)

| Compound No | Structure | $^1$H-RMN / MS(m/z) [M+H]$^+$ |
|---|---|---|
| 55 | 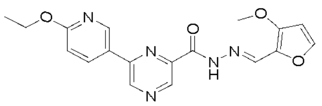 (*E*)-6-(6-ethoxypyridine-3-yl)-*N*'-((3-methoxyfuran-2-yl)methylene)pyrazine-2-carbohydrazide | $^1$H-NMR, 400 MHz, DMSO-$d_6$ $\delta$ = 12.38 (s, 1H), 9.56 (s, 1H), 9.20 (s, 1H), 9.12 (d, J = 2.3 Hz, 1H), 8.55 (dd, J = 2.5, 8.7 Hz, 1H), 7.92 (d, J = 2.1 Hz, 1H), 7.42 (s, 1H), 7.16 (d, J = 8.7 Hz, 1H), 6.98 (d, J = 2.2 Hz, 1H), 4.44 (q, J = 7.0 Hz, 2H), 3.90 (s, 3H), 1.38 (t, J = 7.0 Hz, 3H) [M+H]$^+$: 368.0. |
| 56 | 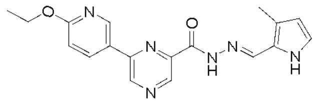 (*E*)-6-(6-ethoxypyridine-3-yl)-*N*'-((3-methyl-1H-pyrrole-2-yl) methylene)pyrazine-2-carbohydrazide | $^1$H-NMR, 400 MHz, DMSO-$d_6$ $\delta$ = 11.71 (s, 1H), 11.28 (br s, 1H), 9.50 (s, 1H), 9.28 (d, J = 2.3 Hz, 1H), 9.15 (s, 1H), 8.75 (dd, J = 2.5, 8.7 Hz, 1H), 8.62 (s, 1H), 7.02 (d, J = 8.7 Hz, 1H), 6.84 (t, J = 2.7 Hz, 1H), 6.00 (t, J = 2.2 Hz, 1H), 4.42 (q, J = 7.0 Hz, 2H), 2.21 (s, 3H), 1.37 (t, J = 7.0 Hz, 3H). [M+H]$^+$: 351.1. |
| 57 | 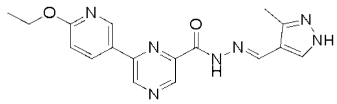 (*E*)-6-(6-ethoxypyridine-3-yl)-*N'*-((3-methyl-1H-pyrazole-4-yl) methylene)pyrazine-2-carbohydrazide | $^1$H-NMR, 400 MHz, DMSO-$d_6$ $\delta$ = 13.05 - 12.81 (m, 1H), 11.74 (br s, 1H), 9.50 (s, 1H), 9.27 (d, J = 2.2 Hz, 1H), 9.14 (s, 1H), 8.75 (dd, J = 2.5, 8.7 Hz, 1H), 8.65 (s, 1H), 8.22 - 7.64 (m, 1H), 7.01 (d, J = 8.8 Hz, 1H), 4.42 (q, J = 7.1 Hz, 2H), 2.43 (br s, 3H), 1.37 (t, J = 7.0 Hz, 3H). [M+H]$^+$: 352.0. |
| 58 | 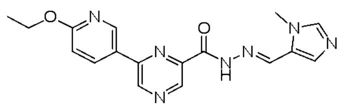 (*E*)-6-(6-ethoxypyridine-3-yl)-*N*'-((1-methyl-1H-imidazole-5-yl) methylene)pyrazine-2-carbohydrazide | $^1$H-NMR, 400 MHz, DMSO-$d_6$ $\delta$ = 11.94 (s, 1H), 9.52 (s, 1H), 9.27 (d, J = 2.4 Hz, 1H), 9.14 (s, 1H), 8.78 - 8.69 (m, 2H), 7.84 (s, 1H), 7.41 (s, 1H), 7.01 (d, J = 8.8 Hz, 1H), 4.42 (q, J = 7.0 Hz, 2H), 3.92 (s, 3H), 1.37 (t, J = 7.0 Hz, 3H). [M+H]$^+$: 352.0. |
| 59 | 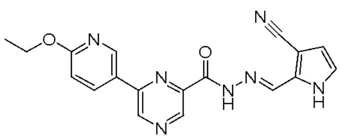 (*E*)-*N'*-((3-cyano-1*H*-pyrrole-2-yl)methylene)-6-(6-ethoxypyridine-3-yl)pyrazine-2-carbohydrazide | $^1$H-NMR, 400 MHz, DMSO-$d_6$ $\delta$ = 12.69 - 12.44 (m, 1H), 12.17 (s, 1H), 9.53 (s, 1H), 9.28 (d, J = 2.0 Hz, 1H), 9.17 (s, 1H), 8.90 - 8.63 (m, 2H), 7.17 - 6.97 (m, 2H), 6.65 (d, J = 2.7 Hz, 1H), 4.43 (q, J = 7.0 Hz, 2H), 1.38 (t, J = 7.0 Hz, 3H) [M+H]$^+$: 362,0. |

(continued)

| Compound No | Structure | $^1$H-RMN / MS(m/z) [M+H]$^+$ |
|---|---|---|
| 60 | 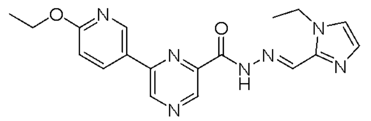 (*E*)-6-(6-ethoxypyridine-3-yl)-*N'*-((1-ethyl-1*H*-imidazole-2-yl) methylene)pyrazine-2-carbohydrazide | $^1$H-NMR, 400 MHz, DMSO-d$_6$ $\delta$ = 12.10 (s, 1H), 9.53 (s, 1H), 9.28 (d, J = 2.5 Hz, 1H), 9.16 (s, 1H), 8.82 - 8.66 (m, 2H), 7.46 (s, 1H), 7.11 (d, J = 0.8 Hz, 1H), 7.01 (d, J = 8.7 Hz, 1H), 4.56 - 4.34 (m, 4H), 1.38 (dt, J = 3.6, 7.1 Hz, 6H). [M+H]$^+$: 366.1. |
| 61 | 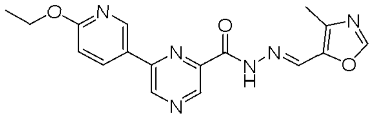 (E)-6-(6-ethoxypyridine-3-yl)-*N'*-((4-methyloxazole-5-yl)methylene)pyrazine-2-carbohydrazide | $^1$H-NMR, 400 MHz, DMSO-d$_6$ $\delta$ = 12.06 (s, 1H), 9.53 (s, 1H), 9.28 (d, J = 2.2 Hz, 1H), 9.17 (s, 1H), 8.85 - 8.67 (m, 2H), 8.48 (s, 1H), 7.02 (d, J = 8.7 Hz, 1H), 4.42 (q, J = 7.0 Hz, 2H), 2.34 (s, 3H), 1.37 (t, J = 7.1 Hz, 3H). [M+H]$^+$: 353.0. |
| 62 | 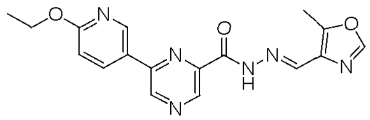 (*E*)-6-(6-ethoxypyridine-3-yl)-*N'*-((5-methyloxazole-4-yl)methylene)pyrazine-2-carbohydrazide | $^1$H-NMR, 400 MHz, DMSO-d$_6$ $\delta$ = 11.97 (s, 1H), 9.52 (s, 1H), 9.27 (d, J = 2.0 Hz, 1H), 9.16 (s, 1H), 8.83 - 8.65 (m, 2H), 8.36 (s, 1H), 7.01 (d, J = 8.7 Hz, 1H), 4.42 (q, J = 7.1 Hz, 2H), 2.58 (s, 3H), 1.37 (t, J = 7.0 Hz, 3H). [M+H]$^+$: 353.0. |
| 63 | 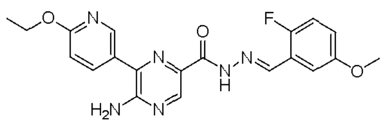 (*E*)-5-amino-6-(6-ethoxypyridine-3-yl)-*N'*-(2-fluoro-5-methoxybenzylidene)pyrazine -2-carbohydrazide | $^1$H-NMR, 400 MHz, DMSO-d$_6$ $\delta$ = 11.71 (s, 1H), 8.81 (s, 1H), 8.65 (d, J = 2.0 Hz, 1H), 8.59 (s, 1H), 8.17 (dd, J = 2.4, 8.4 Hz, 1H), 7.39 (q, 3.2 Hz, 1H), 7.26 (t, J = 9.2 Hz, 1H), 7.10 (s, 2H), 7.07 (m, 1H), 6.95 (q, J = 4.0 Hz, 1H), 4.41 (q, J = 6.8 Hz, 2H), 3.81 (s, 3H), 1.39 (t, J = 7.2 Hz, 3H). [M+H]$^+$: 411.1. |
| 64 | 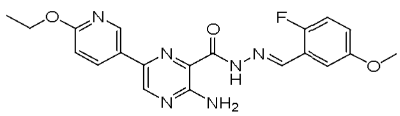 (*E*)-3-amino-6-(6-ethoxypyridine-3-yl)-*N'*-(2-fluoro-5-methoxybenzylidene)pyrazine -2-carbohydrazide | $^1$H-NMR, 400 MHz, DMSO-d$_6$ $\delta$ = 12.00 (s, 1H), 9.04 (d, J = 2.0 Hz, 1H), 8.92 (d, J = 3.6 Hz, 2H), 8.57 (dd, 2.8, 8.8 Hz, 1H), 7.70 (s, 2H), 7.43 (m, 1H), 7.29 (t, J = 9.2 Hz, 1H), 7.09 (m, 1H), 6.93 (d, J = 8.8 Hz, 1H), 4.40 (q, J = 6.8 Hz, 2H), 3.82 (s, 3H), 1.38 (t, J = 6.8 Hz, 3H). [M+H]$^+$: 411.1. |

(continued)

| Compound Nº | Structure | $^1$H-RMN / MS(m/z) [M+H]$^+$ |
|---|---|---|
| 65 | 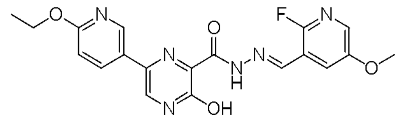 (*E*)-6-(6-ethoxypyridine-3-yl)-N'-((2-fluoro-5-methoxypyridine-3-yl)methylene)-3-hydroxypyrazine-2-carbohydrazide | $^1$H-NMR, 400 MHz, DMSO-$d_6$ $\delta$ = 12.49 (s, 1H), 8.84 (m, 1H), 8.64 (s, 1H), 8.35 (s, 1H), 8.03 (m, 1H), 7.89 (m, 1H), 6.91 (m, 1H), 4.36 (m, 2H), 3.91 (s, 3H), 1.34 (m, 3H) . [M+H]$^+$: 413.1. |
| 66 | 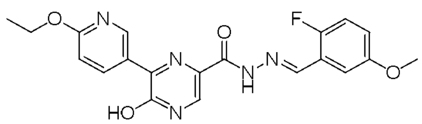 (*E*)-6-(6-ethoxypyridine-3-yl)-*N'*-(2-fluoro-5-methoxybenzylidene)-5-hydroxypyrazine-2-carbohydrazide | $^1$H-NMR, 400 MHz, DMSO-$d_6$ $\delta$ = 13.02 (s, 1H), 11.73 (s, 1H), 9.44 (s, 1H), 8.89 (m, 2H), 8.07 (s, 1H), 7.41 (d, J = 3.2 Hz, 1H), 7.28 (m, 1H), 7.07 (m, 1H), 6.92 (m, 1H), 4.41 (q, J = 2.8 Hz, 2H), 4.37 (s, 3H), 1.37 (t, J = 7.2 Hz, 3H) . [M+H]$^+$: 412.4. |
| 67 | 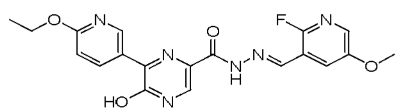 (*E*)-6-(6-ethoxypyridine-3-yl)-*N'*-((2-fluoro-5-methoxypyridine-3-yl)methylene)-5-hydroxypyrazine-2-carbohydrazide | $^1$H-NMR, 400 MHz, DMSO-$d_6$ $\delta$ = 13.06 (s, 1H), 11.86 (s, 1H), 9.43 (s, 1H), 8.87 (m, 1H), 8.82 (s, 1H), 8.08 (m, 1H), 7.89 (m, 1H), 6.91 (d, J = 8.8 Hz, 1H), 4.41 (q, J = 2.8 Hz, 2H), 3.39 (s, 3H), 1.36 (t, J = 7.2 Hz, 3H). [M+H]$^+$: 413.2. |
| 68 | 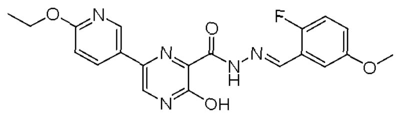 (*E*)-6-(6-ethoxypyridine-3-yl)-*N'*-(2-fluoro-5-methoxybenzylidene)-3-hydroxypyrazine-2-carbohydrazide | $^1$H-NMR, 400 MHz, DMSO-$d_6$ $\delta$ = 12.21 (s, 1H), 8.76 (m, 2H), 8.38 (s, 1H), 8.16 (s, 1H), 7.41 (m, 1H), 7.28 (t, J = 9.6 Hz, 1H), 7.10 (m, 1H), 6.92 (m, 2H), 4.35 (s, 2H), 3.82 (s, 1H), 1.34 (m, 3H). [M+H]$^+$: 412.1. |
| 69 | 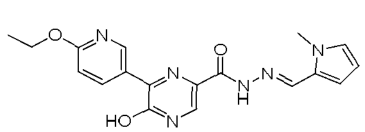 (*E*)-6-(6-ethoxypyridine-3-yl)-5-hydroxy-*N*'-((1-methyl-1*H*-pyrrole-2-yl)methylene)pyrazine-2-carbohydrazide | $^1$H-NMR, 400 MHz, DMSO-$d_6$ $\delta$ = 11.18 (s, 1H) , 9.44 (d, J = 1.8 Hz, 1H) , 8.88 (dd, J = 2.3, 8.7 Hz, 1H) , 8.57 (s, 1H) , 8.15 - 8.05 (m, 1H) , 6.97 (t, J = 2.0 Hz, 1H) , 6.88 (d, J = 8.8 Hz, 1H) , 6.51 (dd, J = 1.7, 3.7 Hz, 1H) , 6.12 (dd, J = 2.7, 3.5 Hz, 1H) , 4.40 (q, J = 7.0 Hz, 2H), 3.89 (s, 3H), 1.36 (t, J = 7.1 Hz, 3H). [M+H]$^+$: 367.1. |
| 70 | 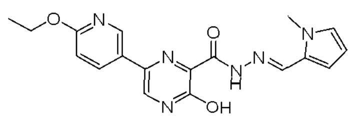 (*E*)-6-(6-ethoxypyridine-3-yl)-3-hydroxy-*N*'-((1-methyl-1*H*-pyrrole-2-yl)methylene)pyrazine-2-carbohydrazide | $^1$H-NMR, 400 MHz, DMSO-$d_6$ $\delta$ = 13.26 - 12.39 (m, 1H), 12.09 - 11.16 (m, 1H), 9.04 - 7.83 (m, 4H), 7.18 - 6.75 (m, 2H), 6.65 - 5.93 (m, 2H), 4.42 (q, J = 6.8 Hz, 2H), 3.99 - 3.33 (m, 3H), 1.37 (br t, J = 6.9 Hz, 3H). [M+H]$^+$: 367.1. |

(continued)

| Compound Nº | Structure | $^1$H-RMN / MS(m/z) [M+H]$^+$ |
|---|---|---|
| 71 | 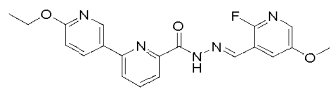 (*E*)-6'-ethoxy-*N*'-((2-fluoro-5-methoxypyridine-3-yl)methylene)-[2,3'-bipyridine]-6-carbohydrazide | $^1$H-NMR, 400 MHz, DMSO-$d_6$ $\delta$ = 12.23 (s, 1H), 9.22 (d, J = 2.3 Hz, 1H), 8.91 (s, 1H), 8.70 (dd, J = 2.5, 8.8 Hz, 1H), 8.26 (dd, J = 0.8, 7.9 Hz, 1H), 8.18 - 7.99 (m, 3H), 7.91 (dd, J = 3.1, 7.6 Hz, 1H), 6.97 (d, J = 8.8 Hz, 1H), 4.41 (q, J = 7.0 Hz, 2H), 3.92 (s, 3H), 1.38 (t, J = 7.1 Hz, 3H). [M+H]$^+$: 396.4. |
| 72 | 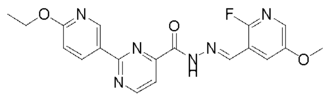 (*E*)-2-(6-ethoxypyridine-3-yl)-*N'*-((2-fluoro-5-methoxypyridine-3-yl)methylene)pyrimidine-4-carbohydrazide | $^1$H-NMR, 400 MHz, DMSO-$d_6$ $\delta$ = 12.41 (s, 1H), 9.49 (d, J = 2.1 Hz, 1H), 9.17 (d, J = 4.9 Hz, 1H), 8.94 (s, 1H), 8.86 (dd, J = 2.4, 8.8 Hz, 1H), 8.07 - 7.87 (m, 3H), 7.00 (d, J = 8.6 Hz, 1H), 4.44 (q, J = 7.1 Hz, 2H), 3.92 (s, 3H), 1.38 (t, J = 7.0 Hz, 3H). [M+H]$^+$: 397.0. |
| 73 | 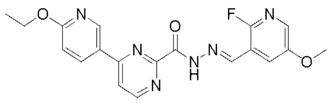 (*E*)-4-(6-ethoxypyridine-3-yl)-*N'*-((2-fluoro-5-methoxypyridine-3-yl)methylene)pyrimidine-2-carbohydrazide | $^1$H-NMR, 400 MHz, DMSO-$d_6$ $\delta$ = 12.47 (s, 1H), 9.25 (d, J = 2.3 Hz, 1H), 9.04 (d, J = 5.4 Hz, 1H), 8.83 (s, 1H), 8.70 (dd, J = 2.6, 8.8 Hz, 1H), 8.30 (d, J = 5.4 Hz, 1H), 8.02 (dd, J = 1.8, 3.0 Hz, 1H), 7.90 (br d, J = 7.6 Hz, 1H), 7.02 (d, J = 8.8 Hz, 1H), 4.43 (q, J = 7.1 Hz, 2H), 3.92 (s, 3H), 1.37 (t, J = 7.1 Hz, 3H). [M+H]$^+$: 396.13. |
| 74 | 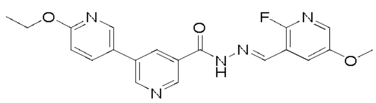 (*E*)-6'-ethoxy-*N*'-((2-fluoro-5-methoxypyridine-3-yl)methylene)-[3,3'-bipyridine]-5-carbohydrazide | $^1$H-NMR, 400 MHz, DMSO-$d_6$ $\delta$ = 11.88 (s, 1H), 9.43 (d, J = 2.00 Hz, 1H), 8.87 (dd, J = 2.40, 8.80 Hz, 1H), 8.83 (s, 1H), 8.58 (s, 1H), 8.01 (d, J = 2.00 Hz, 1H), 7.89 (dd, J = 2.80, 7.80 Hz, 1H), 6.93 (d, J = 8.80 Hz, 1H), 4.41 (q, J = 6.80 Hz, 2H), 3.92 (s, 3H), 3.65 (s, 3H), 1.37 (t, J = 7.20 Hz, 3H). [M+H]$^+$: 395.4. |

(continued)

| Compound No | Structure | $^1$H-RMN / MS(m/z) [M+H]$^+$ |
|---|---|---|
| 75 | 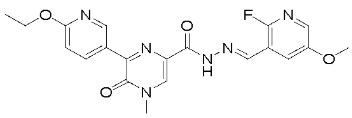 (*E*)-6-(6-ethoxypyridine-3-yl)-*N'*-((2-fluoro-5-methoxypyridine-3-yl)methylene)-4-methyl-5-oxo-4,5-dihydropyrazine-2-carbohydrazide | $^1$H-NMR, 400 MHz, DMSO-$d_6$ $\delta$ = 12.29 - 12.32 (m, 1 H), 9.11 (d, J = 2.00 Hz, 1 H), 9.04 (d, J = 1.50 Hz, 1 H), 8.61 - 8.67 (m, 1 H), 8.57 (s, 1 H), 8.53 (t, J = 1.81 Hz, 1 H), 8.19 (dd, J = 8.57, 2.56 Hz, 1 H), 8.03 (br s, 1 H), 7.88 (dd, J = 7.69, 2.81 Hz, 1 H), 6.98 (d, J = 8.50 Hz, 1 H), 4.38 (q, J = 7.05 Hz, 2 H), 3.88 - 3.93 (m, 3 H), 1.36 (t, J = 7.07 Hz, 3 H). [M+H]$^+$: 427;0. |
| 76 | 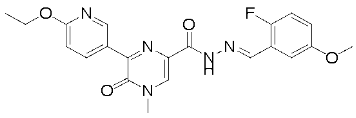 (*E*)-6-(6-ethoxypyridine-3-yl)-*N'*-(2-fluoro-5-methoxybenzylidene)-4-methyl-5-oxo-4,5-dihydropyrazine-2-carbohydrazide | $^1$H-NMR, 400 MHz, DMSO-$d_6$ $\delta$ = 11.76 (s, 1H), 9.43 (s, 1H), 8.90-8.86 (m, 2H), 8.56 (s, 1H), 7.41 (d, J = 2.80 Hz, 1H), 7.27 (t, J = 9.60 Hz, 1H), 7.07 (dd, J = 5.20, 8.00 Hz, 1H), 6.93 (d, J = 8.80 Hz, 1H), 4.41 (q, J = 7.20 Hz, 2H), 3.82 (s, 3H), 3.65 (s, 3H), 1.36 (t, J = 7.20 Hz, 3H). [M+H]$^+$: 426.0. |
| 77 | 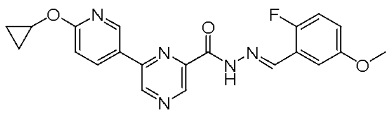 (*E*) -6-(6-cyclopropoxypyridine-3-yl)-*N'*-(2-fluoro-5-methoxybenzylidene)pyrazine -2-carbohydrazide | $^1$H-NMR, 400 MHz, $CDCl_3$ $\delta$ = 10.80 (s, 1H), 9.45 (s, 1H), 9.22 (s, 1H), 8.98 (d, J = 2.3 Hz, 1H), 8.62 (s, 1H), 8.30 (dd, J = 2.5, 8.6 Hz, 1H), 7.64 (dd, J = 3.1, 5.4 Hz, 1H), 7.10 - 6.93 (m, 3H), 4.35 (tt, J = 3.2, 6.1 Hz, 1H), 3.88 (s, 3H), 0.95 - 0.82 (m, 4H) . [M+H]$^+$: 408.2. |
| 78 | 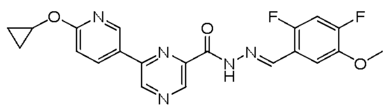 (*E*)-6-(6-cyclopropoxypyridine-3-yl)-*N'*-(2,4-difluoro-5-methoxybenzylidene)pyrazine -2-carbohydrazide | $^1$H-NMR, 400 MHz, $CDCl_3$ $\delta$ = 10.80 (s, 1H), 9.45 (br s, 1H), 9.22 (br s, 1H), 8.97 (br s, 1H), 8.57 (s, 1H), 8.28 (dd, J = 2.1, 8.6 Hz, 1H), 7.73 (dd, J = 6.8, 9.3 Hz, 1H), 7.03 - 6.83 (m, 2H), 4.34 (tt, J = 3.2, 6.1 Hz, 1H), 3.98 (s, 3H), 0.96 - 0.78 (m, 4H). [M+H]$^+$: 426.2. |

(continued)

| Compound No | Structure | $^1$H-RMN / MS(m/z) [M+H]$^+$ |
|---|---|---|
| 79 | 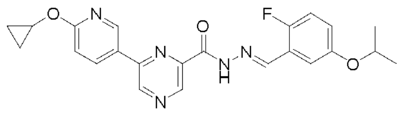 (*E*)-6-(6-cyclopropoxypyridine-3-yl)-*N'*-(2-fluoro-5-isopropoxybenzylidene)pyraz ine-2-carbohydrazide | $^1$H-NMR, 400 MHz, DMSO-$d_6$ $\delta$ = 12.21 (s, 1H), 9.53 (s, 1H), 9.28 (d, J = 2.0 Hz, 1H), 9.17 (s, 1H), 8.95 (s, 1H), 8.76 (dd, J = 2.4, 8.8 Hz, 1H), 7.42 (dd, J = 3.4, 5.9 Hz, 1H), 7.28 - 7.22 (m, 1H), 7.08 (td, J = 3.8, 8.6 Hz, 1H), 7.01 (d, J = 8.8 Hz, 1H), 4.61 (td, J = 5.9, 12.1 Hz, 1H), 4.42 (q, J = 7.2 Hz, 2H), 1.38 (t, J = 7.1 Hz, 3H), 1.30 (d, J = 5.9 Hz, 6H). [M+H]$^+$: 436.2. |
| 80 | 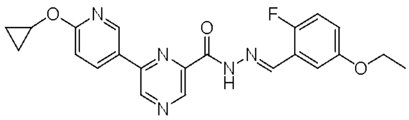 (*E*)-6-(6-cyclopropoxypyridine-3-yl)-*N'*-(5-ethoxy-2-fluorobenzy-lidene)pyrazine-2-carbohydrazide | $^1$H-NMR, 400 MHz, DMSO-$d_6$ $\delta$ = 12.21 (s, 1H), 9.54 (s, 1H), 9.33 (d, J = 2.4 Hz, 1H), 9.19 (s, 1H), 8.96 (s, 1H), 8.78 (dd, J = 2.7, 8.6 Hz, 1H), 7.42 (dd, J = 3.2, 5.6 Hz, 1H), 7.26 (t, J = 9.5 Hz, 1H), 7.14 - 7.02 (m, 2H), 4.33 (td, J = 3.0, 6.2 Hz, 1H), 4.08 (q, J = 6.8 Hz, 2H), 1.36 (t, J = 7.1 Hz, 3H), 0.86 - 0.69 (m, 4H). [M+H]$^+$: 422.2. |
| 81 | 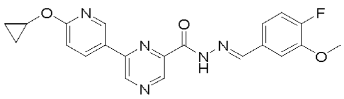 (*E*)-6-(6-cyclopropoxypyridine-3-yl)-*N'*-(4-fluoro-3-methoxyben-zylidene)pyrazine -2-carbohydrazide | $^1$H-NMR, 400 MHz, $CDCl_3$ $\delta$ = 10.63 (s, 1H), 9.36 (s, 1H), 9.13 (s, 1H), 8.90 (d, J = 2.3 Hz, 1H), 8.32 (s, 1H), 8.19 (dd, J = 2.4, 8.7 Hz, 1H), 7.57 (dd, J = 1.6, 8.3 Hz, 1H), 7.14 - 7.02 (m, 2H), 6.89 (d, J = 8.7 Hz, 1H), 4.26 (tt, J = 3.2, 6.1 Hz, 1H), 3.92 (s, 3H), 0.77 - 0.76 (m, 1H), 0.85 - 0.73 (m, 3H). [M+H]$^+$: 408.1. |
| 82 | 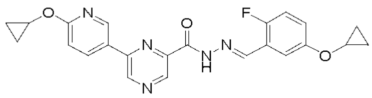 (*E*)-*N'*-(5-cyclopropoxy-2-fluorobenzylidene)-6-(6-cyclopropoxypyridine-3-yl)pyrazine-2-carbohydrazide | $^1$H-NMR, 400 MHz, DMSO-$d_6$ $\delta$ = 12.23 (s, 1H), 9.55 (s, 1H), 9.33 (d, J = 2.4 Hz, 1H), 9.19 (s, 1H), 9.03 - 8.95 (m, 1H), 8.78 (dd, J = 2.4, 8.8 Hz, 1H), 7.61 (dd, J = 3.2, 5.7 Hz, 1H), 7.35 - 7.23 (m, 1H), 7.20 - 7.04 (m, 2H), 4.33 (tt, J = 3.0, 6.2 Hz, 1H), 3.94 (tt, J = 2.8, 5.9 Hz, 1H), 0.90 - 0.64 (m, 8H). [M+H]$^+$: 434.1. |

(continued)

| Compound No | Structure | $^1$H-RMN / MS(m/z) [M+H]$^+$ |
|---|---|---|
| 83 | 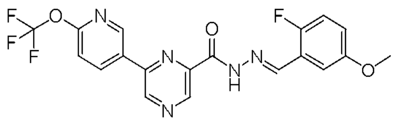 (*E*)-*N'*-(2-fluoro-5-methoxybenzylidene)-6-(6-(trifluoromethoxy) pyridine-3-yl)pyrazine-2-carbohydrazide | $^1$H-NMR, 400 MHz, DMSO-$d_6$ $\delta$ = 12.26 (s, 1H), 9.64 (s, 1H), 9.48 (d, J = 2.3 Hz, 1H), 9.29 (s, 1H), 9.06 (dd, J = 2.4, 8.6 Hz, 1H), 8.97 (s, 1H), 7.56 (d, J = 8.6 Hz, 1H), 7.44 (dd, J = 3.2, 5.6 Hz, 1H), 7.29 (t, J = 9.5 Hz, 1H), 7.11 (td, J = 3.8, 9.0 Hz, 1H), 3.83 (s, 3H). [M+H]$^+$: 436.0. |
| 84 | 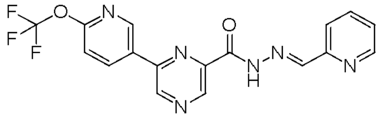 (*E*)-*N'*-(pyridine-2-ylmethylene)-6-(6-(trifluoromethoxy)pyridine-3-yl)pyrazine-2-carbohydrazide | $^1$H-NMR, 400 MHz, DMSO-$d_6$ $\delta$ = 12.32 (s, 1H), 9.65 (s, 1H), 9.49 (d, J = 2.3 Hz, 1H), 9.30 (s, 1H), 9.06 (dd, J = 2.5, 8.6 Hz, 1H), 8.80 (s, 1H), 8.68 (d, J = 4.3 Hz, 1H), 8.05 (d, J = 7.9 Hz, 1H), 7.93 (dt, J = 1.5, 7.7 Hz, 1H), 7.56 (d, J = 8.6 Hz, 1H), 7.47 (ddd, J = 1.0, 4.9, 7.4 Hz, 1H). [M+H]$^+$: 389.0. |
| 85 | 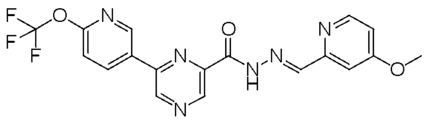 (*E*)-*N'*-((4-methoxypyridine-2-yl)methylene)-6-(6-(trifluoromethoxy)pyridine-3-yl)pyrazine-2-carbohydrazide | $^1$H-NMR, 400 MHz, DMSO-$d_6$ $\delta$ = 12.31 (s, 1H), 9.65 (s, 1H), 9.49 (d, J = 2.3 Hz, 1H), 9.40 - 9.27 (m, 1H), 9.11 - 9.01 (m, 1H), 8.84 - 8.68 (m, 1H), 8.49 (d, J = 5.7 Hz, 1H), 7.89 - 7.41 (m, 2H), 7.07 (dd, J = 2.6, 5.7 Hz, 1H), 3.98 - 3.82 (m, 3H). [M+H]$^+$: 419.1. |
| 86 | 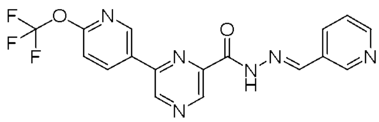 (*E*)-*N'*-(pyridine-3-ylmethylene)-6-(6-(trifluoromethoxy)pyridine-3-yl)pyrazine-2-carbohydrazide | $^1$H-NMR, 400 MHz, DMSO-$d_6$ $\delta$ = 12.27 (s, 1H), 9.64 (s, 1H), 9.49 (d, J = 2.3 Hz, 1H), 9.29 (s, 1H), 9.05 (dd, J = 2.4, 8.6 Hz, 1H), 8.95 - 8.89 (m, 1H), 8.81 (s, 1H), 8.67 (dd, J = 1.7, 4.7 Hz, 1H), 8.25 - 8.20 (m, 1H), 7.60 - 7.52 (m, 2H) . [M+H]$^+$: 389.1. |
| 87 | 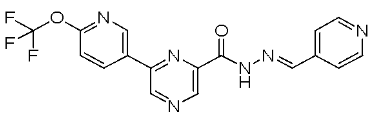 (*E*)-*N'*-(pyridine-4-ylmethylene)-6-(6-(trifluoromethoxy)pyridine-3-yl)pyrazine-2-carbohydrazide | $^1$H-NMR, 400 MHz, DMSO-$d_6$ $\delta$ = 12.35 (s, 1H), 9.65 (s, 1H), 9.48 (d, J = 2.4 Hz, 1H), 9.30 (s, 1H), 9.05 (dd, J = 2.4, 8.6 Hz, 1H), 8.80 - 8.68 (m, 3H), 7.73 (d, J = 5.8 Hz, 2H), 7.56 (d, J = 8.6 Hz, 1H). [M+H]$^+$: 389.0. |

(continued)

| Compound No | Structure | $^1$H-RMN / MS(m/z) [M+H]$^+$ |
|---|---|---|
| 88 | 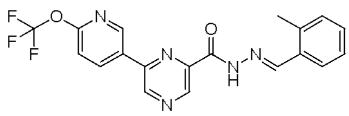 (*E*)-*N'*-(2-methylbenzylidene)-6-(6-(trifluoromethoxy)pyridine-3-yl)pyrazine-2-carbohydrazide | $^1$H-NMR, 400 MHz, DMSO-$d_6$ $\delta$ = 12.10 (s, 1H), 9.62 (s, 1H), 9.47 (d, J = 2.4 Hz, 1H), 9.28 (s, 1H), 9.06 - 8.95 (m, 2H), 7.90 (d, J = 7.7 Hz, 1H), 7.56 (d, J = 8.6 Hz, 1H), 7.39 - 7.21 (m, 3H), 2.51 (s, 3H). [M+H]$^+$: 402.1. |
| 89 | 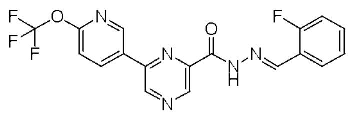 (*E*)-*N'*-(2-fluorobenzylidene)-6-(6-(trifluoromethoxy)pyridine-3-yl)pyrazine-2-carbohydrazide | $^1$H-NMR, 400 MHz, DMSO-$d_6$ $\delta$ = 12.25 (s, 1H), 9.64 (s, 1H), 9.48 (d, J = 2.1 Hz, 1H), 9.29 (s, 1H), 9.05 (dd, J = 2.5, 8.6 Hz, 1H), 9.00 (s, 1H), 8.02 (dt, J = 1.5, 7.6 Hz, 1H), 7.60 - 7.49 (m, 2H), 7.40 - 7.29 (m, 2H) . [M+H]$^+$: 406.1. |
| 90 | 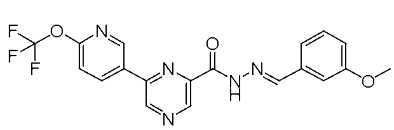 (*E*)-*N'*-(3-methoxybenzylidene)-6-(6-(trifluoromethoxy)pyridine-3-yl)pyrazine-2-carbohydrazide | $^1$H-NMR, 400 MHz, DMSO-$d_6$ $\delta$ = 12.11 (s, 1H), 9.62 (s, 1H), 9.47 (s, 1H), 9.27 (s, 1H), 9.04 (br d, J = 8.5 Hz, 1H), 8.71 (s, 1H), 7.54 (d, J = 8.6 Hz, 1H), 7.46 - 7.39 (m, 1H), 7.37 - 7.29 (m, 2H), 7.06 (br d, J = 8.1 Hz, 1H), 3.83 (s, 3H). [M+H]$^+$: 418.0. |
| 91 | 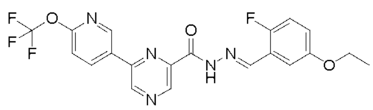 (*E*)-*N'*-(5-ethoxy-2-fluorobenzylidene)-6-(6-(trifluoromethoxy) pyridine-3-yl)pyrazine-2-carbohydrazide | $^1$H-NMR, 400 MHz, DMSO-$d_6$ $\delta$ = 12.23 (s, 1H), 9.62 (s, 1H), 9.46 (d, J = 2.3 Hz, 1H), 9.26 (s, 1H), 9.04 (dd, J = 2.5, 8.6 Hz, 1H), 8.94 (s, 1H), 7.54 (d, J = 8.6 Hz, 1H), 7.40 (dd, J = 3.2, 5.6 Hz, 1H), 7.25 (t, J = 9.5 Hz, 1H), 7.13 - 7.00 (m, 1H), 4.07 (q, J = 7.0 Hz, 2H), 1.35 (t, J = 6.9 Hz, 3H). [M+H]$^+$: 450.0. |
| 92 | 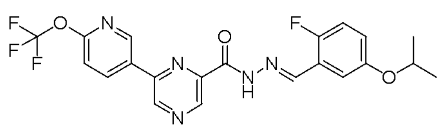 (*E*)-*N'*-(2-fluoro-5-isopropoxybenzylidene)-6-(6-(trifluoromethoxy)pyridine-3-yl)pyrazine-2-carbohydrazide | $^1$H-NMR, 400 MHz, DMSO-$d_6$ $\delta$ = 12.25 (s, 1H), 9.63 (s, 1H), 9.47 (d, J = 2.4 Hz, 1H), 9.28 (s, 1H), 9.05 (dd, J = 2.4, 8.6 Hz, 1H), 8.94 (s, 1H), 7.55 (d, J = 8.6 Hz, 1H), 7.42 (dd, J = 3.2, 5.7 Hz, 1H), 7.26 (t, J = 9.6 Hz, 1H), 7.16 - 7.00 (m, 1H), 4.61 (td, J = 6.0, 12.1 Hz, 1H), 1.29 (d, J = 6.0 Hz, 6H). [M+H]$^+$: 464.0. |

(continued)

| Compound No | Structure | $^1$H-RMN / MS(m/z) [M+H]$^+$ |
|---|---|---|
| 93 | 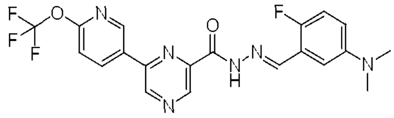 (*E*)-*N*'-(5-(dimethylamino)-2-fluorobenzylidene)-6-(6-(trifluoromethoxy)pyridine-3-yl)pyrazine-2-carbohydrazide | $^1$H-NMR, 400 MHz, DMSO-$d_6$ $\delta$ = 12.20 (s, 1H), 9.64 (s, 1H), 9.48 (d, J = 2.3 Hz, 1H), 9.28 (s, 1H), 9.06 (dd, J = 2.5, 8.6 Hz, 1H), 8.96 (s, 1H), 7.56 (d, J = 8.6 Hz, 1H), 7.24 - 7.13 (m, 2H), 6.92 (td, J = 3.9, 8.9 Hz, 1H), 2.94 (s, 6H) . [M+H]$^+$: 449.0. |
| 94 | 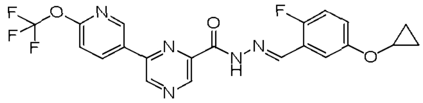 (*E*)-*N'*-(5-cyclopropoxy-2-fluorobenzylidene)-6-(6-(trifluoromethoxy)pyridine-3-yl)pyrazine-2-carbohydrazide | $^1$H-NMR, 400 MHz, DMSO-$d_6$ $\delta$ = 12.27 (s, 1H), 9.64 (s, 1H), 9.48 (d, J = 2.3 Hz, 1H), 9.28 (s, 1H), 9.06 (dd, J = 2.5, 8.6 Hz, 1H), 8.99 - 8.94 (m, 1H), 7.62 (dd, J = 3.2, 5.7 Hz, 1H), 7.56 (d, J = 8.5 Hz, 1H), 7.34 - 7.25 (m, 1H), 7.21 - 7.13 (m, 1H), 0.86 - 0.79 (m, 2H), 0.75 - 0.64 (m, 2H). [M+H]$^+$: 462.0. |
| 95 | 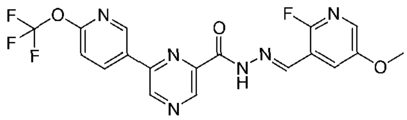 (*E*)-*N'*-((2-fluoro-5-methoxypyridine-3-yl)methylene)-6-(6-(trifluoromethoxy)pyridine-3-yl)pyrazine-2-carbohydrazide | $^1$H-NMR, 400 MHz, DMSO-$d_6$ $\delta$ = 12.38 (s, 1H), 9.65 (s, 1H), 9.48 (d, J = 2.00 Hz, 1H), 9.29 (s, 1H), 9.05 (dd, J = 2.80, 8.60 Hz, 1H), 8.89 (s, 1H), 8.05 (dd, J = 1.60, 3.00 Hz, 1H), 7.92 (dd, J = 2.80, 7.80 Hz, 1H), 7.57 (d, J = 8.80 Hz, 1H), 3.93 (s, 1H). [M+H]$^+$: 437.1. |
| 96 | 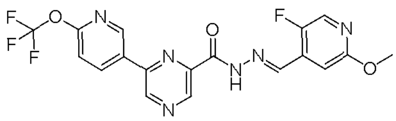 (*E*)-*N'*-((5-fluoro-2-methoxypyridine-4-yl)methylene)-6-(6-(trifluoromethoxy)pyridine-3-yl)pyrazine-2-carbohydrazide | $^1$H-NMR, 400 MHz, DMSO-$d_6$ $\delta$ = 12.46 (s, 1H), 9.66 (s, 1H), 9.47 (d, J = 2.00 Hz, 1H), 9.30 (s, 1H), 9.04 (dd, J = 2.40, 8.60 Hz, 1H), 8.92 (s, 1H), 8.33 (d, J = 1.60 Hz, 1H), 7.56 (t, J = 0.40 Hz, 1H), 7.22 (d, J = 4.80 Hz, 1H), 3.90 (s, 3H). [M+H]$^+$: 437.1. |
| 97 | 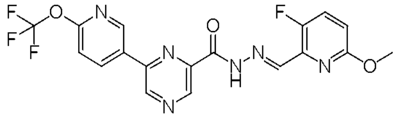 (*E*)-*N'*-((3-fluoro-6-methoxypyridine-2-yl)methylene)-6-(6-(trifluoromethoxy)pyridine-3-yl)pyrazine-2-carbohydrazide | $^1$H-NMR, 400 MHz, DMSO-$d_6$ $\delta$ = 14.49 (s, 1H), 9.58 (s, 1H), 9.34 (s, 1H), 9.19 (d, J = 2.00 Hz, 1H), 8.73 (dd, J = 2.40, 8.60 Hz, 1H), 8.02 (t, J = 9.20 Hz, 1H), 7.88 (d, J = 2.00 Hz, 1H), 7.53 (dd, J = 0.40, 8.40 Hz, 1H), 7.16 (dd, J = 3.20, 9.20 Hz, 1H), 3.54 (s, 3H). [M+H]$^+$: 437.1. |

(continued)

| Compound Nº | Structure | $^1$H-RMN / MS(m/z) $[M+H]^+$ |
|---|---|---|
| 98 | 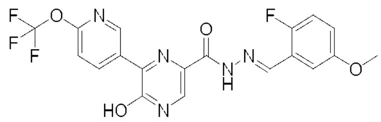 (*E*)-*N'*-(2-fluoro-5-methoxybenzylidene)-5-hydroxy-6-(6-(trifluoromethoxy)pyridine-3-yl)pyrazine-2-carbohydrazide | $^1$H-NMR, 400 MHz, DMSO-$d_6$ $\delta$ = 13.24 (br d, J = 1.5 Hz, 1H), 11.77 (s, 1H), 9.53 (d, J = 2.1 Hz, 1H), 9.14 (dd, J = 2.4, 8.6 Hz, 1H), 8.89 (s, 1H), 8.18 (s, 1H), 7.48 - 7.39 (m, 2H), 7.27 (t, J = 9.6 Hz, 1H), 7.07 (td, J = 3.9, 8.7 Hz, 1H), 3.82 (s, 3H). $[M+H]^+$: 452.0. |
| 99 | 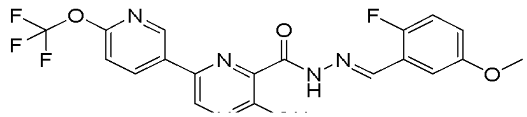 (*E*)-*N'*-(2-fluoro-5-methoxybenzylidene)-3-hydroxy-6-(6-(trifluoromethoxy)pyridine-3-yl)pyrazine-2-carbohydrazide | $^1$H-NMR, 400 MHz, DMSO-$d_6$ $\delta$ = 13.59 - 12.02 (m, 2H), 9.09 - 8.14 (m, 4H), 7.46 - 6.78 (m, 4H), 3.85 - 3.57 (m, 4H). $[M+H]^+$: 452.0. |
| 100 | 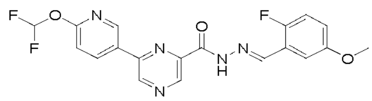 (*E*)-6-(6-(difluoromethoxy)pyridine-3-yl)-*N'*-(2-fluoro-5-methoxybenzylidene)pyrazine -2-carbohydrazide | $^1$H-NMR, 400 MHz, DMSO-$d_6$ $\delta$ = 12.27 (s, 1H), 9.62 (s, 1H), 9.38 (d, J = 2.2 Hz, 1H), 9.25 (s, 1H), 9.01 - 8.96 (m, 2H), 7.85 (t, J = 72.6 Hz, 1H), 7.44 (dd, J = 3.2, 5.5 Hz, 1H), 7.36 (d, J = 8.7 Hz, 1H), 7.33 - 7.25 (m, 1H), 7.10 (td, J = 3.8, 8.8 Hz, 1H), 3.83 (s, 3H). $[M+H]^+$: 418.0. |
| 101 | 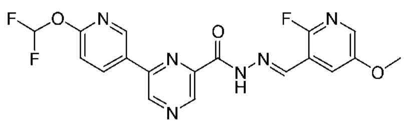 (*E*)-6-(6-(difluoromethoxy)pyridine-3-yl)-*N'*-((2-fluoro-5-methoxypyridine-3-yl)methylene)pyrazine-2-carbohydrazide | $^1$H-NMR, 400 MHz, DMSO-$d_6$ $\delta$ = 12.39 (s, 1H), 9.63 (s, 1H), 9.37 (d, J = 2.0 Hz, 1H), 9.26 (s, 1H), 8.99 (dd, J = 2.8, 8.8 Hz, 1H), 8.89 (s, 1H), 8.05-7.66 (m, 3H), 7.37 (d, J = 8.4 Hz, 1H), 3.93 (s, 3H). $[M+H]^+$: 419.1. |
| 102 | 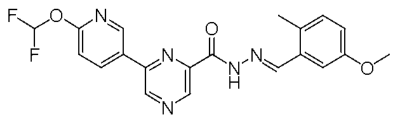 (*E*)-6-(6-(difluoromethoxy)pyridine-3-yl)-*N'*-(5-methoxy-2-methylbenzylidene)pyrazine-2-carbohydrazide | $^1$H-NMR, 400 MHz, DMSO-$d_6$ $\delta$ = 12.13 (s, 1H), 9.61 (s, 1H), 9.37 (d, J = 2.4 Hz, 1H), 9.25 (s, 1H), 8.98 to 8.95 (m, 2H), 8.03 to 7.67 (m, 1H), 7.44 (d, J = 2.8 Hz, 1H), 7.37 (d, J = 8.8 Hz, 1H), 7.23 (d, J = 8.8 Hz, 1H), 6.98 to 6.96 (dd, J = 2.8, 8.4 Hz, 1H). $[M+H]^+$: 414.0. |
| 103 | 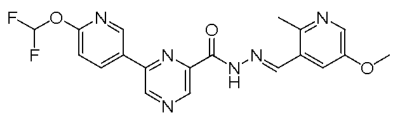 (*E*)-6-(6-(difluoromethoxy)pyridine-3-yl)-*N'*-((5-methoxy-2-methylpyridine-3-yl)methylene)pyrazine-2-carbohydrazide | $^1$H-NMR, 400 MHz, DMSO-$d_6$ $\delta$ = 12.25 (s, 1H), 9.62 (s, 1H), 9.37 (d, J = 2.0 Hz, 1H), 9.26 (s, 1H), 8.99-8.95 (m, 2H), 8.27 (d, J = 2.8 Hz, 1H), 8.03-7.67 (m, 2H), 7.38 (s, 1H), 3.89 (s, 3H), 2.62 (s, 3H). $[M+H]^+$: 415.2. |

(continued)

| Compound No | Structure | $^1$H-RMN / MS(m/z) [M+H]$^+$ |
|---|---|---|
| 104 | fluorobenzylidene)pyrazine-2-carbohydrazide | $^1$H-NMR, 400 MHz, DMSO-$d_6$ $\delta$ = 12.20 (s, 1H), 9.61 (s, 1H), 9.38 (s, 1H), 9.00 (s, 1H), 8.98-8.03 (m, 2H), 7.85 (t, J = 7.24 Hz, 1H), 7.75 (d, J = 7.24 Hz, 1H), 7.37-7.34 (m, 2H), 7.21-7.15 (m, 1H), 2.94 (s, 6H). [M+H]$^+$: 431.2. |
| 105 | 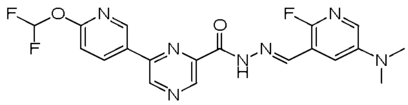 (*E*)-6-(6-(difluoromethoxy)pyridine-3-yl)-*N'*-((5-(dimethylamino)-2-fluoropyridine-3-yl)methylene)pyrazine-2-carbohydrazide | H-NMR, 400 MHz, DMSO-$d_6$ $\delta$ = 12.32 (s, 1H), 9.62 (s, 1H), 9.37 (s, 1H), 9.31 (s, 1H), 9.00-8.99 (m, 1H), 8.97-8.89 (m, 1H), 8.03-7.79 (m, 3H), 7.63-7.35 (m, 1H), 3.00 (s, 6H). [M+H]$^+$: 431.37. |
| 106 | 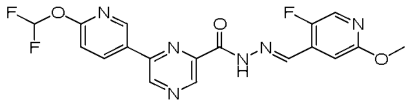 (*E*)-6-(6-(difluoromethoxy)pyridine-3-yl)-*N'*-((5-fluoro-2-methoxypyridine-4-yl)methylene)pyrazine-2-carbohydrazide | $^1$H-NMR, 400 MHz, DMSO-$d_6$ $\delta$ = 12.47 (s, 1H), 9.63 (s, 1H), 9.37 (d, J = 2.4 Hz,1H), 9.26 (s, 1H), 8.99-8.96 (m, 1H), 8.92 (s, 1H), 8.33 (d, J = 1.6 Hz, 1H), 8.02-7.66 (m, 1H), 7.37 (d, J = 8.4 Hz, 1H), 7.22 (d, J = 4.8 Hz, 1H), 3.89 (s, 3H). [M+H]$^+$: 419.1. |
| 107 | 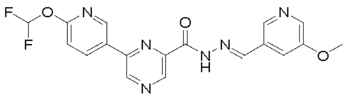 (*E*)-6-(6-(difluoromethoxy)pyridine-3-yl)-*N'*-((5-methoxypyridine-3-yl)methylene)pyrazine-2-carbohydrazide | $^1$H-NMR, 400 MHz, DMSO-$d_6$ $\delta$ = 12.28 (s, 1H), 9.62 (s, 1H), 9.37 (d, J = 2.3 Hz, 1H), 9.25 (s, 1H), 8.98 (dd, J = 2.4, 8.7 Hz, 1H), 8.80 (s, 1H), 8.50 (d, J = 1.2 Hz, 1H), 8.40 (d, J = 2.9 Hz, 1H), 8.04 - 7.65 (m, 2H), 7.36 (d, J = 8.6 Hz, 1H), 3.93 (s, 3H). [M+H]$^+$: 401.0. |
| 108 | 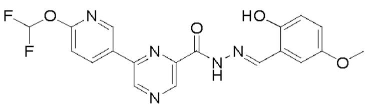 (*E*)-6-(6-(difluoromethoxy)pyridine-3-yl)-*N'*-(2-hydroxy-5-methoxybenzylidene)pyrazine -2-carbohydrazide | $^1$H-NMR, 400 MHz, DMSO-$d_6$ $\delta$ = 3.75 (s, 3 H) 6.84 - 7.00 (m, 2 H) 7.18 (d, J=2.88 Hz, 1 H) 7.35 (d, J=8.50 Hz, 1 H) 7.84 (t, J=72.54 Hz, 1 H) 8.86 - 9.03 (m, 2 H) 9.24 (s, 1 H) 9.36 (d, J=1.88 Hz, 1 H) 9.60 (s, 1 H) 10.54 (br s, 1 H) 12.30 (br s, 1 H). [M+H]$^+$: 416.4. |

(continued)

| Compound No | Structure | $^1$H-RMN / MS(m/z) [M+H]$^+$ |
|---|---|---|
| 109 | 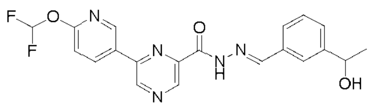 (*E*)-6-(6-(difluoromethoxy)pyridine-3-yl)-*N'*-(3-(1-hydroxyethyl) benzylidene)py razine-2-carbohydrazide | $^1$H-NMR, 400 MHz, DMSO-$d_6$ $\delta$ = 12.09 (1H, s), 9.61 (1H, s), 9.38 (1H, d, J = 2.3 Hz), 9.25 (1H, s), 8.98 (1H, dd, J = 2.5, 8.6 Hz), 8.74 (1H, s), 8.04-7.67 (2H, m), 7.63-7.58 (1H, m), 7.45 (2H, d, J = 5.0 Hz), 7.35 (1H, d, J = 8.6 Hz), 5.30 (1H, d, J = 4.1 Hz), 4.82-4.77 (1H, m), 1.37 (3H, d, J = 6.4 Hz) . [M+H]$^+$: 414.4. |
| 110 | 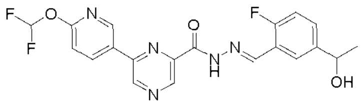 (*E*)-6-(6-(difluoromethoxy)pyridine-3-yl)-*N'*-(2-fluoro-5-(1-hydroxyethyl)benzylidene)py razine-2-carbohydrazide | $^1$H-NMR, 400 MHz, DMSO-$d_6$ $\delta$ = 12.24 (1H, s), 9.61 (1H, s), 9.37 (1H, d, J = 2.1 Hz), 9.25 (1H, s), 9.00-8.96 (2H, m), 8.03-8.00 (1H, m), 7.84 (1H, t, J = 72.5 Hz), 7.50-7.44 (1H, m), 7.35 (1H, dd, J = 0.5, 8.6 Hz), 7.28 (1H, dd, J = 8.5, 10.5 Hz), 5.36 (1H, d, J = 4.1 Hz), 4.83-4.77 (1H, m), 1.35 (3H, d, J = 6.5 Hz) . [M+H]$^+$: 432.3. |
| 111 | 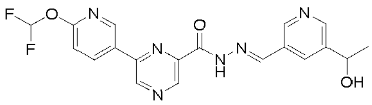 (*E*)-6-(6-(difluoromethoxy)pyridine-3-yl)-*N'*-((5-(1-hydroxyethyl) pyridine-3-yl)methylene)pyrazine-2-carbohydrazide | $^1$H-NMR, 400 MHz, DMSO-$d_6$ $\delta$ = 12.26 (1H, s), 9.62 (1H, s), 9.38 (1H, d, J = 2.1 Hz), 9.26 (1H, s), 8.98 (1H, dd, J = 2.5, 8.6 Hz), 8.81 (1H, s), 8.75 (1H, d, J = 2.0 Hz), 8.63 (1H, d, J = 2.0 Hz), 8.22 (1H, t, J = 1.9 Hz), 7.85 (1H, t, J = 72.1 Hz), 7.36 (1H, d, J = 8.6 Hz), 5.49 (1H, d, J = 4.4 Hz), 4.93 to 4.85 (1H, m), 1.41 (3H, d, J = 6.4 Hz). [M+H]$^+$: 415.4. |
| 112 | 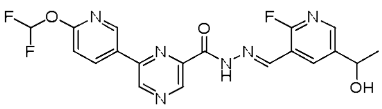 (*E*)-6-(6-(difluoromethoxy)pyridine-3-yl)-*N'*-((2-fluoro-5-(1-hydroxyethyl)pyridine-3-yl)methylene)pyrazine-2-carbohydrazide | $^1$H-NMR, 400 MHz, DMSO-$d_6$ $\delta$ = 12.37 (1H, s), 9.62 (1H, s), 9.37 (1H, d, J = 2.1 Hz), 9.26 (1H, s), 8.98 (1H, dd, J = 2.6, 8.8 Hz), 8.92 (1H, s), 8.46 (1H, dd, J = 2.2, 9.4 Hz), 8.28 (1H, d, J = 1.4 Hz), 7.84 (1H, t, J = 72.7 Hz), 7.36 (1H, d, J = 8.8 Hz), 5.55 (1H, d, J = 4.4 Hz), 4.94-4.88 (1H, m), 1.40 (3H, d, J = 6.5 Hz). [M+H]$^+$: 433.2. |

(continued)

| Compound № | Structure | $^1$H-RMN / MS(m/z) [M+H]$^+$ |
|---|---|---|
| 113 | 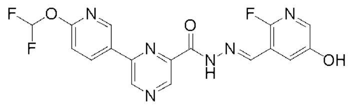 (*E*)-6-(6-(difluoromethoxy)pyridine-3-yl)-*N'*-((2-fluoro-5-hydroxypyridine-3-yl)methylene)pyrazine-2-carbohydrazide | $^1$H-NMR, 400 MHz, DMSO-d$_6$ $\delta$ = 12.36 (s, 1H) , 10.27 (s, 1H) , 9.62 (s, 1H), 9.37 (d, J = 2.3 Hz, 1H) , 9.26 (s, 1H) , 8.98 (dd, J = 2.4, 8.6 Hz, 1H) , 8.84 (s, 1H) , 8.05 - 7.64 (m, 3H) , 7.36 (d, J = 8.6 Hz, 1H).<br>[M+H]$^+$: 405.3. |
| 114 | 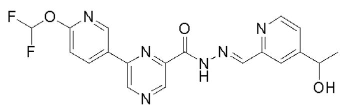 (*E*)-6-(6-(difluoromethoxy)pyridine-3-yl)-*N'*-((4-(1-hydroxyethyl) pyridine-2-yl)methylene)pyrazine-2-carbohydrazide | $^1$H-NMR, 400 MHz, DMSO-d$_6$ $\delta$ = 9.61 (s, 1H), 9.38 (d, J = 2.3 Hz, 1H), 9.26 (s, 1H), 8.98 (dd, J = 2.4, 8.6 Hz, 1H), 8.78 (s, 1H), 8.58 (d, J = 5.1 Hz, 1H), 8.07 (s, 1H), 7.84 (t, J = 72.5 Hz, 1H), 7.28 - 7.45 (m, 2H), 5.55 (d, J = 4.4 Hz, 1H), 4.77 - 4.89 (m, 1H), 1.37 (d, J = Hz, 3H).<br>6.5 [M+H]$^+$: 414.37. |
| 115 | 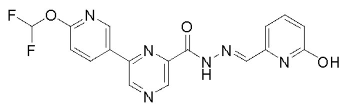 (*E*)-6-(6-(difluoromethoxy)pyridine-3-yl)-*N'*-((6-hydroxypyridine-2-yl)methylene)pyrazine-2-carbohydrazide | $^1$H-NMR, 400 MHz, DMSO-d$_6$ $\delta$ = 6.40 (br d, J=1.50 Hz, 1 H) 7.24 (s, 1 H) 7.71 (br d, J=2.13 Hz, 1 H) 7.77 - 7.91 (m, 1 H) 7.96 - 8.13 (m, 1 H) 8.36 (br d, J=6.88 Hz, 1 H) 8.84 (s, 1 H) 9.16 - 9.42 (m, 2 H) 9.49 - 9.80 (m, 1 H) 11.40 (s, 1 H) 12.31 - 12.66 (m, 1 H).<br>[M+H]$^+$: 387.4. |
| 116 | 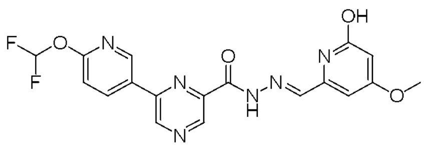 (*E*)-6-(6-(difluoromethoxy)pyridine-3-yl)-*N'*-((6-hydroxy-4-methoxypyridine-2-yl)methylene)pyra-zine-2-carbohydrazide | |
| 117 | 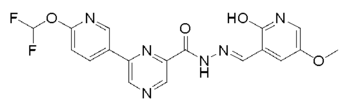 *(E)-6-(6*-(difluoromethoxy)pyridine-3-yl)-*N'*-((2-hydroxy-5-methoxypyridine-3-yl)methylene)pyrazine-2-carbohydrazide | $^1$H-NMR, 400 MHz, DMSO-d$_6$ $\delta$ = 12.22 (s, 1H) , 11.82 (br s, 1H) , 9.60 (s, 1H) , 9.37 (d, J = 2.4 Hz, 1H) , 9.23 (s, 1H) , 9.02 (dd, J = 2.4, 8.7 Hz, 1H) , 8.88 (s, 1H) , 8.03 - 7.64 (m, 2H) , 7.39 - 7.23 (m, 2H) , 3.73 (s, 3H).<br>[M+H]$^+$: 417.4. |
| 118 | 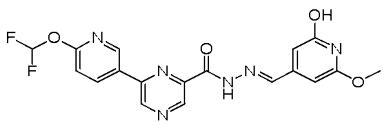 (*E*)-6-(6-(difluoromethoxy)pyridine-3-yl)-*N'*-((2-hydroxy-6-methoxypyridine-4-yl)methylene)pyrazine-2-carbohydrazide | $^1$H-NMR, 400 MHz, DMSO-d$_6$ $\delta$ = 12.29 - 12.20 (m, 1H) , 11.04 - 10.69 (m, 1H) , 9.62 (s, 1H) , 9.37 (d, J = 2.3 Hz, 1H) , 9.25 (s, 1H) , 9.01 - 8.91 (m, 1H) , 8.64 - 8.53 (m, 1H) , 7.84 (t, J = 72.5 Hz, 1H) , 7.35 (d, J = 8.5 Hz, 1H) , 6.73 - 6.29 (m, 2H) , 3.85 (s, 3H).<br>[M+H]$^+$: 417.3. |

(continued)

| Compound No | Structure | $^1$H-RMN / MS(m/z) [M+H]$^+$ |
|---|---|---|
| 119 | 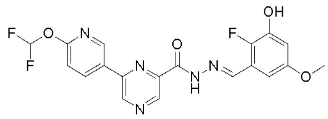 (*E*)-6-(6-(difluoromethoxy)pyridine-3-yl)-*N'*-(2-fluoro-3-hydroxy-5-methoxybenzylidene)pyrazine -2-carbohydrazide | $^1$H-NMR, 400 MHz, DMSO-d$_6$ $\delta$ = 3.76 (s, 3 H) 6.61 (dd, J=7.38, 3.13 Hz, 1 H) 6.75 - 6.97 (m, 1 H) 7.34 (d, J=8.75 Hz, 1 H) 7.83 (t, J=72.54 Hz, 1 H) 8.91 - 9.03 (m, 2 H) 9.24 (s, 1 H) 9.36 (d, J=2.25 Hz, 1 H) 9.60 (s, 1 H) 9.84 - 10.59 (m, 1 H) 12.22 (br s, 1 H). [M+H]$^+$: 434.3. |
| 120 | 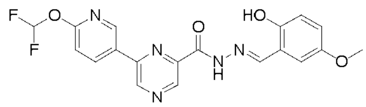 (*E*)-6-(6-(difluoromethoxy)pyridine-3-yl)-*N'*-(2-hydroxy-5-methoxybenzylidene)pyrazine -2-carbohydrazide | $^1$H-NMR, 400 MHz, DMSO-d$_6$ $\delta$ = 3.75 (s, 3 H) 6.84 - 7.00 (m, 2 H) 7.18 (d, J=2.88 Hz, 1 H) 7.35 (d, J=8.50 Hz, 1 H) 7.84 (t, J=72.54 Hz, 1 H) 8.86 - 9.03 (m, 2 H) 9.24 (s, 1 H) 9.36 (d, J=1.88 Hz, 1 H) 9.60 (s, 1 H) 10.54 (br s, 1 H) 12.30 (br s, 1 H). [M+H]$^+$: 416.4. |
| 121 | 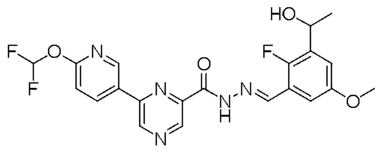 (*E*)-6-(6-(difluoromethoxy)pyridine-3-yl)-*N'*-(2-fluoro-3-(1-hydroxyethyl)-5-methoxybenzylidene)pyrazine-2-carbohydrazide | |
| 122 | 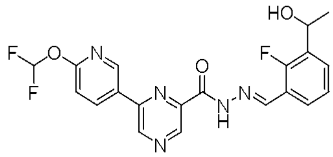 (*E*)-6-(6-(difluoromethoxy)pyridine-3-yl)-*N'*-(2-fluoro-3-(1-hydroxyethyl)benzylidene)pyrazine-2-carbohydrazide | |
| 123 | 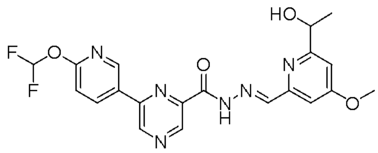 (*E*)-6-(6-(difluoromethoxy)pyridine-3-yl)-*N'*-((6-(1-hydroxyethyl)-4-methoxypyridine-2-yl)methylene) pyrazine-2-carbohydrazide | |
| 124 | 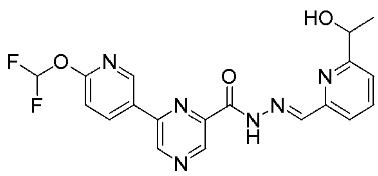 (*E*)-6-(6-(difluoromethoxy)pyridine-3-yl)-*N'*-((6-(1-hydroxyethyl)pyridine-2-yl)methylene)pyrazine-2-carbohydrazide | |

(continued)

| Compound No | Structure | $^1$H-RMN / MS(m/z) [M+H]$^+$ |
|---|---|---|
| 125 | 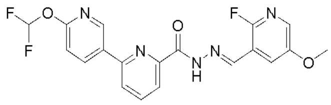 (*E*)-6'-(difluoromethoxy)-*N'*-((2-fluoro-5-methoxypyridine-3-yl) methylene)-[2,3'-bipyridine]-6-carbohydrazide | $^1$H-NMR, 400 MHz, DMSO-$d_6$ $\delta$ = 12.27 (s, 1H), 9.32 (d, J = 2.3 Hz, 1H), 8.95 - 8.89 (m, 2H), 8.39 - 8.34 (m, 1H), 8.23 - 8.11 (m, 2H), 8.05 - 7.60 (m, 3H), 7.30 (d, J = 8.6 Hz, 1H), 3.93 (s, 3H). [M+H]$^+$: 418.4. |
| 126 | 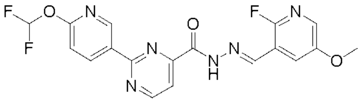 (*E*)-2-(6-(difluoromethoxy)pyridine-3-yl)-*N'*-((2-fluoro-5-methoxypyridine-3-yl)methylene)pyrimidine-4-carbohydrazide | $^1$H-NMR, 400 MHz, DMSO-$d_6$ $\delta$ = 12.58 - 12.28 (m, 1H), 9.55 (d, J = 2.0 Hz, 1H), 9.24 (s, 1H), 9.08 (dd, J = 2.4, 8.6 Hz, 1H), 8.94 (s, 1H), 8.13 - 7.65 (m, 4H), 7.34 (d, J = 8.8 Hz, 1H), 3.93 (s, 3H). [M+H]$^+$: 440.9. |
| 127 | 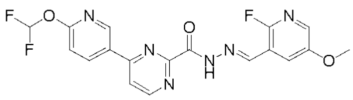 *(E)*-4-(6-(difluoromethoxy)pyridine-3-yl)-*N'*-((2-fluoro-5-methoxypyridine-3-yl)methylene)pyrimidine-2-carbohydrazide | $^1$H-NMR, 400 MHz, DMSO-$d_6$ $\delta$ = 12.49 (s, 1H), 9.32 (d, J = 2.3 Hz, 1H), 9.12 (d, J = 5.3 Hz, 1H), 8.91 (dd, J = 2.4, 8.7 Hz, 1H), 8.83 (s, 1H), 8.39 (d, J = 5.3 Hz, 1H), 8.01 -8.04 (m, 1H), 7.90 (dd, J = 3.1, 7.6 Hz, 1H), 7.61 - 7.87 (m, 1H), 7.35 (d, J = 8.6 Hz, 1H), 3.92 (s, 3H). [M+H]$^+$: 418.1. |
| 128 | 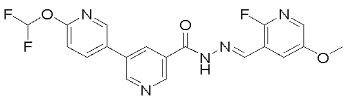 (*E*)-6'-(difluoromethoxy)-*N'*-((2-fluoro-5-methoxypyridine-3-yl) methylene)-[3,3'-bipyridine]-5-carbohydrazide | $^1$H-NMR, 400 MHz, DMSO-$d_6$ $\delta$ = 3.88 (br s, 3 H), 7.25 (d, J = 8.50 Hz, 1 H), 7.73 (t, J = 72.85 Hz, 2 H), 7.98 (br s, 1 H), 8.36 (dd, J = 8.57, 2.19 Hz, 1 H), 8.55 (br s, 2 H), 8.72 (d, J = 2.00 Hz, 1 H), 9.10 (br d, J = 12.01 Hz, 2 H) 12.11 (br s, 1 H). [M+H]$^+$: 417.1. |
| 129 | 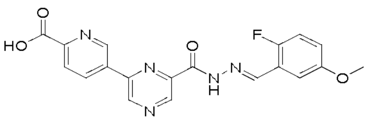 (*E*)-5-(6-(2-(2-(2-fluoro-5-methoxybenzylidene)hydrazin e-1-carbonyl)pyrazine-2-yl)picolinic acid | $^1$H-NMR, 400 MHz, DMSO-$d_6$ $\delta$ = 3.88 (br s, 3 H), 7.25 (d, J = 8.50 Hz, 1 H), 7.73 (t, J = 72.85 Hz, 2 H), 7.98 (br s, 1 H), 8.36 (dd, J = 8.57, 2.19 Hz, 1 H), 8.55 (br s, 2 H), 8.72 (d, J = 2.00 Hz, 1 H), 9.10 (br d, J = 12.01 Hz, 2 H) 12.11 (br s, 1 H). [M+H]$^+$: 417.1. |

(continued)

| Compound Nº | Structure | $^1$H-RMN / MS(m/z) [M+H]$^+$ |
|---|---|---|
| 130 | 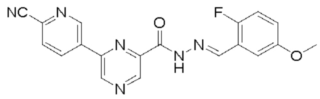 (*E*)-6-(6-cyanopyridine-3-yl)-*N'*-(2-fluoro-5-methoxybenzylidene) pyrazine -2-carbohydrazide | $^1$H-NMR, 400 MHz, DMSO-$d_6$ $\delta$ = 12.34 (s, 1H), 9.80 (d, J = 2.00 Hz, 1H), 9.68 (s, 1H), 9.33 (s, 1H), 9.06 (dd, J = 2.40, 8.20 Hz, 1H), 8.91 (s, 1H), 8.32 (dd, J = 0.80, 8.20 Hz, 1H), 7.44 (dd, J = 3.20, 5.60 Hz, 1H), 7.28 (t, J = 10.00 Hz, 1H), 7.09 (dd, J = 1.60, 6.60 Hz, 1H), 3.83 (s, 3H) . [M+H]$^+$: 377.1. |
| 131 | 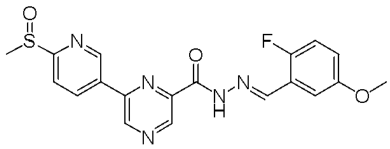 (*E*)-*N'*-(2-fluoro-5-methoxybenzylidene)-6-(6-(methylsulfinyl)pyridine-3-yl)pyrazine-2-carbohydrazide | $^1$H-NMR, 400 MHz, DMSO-$d_6$ $\delta$ = 12.31 (s, 1H), 9.73 (d, J = 1.60 Hz, 1H), 9.69 (s, 1H), 9.31 (s, 1H), 9.12 (dd, J = 2.40, 8.20 Hz, 1H), 8.98 (s, 1H), 8.12 (dd, J = 0.40, 8.00 Hz, 1H), 7.44 (dd, J = 3.20, 5.60 Hz, 1H), 7.32-7.27 (m, 1H), 7.13-7.09 (m, 1H), 3.83 (s, 3H), 2.90 (s, 3H). [M+H]$^+$: 414.0. |
| 132 | 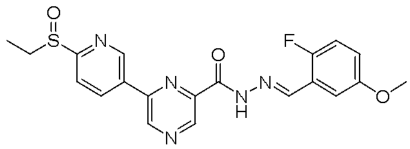 (*E*)-6-(6-(ethylsulfinyl)pyridine-3-yl)-*N'*-(2-fluoro-5-methoxybenzylidene)pyrazine -2-carbohydrazide | $^1$H-NMR, 400 MHz, DMSO-$d_6$ $\delta$ = 12.31 (s, 1H), 9.74 (d, J = 1.60 Hz, 1H), 9.68 (s, 1H), 9.31 (s, 1H), 9.10 (dd, J = 2.40, 8.20 Hz, 1H), 8.97 (s, 1H), 8.07 (d, J = 8.40 Hz, 1H), 7.44 (dd, J = 3.20, 5.60 Hz, 1H), 7.29 (t, J = 9.60 Hz, 1H), 7.12-7.08 (m, 1H), 3.83 (s, 3H), 3.28-3.23 (m, 1H), 3.00-2.91 (m, 1H), 1.07 (t, J = 2.00 Hz, 3H). [M+H]$^+$: 428.1. |
| 133 | 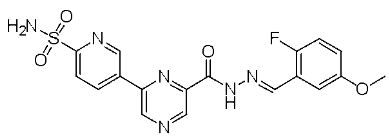 (*E*)-5-(6-(2-(2-fluoro-5-methoxybenzylidene)hydrazin e-1-carbonyl)pyrazine-2-yl)pyridine-2-sulfonamide | $^1$H-NMR, 400 MHz, DMSO-$d_6$ $\delta$ = 2.34 (s, 1H), 9.73 (dd, J = 0.40, 2.20 Hz, 1H), 9.71 (s, 1H), 9.07 (dd, J = 2.00, 8.20 Hz, 1H), 8.98 (d, J = Hz, 1H), 8.13 (dd, J = 0.80, 8.40 Hz, 1H), 7.65 (s, 2H), 7.44 (dd, J = 3.20, 5.60 Hz, 1H), 7.32-7.27 (m, 1H), 7.13-7.12 (m, 1H), 3.83 (s, 3H). [M+H]$^+$: 431.0. |
| 134 | 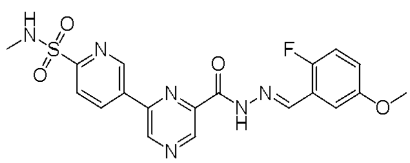 (*E*)-5-(6-(2-(2-fluoro-5-methoxybenzylidene)hydrazin e-1-carbonyl)pyrazine-2-yl)-*N*-methylpyridine-2-sulfonamide | $^1$H-NMR, 400 MHz, DMSO-$d_6$ $\delta$ = 12.34 (s, 1H), 9.77 (d, J = 1.60 Hz, 1H), 9.71 (s, 1H), 9.33 (s, 1H), 9.08 (dd, J = 2.00, 8.00 Hz, 1H), 8.98 (s, 1H), 8.14 (d, J = 8.40 Hz, 1H), 7.46-7.44 (m, 1H), 7.30 (t, J = 9.60 Hz, 1H), 7.13-7.10 (m, 1H), 3.83 (s, 3H), 2.63 (s, 3H). [M+H]$^+$: 445.2. |

(continued)

| Compound No | Structure | $^1$H-RMN / MS(m/z) [M+H]$^+$ |
|---|---|---|
| 135 | 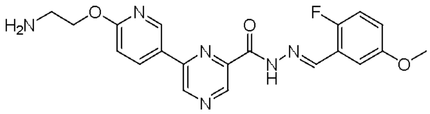 (*E*)-6-(6-(2-aminoethoxy)pyridine-3-yl)-*N'*-(2-fluoro-5-methoxy-benzylidene)pyrazine -2-carbohydrazide | $^1$H-NMR, 400 MHz, DMSO-$d_6$ $\delta$ = 12.24 (br s, 1H), 9.56 (s, 1H), 9.30 (d, J = 2.3 Hz, 1H), 9.20 (s, 1H), 8.97 (s, 1H), 8.83 (dd, J = 2.4, 8.7 Hz, 1H), 7.86 (br d, J = 4.1 Hz, 2H), 7.43 (dd, J = 3.3, 5.6 Hz, 1H), 7.29 (t, J = 9.5 Hz, 1H), 7.14 - 7.04 (m, 2H), 4.55 (t, J = 5.1 Hz, 2H), 3.82 (s, 3H), 3.29 - 3.26 (m, 2H). [M+H]$^+$: 411.1. |
| 136 | 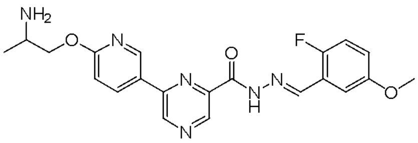 (*E*)-6-(6-(2-aminopropoxy)pyridine-3-yl)-*N'*-(2-fluoro-5-methoxy-benzylidene)pyrazine -2-carbohydrazide | $^1$H-NMR, 400 MHz, DMSO-$d_6$ $\delta$ = 9.48 (s, 1H), 9.22 (br s, 1H), 9.16 (s, 1H), 8.91 (s, 1H), 8.74 (br d, J = 7.9 Hz, 1H), 7.43 (br s, 1H), 7.26 (br t, J = 9.4 Hz, 1H), 7.12 - 6.98 (m, 2H), 4.12 (br d, J = 5.9 Hz, 2H), 3.82 (s, 3H), 3.25 - 3.11 (m, 1H), 1.07 (br d, J = 6.3 Hz, 3H). [M+H]$^+$: 425.1 |
| 137 | 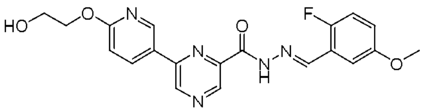 (*E*)-*N'*-(2-fluoro-5-methoxybenzylidene)-6-(6-(2-hydroxyethoxy) pyridine-3-yl)pyrazine-2-carbohydrazide | $^1$H-NMR, 400 MHz, DMSO-$d_6$ $\delta$ = 12.23 (s, 1H) , 9.55 (s, 1H) , 9.28 (d, J = 2.4 Hz, 1H) , 9.18 (s, 1H) , 8.98 (s, 1H) , 8.78 (dd, J = 2.5, 8.8 Hz, 1H) , 7.44 (dd, J = 3.3, 5.6 Hz, 1H) , 7.29 (t, J = 9.5 Hz, 1H) , 7.14 - 7.02 (m, 2H) , 4.90 (t, J = 5.5 Hz, 1H) , 4.40 (t, J = 5.1 Hz, 2H), 3.83 (s, 3H), 3.77 (q, J = 5.3 Hz, 2H). [M+H]$^+$: 412.1. |
| 138 | 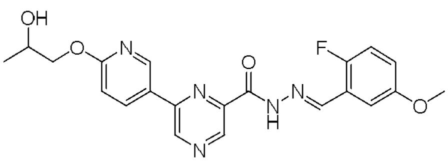 (*E*)-*N'*-(2-fluoro-5-methoxybenzylidene)-6-(6-(2-hydroxypropoxy)pyridine-3-yl)pyrazine-2-carbohydrazide | $^1$H-NMR, 400 MHz, DMSO-$d_6$ $\delta$ = 12.24 (s, 1H) , 9.55 (s, 1H) , 9.28 (d, J = 2.4 Hz, 1H) , 9.18 (s, 1H) , 8.98 (s, 1H) , 8.78 (dd, J = 2.4, 8.7 Hz, 1H) , 7.44 (dd, J = 3.2, 5.5 Hz, 1H) , 7.33 - 7.24 (m, 1H) , 7.15 - 7.02 (m, 2H) , 4.95 - 4.82 (m, 1H) , 4.31 - 4.16 (m, 2H) , 4.08 - 3.96 (m, 1H) , 3.83 (s, 3H), 1.18 (d, J = 6.3 Hz, 3H) . [M+H]$^+$: 426.1. |

(continued)

| Compound No | Structure | $^1$H-RMN / MS(m/z) [M+H]$^+$ |
|---|---|---|
| 139 | 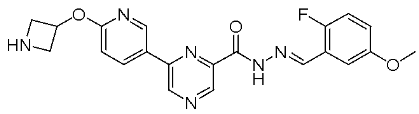 (*E*)-6-(6-(azetidine-3-iloxy)pyridine-3-yl)-*N*'-(2-fluoro-5-methoxy-benzylidene)pyrazine -2-carbohydrazide | $^1$H-NMR, 400 MHz, methanol-$d_4$ $\delta$ = 9.75 (s, 1H), 9.56 - 9.41 (m, 3H), 8.84 (s, 1H), 7.88 - 7.77 (m, 2H), 7.21 - 7.01 (m, 2H), 5.94 - 5.79 (m, 1H), 5.36 (dd, J = 9.8, 12.5 Hz, 1H), 5.00 (dd, J = 8.1, 12.6 Hz, 1H), 3.88 (s, 3H), 3.69 (d, J = 6.1 Hz, 2H). [M+H]$^+$: 423.1. |
| 140 | 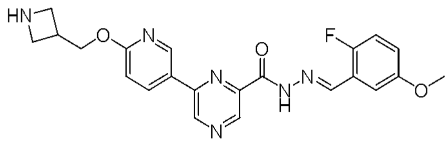 (*E*)-6-(6-(azetidine-3-ylmethoxy)pyridine-3-yl)-*N'*-(2-fluoro-5-methoxybenzylidene)pyrazine -2-carbohydrazide | $^1$H-NMR, 400 MHz, methanol-$d_4$ $\delta$ = 9.38 (s, 1H), 9.28 (s, 1H), 9.12 (d, J = 2.1 Hz, 1H), 8.84 (s, 1H), 8.73 (dd, J = 2.4, 8.3 Hz, 1H), 7.82 (dd, J = 3.1, 5.5 Hz, 1H), 7.14 - 7.07 (m, 2H), 7.06 - 7.00 (m, 1H), 4.58 (d, J = 5.0 Hz, 2H), 4.31 - 4.09 (m, 4H), 3.87 (s, 3H), 3.50 - 3.36 (m, 1H). [M+H]$^+$: 437.1. |
| 141 | 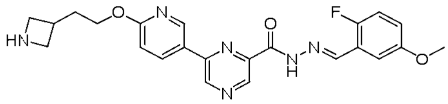 (*E*)-6-(6-(2-(azetidine-3-yl)ethoxy)pyridine-3-yl)-N'-(2-fluoro-5-methoxybenzylidene)pyrazine -2-carbohydrazide | $^1$H-NMR, 400 MHz, methanol-$d_4$ $\delta$ = 9.36 (s, 1H), 9.27 (s, 1H), 9.09 (d, J = 2.4 Hz, 1H), 8.84 (s, 1H), 8.68 (dd, J = 2.6, 8.7 Hz, 1H), 7.82 (dd, J = 3.2, 5.6 Hz, 1H), 7.16 - 7.07 (m, 1H), 7.04 (dd, J = 3.2, 4.3 Hz, 1H), 7.03 - 7.00 (m, 1H), 7.00 - 6.95 (m, 1H), 4.49 - 4.42 (m, 2H), 4.23 - 3.92 (m, 4H), 3.87 (s, 3H), 3.20 (td, J = 8.1, 16.1 Hz, 1H), 2.25 - 2.15 (m, 2H) . [M+H]$^+$: 451.2. |
| 142 | 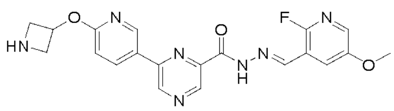 (*E*)-6-(6-(azetidine-3-iloxy)pyridine-3-yl)-*N*'-((2-fluoro-5-methoxypyridine-3-yl)methylene)pyrazine-2-carbohydrazide | $^1$H-NMR, 400 MHz, DMSO-$d_6$ $\delta$ = 12.49 (s, 1H), 9.88 (s, 1H), 9.66 (s, 1H), 9.52 - 9.44 (m, 1H), 9.36 (d, J = 1.8 Hz, 1H), 8.88 (s, 1H), 8.51 - 8.37 (m, 3H), 8.06 (br s, 1H), 7.98 - 7.89 (m, 2H), 5.84 - 5.71 (m, 1H), 5.31 - 5.14 (m, 1H), 4.97 - 4.82 (m, 1H), 3.92 (d, J = 1.8 Hz, 3H), 3.76 - 3.61 (m, 2H). [M+H]$^+$: 424.1 |

(continued)

| Compound No | Structure | $^1$H-RMN / MS(m/z) [M+H]$^+$ |
|---|---|---|
| 143 | 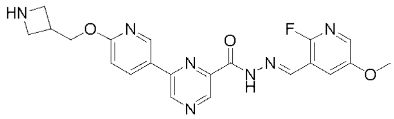 (*E*)-6-(6-(azetidine-3-ylmethoxy)pyridine-3-yl)-*N'*-((2-fluoro-5-methoxypyridine-3-yl)methylene)pyrazine-2-carbohydrazide | $^1$H-NMR, 400 MHz, methanol-$d_4$ $\delta$ = 9.38 (s, 1H), 9.27 (s, 1H), 9.12 (d, J = 2.3 Hz, 1H), 8.75 - 8.69 (m, 2H), 8.38 (dd, J = 3.0, 7.5 Hz, 1H), 7.93 (dd, J = 1.5, 2.9 Hz, 1H), 7.11 (d, J = 8.6 Hz, 1H), 4.58 (d, J = 4.9 Hz, 2H), 4.32 - 4.08 (m, 4H), 3.95 (s, 3H), 3.48 - 3.37 (m, 1H). [M+H]$^+$: 438.1. |
| 144 | 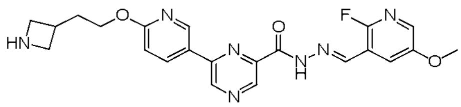 (*E*)-6-(6-(2-(azetidine-3-yl)ethoxy)pyridine-3-yl)-*N'*-((2-fluoro-5-methoxypyridine-3-yl)methylene)pyrazine-2-carbohydrazide | $^1$H-NMR, 400 MHz, DMSO-$d_6$ $\delta$ = 12.36 (s, 1H), 9.55 (s, 1H), 9.27 (d, J = 2.0 Hz, 1H), 9.19 (s, 1H), 8.89 (s, 1H), 8.78 (dd, J = 2.5, 8.8 Hz, 1H), 8.72 - 8.39 (m, 2H), 8.04 (dd, J = 1.8, 3.1 Hz, 1H), 7.90 (dd, J = 3.1, 7.6 Hz, 1H), 7.03 (d, J = 8.8 Hz, 1H), 4.37 (t, J = 6.2 Hz, 2H), 4.09 - 3.97 (m, 2H), 3.92 (s, 3H), 3.84 - 3.73 (m, 2H), 2.99 (td, J = 7.8, 15.7 Hz, 1H), 2.09 (q, J = 6.4 Hz, 2H). [M+H]$^+$: 452.2. |
| 145 | 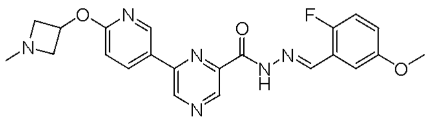 (*E*)-*N'*-(2-fluoro-5-methoxybenzylidene)-6-(6-((1-methylazetidine-3-yl)oxy)pyridine-3-yl)pyrazine-2-carbohydrazide | |
| 146 | 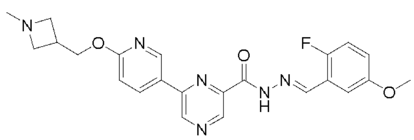 (*E*)-*N'*-(2-fluoro-5-methoxybenzylidene)-6-(6-((1-methylazetidine3-yl)methoxy)pyridine-3-yl)pyrazine-2-carbohydrazide | $^1$H-NMR, 400 MHz, methanol-$d_4$ $\delta$ = 9.39 (s, 1H), 9.34 (s, 1H), 9.10 (d, J = 1.9 Hz, 1H), 8.87 (dd, J = 2.1, 9.6 Hz, 1H), 8.82 (s, 1H), 7.81 (dd, J = 3.1, 5.6 Hz, 1H), 7.42 (d, J = 9.6 Hz, 1H), 7.15 - 7.08 (m, 1H), 7.07 - 7.01 (m, 1H), 4.68 - 4.61 (m, 1H), 4.36 (dd, J = 9.4, 13.2 Hz, 1H), 3.87 (s, 3H), 3.83 - 3.75 (m, 2H), 3.73 - 3.58 (m, 2H), 3.38 (s, 3H), 2.66 - 2.54 (m, 1H). [M+H]$^+$: 451.3. |

(continued)

| Compound No | Structure | $^{1}$H-RMN / MS(m/z) [M+H]$^{+}$ |
|---|---|---|
| 147 | 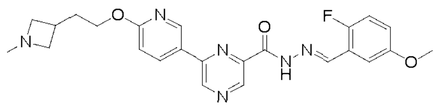 (*E*)-*N'*-(2-fluoro-5-methoxybenzylidene)-6-(6-(2-(1-methylazetidine-3-yl)ethoxy)pyridine-3-yl)pyrazine-2-carbohydrazide | $^{1}$H-NMR, 400 MHz, methanol-$d_4$ $\delta$ = 9.36 (s, 1H), 9.28 (s, 1H), 9.10 (d, J = 2.3 Hz, 1H), 8.84 (s, 1H), 8.68 (dd, J = 2.5, 8.8 Hz, 1H), 7.82 (dd, J = 3.1, 5.5 Hz, 1H), 7.15 - 7.08 (m, 1H), 7.04 (dt, J = 3.3, 4.5 Hz, 1H), 6.98 (d, J = 8.6 Hz, 1H), 4.46 (t, J = 5.8 Hz, 2H), 4.40 - 3.92 (m, 4H), 3.88 (s, 3H), 3.20 - 3.09 (m, 1H), 2.94 (s, 3H), 2.20 (br d, J = 6.8 Hz, 2H). [M+H]$^{+}$: 465.4. |
| 148 | 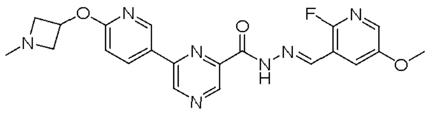 (*E*)-*N'*-((2-fluoro-5-methoxypyridine-3-yl)methylene)-6-(6-((1-methylazetidine-3-yl)oxy)pyridine-3-yl) pyrazine-2-carbohydrazide | |
| 149 | 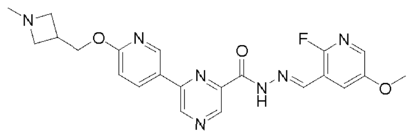 (*E*)-*N'*-((2-fluoro-5-methoxypyridine-3-yl)methylene)-6-(6-((1-methylazetidine-3-yl)methoxy)pyridine-3-yl)pyrazine-2-carbohydrazide | |
| 150 | 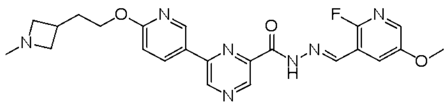 (*E*)-*N'*-((2-fluoro-5-methoxypyridin-3-yl)methylene)-6-(6-(2-(1-methylazetidine-3-yl)ethoxy)pyridine-3-yl)pyrazine-2-carbohydrazide | |
| 151 | 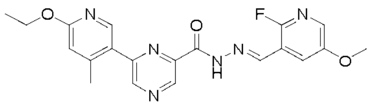 (*E*)-6-(6-ethoxy-4-methylpyridine-3-yl)-*N'*-((2-fluoro-5-methoxypyridine-3-yl)methylene)pyrazine-2-carbohydrazide | $^{1}$H-NMR, 400 MHz, DMSO-$d_6$ $\delta$ = 12.36 (s, 1H), 9.23 (s, 1H), 9.13 (s, 1H), 8.82 (s, 1H), 8.46 (s, 1H), 8.02 (br s, 1H), 7.88 (dd, J = 2.9, 7.4 Hz, 1H), 6.86 (s, 1H), 4.38 (q, J = 6.9 Hz, 2H), 3.91 (s, 3H), 2.45 (s, 3H), 1.35 (t, J = 7.0 Hz, 3H). [M+H]$^{+}$: 411.1. |
| 152 | 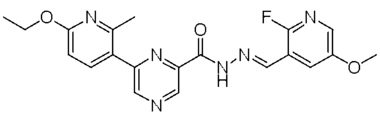 (*E*)-6-(6-ethoxy-2-methylpyridine-3-yl)-*N'*-((2-fluoro-5-methoxypyridine-3-yl)methylene)pyrazine-2-carbohydrazide | $^{1}$H-NMR, 400 MHz, DMSO-$d_6$ $\delta$ = 12.36 (br s, 1H), 9.21 (s, 1H), 9.12 (s, 1H), 8.82 (s, 1H), 8.05 (d, J = 8.4 Hz, 1H), 8.02 (dd, J = 1.7, 2.9 Hz, 1H), 7.88 (dd, J = 3.1, 7.6 Hz, 1H), 6.83 (d, J = 8.5 Hz, 1H), 4.39 (q, J = 7.0 Hz, 2H), 3.91 (s, 3H), 2.57 (s, 3H), 1.36 (t, J = 7.1 Hz, 3H). [M+H]$^{+}$: 411.1. |

(continued)

| Compound № | Structure | $^1$H-RMN / MS(m/z) [M+H]$^+$ |
|---|---|---|
| 153 | 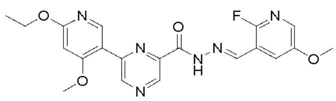 (*E*)-6-(6-ethoxy-4-methoxypyridine-3-yl)-*N*'-((2-fluoro-5-methoxypyridine-3-yl)methylene)pyrazine-2-carbohydrazide | $^1$H-NMR, 400 MHz, DMSO-d$_6$ $\delta$ = 12.31 (s, 1H), 9.32 (s, 1H), 9.14 (s, 1H), 8.93 (s, 1H), 8.85 (s, 1H), 8.02 (dd, J = 1.8, 3.0 Hz, 1H), 7.90 (dd, J = 3.1, 7.6 Hz, 1H), 6.60 (s, 1H), 4.41 (q, J = 7.1 Hz, 2H), 3.97 (s, 3H), 3.91 (s, 3H), 1.36 (t, J = 7.1 Hz, 3H) . [M+H]$^+$: 427.0. |
| 154 | 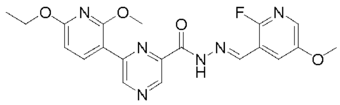 (*E*)-6-(6-ethoxy-2-methoxypyridine-3-yl)-*N*'-((2-fluoro-5-methoxypyridine-3-yl)methylene)pyrazine-2-carbohydrazide | $^1$H-NMR, 400 MHz, DMSO-d$_6$ $\delta$ = 12.38 - 12.29 (m, 1H), 9.49 (s, 1H), 9.10 (s, 1H), 8.88 - 8.83 (m, 1H), 8.78 (d, J = 8.4 Hz, 1H), 8.03 (dd, J = 1.8, 3.1 Hz, 1H), 7.90 (dd, J = 3.1, 7.8 Hz, 1H), 6.66 (d, J = 8.3 Hz, 1H), 4.44 (q, J = 7.1 Hz, 2H), 4.06 (s, 3H), 3.92 (s, 3H), 1.41 - 1.36 (m, 3H). [M+H]$^+$: 427.1. |
| 155 | 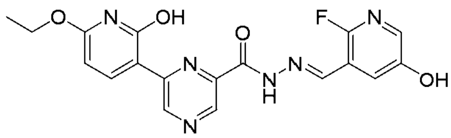 (*E*)-6-(6-ethoxy-2-hydroxypyridine-3-yl)-*N*'-((2-fluoro-5-hydroxypyridine-3-yl)methylene)pyrazine-2-carbohydrazide | |
| 156 | 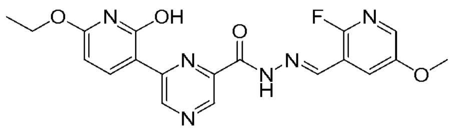 (*E*)-6-(6-ethoxy-2-hydroxypyridine-3-yl)-*N*'-((2-fluoro-5-methoxypyridine-3-yl)methylene)pyrazine-2-carbohydrazide | |
| 157 | 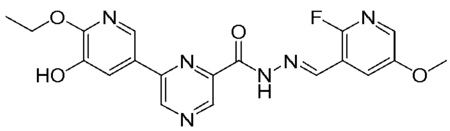 (*E*)-6-(6-ethoxy-5-hydroxypyridine-3-yl)-*N*'-((2-fluoro-5-methoxypyridine-3-yl)methylene)pyrazine-2-carbohydrazide | $^1$H-NMR, 400 MHz, DMSO-d$_6$ $\delta$ = 1.39 (t, J=7.00 Hz, 3 H) 3.92 (s, 3 H) 4.44 (q, J=6.96 Hz, 2 H) 7.90 (dd, J=7.63, 3.13 Hz, 1 H) 8.00 - 8.12 (m, 2 H) 8.69 (d, J=2.13 Hz, 1 H) 8.88 (s, 1 H) 9.14 (s, 1 H) 9.44 (s, 1 H) 9.51 - 9.93 (m, 1 H) 12.13 - 12.60 (m, 1 H). [M+H]$^+$: 413.3. |
| 158 | 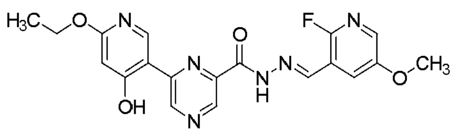 (*E*)-6-(6-ethoxy-4-hydroxypyridine-3-yl)-*N*'-((2-fluoro-5-methoxypyridine-3-yl)methylene)pyrazine-2-carbohydrazide | |

(continued)

| Compound Nο | Structure | $^1$H-RMN / MS(m/z) [M+H]$^+$ |
|---|---|---|
| 159 | 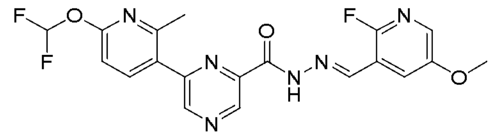 (*E*)-6-(6-(difluoromethoxy)-2-methylpyridine-3-yl)-*N*'-((2-fluoro-5-methoxypyridine-3-yl)methylene)pyrazine-2-carbohydrazide | $^1$H-NMR, 400 MHz, DMSO-$d_6$ $\delta$ = 12.39 (s, 1H), 9.28 (s, 1H), 9.19 (s, 1H), 8.82 (s, 1H), 8.26 (d, J = 8.40 Hz, 1H), 8.04-8.03 (m, 1H), 7.90-7.88 (m, 1H), 7.74 (s, 1H), 7.17-7.15 (m, 1H), 3.92 (s, 3H), 2.61 (s, 3H). [M+H]$^+$: 433.0. |
| 160 | 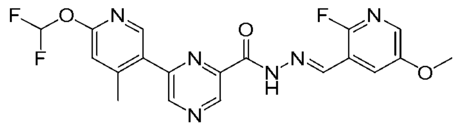 (*E*)-6-(6-(difluoromethoxy)-4-methylpyridine-3-yl)-*N*'-((2-fluoro-5-methoxypyridine-3-ylene)methylene)pyrazine-2-carbohydrazide | $^1$H-NMR, 400 MHz, DMSO-$d_6$ $\delta$ = 10.77 (s, 1H), 9.56 (s, 1H), 8.99 (s, 1H), 8.48 (s, 1H), 8.32 (s, 1H), 8.10-8.07 (m, 1H), 7.96-7.95 (m, 1H), 7.75-7.39 (m, 1H), 3.95 (s, 3H), 2.51 (s, 3H). [M+H]$^+$: 433.2. |
| 161 | 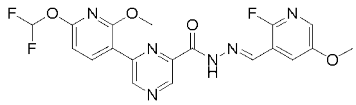 (*E*)-6-(6-(difluoromethoxy)-2-methoxypyridine-3-yl)-*N*'-((2-fluoro-5-methoxypyridine-3-yl)methylene) pyrazine-2-carbohydrazide | |
| 162 | 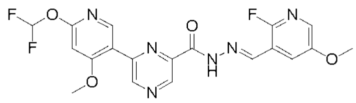 (*E*)-6-(6-(difluoromethoxy)-4-methoxypyridine-3-yl)-*N'*-((2-fluoro-5-methoxypyridine3-ylene)pyrazine-2-carbohydrazide | |
| 163 | 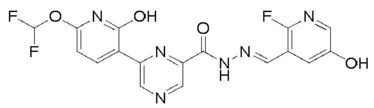 (*E*)-6-(6-(difluoromethoxy)-2-hydroxypyridine-3-yl)-*N*'-((2-fluoro-5-hydroxypyridine-3-yl)methylene) pyrazine-2-carbohydrazide | |
| 164 | 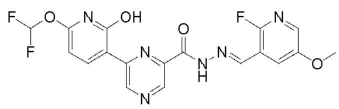 (*E*)-6-(6-(difluoromethoxy)-2-hydroxypyridine-3-yl)-*N*'-((2-fluoro-5-methoxypyridine-3-yl)methylene) pyrazine-2-carbohydrazide | |
| 165 | 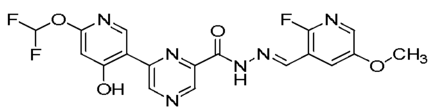 (*E*)-6-(6-(difluoromethoxy)-4-hydroxypyridine-3-yl)-*N*'-((2-fluoro-5-methoxypyridine-3-yl)methylene) pyrazine-2-carbohydrazide | |

(continued)

| Compound N<u>o</u> | Structure | $^1$H-RMN / MS(m/z) [M+H]$^+$ |
|---|---|---|
| 166 | 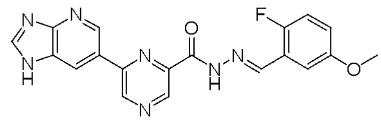<br>(*E*)-*N*'-(2-fluoro-5-methoxybenzylidene)-6-(1*H*-imidazo[4,5-*b*] pyridine-6-yl)pyrazine-2-carbohydrazide | $^1$H-NMR, 400 MHz, DMSO-d$_6$ $\delta$ = 13.37 (br s, 1H), 13.10 (br s, 1H), 12.45 - 12.26 (m, 1H), 9.67 (s, 1H), 9.53 - 9.39 (m, 1H), 9.27 - 9.14 (m, 1H), 9.05 - 8.90 (m, 1H), 8.67 - 8.51 (m, 1H), 7.44 (dd, J = 3.3, 5.6 Hz, 1H), 7.29 (t, J = 9.6 Hz, 1H), 7.10 (td, J = 3.8, 8.9 Hz, 1H), (s, 3H).<br>3.83 [M+H]$^+$: 392.1. |
| 167 | 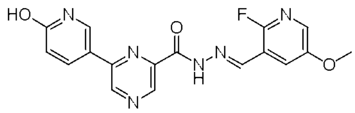<br>(*E*)-*N*'-((2-fluoro-5-methoxypyridine-3-yl)methylene)-6-(6-hydroxypyridine-3-yl)pyrazine-2-carbohydrazide | $^1$H-NMR, 400 MHz, DMSO-d$_6$ $\delta$ = 12.41 - 12.20 (m, 2H), 9.40 (s, 1H), 9.07 (s, 1H), 8.87 (s, 1H), 8.68 - 8.46 (m, 2H), 8.04 (br s, 1H), 7.91 (br d, J = 4.4 Hz, 1H), 6.55 (br d, J = 9.7 Hz, 1H), 3.92 (s, 3H).<br>[M+H]$^+$: 369.0. |
| 168 | 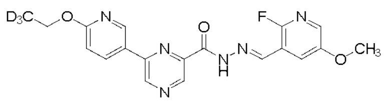<br>(*E*)-6-(6-(ethoxy-2,2,2-*d$_3$*)pyridine-3-yl)-*N'*-((2-fluoro-5-methoxypyridine-3-yl)methylene)pyrazine-2-carbohydrazide | $^1$H-NMR, 400 MHz, DMSO-d$_6$ $\delta$ = 12.33 (s, 1H), 9.53 (s, 1H), 9.27 (d, J = 2.0 Hz, 1H), 9.17 (s, 1H), 8.88 (s, 1H), 8.75 (dd, J = 2.3, 8.7 Hz, 1H), 8.03 (br s, 1H), 7.89 (dd, J = 2.9, 7.5 Hz, 1H), 7.01 (d, J = 8.7 Hz, 1H), 4.40 (s, 2H), 3.92 (s, 3H).<br>[M+H]$^+$: 400.2. |
| 169 | 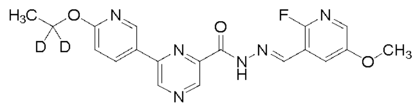<br>(*E*)-6-(6-(ethoxy-1,1-*d$_2$*)pyridine-3-yl)-*N'*-(((2-fluoro-5-methoxypyridine-3-yl)methylene)pyrazine-2-carbohydrazide | $^1$H-NMR, 400 MHz, DMSO-d$_6$ $\delta$ = 12.32 (s, 1H), 9.53 (s, 1H), 9.27 (d, J = 2.2 Hz, 1H), 9.16 (s, 1H), 8.88 (s, 1H), 8.74 (dd, J = 2.4, 8.7 Hz, 1H), 8.02 (br s, 1H), 7.89 (dd, J = 3.0, 7.5 Hz, 1H), 7.10 - 6.85 (m, 1H), 3.91 (s, 3H), 1.35 (s, 3H).<br>[M+H]$^+$: 399.1. |
| 170 | 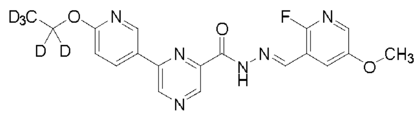<br>(*E*)-6-(6-(ethoxy-*d$_5$*)pyridine-3-yl)-*N'*-((2-fluoro-5-methoxypyridine-3-yl)methylene)pyrazine-2-carbohydrazide | $^1$H-NMR, 400 MHz, DMSO-d$_6$ $\delta$ = 12.33 (s, 1H), 9.54 (s, 1H), 9.28 (d, J = 1.8 Hz, 1H), 9.17 (s, 1H), 8.89 (s, 1H), 8.75 (dd, J = 2.5, 8.8 Hz, 1H), 8.03 (dd, J = 1.8, 2.9 Hz, 1H), 7.90 (dd, J = 3.1, 7.6 Hz, 1H), 7.04 - 6.98 (m, 1H), 3.92 (s, 3H).<br>[M+H]$^+$: 402.1. |

(continued)

| Compound Nº | Structure | $^1$H-RMN / MS(m/z) [M+H]$^+$ |
|---|---|---|
| 171 | 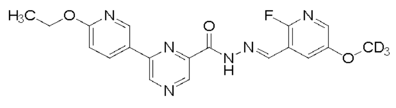 (*E*)-6-(6-ethoxypyridine-3-yl)-*N'*-((2-fluoro-5-(methoxy-$d_3$)pyridine-3-yl)methylene)pyrazine-2-carbohydrazide | $^1$H-NMR, 400 MHz, DMSO-$d_6$ $\delta$ = 12.32 (br s, 1H), 9.53 (s, 1H), 9.27 (d, J = 2.3 Hz, 1H), 9.16 (s, 1H), 8.88 (s, 1H), 8.75 (dd, J = 2.4, 8.7 Hz, 1H), 8.02 (br s, 1H), 7.89 (dd, J = 3.1, 7.5 Hz, 1H), 7.01 (d, J = 8.7 Hz, 1H), 4.41 (q, J = 7.0 Hz, 2H), 1.37 (t, J = 7.0 Hz, 3H).<br>[M+H]$^+$: 400.1. |
| 172 | 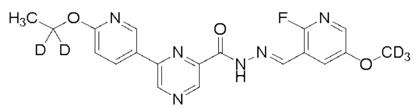 (*E*)-6-(6-(ethoxy-1,1-$d_2$)pyridine-3-yl)-*N'*-((2-fluoro-5-(methoxy-$d_3$)pyridine-3-yl)methylene)pyrazine-2-carbohydrazide | $^1$H-NMR, 400 MHz, DMSO-$d_6$ $\delta$ = 12.33 (br s, 1H), 9.53 (s, 1H), 9.27 (d, J = 2.1 Hz, 1H), 9.17 (s, 1H), 8.88 (s, 1H), 8.75 (dd, J = 2.6, 8.8 Hz, 1H), 8.02 (dd, J = 1.8, 3.1 Hz, 1H), 7.89 (dd, J = 3.1, 7.6 Hz, 1H), 7.01 (d, J = 8.7 Hz, 1H), 1.35 (s, 3H).<br>[M+H]$^+$: 402.1. |
| 173 | 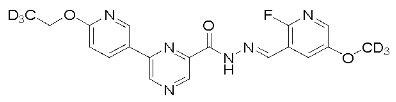 (*E*)-6-(6-(ethoxy-2,2,2-$d_3$)pyridine-3-yl)-*N'*-((2-fluoro-5-(methoxy-$d_3$)pyridine-3-yl)methylene)pyrazine-2-carbohydrazide | $^1$H-NMR, 400 MHz, DMSO-$d_6$ $\delta$ = 12.33 (br s, 1H), 9.53 (s, 1H), 9.27 (d, J = 2.4 Hz, 1H), 9.17 (s, 1H), 8.88 (s, 1H), 8.75 (dd, J = 2.5, 8.7 Hz, 1H), 8.02 (dd, J = 1.7, 3.0 Hz, 1H), 7.89 (dd, J = 3.1, 7.6 Hz, 1H), 7.01 (d, J = 8.7 Hz, 1H), 4.40 (s, 2H).<br>[M+H]$^+$: 403.1. |
| 174 | 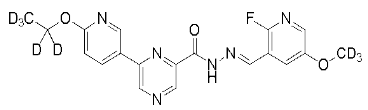 (*E*)-6-(6-(ethoxy-$d_5$)pyridine-3-yl)-*N'*-((2-fluoro-5-(methoxy-$d_3$) pyridine-3-yl)methylene)pyrazine-2-carbohydrazide | $^1$H-NMR, 400 MHz, DMSO-$d_6$ $\delta$ = 12.33 (br s, 1H), 9.53 (s, 1H), 9.27 (d, J = 1.8 Hz, 1H), 9.17 (s, 1H), 8.88 (s, 1H), 8.81 - 8.70 (m, 1H), 8.03 (dd, J = 1.7, 2.9 Hz, 1H), 7.89 (dd, J = 3.1, 7.6 Hz, 1H), 7.09 - 6.92 (m, 1H).<br>[M+H]$^+$: 405.2. |

**EXAMPLE 2. OBTAINING THE COMPOUNDS OF FORMULA IB COMPOUNDS 175 - 176**

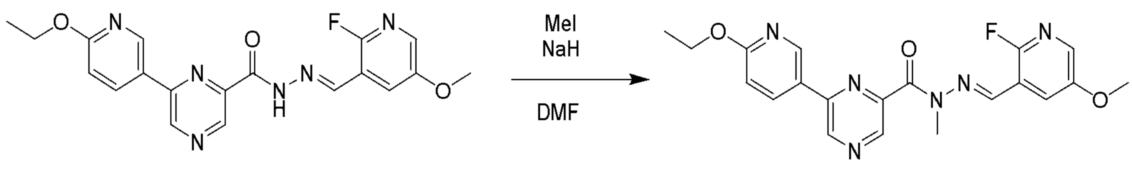


In a flask containing 3.57 mmol of the respective starting hydrazone, dissolved in 50 mL of DMF, 4.28 mmol of NaH (60% purity) at 0 °C were added. The mixture was heated to 20 °C for 2 hours, and subsequently 9.86 mmol of iodomethane was added. Subsequently, the reaction mixture was kept in agitation at 20 °C for 2 hours. After total consumption of the starting reagent (monitored by CCF), the reaction mixture was diluted with a solution of $NH_4Cl$. The product was filtered and used without subsequent purification. 440 mg (30% yield) of the compound of interest were

obtained.

**TABLE 19. COMPOUNDS OBTAINED FROM THE CORRESPONDING REAGENTS FOLLOWING THE METHOD DESCRIBED.**

| Compound No. | Structure | $^1$H-RMN / MS(m/z) $[M+H]^+$ |
|---|---|---|
| 175 | 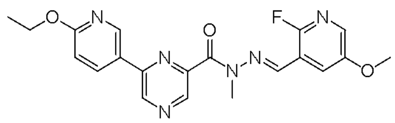 (*E*)-6-(6-ethoxypyridine-3-yl)-*N*'-((2-fluoro-5-methoxypyridine-3-yl)methylene)-*N*-methylpyrazine-2-carbohydrazide | $^1$H-NMR, 400 MHz, DMSO-$d_6$ $\delta$ = 9.36 (s, 1H), 8.95 (d, J = 2.3 Hz, 1H), 8.79 (s, 1H), 8.40 (dd, J = 2.4, 8.7 Hz, 1H), 7.98 (s, 1H), 7.89 (br d, J = 2.0 Hz, 1H), 7.17 (dd, J = 2.8, 7.3 Hz, 1H), 6.94 (d, J = 8.7 Hz, 1H), 4.38 (q, J = 7.1 Hz, 2H), 3.57 (d, J = 8.6 Hz, 6H), 1.34 (t, J = 7.0 Hz, 3H). $[M+H]^+$: 411.1. |
| 176 | 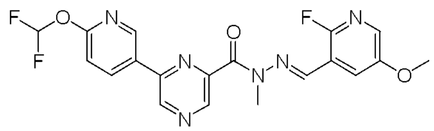 (*E*)-6-(6-(difluoromethoxy)pyridine-3-yl)-*N*'-((2-fluoro-5-methoxypyridine-3-yl)methylene)-*N*-methylpyrazine-2-carbohydrazide | $^1$H-NMR, 400 MHz, DMSO-$d_6$ $\delta$ = 9.43 (s, 1H), 9.03 (d, J = 2.3 Hz, 1H), 8.87 (s, 1H), 8.61 (dd, J = 2.4, 8.7 Hz, 1H), 8.02 - 7.58 (m, 3H), 7.26 (d, J = 8.8 Hz, 1H), 7.18 (dd, J = 2.9, 7.5 Hz, 1H), 3.58 (d, J = 2.4 Hz, 6H). $[M+H]^+$: 433.1. |

**EXAMPLE 3. TESTS *IN VITRO:***

Biological assays were performed on Chinese hamster ovary (CHO) cells that express human sodium channels Nav 1.8 or Nav 1.7 in a stable manner.

The experimental protocol *voltage-clamp* using the whole cell configuration was established on the automated ScreenPatch® 384P platform (SP384PE, Nanion Technologies, Livingston, NJ) and the results were recorded with a Nanion 384-well Patch Clamp chip (NPC) (Nanion Technologies, Livingston, NJ).

Formula I compounds were diluted in eight concentrations in the extracellular solution composed of saline solution, buffered with HEPES (mM): NaCl, 137; KCl, 4; $CaCl_2$, 3,8; $MgCl_2$, 1; HEPES, 10; Glucose, 10; pH 7.4. The extracellular solution is composed of (mM) CsCl, 50; CsF, 90; $MgCl_2$, 5; EGTA, 5; HEPES, 10; pH 7.2. The duration of exposure of each compound with cells expressing Nav 1.7 or Nav 1.8 was at least five minutes and the assays were performed at room temperature.

The measurements of the sodium currents of Nav 1.8 and Nav 1.7 were obtained using the voltage protocol described below.

A holding voltage of -100 mV was established followed by an inactivation voltage step at -40 mV for 8 seconds, followed by a step of -100 mV for 20 ms, followed by a 20 ms step for 10 mV for Nav 1.8 or 0 mV for Nav 1.7 (TP1A) before returning to the holding voltage of -100 mV.

The protocol was repeated at a frequency of 0.05 Hz and the amplitude of the current was quantified throughout the recording of the TP1A phase. The variation in the amplitude of the peak current was evaluated according to the formula described below, after exposing the cells expressing the channels to each concentration of the different compounds:

$$\% \text{ Block} = (1 - (I_{TP1A,molecule} / I_{TP1A,basal}) \times 100\%,$$

wherein $I_{TP1A,basal}$ and $I_{TP1A,molecule}$ represent the peaks of sodium current input into TP1A before exposure to the compound and in the presence of the compound, respectively.

The decrease in the amplitude of the peak current, after the exposure of the cells to the compounds, was used to calculate the percentage of relative blockage of the channels in relation to the positive control according to the formula below:

$$\% \text{ Block}' = 100\% - ((\% \text{ Block} - \% \text{ CP}) * (100\% / (\%V - \% \text{ CP})),$$

wherein %V and %CP represent the means of the values of the current block with a vehicle (DMSO) and positive controls, respectively. The sodium currents of the positive control were considered as 100%:

$$\% \text{ Block}' = (100\% / [1 + ([\text{Test}] / CI_{50})^{N}],$$

wherein [Test] represents the concentration of the molecule evaluated, $IC_{50}$ is the concentration of the compound that generates half of the maximum blockage, N is the Hill coefficient, and % Block' is the percentage of the sodium channel current (Nav 1.8 and Nav 1.7) blocked at each concentration of the molecule evaluated. Data were obtained by nonlinear regression (*nonlinear least squares*) with XLfit for Excel (Microsoft, Redmond, WA).

The compounds were tested in at least one assay to obtain the value of $CI_{50.}$ For compounds that were tested in two or more assays, the results are described as the means of the $CI_{50}$ values.

Table 20 shows the efficacy *in vitro* of selected compounds against Nav1.8 and Nav1.7. $CI_{50}$ values less than 500 nM are represented with the legend (+++); $CI_{50}$ values between 500 nM and 1000 nM are represented with the legend (++), $CI_{50}$ values greater than 1000 nM are demonstrated with the symbology (+).

**TABLE 20. $CI_{50}$ VALUES OF THE COMPOUNDS OF THIS INVENTION IN NAV 1.8 AND NAV 1.7 CHANNELS.**

| Compound № | Nav1.8 $IC_{50}$ (nM) | Nav1.7 $IC_{50}$ (nM) |
|---|---|---|
| 1 | +++ | ++ |
| 2 | +++ | ++ |
| 3 | +++ | ++ |
| 4 | +++ | + |
| 5 | +++ | +++ |
| 6 | +++ | + |
| 7 | +++ | + |
| 8 | ++ | + |
| 9 | +++ | + |
| 10 | + | + |
| 11 | +++ | + |
| 13 | + | + |
| 14 | + | + |
| 15 | +++ | + |
| 16 | + | + |
| 17 | +++ | ++ |
| 18 | +++ | + |
| 19 | ++ | + |
| 20 | + | + |
| 21 | +++ | ++ |
| 22 | ++ | + |
| 23 | +++ | + |
| 24 | + | + |
| 25 | ++ | + |
| 26 | +++ | + |
| 27 | +++ | + |
| 28 | ++ | + |
| 29 | + | + |
| 31 | + | + |
| 34 | + | + |

(continued)

| Compound № | Nav1.8 $IC_{50}$ (nM) | Nav1.7 $IC_{50}$ (nM) |
|---|---|---|
| 36 | +++ | + |
| 37 | + | + |
| 38 | +++ | + |
| 39 | + | + |
| 40 | + | + |
| 42 | + | + |
| 43 | + | + |
| 44 | + | + |
| 45 | ++ | + |
| 50 | +++ | + |
| 51 | + | + |
| 52 | +++ | + |
| 53 | + | + |
| 54 | +++ | + |
| 55 | + | + |
| 56 | ++ | + |
| 57 | + | + |
| 58 | + | + |
| 59 | + | + |
| 60 | ++ | + |
| 61 | + | + |
| 62 | ++ | + |
| 63 | ++ | + |
| 64 | ++ | + |
| 65 | + | + |
| 66 | + | + |
| 67 | + | + |
| 68 | + | + |
| 69 | + | + |
| 70 | + | + |
| 71 | +++ | ++ |
| 72 | +++ | + |
| 73 | ++ | + |
| 74 | + | + |
| 75 | + | + |
| 76 | + | + |
| 77 | +++ | +++ |
| 78 | +++ | +++ |
| 79 | +++ | +++ |

(continued)

| Compound № | Nav1.8 $IC_{50}$ (nM) | Nav1.7 $IC_{50}$ (nM) |
|---|---|---|
| 80 | +++ | + |
| 81 | +++ | ++ |
| 82 | +++ | +++ |
| 83 | +++ | + |
| 84 | + | + |
| 85 | +++ | + |
| 86 | + | + |
| 87 | + | + |
| 88 | +++ | +++ |
| 89 | +++ | + |
| 90 | +++ | + |
| 91 | +++ | + |
| 92 | +++ | +++ |
| 93 | +++ | + |
| 94 | +++ | +++ |
| 95 | +++ | + |
| 96 | +++ | + |
| 97 | + | + |
| 98 | + | + |
| 99 | + | + |
| 100 | +++ | ++ |
| 101 | +++ | + |
| 102 | +++ | + |
| 103 | +++ | + |
| 104 | +++ | + |
| 105 | + | + |
| 106 | ++ | + |
| 107 | + | + |
| 108 | + | + |
| 109 | ++ | + |
| 110 | +++ | + |
| 111 | + | + |
| 112 | ++ | + |
| 113 | ++ | + |
| 114 | + | + |
| 115 | + | + |
| 117 | + | + |
| 118 | + | + |
| 119 | ++ | + |

(continued)

| Compound № | Nav1.8 $IC_{50}$ (nM) | Nav1.7 $IC_{50}$ (nM) |
|---|---|---|
| 120 | + | + |
| 125 | +++ | +++ |
| 126 | ++ | + |
| 127 | ++ | + |
| 128 | + | + |
| 129 | ++ | + |
| 130 | + | + |
| 131 | ++ | + |
| 132 | + | + |
| 133 | + | + |
| 134 | + | + |
| 135 | + | + |
| 136 | + | + |
| 137 | + | + |
| 138 | + | + |
| 139 | + | + |
| 140 | + | + |
| 141 | + | + |
| 142 | + | + |
| 143 | + | + |
| 144 | + | + |
| 146 | + | + |
| 147 | + | + |
| 151 | +++ | + |
| 152 | +++ | + |
| 153 | + | + |
| 154 | +++ | +++ |
| 157 | ++ | + |
| 159 | +++ | + |
| 160 | +++ | +++ |
| 166 | + | + |
| 167 | + | + |
| 168 | +++ | + |
| 169 | +++ | + |
| 170 | +++ | + |
| 171 | +++ | + |
| 172 | +++ | + |
| 173 | +++ | + |
| 174 | +++ | + |

(continued)

| Compound № | Nav1.8 $IC_{50}$ (nM) | Nav1.7 $IC_{50}$ (nM) |
|---|---|---|
| 175 | + | + |
| 176 | + | + |

From these results, the blocking activity of Nav 1.7 and/or 1.8 is proven, whose application is readily performed by those skilled in the art in pharmaceutical compositions, which may comprise one or more of the aforementioned Formula I compounds, kits, in addition to uses in the treatment of pain-related pathologies.

In particular, these results indicate the possibility of using Formula (I) compounds in the preparation of drugs for the treatment of conditions such as peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia, and postsurgical neuralgia.

It should be understood that the embodiments described above are merely illustrative and that several modifications can be made thereto by a person skilled in the art without departing from the scope of this invention. Consequently, the present invention should not be regarded as being limited to the illustrative specifications described in this application.

## Claims

1. COMPOUND, **characterized by** being of Formula (I):

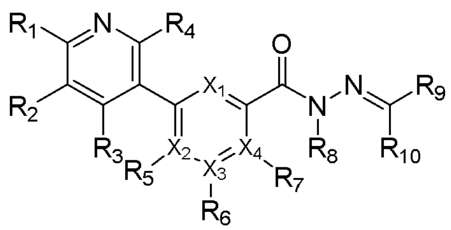

Formula (I)

or a pharmaceutically acceptable salt, hydrate, solvate and isomer thereof, wherein:

- $X_1$, $X_2$, $X_3$, and $X_4$ are independently selected from the group consisting of carbon, CH, or nitrogen, where:

  - when at least one of the substituents $X_2$, $X_3$ and $X_4$ is N or CH, at least one of $R_5$, $R_6$ and/or $R_7$, respectively, is null; and
  - when two of the substituents $X_1$, $X_2$, $X_3$ and $X_4$ are nitrogen, the other substituents must be carbon;
  - $R_1$, $R_2$, $R_3$, and $R_4$ are independently selected from the group consisting of hydrogen, halogen, hydroxy, hydroxy(alkyl $C_2$-$C_4$ linear or branched), alkyl $C_1$-$C_6$ linear or branched, alkoxy $C_1$-$C_6$ linear or branched, cycloalkoxy $C_3$-$C_7$, haloalkoxy $C_1$-$C_6$, haloalkyl $C_1$-$C_6$ hydroxy, hydroxyalkoxy $C_1$-$C_6$ linear or branched, difluoromethyl, trifluoromethyl, azetidinyloxy, azetidinylalkoxy $C_1$-$C_3$, *N*-alkyl($C_1$-$C_3$ linear) -azetidinyloxy, *N*-alkyl ($C_1$-$C_3$ linear)azetidinylalkoxy $C_1$-$C_3$, carboxyl, carboxyalkyl ester $C_1$-$C_3$, imidazole, nitrile, sulfinyl, alkylsulfinyl $C_1$-$C_6$, sulfonamide, alkylsulfonylamide $C_1$-$C_6$, *N,N*-(dialkyl)sulfonylamide $C_1$-$C_6$, sulfonyl, alkylsulfonyl $C_1$-$C_6$, amino (alkyl $C_2$-$C_4$ linear or branched) alkoxy, ($C_1$-$C_6$ alkylamino) alkoxy $C_1$-$C_6$, aminoalkoxy $C_1$-$C_6$, (-S-alkyl $C_1$-$C_6$) alkoxy $C_1$-$C_6$, carbamoyl, *N*-alkylcarbamoyl $C_1$-$C_6$, *N,N*-dialkylcarbamoyl $C_1$-$C_6$, alkoxy $C_1$-$C_2$ containing one or more isotopically enriched atoms such as hydrogen isotope $^2$H, but not limited to the same;
  - $R_5$ and $R_7$ are independently selected from the group consisting of hydrogen, halogen, $C_1$-$C_6$ linear or branched alkyl, amine, hydroxy, carbonyl, $C_1$-$C_6$ linear or branched alkoxy, haloalkyl $C_1$-$C_6$; wherein when $R_5$ is carbonyl, the ring of $X_2$ will have only two unsaturations and $X_2$ will be carbon;
  - $R_6$ is selected from hydrogen, halogen, or alkyl $C_1$-$C_2$;
  - $R_8$ and $R_{10}$ are independently selected from hydrogen and alkyl $C_1$-$C_6$ linear or branched;
  - $R_9$ is selected from the group consisting of aryl, pyridinyl or substituted pyridinyl, pyridone, cycloalkyl $C_3$-$C_7$ saturated or unsaturated, cycloalkyl $C_3$-$C_7$ saturated or unsaturated alkylsubstituted $C_1$-$C_5$, cycloalkyl $C_3$-$C_7$ saturated or unsaturated, halosubstituted or $R_{11}$ or $R_{17}$;

- $R_{11}$ is:

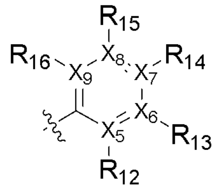

wherein:

- $X_5$, $X_6$, $X_7$, $X_8$, $X_9$ are independently selected from the group consisting of carbon, CH, or nitrogen, wherein:

- when at least one of the substituents $X_5$, $X_6$, $X_7$, $X_8$ and $X_9$ is N or CH, at least one of $R_{12}$, $R_{13}$, $R_{14}$, $R_{15}$ and/or $R_{16}$, respectively, is null;
- $R_{12}$, $R_{13}$, $R_{14}$, $R_{15}$, and $R_{16}$ are independently selected from the group consisting of hydrogen, halogen, hydroxy, hydroxy (alkyl $C_2$-$C_4$ linear or branched), alkyl $C_1$-$C_4$ linear or branched, $C_1$-$C_4$ linear or branched alkoxy, $C_3$-$C_7$cycloalkoxy, haloalkoxy, carboxyl, carboxyalkyl ester $C_1$-$C_3$, amine, *N*-alkyl $C_1$-$C_4$-amine, *N,N*-dialkyl $C_1$-$C_4$-amine, amino(alkyl $C_2$-$C_4$ linear or branched), sulfonamide, alkylsulfonylamide $C_1$-$C_6$, *N,N*-(dialkyl) sulfonylamide $C_1$-$C_6$, sulfonyl, alkyl sulfonyl $C_1$-$C_6$, sulfinyl, alkylsulfinyl $C_1$-$C_6$, azetidinyloxy, azetidinylalkoxy $C_1$-$C_3$, alkoxy $C_1$-$C_2$ linear or branched , hydroxyazetidinyl, oxo-dihydropyridinyl, oxo-dihydropyridinylalkoxy $C_1$-$C_4$ linear or branched, alkoxy $C_1$-$C_6$ containing one or more isotopically enriched atoms such as hydrogen isotope $^2$H but not being limited to the same;
- $R_{17}$ is selected from the group consisting of:

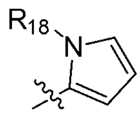
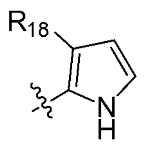
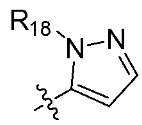
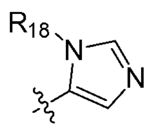
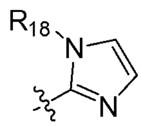
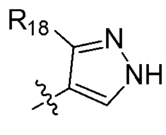
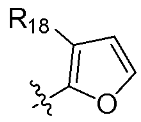
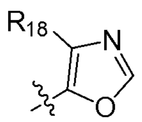
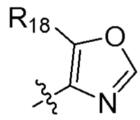
,

wherein:

- $R_{18}$ is selected from the group consisting of hydrogen, halogen, hydroxy, hydroxy(alkyl $C_2$-$C_4$ linear or branched), alkyl $C_1$-$C_4$ linear or branched, alkoxy $C_1$-$C_4$ linear or branched, $C_3$-$C_7$ cycloalkoxy, haloalkoxy, nitrile, carboxyl, carboxyalkyl ester $C_1$-$C_3$, amine, *N*-alkylamine, *N,N*-dialkylamine, amino (alkyl $C_2$-$C_4$ linear or branched), sulfonamide, alkylsulfonylamide $C_1$-$C_6$, *N,N*-(dialkyl)sulfonylamide $C_1$-$C_6$, sulfonyl, alkylsulfonyl $C_1$-$C_6$, sulfinyl, alkylsulfinyl $C_1$-$C_6$, azetidinyloxy, azetidinylalkoxy $C_1$-$C_3$, alkoxy $C_1$-$C_2$ linear or branched, hydroxyazetidinyl, alkoxy $C_1$-$C_6$ containing one or more isotopically enriched atoms, such as hydrogen isotopes $^2$H, but not limited to the same.

**2.** COMPOUND, in accordance with claim 1, **characterized by** being selected from the group consisting of:

(*E*)-6-(6-ethoxypyridine-3-yl)-*N*'-(2-fluoro-5-methoxybenzylidene)pyrazine-2-carbohydrazide (Compound 1);
(*E*)-6-(6-ethoxypyridine-3-yl)-*N*'-(4-fluoro-3-methoxybenzylidene)pyrazine-2-carbohydrazide (Compound 2);
(*E*)-*N*'-(2,4-difluoro-5-methoxybenzylidene)-6-(6-ethoxypyridine-3-yl)pyrazine-2-carbohydrazide (Compound 3);
(*E*)-N'-(5-ethoxy-2-fluorobenzylidene)-6-(6-ethoxypyridine-3-yl)pyrazine-2-carbohydrazide (Compound 4);
(*E*)-6-(6-ethoxypyridine-3-yl)-*N*'-(2-fluoro-5-isopropoxybenzylidene)pyrazine-2-carbohydrazide (Compound 5);

(*E*)-N'-(5-cyclopropoxy-2-fluorobenzylidene)-6-(6-ethoxypyridine-3-yl)pyrazine-2-carbohydrazide (Compound 6);
(*E*)-6-(6-ethoxypyridine-3-yl)-*N*'-((2-fluoro-5-methoxypyridine-3-yl)methylene) pyrazine-2-carbohydrazide (Compound 7);
(*E*)-6-(6-ethoxypyridine-3-yl)-*N*'-((5-fluoro-2-methoxypyridine-4-yl)methylene) pyrazine-2-carbohydrazide (Compound 8);
(*E*)-6-(6-ethoxypyridine-3-yl)-*N*'-((4-methoxypyridine-2-yl)methylene)pyrazine-2-carbohydrazide (Compound 9);
(*E*)-3-(2-(6-(6-(6-ethoxypyridine-3-yl)pyrazine-2-carbonyl)hydrazinylidene) methyl)-4-fluorobenzoic acid (Compound 10);
(*E*)-*N*'-(5-(dimethylamino)-2-fluorobenzylidene)-6-(6-ethoxypyridine-3-yl)pyrazine-2-carbohydrazide (Compound 11);
(*E*)-*N*'-((5-(dimethylamino)-2-fluoropyridine-3-yl)methylene)-6-(6-ethoxypyridine-3-yl)pyrazine-2-carbohydrazide (Compound 12);
(*E*)-N'-(5-(aminomethyl)-2-fluorobenzylidene)-6-(6-ethoxypyridine-3-yl)pyrazine-2-carbohydrazide (Compound 13);
(*E*)-3-((2-(6-(6-ethoxypyridine-3-yl)pyrazine-2-carbonyl)hydrazinoylidene)methyl)-4-fluorobenzenesulfonamide (Compound 14);
(*E*)-6-(6-ethoxypyridine-3-yl)-*N*'-((3-fluoro-6-methoxypyridine-2-yl)methylene) pyrazine-2-carbohydrazide (Compound 15);
(*E*)-6-(6-ethoxypyridine-3-yl)-*N*'-(2-fluoro-5-(methylsulfinyl)benzylidene)pyrazine-2-carbohydrazide (Compound 16);
(*E*)-6-(6-ethoxypyridine-3-yl)-*N*'-(5-methoxy-2-methylbenzylidene)pyrazine-2-carbohydrazide (Compound 17);
(*E*)-6-(6-ethoxypyridine-3-yl)-*N*'-((5-methoxypyridine-3-yl)methylene)pyrazine-2-carbohydrazide (Compound 18);
(*E*)-6-(6-ethoxypyridine-3-yl)-*N*'-(2-hydroxy-5-methoxybenzylidene)pyrazine-2-carbohydrazide (Compound 19);
(*E*)-*N*'-(5-(azetidine-3-yloxy)-2-fluorobenzylidene)-6-(6-ethoxypyridine-3-yl) pyrazine-2-carbohydrazide (Compound 20);
(*E*)-*N*'-(5-cyclobutoxy-2-fluorobenzylidene)-6-(6-ethoxypyridine-3-yl)pyrazine-2-carbohydrazide (Compound 21);
(*E*)-6-(6-ethoxypyridine-3-yl)-*N*'-(3-(1-hydroxyethyl)benzylidene)pyrazine-2-carbohydrazide (Compound 22);
(*E*)-6-(6-ethoxypyridine-3-yl)-*N*'-(2-fluoro-5-(1-hydroxyethyl)benzylidene) pyrazine-2-carbohydrazide (Compound 23);
(*E*)-6-(6-ethoxypyridine-3-yl)-*N*'-((5-(1-hydroxyethyl)pyridine-3-yl)methylene) pyrazine-2-carbohydrazide (Compound 24);
(*E*)-6-(6-ethoxypyridine-3-yl)-*N*'-((2-fluoro-5-(1-hydroxyethyl)pyridine-3-yl) methylene)pyrazine-2-carbohydrazide (Compound 25);
(*E*)-6-(6-ethoxypyridine-3-yl)-*N*'-(3-(1-hydroxyethyl)-5-methoxybenzylidene) pyrazine-2-carbohydrazide (Compound 26);
(*E*)-6-(6-ethoxypyridine-3-yl)-*N*'-((5-methoxy-2-methylpyridine-3-yl)methylene) pyrazine-2-carbohydrazide (Compound 27);
(*E*)-6-(6-ethoxypyridine-3-yl)-*N*'-((2-fluoro-5-hydroxypyridine-3-yl)methylene) pyrazine-2-carbohydrazide (Compound 28);
(*E*)-*N*'-(3-(1-aminoethyl)-5-methoxybenzylidene)-6-(6-ethoxypyridine-3-yl) pyrazine-2-carbohydrazide (Compound 29);
(*E*)-*N*'-(5-(1-aminoethyl)-2-fluorobenzyllidene)-6-(6-ethoxypyridine-3-yl)pyrazine-2-carbohydrazide (Compound 30);
(*E*)-6-(6-ethoxypyridine-3-yl)-*N*'-((4-(1-hydroxyethyl)pyridine-2-yl)methylene) pyrazine-2-carbohydrazide (Compound 31);
(*E*)-6-(6-ethoxypyridine-3-yl)-*N*'-((2-fluoro-5-(3-hydroxyzetidine-1-yl)pyridine-3-yl) methylene)pyrazine-2-carbohydrazide (Compound 32);
(*E*)-*N*'-((5-(azetidine-1-yl)-2-fluoropyridine-3-yl)methylene)-6-(6-ethoxypyridine-3-yl)pyrazine-2-carbohydrazide (Compound 33);
(*E*)-*N*'-((5-(azetidine-3-yloxy)-2-fluoropyridine-3-yl)methylene)-6-(6-ethoxypyridine-3-yl)pyrazine-2-carbohydrazide (Compound 34);
(*E*)-6-(6-ethoxypyridine-3-yl)-*N*'-((2-fluoro-5-((1-methylazetidine-3-yl)oxy)pyridine-3-yl)methylene)pyrazine-2-carbohydrazide (Compound 35);

(*E*)-*N'*-((5-cyclobutoxy-2-fluoropyridine-3-yl)methylene)-6-(6-ethoxypyridine-3-yl)pyrazine-2-carbohydrazide (Compound 36);
(*E*)-6-(6-ethoxypyridine-3-yl)-*N'*-(2-fluoro-5-(3-hydroxyzetidine-1-yl)benzylidene)pyrazine-2-carbohydrazide (Compound 37);
(*E*)-*N'*-(5-(azetidine1-yl)-2-fluorobenzylidene)-6-(6-ethoxypyridine-3-yl) pyrazine-2-carbohydrazide (Compound 38);
(*E*)-6-(6-ethoxypyridine-3-yl)-*N'*-(2-fluoro-5-((1-methylazetidine-3-yl)oxy)benzylidene) pyrazine-2-carbohydrazide (Compound 39);
(*E*)-6-(6-ethoxypyridine-3-yl)-*N'*-((6-hydroxypyridine-2-yl)methylene)pyrazine-2-carbohydrazide (Compound 40);
(*E*)-6-(6-ethoxypyridine-3-yl)-*N'*-((6-hydroxy-4-methoxypyridine-2-yl)methylene) pyrazine-2-carbohydrazide (Compound 41);
(*E*)-6-(6-ethoxypyridine-3-yl)-*N'*-((2-hydroxy-5-methoxypyridine-3-yl)methylene) pyrazine-2-carbohydrazide (Compound 42);
(*E*)-6-(6-ethoxypyridine-3-yl)-*N'*-((2-hydroxy-6-methoxypyridine-4-yl)methylene) pyrazine-2-carbohydrazide (Compound 43);
(*E*)-6-(6-ethoxypyridine-3-yl)-*N'*-(2-fluoro-3-hydroxy-5-methoxybenzylidene) pyrazine-2-carbohydrazide (Compound 44);
(*E*)-6-(6-ethoxypyridine-3-yl)-N'-(2-hydroxy-5-methoxybenzylidene)pyrazine-2-carbohydrazide (Compound 45);
(*E*)-6-(6-ethoxypyridine-3-yl)-*N'*-(2-fluoro-3-(1-hydroxyethyl)-5-methoxybenzylidene)pyrazine-2-carbohydrazide (Compound 46);
(*E*)-6-(6-ethoxypyridine-3-yl)-*N'*-(2-fluoro-3-(1-hydroxyethyl)benzylidene) pyrazine-2-carbohydrazide (Compound 47);
(*E*)-6-(6-ethoxypyridine-3-yl)-*N'*-((6-(1-hydroxyethyl)-4-methoxypyridine-2-yl) methylene)pyrazine-2-carbohydrazide (Compound 48);
(*E*)-6-(6-ethoxypyridine-3-yl)-*N'*-((6-(1-hydroxyethyl)pyridine-2-yl)methylene) pyrazine-2-carbohydrazide (Compound 49);
(*E*)-6-(6-ethoxypyridine-3-yl)-*N'*-((1-methyl-1*H*-pyrrole-2-yl)methylene)pyrazine-2-carbohydrazide (Compound 50);
(*E*)-6-(6-ethoxypyridine-3-yl)-*N'*-((1-methyl-1*H*-pyrazole-5-yl)methylene)pyrazine-2-carbohydrazide (Compound 51);
(*E*)-6-(6-ethoxypyridine-3-yl)-*N'*-((1-ethyl-1*H*-pyrazole-5-yl)methylene)pyrazine-2-carbohydrazide (Compound 52);
(*E*)-6-(6-ethoxypyridine-3-yl)-*N'*-((1-isopropyl-1*H*-imidazole-5-yl)methylene) pyrazine-2-carbohydrazide (Compound 53);
(*E*)-6-(6-ethoxypyridine-3-yl)-*N'*-((1-isopropyl-1*H*-pyrazole-5-yl)methylene) pyrazine-2-carbohydrazide (Compound 54);
(*E*)-6-(6-ethoxypyridine-3-yl)-*N'*-((3-methoxyfuran-2-yl)methylene)pyrazine-2-carbohydrazide (Compound 55);
(*E*)-6-(6-ethoxypyridine-3-yl)-*N'*-((3-methyl-1*H*-pyrrole-2-yl)methylene)pyrazine-2-carbohydrazide (Compound 56);
(*E*)-6-(6-ethoxypyridine-3-yl)-*N'*-((3-methyl-1*H*-pyrazole-4-yl)methylene)pyrazine-2-carbohydrazide (Compound 57);
(*E*)-6-(6-ethoxypyridine3-yl)-*N'*-((1-methyl-1*H*-imidazole-5-yl)methylene)pyrazine-2-carbohydrazide (Compound 58);
(*E*)-N'-((3-cyano-1H-pyrrole-2-yl)methylene)-6-(6-ethoxypyridine-3-yl)pyrazine-2-carbohydrazide (Compound 59);
(*E*)-6-(6-ethoxypyridine-3-yl)-*N'*-((1-ethyl-1*H*-imidazole-2-yl)methylene)pyrazine-2-carbohydrazide (Compound 60);
(*E*)-6-(6-ethoxypyridine-3-yl)-*N'*-((4-methyloxazole-5-yl)methylene)pyrazine-2-carbohydrazide (Compound 61);
(*E*)-6-(6-ethoxypyridine-3-yl)-*N'*-((5-methyloxazole-4-yl)methylene)pyrazine-2-carbohydrazide (Compound 62);
(*E*)-5-amino-6-(6-ethoxypyridine-3-yl)-*N'*-(2-fluoro-5-methoxybenzylidene) pyrazine-2-carbohydrazide (Compound 63);
(*E*)-3-amino-6-(6-ethoxypyridine-3-yl)-*N'*-(2-fluoro-5-methoxybenzylidene) pyrazine-2-carbohydrazide (Compound 64);
(*E*)-6-(6-ethoxypyridine-3-yl)-*N'*-((2-fluoro-5-methoxypyridine-3-yl)methylene)-3-hydroxypyrazine-2-carbohydrazide (Compound 65);
(*E*)-6-(6-ethoxypyridine-3-yl)-*N*'-(2-fluoro-5-methoxybenzylidene)-5-hydroxypyrazine-2-carbohydrazide (Compound 66);
(*E*)-6-(6-ethoxypyridine-3-yl)-*N*'-((2-fluoro-5-methoxypyridine-3-yl)methylene)-5-hydroxypyrazine-2-carbohydrazide (Compound 67);
(*E*)-6-(6-ethoxypyridine-3-yl)-*N*'-(2-fluoro-5-methoxybenzylidene)-3-hydroxypyrazine-2-carbohydrazide (Compound 68);
(*E*)-6-(6-ethoxypyridine-3-yl)-5-hydroxy-*N*'-((1-methyl-1H-pyrrole-2-yl)methylene)pyrazine-2-carbohydrazide (Compound 69);
(*E*)-6-(6-ethoxypyridine-3-yl)-3-hydroxy-*N*'-((1-methyl-1*H*-pyrrole-2-yl)methylene)pyrazine-2-carbohydrazide (Compound 70);
(*E*)-6'-ethoxy-*N'*-((2-fluoro-5-methoxypyridine-3-yl)methylene)-[2,3'-bipyridine]-6-carbohydrazide (Compound 71);
(*E*)-2-(6-ethoxypyridine-3-yl)-*N'*-((2-fluoro-5-methoxypyridine-3-yl)methylene) pyrimidine-4-carbohydrazide (Compound 72);
(*E*)-4-(6-ethoxypyridine-3-yl)-*N*'-((2-fluoro-5-methoxypyridine-3-yl)methylene) pyrimidine-2-carbohydrazide (Compound 73);
(*E*)-6'-ethoxy-*N'*-((2-fluoro-5-methoxypyridine-3-yl)methylene)-[3,3'-bipyridine]-5-carbohydrazide (Compound 74);
(*E*)-6-(6-ethoxypyridine-3-yl)-*N'*-((2-fluoro-5-methoxypyridine-3-yl)methylene)-4-methyl-5-oxo-4,5-dihydropyrazine-2-carbohydrazide (Compound 75);
(*E*)-6-(6-ethoxypyridine-3-yl)-N'-(2-fluoro-5-methoxybenzylidene)-4-methyl-5-oxo-4,5-dihydropyrazine-2-carbohydrazide (Compound 76);
(*E*)-6-(6-cyclopropoxypyridine-3-yl)-*N'*-(2-fluoro-5-methoxybenzylidene) pyrazine-2-carbohydrazide (Compound 77);
(*E*)-6-(6-cyclopropoxypyridine-3-yl)-*N'*-(2,4-difluoro-5-methoxybenzylidene) pyrazine-2-carbohydrazide (Compound 78);
(*E*)-6-(6-cyclopropoxypyridine-3-yl)-*N'*-(2-fluoro-5-isopropoxybenzylidene) pyrazine-2-carbohydrazide (Compound 79);
(*E*)-6-(6-cyclopropoxypyridine-3-yl)-*N'*-(5-ethoxy-2-fluorobenzylidene)pyrazine-2-carbohydrazide (Compound 80);
(*E*)-6-(6-cyclopropoxypyridine-3-yl)-*N'*-(4-fluoro-3-methoxybenzylidene) pyrazine-2-carbohydrazide (Compound 81);
(*E*)-*N*'-(5-cyclopropoxy-2-fluorobenzylidene)-6-(6-cyclopropoxypyridine-3-yl)pyrazine-2-carbohydrazide (Compound 82);
(*E*)-*N*'-(2-fluoro-5-methoxybenzylidene)-6-(6-(trifluoromethoxy)pyridine-3-yl)pyrazine-2-carbohydrazide (Compound 83);
(*E*)-*N*'-(pyridine-2-ylmethylene)-6-(6-(trifluoromethoxy)pyridine-3-yl)pyrazine-2-carbohydrazide (Compound 84);
(*E*)-*N'*-((4-methoxypyridine-2-yl)methylene)-6-(6-(trifluoromethoxy)pyridine-3-yl) pyrazine-2-carbohydrazide (Compound 85);
(*E*)-N'-(pyridine-3-ylmethylene)-6-(6-(trifluoromethoxy)pyridine-3-yl)pyrazine-2-carbohydrazide (Compound 86);
(*E*)-*N*'-(pyridine-4-ylmethylene)-6-(6-(trifluoromethoxy)pyridine-3-yl)pyrazine-2-carbohydrazide (Compound 87);
(*E*)-*N*'-(2-methylbenzylidene)-6-(6-(trifluoromethoxy)pyridine-3-yl)pyrazine-2-carbohydrazide (Compound 88);
(*E*)-*N*'-(2-fluorobenzylidene)-6-(6-(trifluoromethoxy)pyridine-3-yl)pyrazine-2-carbohydrazide (Compound 89);
(*E*)-*N*'-(3-methoxybenzylidene)-6-(6-(trifluoromethoxy)pyridine-3-yl)pyrazine-2-carbohydrazide (Compound 90);
(*E*)-*N*'-(5-ethoxy-2-fluorobenzylidene)-6-(6-(trifluoromethoxy)pyridine-3-yl) pyrazine-2-carbohydrazide (Compound 91);
(*E*)-*N'*-(2-fluoro-5-isopropoxybenzylidene)-6-(6-(trifluoromethoxy)pyridine-3-yl)pyrazine-2-carbohydrazide (Compound 92);
(*E*)-*N*'-(5-(dimethylamino)-2-fluorobenzylidene)-6-(6-(trifluoromethoxy)pyridine-3-yl)pyrazine-2-carbohydrazide (Compound 93);
(*E*)-*N*'-(5-cyclopropoxy-2-fluorobenzylidene)-6-(6-(trifluoromethoxy)pyridine-3-yl)pyrazine-2-carbohydrazide (Compound 94);
(*E*)-*N*'-((2-fluoro-5-methoxypyridine-3-yl)methylene)-6-(6-(trifluoromethoxy)pyridine-3-yl)pyrazine-2-carbohydrazide (Compound 95);
(*E*)-*N*'-((5-fluoro-2-methoxypyridine-4-yl)methylene)-6-(6-(trifluoromethoxy)pyridine-3-yl)pyrazine-2-carbohydrazide (Compound 96);
(*E*)-*N*'-((3-fluoro-6-methoxypyridine-2-yl)methylene)-6-(6-(trifluoromethoxy)pyridine-3-yl)pyrazine-2-carbohydrazide (Compound 97);
(*E*)-*N*'-(2-fluoro-5-methoxybenzylidene)-5-hydroxy-6-(6-(trifluoromethoxy)pyridine-3-yl)pyrazine-2-carbohydrazide (Compound 98);
(*E*)-*N*'-(2-fluoro-5-methoxybenzylidene)-3-hydroxy-6-(6-(trifluoromethoxy)pyridine-3-yl)pyrazine-2-carbohydrazide (Compound 99);
(*E*)-6-(6-(difluoromethoxy)pyridine-3-yl)-*N*'-(2-fluoro-5-methoxybenzylidene) pyrazine-2-carbohydrazide (Compound 100);
(*E*)-6-(6-(difluoromethoxy)pyridine-3-yl)-N'-((2-fluoro-5-methoxypyridine-3-yl)methylene)pyrazine-2-carbohydrazide (Compound 101);
(*E*)-6-(6-(difluoromethoxy)pyridine-3-yl)-N'-(5-methoxy-2-methylbenzylidene) pyrazine-2-carbohydrazide (Compound 102);
(*E*)-6-(6-(difluoromethoxy)pyridine-3-yl)-N'-((5-methoxy-2-methylpyridine-3-yl)methylene)pyrazine-2-carbohydrazide (Compound 103);
(*E*)-6-(6-(difluoromethoxy)pyridine-3-yl)-*N*'-(5-(dimethylamino)-2-fluorobenzylidene)pyrazine-2-carbohydrazide (Compound 104);
(*E*)-6-(6-(difluoromethoxy)pyridine-3-yl)-*N*'-((5-(dimethylamino)-2-fluoropyridine-3-yl)methylene)pyrazine-2-carbohydrazide (Compound 105);
(*E*)-6-(6-(difluoromethoxy)pyridine-3-yl)-*N*'-((5-fluoro-2-methoxypyridine-4-yl)methylene)pyrazine-2-carbohydrazide (Compound 106);
(*E*)-6-(6-(difluoromethoxy)pyridine-3-yl)-*N*'-((5-methoxypyridine-3-yl)methylene) pyrazine-2-carbohydrazide (Compound 107);
(*E*)-6-(6-(difluoromethoxy)pyridine-3-yl)-*N*'-(2-hydroxy-5-methoxybenzylidene) pyrazine-2-carbohydrazide (Compound 108);
(*E*)-6-(6-(difluoromethoxy)pyridine-3-yl)-*N*'-(3-(1-hydroxyethyl)benzylidene) pyrazine-2-carbohydrazide (Compound 109);
(*E*)-6-(6-(difluoromethoxy)pyridine-3-yl)-*N*'-(2-fluoro-5-(1-hydroxyethyl)benzylidene)pyrazine-2-carbohydrazide (Compound 110);
(*E*)-6-(6-(difluoromethoxy)pyridine-3-yl)-*N*'-((5-(1-hydroxyethyl)pyridine-3-yl)methylene)pyrazine-2-carbohydrazide (Compound 111);
(*E*)-6-(6-(difluoromethoxy)pyridine-3-yl)-*N*'-((2-fluoro-5-(1-hydroxyethyl)pyridine-3-yl)methylene)pyrazine-2-carbohydrazide (Compound 112);
(*E*)-6-(6-(difluoromethoxy)pyridine-3-yl)-N'-((2-fluoro-5-hydroxypyridine-3-yl)methylene)pyrazine-2-carbohydrazide (Compound 113);
(*E*)-6-(6-(difluoromethoxy)pyridine-3-yl)-*N*'-((4-(1-hydroxyethyl)pyridine-2-yl)methylene)pyrazine-2-carbohydrazide (Compound 114);
(*E*)-6-(6-(difluoromethoxy)pyridine-3-yl)-*N*'-((6-hydroxypyridine-2-yl)methylene) pyrazine-2-carbohydrazide (Compound 115);
(*E*)-6-(6-(difluoromethoxy)pyridine-3-yl)-*N*'-((6-hydroxy-4-methoxypyridine-2-yl)methylene)pyrazine-2-carbohydrazide (Compound 116);
(*E*)-6-(6-(difluoromethoxy)pyridine-3-yl)-*N*'-((2-hydroxy-5-methoxypyridine-3-yl)methylene)pyrazine-2-carbohydrazide (Compound 117);
(*E*)-6-(6-(difluoromethoxy)pyridine-3-yl)-*N*'-((2-hydroxy-6-methoxypyridine-4-yl)methylene)pyrazine-2-carbohydrazide (Compound 118);
(*E*)-6-(6-(difluoromethoxy)pyridine-3-yl)-*N*'-(2-fluoro-3-hydroxy-5-methoxybenzylidene)pyrazine-2-carbohydrazide (Compound 119);
(*E*)-6-(6-(difluoromethoxy)pyridine-3-yl)-*N*'-(2-hydroxy-5-methoxybenzylidene) pyrazine-2-carbohydrazide (Compound 120);
(*E*)-6-(6-(difluoromethoxy)pyridine-3-yl)-*N*'-(2-fluoro-3-(1-hydroxyethyl)-5-methoxybenzylidene)pyrazine-2-carbohydrazide (Compound 121);
(*E*)-6-(6-(difluoromethoxy)pyridine-3-yl)-*N*'-(2-fluoro-3-(1-hydroxyethyl)benzylidene)pyrazine-2-carbohydrazide (Compound 122);
(*E*)-6-(6-(difluoromethoxy)pyridine-3-yl)-*N*'-((6-(1-hydroxyethyl)-4-methoxypyridine-2-yl)methylene)pyrazine-2-carbohydrazide (Compound 123);
(*E*)-6-(6-(difluoromethoxy)pyridine-3-yl)-*N*'-((6-(1-hydroxyethyl)pyridine-2-yl)methylene)pyrazine-2-carbohydrazide (Compound 124);
(*E*)-6'-(difluoromethoxy)-*N*'-((2-fluoro-5-methoxypyridine-3-yl)methylene)-[2,3'-bipyridine]-6-carbohydrazide (Compound 125);
(*E*)-2-(6-(difluoromethoxy)pyridine-3-yl)-*N'*-((2-fluoro-5-methoxypyridine-3-yl)methylene)pyrimidine-4-carbohydrazide (Compound 126);
(*E*)-4-(6-(difluoromethoxy)pyridine-3-yl)-*N*'-((2-fluoro-5-methoxypyridine-3-yl)methylene)pyrimidine-2-carbohydrazide (Compound 127);
(*E*)-6'-(difluoromethoxy)-*N*'-((2-fluoro-5-methoxypyridine-3-yl)methylene)-[3,3'-bipyridine]-5-carbohydrazide (Compound 128);
(*E*)-5-(6-(2-(2-(2-fluoro-5-methoxybenzylidene)hydrazine-1-carbonyl)pyrazine-2-yl)picolinic acid (Compound 129);
(*E*)-6-(6-cyanopyridine-3-yl)-*N*'-(2-fluoro-5-methoxybenzylidene)pyrazine-2-carbohydrazide (Compound 130);
(*E*)-*N*'-(2-fluoro-5-methoxybenzylidene)-6-(6-(methylsulfinyl)pyridine-3-yl)pyrazine-2-carbohydrazide (Compound 131);
(*E*)-6-(6-(ethylsulfinyl)pyridine-3-yl)-*N*'-(2-fluoro-5-methoxybenzylidene)pyrazine-2-carbohydrazide (Compound 132);
(*E*)-5-(6-(2-(2-fluoro-5-methoxybenzylidene)hydrazine-1-carbonyl)pyrazine-2-yl)pyridine-2-sulfonamide (Compound 133);
(*E*)-5-(6-(2-(2-fluoro-5-methoxybenzylidene)hydrazine-1-carbonyl)pyrazine-2-yl)-N-methylpyridine-2-sulfonamide (Compound 134);
(*E*)-6-(6-(2-aminoethoxy)pyridine-3-yl)-*N*'-(2-fluoro-5-methoxybenzylidene) pyrazine-2-carbohydrazide (Compound 135);
(*E*)-6-(6-(2-aminopropoxy)pyridine-3-yl)-*N*'-(2-fluoro-5-methoxybenzylidene) pyrazine-2-carbohydrazide (Compound 136);
(*E*)-*N*'-(2-fluoro-5-methoxybenzylidene)-6-(6-(2-hydroxyethoxy)pyridine-3-yl)pyrazine-2-carbohydrazide (Compound 137);
(*E*)-*N*'-(2-fluoro-5-methoxybenzylidene)-6-(6-(2-hydroxypropoxy)pyridine-3-yl) pyrazine-2-carbohydrazide (Compound 138);
(E)-6-(6-(azetidin-3-yloxy)pyridine-3-yl)-*N'*-(2-fluoro-5-methoxybenzylidene) pyrazine-2-carbohydrazide (Compound 139);
(*E*)-6-(6-(azetidin-3-ylmethoxy)pyridine-3-yl)-*N'*-(2-fluoro-5-methoxybenzylidene)pyrazine-2-carbohydrazide (Compound 140);
(*E*)-6-(6-(2-(azetidine-3-yl)ethoxy)pyridine-3-yl)-N'-(2-fluoro-5-methoxybenzylidene)pyrazine-2-carbohydrazide (Compound 141);
(*E*)-6-(6-(azetidine-3-yloxy)pyridine-3-yl)-*N'*-((2-fluoro-5-methoxypyridine-3-yl)methylene)pyrazine-2-carbohydrazide (Compound 142);
(E)-6-(6-(azetidine-3-ylmethoxy)pyridine-3-yl)-N'-((2-fluoro-5-methoxypyridine-3-yl)methylene)pyrazine-2-carbohydrazide (Compound 143);
(*E*)-6-(6-(2-(azetidine-3-yl)ethoxy)pyridine-3-yl)-*N*'-((2-fluoro-5-methoxypyridine-3-yl)methylene)pyrazine-2-carbohydrazide (Compound 144);
(*E*)-*N*'-(2-fluoro-5-methoxybenzylidene)-6-(6-((1-methylazetidine-3-yl)oxy)pyridine-3-yl)pyrazine-2-carbohydrazide (Compound 145);
(*E*)-*N*'-(2-fluoro-5-methoxybenzylidene)-6-(6-((1-methylazetidine-3-yl)methoxy)pyridine-3-yl)pyrazine-2-carbohydrazide (Compound 146);
(*E*)-N'-(2-fluoro-5-methoxybenzylidene)-6-(6-(2-(1-methylazetidine-3-yl)ethoxy)pyridine-3-yl)pyrazine-2-carbohydrazide (Compound 147);
(*E*)-N'-((2-fluoro-5-methoxypyridine-3-yl)methylene)-6-(6-((1-methylazetidine-3-yl)oxy)pyridine-3-yl)pyrazine-2-carbohydrazide (Compound 148);
(*E*)-N'-((2-fluoro-5-methoxypyridine-3-yl)methylene)-6-(6-((1-methylazetidine-3-yl)methoxy)pyridine-3-yl)pyrazine-2-carbohydrazide (Compound 149);
(*E*)-*N*'-((2-fluoro-5-methoxypyridine-3-yl)methylene)-6-(6-(2-(1-methylazetidine-3-yl)ethoxy)pyridine-3-yl)pyrazine-2-carbohydrazide (Compound 150);
(*E*)-6-(6-ethoxy-4-methylpyridine-3-yl)-*N*'-((2-fluoro-5-methoxypyridine-3-yl) methylene)pyrazine-2-carbohydrazide (Compound 151);
(*E*)-6-(6-ethoxy-2-methylpyridine-3-yl)-*N*'-((2-fluoro-5-methoxypyridine-3-yl) methylene)pyrazine-2-carbohydrazide (Compound 152);
(*E*)-6-(6-ethoxy-4-methoxypyridine-3-yl)-*N*'-((2-fluoro-5-methoxypyridine-3-yl)methylene)pyrazine-2-carbohydrazide (Compound 153);

(*E*)-6-(6-ethoxy-2-methoxypyridine-3-yl)-*N*'-((2-fluoro-5-methoxypyridine-3-yl)methylene)pyrazine-2-carbohydrazide (Compound 154);
(*E*)-6-(6-ethoxy-2-hydroxypyridine-3-yl)-*N*'-((2-fluoro-5-hydroxypyridine-3-yl)methylene)pyrazine-2-carbohydrazide (Compound 155);
(*E*)-6-(6-ethoxy-2-hydroxypyridine-3-yl)-*N*'-((2-fluoro-5-methoxypyridine-3-yl) methylene)pyrazine-2-carbohydrazide (Compound 156);
(*E*)-6-(6-ethoxy-5-hydroxypyridine-3-yl)-*N*'-((2-fluoro-5-methoxypyridine-3-yl) methylene)pyrazine-2-carbohydrazide (Compound 157);
(*E*)-6-(6-ethoxy-4-hydroxypyridine-3-yl)-*N*'-((2-fluoro-5-methoxypyridine-3-yl)methylene)pyrazine-2-carbohydrazide (Compound 158);
(*E*)-6-(6-(difluoromethoxy)-2-methylpyridine-3-yl)-*N*'-((2-fluoro-5-methoxypyridine-3-yl)methylene)pyrazine-2-carbohydrazide (Compound 159);
(*E*)-6-(6-(difluoromethoxy)-4-methylpyridine-3-yl)-*N*'-((2-fluoro-5-methoxypyridine-3-yl)methylene)pyrazine-2-carbohydrazide (Compound 160);
(*E*)-6-(6-(difluoromethoxy)-2-methoxypyridine-3-yl)-*N*'-((2-fluoro-5-methoxypyridine-3-yl)methylene)pyrazine-2-carbohydrazide (Compound 161);
(*E*)-6-(6-(difluoromethoxy)-4-methoxypyridine-3-yl)-*N*'-((2-fluoro-5-methoxypyridine-3-yl)methylene)pyrazine-2-carbohydrazide (Compound 162);
(*E*)-6-(6-(difluoromethoxy)-2-hydroxypyridine-3-yl)-*N*'-((2-fluoro-5-hydroxypyridine-3-yl)methylene)pyrazine-2-carbohydrazide (Compound 163);
(*E*)-6-(6-(difluoromethoxy)-2-hydroxypyridine-3-yl)-*N*'-((2-fluoro-5-methoxypyridine-3-yl)methylene)pyrazine-2-carbohydrazide (Compound 164);
(*E*)-6-(6-(difluoromethoxy)-4-hydroxypyridine-3-yl)-*N*'-((2-fluoro-5-methoxypyridine-3-yl) methylene)pyrazine-2-carbohydrazide (Compound 165);
(*E*)-*N'*-(2-fluoro-5-methoxybenzylidene)-6-(1*H*-imidazo[4,5-b]pyridine-6-yl) pyrazine-2-carbohydrazide (Compound 166);
(*E*)-*N*'-((2-fluoro-5-methoxypyridine-3-yl)methylene)-6-(6-hydroxypyridine-3-yl) pyrazine-2-carbohydrazide(Compound 167);
(*E*)-6-(6-(ethoxy-2,2,2-d3)pyridine3-yl)-*N'*-((2-fluoro-5-methoxypyridine-3-yl)methylene)pyrazine-2-carbohydrazide (Compound 168);
(*E*)-6-(6-(ethoxy-1,1-d2)pyridine-3-yl)-*N'*-((2-fluoro-5-methoxypyridine-3-yl)methylene)pyrazine-2-carbohydrazide (Compound 169);
(*E*)-6-(6-(ethoxy-d5)pyridine-3-yl)-*N'*-((2-fluoro-5-methoxypyridine-3-yl)methylene) pyrazine-2-carbohydrazide (Compound 170);
(*E*)-6-(6-ethoxypyridine-3-yl)-*N*'-((2-fluoro-5-(methoxy-d3)pyridine-3-yl)methylene) pyrazine-2-carbohydrazide (Compound 171);
(*E*)-6-(6-(ethoxy-1,1-d2)pyridine-3-yl)-*N'*-((2-fluoro-5-(methoxy-d3)pyridine-3-yl) methylene) pyrazine-2-carbohydrazide (Compound 172);
(*E*)-6-(6-(ethoxy-2,2,2-d3)pyridine-3-yl)-*N'*-((2-fluoro-5-(methoxy-d3)pyridine-3-yl)methylene)pyrazine-2-carbohydrazide (Compound 173);
(*E*)-6-(6-(ethoxy-d5)pyridine-3-yl)-*N'*-((2-fluoro-5-(methoxy-d3)pyridine3-yl) methylene)pyrazine-2-carbohydrazide (Compound 174);
(*E*)-6-(6-ethoxypyridine-3-yl)-*N*'-((2-fluoro-5-methoxypyridine-3-yl)methylene)-*N*-methylpyrazine-2-carbohydrazide (Compound 175); and
(*E*)-6-(6-(difluoromethoxy)pyridine-3-yl)-*N*'-((2-fluoro-5-methoxypyridine-3-yl)methylene)-*N*-methylpyrazine-2-carbohydrazide (Compound 176).

**3.** COMPOUND, according to any of claims 1 or 2, **characterized by** being a blocker of voltage-gated sodium channels Nav 1.7 and/or Nav 1.8.

**4.** PHARMACEUTICAL COMPOSITION, **characterized by** comprising a therapeutically effective amount of one or more compounds of Formula (I) or of a pharmaceutically acceptable salt, hydrate, solvate, and isomer thereof, as defined in any of claims 1 through 3, and one or more pharmaceutically acceptable excipients.

**5.** PHARMACEUTICAL COMPOSITION, according to claim 4, **characterized by** being formulated as an oral, sublingual, nasal, parenteral, injectable, submuscular, topical, transdermal, ocular, or rectal composition.

**6.** USE OF FORMULA (I) COMPOUND(S), as defined in any of claims 1 through 3, **characterized by** being to prepare a

drug to treat pathologies related to neuropathic pain.

**7.** USE, according to claim 6, **characterized by** said pathologies being selected from the group consisting of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia, and postsurgical neuralgia.

**8.** METHOD OF TREATMENT, PREVENTION, RELIEF, SUPPRESSION AND/OR CONTROL of pathologies related to neuropathic pain, **characterized by the** administration of an effective amount of at least one compound of Formula (I) or a salt, hydrate, solvate and pharmaceutically acceptable isomer thereof, as defined in any of claims 1 to 3.

**9.** METHOD, according to claim 8, **characterized by** being for the treatment or prophylaxis of peripheral neuropathic pain, chemotherapy-induced neuropathy, complex regional pain, neuropathy related to viral infection, neuropathy secondary to tumor infiltration, diabetic neuropathy, phantom limb pain, postherpetic neuralgia, trigeminal neuralgia, and postsurgical neuralgia.

**10.** METHOD, according to any of claims 8 or 9, **characterized by** the administration of at least one compound of Formula (I) being selected from the group comprising oral, sublingual, nasal, parenteral, injectable, submuscular, topical, transdermal, ocular, and rectal routes.

**11.** PROCESS OF OBTAINING GENERAL FORMULA (I) COMPOUND, as defined in any of claims 1 through 3, **characterized by** comprising the steps:

(a) formation of the Formula III intermediate:

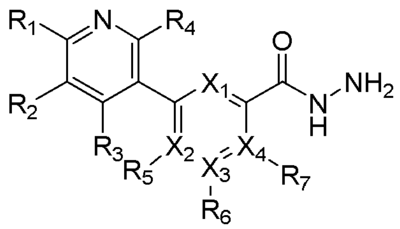

Formula III

from the hydrazinolysis reaction of a Formula IV intermediate:

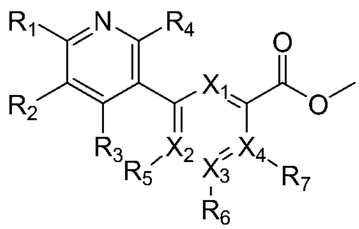

Formula IV

(b) obtaining a Formula I compound wherein $R_6$ is hydrogen;

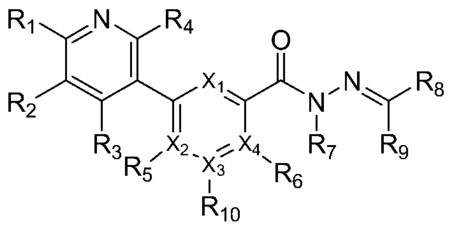

Formula (I)

from the condensation of Formula II intermediates:

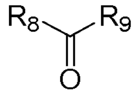

Formula II

and Formula III, with or without the presence of a catalyst and a suitable solvent, wherein:

- $X_1$, $X_2$, $X_3$, and $X_4$ are independently selected from the group consisting of carbon, CH, or nitrogen, where:

  - when at least one of the substituents $X_2$, $X_3$ and $X_4$ is N or CH, at least one of $R_5$, $R_6$ and/or $R_7$, respectively, is null;
  - $R_1$, $R_2$, $R_3$, and $R_4$ are independently selected from the group consisting of hydrogen, halogen, hydroxy, hydroxy(alkyl $C_2$-$C_4$ linear or branched), alkyl $C_1$-$C_6$ linear or branched, alkoxy $C_1$-$C_6$ linear or branched, cycloalkoxy $C_3$-$C_7$, haloalkoxy $C_1$-$C_6$, haloalkyl $C_1$-$C_6$ hydroxy, hydroxyalkoxy $C_1$-$C_6$ linear or branched, difluoromethyl, trifluoromethyl, azetidinyloxy, azetidinylalkoxy $C_1$-$C_3$, *N*-alkyl($C_1$-$C_3$ linear) -azetidinyloxy, N-alkyl ($C_1$-$C_3$ linear)azetidinylalkoxy $C_1$-$C_3$, carboxyl, carboxyalkyl ester $C_1$-$C_3$, imidazole, nitrile, sulfinyl, alkylsulfinyl $C_1$-$C_6$, sulfonamide, alkylsulfonylamide $C_1$-$C_6$, *N,N*-(dialkyl)sulfonylamide $C_1$-$C_6$, sulfonyl, alkylsulfonyl $C_1$-$C_6$, amino (alkyl $C_2$-$C_4$ linear or branched) alkoxy, ($C_1$-$C_6$ alkyl amino) alkoxy $C_1$-$C_6$, (-S-alkyl $C_1$-$C_6$) alkoxy $C_1$-$C_6$, carbamoyl, N-alkylcarbamoyl $C_1$-$C_6$, N,N-dialkylcarbamoyl $C_1$-$C_6$, alkoxy $C_1$-$C_2$ containing one or more isotopically enriched atoms, such as hydrogen isotope $^2$H, but not limited to the same;
  - $R_5$ and $R_7$ are independently selected from the group consisting of hydrogen, halogen, $C_1$-$C_6$ linear or branched alkyl, amine, hydroxy, carbonyl, $C_1$-$C_6$ linear or branched alkoxy, haloalkyl $C_1$-$C_6$; wherein when $R_5$ is carbonyl, the ring of $X_2$ will have only two unsaturations and $X_2$ will be carbon;
  - $R_6$ is selected from hydrogen, halogen, or alkyl $C_1$-$C_2$;
  - $R_8$ and $R_{10}$ are independently selected from hydrogen and alkyl $C_1$-$C_6$ linear or branched;
  - $R_9$ is selected from the group consisting of aryl, pyridinyl or substituted pyridinyl, pyridone, cycloalkyl $C_3$-$C_7$ saturated or unsaturated, cycloalkyl $C_3$-$C_7$ saturated or unsaturated alkyl substituted $C_1$-$C_5$, cycloalkyl $C_3$-$C_7$ saturated or unsaturated, halosubstituted or $R_{11}$ or $R_{17}$;
  - $R_{11}$ is:

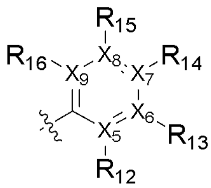

  wherein:

  - $X_5$, $X_6$, $X_7$, $X_8$, $X_9$ are independently selected from the group consisting of carbon, CH, or nitrogen, wherein:

    - when at least one of the substituents $X_5$, $X_6$, $X_7$, $X_8$ and $X_9$ is N or CH, at least one of $R_{12}$, $R_{13}$, $R_{14}$, $R_{15}$ and/or $R_{16}$, respectively, is null;
    - $R_{12}$, $R_{13}$, $R_{14}$, $R_{15}$ , and $R_{16}$ are independently selected from the group consisting of hydrogen, halogen, hydroxy, hydroxy (alkyl $C_2$-$C_4$ linear or branched), alkyl $C_1$-$C_4$ linear or branched, alcoxy $C_1$-$C_4$ linear or branched, $C_3$-$C_7$ cycloalkoxy, haloalkoxy, carboxyl, carboxyalkyl ester $C_1$-$C_3$, amine, N-alkylamine, *N, N*-dialkylamine, amino (alkyl $C_2$-$C_4$ linear or branched), sulfonamide, alkylsulfonylamide $C_1$-$C_6$, *N,N*-(dialkyl)sulfonylamide $C_1$-$C_6$, sulfonyl, alkylsulfonyl $C_1$-$C_6$, sulfinyl, alkylsulfinyl $C_1$-$C_6$, azetidinyloxy, azetidinylalkoxy $C_1$-$C_3$, alkoxy $C_1$-$C_2$ linear or branched, hydroxyazetidinyl, oxo-dihydropyridinyl, oxo-dihydropyridinylalkoxy $C_1$-$C_4$ linear or branched, alkoxy $C_1$-$C_6$ containing one or more isotopically enriched atoms such as hydrogen isotope $^2$H, but not limited to the same;
    - $R_{17}$ is selected from the group consisting of:

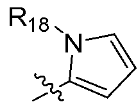 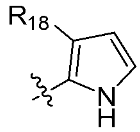 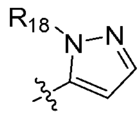 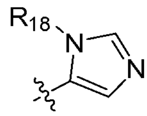 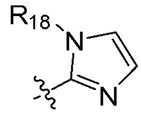

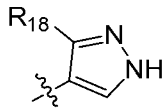 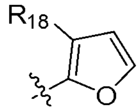 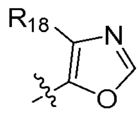 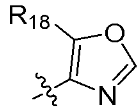 ,

wherein:

- $R_{18}$ is selected from the group consisting of hydrogen, halogen, hydroxy, hydroxy(alkyl $C_2$-$C_4$ linear or branched), alkyl $C_1$-$C_4$ linear or branched, alkoxy $C_1$-$C_4$ linear or branched, $C_3$-$C_7$ cycloalkoxy, haloalkoxy, nitrile, carboxyl, carboxyalkyl ester $C_1$-$C_3$, amine, N-alkylamine, N,N-dialkylamine, amino (alkyl $C_2$-$C_4$ linear or branched), sulfonamide, alkylsulfonylamide $C_1$-$C_6$, *N,N*-(dialkyl)sulfonylamide $C_1$-$C_6$, sulfonyl, alkylsulfonyl $C_1$-$C_6$, sulfinyl, alkylsulfinyl $C_1$-$C_6$, azetidinyloxy, azetidinylalkoxy $C_1$-$C_3$, alkoxy $C_1$-$C_2$ linear or branched, hydroxyazetidinyl, alkoxy $C_1$-$C_6$ containing one or more isotopically enriched atoms, such as hydrogen isotopes $^2$H, but not limited to the same.

**12.** PROCESS, according to claim 11, **characterized by** additionally comprising a compound formation step (c) of Formula (I) in which $R_7$ is linear or branched $C_1$-$C_6$ alkyl from the nucleophilic substitution reaction of a compound obtained in step (b), with linear or branched $C_1$-$C_6$ alkyl halides in the presence of inorganic base and polar aprotic solvents.

**13.** PROCESS, according to any of claims 11 or 12, **characterized by** said catalyst of step (b) being selected from concentrated hydrochloric acid, acetic acid, trifluoroacetic acid, formic acid, or combinations of these and said solvent being selected from dimethylformamide, dimethylsulfoxide, alcohols, or combinations thereof.

**14.** PROCESS, according to any of claims 12 or 13, **characterized by** said inorganic basis of step (c) being selected from $K_2CO_3$ or NaH.

**15.** KIT, **characterized by** comprising a pharmaceutical composition, as defined in claim 4, and an application device.

## INTERNATIONAL SEARCH REPORT

International application No.
**PCT/BR2024/050025**

**A. CLASSIFICATION OF SUBJECT MATTER**

***C07C 233/64***(2006.01)i; ***C07C 243/38***(2006.01)i; ***C07C 243/16***(2006.01)i; **C07C 15/00**(2006.01)i; **C07D 213/00**(2006.01)i; **C07D 401/00**(2006.01)i; **A61K 31/166**(2006.01)i; ***A61P 25/02***(2006.01)i; ***A61P 25/04***(2006.01)i; **A61P 29/00**(2006.01)i
CPC: C07C 233/64, C07C 243/38, C07C 243/16, C07C 15/00, C07D 213/00

According to International Patent Classification (IPC) or to both national classification and IPC

**B. FIELDS SEARCHED**

Minimum documentation searched (classification system followed by classification symbols)

C07C 233/64; C07C 243/38; C07C 243/16; C07C 15/00; C07D 213/00; C07D 401/00; A61K 31/166; A61P 25/02; A61P 25/04; A61P 29/00
CPC: C07C 233/64, C07C 243/38, C07C 243/16, C07C 15/00, C07D 213/00

Documentation searched other than minimum documentation to the extent that such documents are included in the fields searched

Database INPI-BR; Periódicos Capes

Electronic data base consulted during the international search (name of data base and, where practicable, search terms used)

Espacenet; Derwent Innovation; REGISTRY, CAPLUS, MARPAT (STN®)

**C. DOCUMENTS CONSIDERED TO BE RELEVANT**

| Category* | Citation of document, with indication, where appropriate, of the relevant passages | Relevant to claim No. |
|---|---|---|
| P,X | WO 2023010191 A1 (EUROFARMA LABORATORIOS S A [BR]) 09 February 2023 (2023-02-09)<br>See abstract and claims. | 1-7, 11-15 |
| X | CN 103880707 A (SHANGHAI JIAOTONG UNIV SCHOOL MEDICINE) 25 June 2014 (2014-06-25)<br>See abstract; paragraph 0024; claims. | 1-7, 11-15 |
| Y | Lopes A.B. et al. Characterization of amide bond conformers for a novel heterocyclic template of N-acylhydrazone derivatives. Molecules. 2013 Sep 25;18(10):11683-704. doi: 10.3390/molecules181011683. PMID: 24071978; PMCID: PMC6270085. See the whole document, especially the abstract and pages 11684 and 11685. | 1-7, 11-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 April 2024** | **24 April 2024** |
| Name and mailing address of the ISA/BR<br>**National Institute of Industrial Property (Brazil)**<br>**Rua Mayrink Veiga, 9, 6º andar, CEP 20.090-910 Rio de Janeiro – RJ**<br>**Brazil** | Authorized officer<br>**Rodinelli OLIVEIRA** |
| Telephone No. **(55 21) 3037-3742, 3037-3984** | Telephone No. **+55 21 3037 4528 - 3037 3319** |

Form PCT/ISA/210 (second sheet) (July 2022)

**INTERNATIONAL SEARCH REPORT**

International application No. **PCT/BR2024/050025**

**C. DOCUMENTS CONSIDERED TO BE RELEVANT**

| Category* | Citation of document, with indication, where appropriate, of the relevant passages | Relevant to claim No. |
|---|---|---|
| Y | Da Silva Y.K. et al. Synthesis and pharmacological evaluation of pyrazine N-acylhydrazone derivatives designed as novel analgesic and anti-inflammatory drug candidates. Bioorg Med Chem. 2010 Jul 15;18(14):5007-15. doi: 10.1016/j.bmc.2010.06.002. Epub 2010 Jun 8. PMID: 20598893. See the whole document, especially the abstract and pages 5007-5008 and 5014. | 1-7, 11-15 |
| Y | US 2008300240 A1 (OMEROS CORP [US]) 04 December 2008 (2008-12-04) See the whole document, especially the abstract, pages 2-10, example 7 and claims 1, 2, 3 and 10. | 1-7, 11-15 |
| Y | US 2006111409 A1 (MUTO SUSUMU [JP]) 25 May 2006 (2006-05-25) See abstract; pages 30-104; compounds 15, 304, 306 and 321; claim 1. | 1-7, 11-15 |
| Y | US 2006100257 A1 (MUTO SUSUMU [JP]) 11 May 2006 (2006-05-11) See abstract; pages 28-128; compounds 15, 304, 306 and 321; claim 1. | 1-7, 11-15 |
| Y | US 2006019958 A1 (MUTO SUSUMU [JP]) 26 January 2006 (2006-01-26) See abstract; pages 29-103; compounds 15, 304, 306 and 321; claim 1. | 1-7, 11-15 |
| Y | WO 2011069039 A1 (US HEALTH [US]) 09 June 2011 (2011-06-09) See abstract; pages 3, 4, 13-20, 30; claims 1, 14, 23. | 1-7, 11-15 |
| Y | WO 2012129562 A2 (BOUCHEZ LAURE [CH]) 27 September 2012 (2012-09-27) See abstract; claim 1. | 1-7, 11-15 |
| Y | WO 2020023802 A1 (HOPE CITY [US]) 30 January 2020 (2020-01-30) See abstract; pages 49-84, 88-98, 174-175. | 1-7, 11-15 |
| Y | EP 1514544 A1 (INST MED MOLECULAR DESIGN INC [JP]) 16 March 2005 (2005-03-16) See abstract; pages 41-108; compounds 15, 304, 306 and 321; claim 1. | 1-7, 11-15 |
| A | Rodrigues, F.A.R. et al. Biological evaluation of pyrazinamide derivatives as an anticancer class. ECB. 2014; 3(4): 358-361. https://www.eurchembull.com/uploads/paper/c5e03c07c95123429c53bc95785161d9.pdf | 1-7, 11-15 |
| A | WO 2012080729 A2 (PIKE IAN [GB]) 21 June 2012 (2012-06-21) The whole document | 1-7, 11-15 |
| A | CN 111925357 A (KUNMING INST BOTANY CAS) 13 November 2020 (2020-11-13) The whole document | 1-7, 11-15 |
| A | CN 110128343 A (SICHUAN PROVINCIAL PEOPLES HOSPITAL) 16 August 2019 (2019-08-16) The whole document | 1-7, 11-15 |
| A | WO 2013123071 A1 (CLEAVE BIOSCIENCES INC [US]) 22 August 2013 (2013-08-22) The whole document | 1-7, 11-15 |
| A | CN 105481765 A (JIANGSU AIFAN BIOLOGICAL PHARMACEUTICAL CO LTD) 13 April 2016 (2016-04-13) The whole document | 1-7, 11-15 |
| A | US 2010035932 A1 (SCHEPETKIN IGOR A [US]) 11 February 2010 (2010-02-11) The whole document | 1-7, 11-15 |
| A | WO 2004105488 A1 (BASF AG [DE]) 09 December 2004 (2004-12-09) The whole document | 1-7, 11-15 |

Form PCT/ISA/210 (second sheet) (July 2022)

**INTERNATIONAL SEARCH REPORT**

International application No.
**PCT/BR2024/050025**

**C. DOCUMENTS CONSIDERED TO BE RELEVANT**

| Category* | Citation of document, with indication, where appropriate, of the relevant passages | Relevant to claim No. |
|---|---|---|
| A | Shutske, G.M. Synthesis of Anhydro-3-mercapto-5-pyrazinyl-1,2,4-triazolium Hydroxides. J. Heterocyclic Chern., 18, 1017 (1981). https://onlinelibrary.wiley.com/doi/pdf/10.1002/jhet.5570180533 | 1-7, 11-15 |
| A | Gomes LR, Low JN, Rodrigues AS, Wardell JL, Lima CH, de Souza MV. Five N'-benzylidene-N-methylpyrazine-2-carbohydrazides. Acta Crystallogr C. 2013 May;69(Pt 5):549-55. doi: 10.1107/S0108270113010056. Epub 2013 Apr 17. PMID: 23629912. | 1-7, 11-15 |
| A | Howie, R. Alan, da Silva Lima, Camilo H., Kaiser, Carlos R., de Souza, Marcus V. N., Wardell, James L. and Wardell, Solange M. S. V. "Structures of (pyrazinecarbonyl)hydrazones of substituted benzaldehydes: different supramolecular arrangements from C—H···X (X = O, N, π) hydrogen bonds" Zeitschrift für Kristallographie, vol. 225, no. 4, 2010, pp. 158-166. https://doi.org/10.1524/zkri.2010.1235 | 1-7, 11-15 |

Form PCT/ISA/210 (second sheet) (July 2022)

**INTERNATIONAL SEARCH REPORT**

International application No.
**PCT/BR2024/050025**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **8-10**
because they relate to subject matter not required to be searched by this Authority, namely:

Claims 8-10 contain subject matter that falls under the provisions of PCT Rule 39.1(iv) concerning methods for treatment of the human or animal body by surgery or therapy.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/BR2024/050025**

| Patent document cited in search report | | | Publication date (day/month/year) | Patent family member(s) | | | Publication date (day/month/year) |
|---|---|---|---|---|---|---|---|
| WO | 2023010191 | A1 | 09 February 2023 | AR | 126669 | A1 | 01 November 2023 |
| | | | | CA | 3227730 | A1 | 09 February 2023 |
| | | | | IL | 310568 | A | 01 March 2024 |
| CN | 103880707 | A | 25 June 2014 | CN | 103880707 | B | 13 April 2016 |
| US | 2008300240 | A1 | 04 December 2008 | US | 7786139 | B2 | 31 August 2010 |
| | | | | US | 2011021509 | A1 | 27 January 2011 |
| | | | | US | 8278327 | B2 | 02 October 2012 |
| US | 2006111409 | A1 | 25 May 2006 | AU | 2003242137 | A1 | 22 December 2003 |
| | | | | CA | 2488342 | A1 | 18 December 2003 |
| | | | | CN | 1658850 | A | 24 August 2005 |
| | | | | CN | 100379410 | C | 09 April 2008 |
| | | | | CN | 101103977 | A | 16 January 2008 |
| | | | | EA | 200401609 | A1 | 30 June 2005 |
| | | | | EA | 008769 | B1 | 31 August 2007 |
| | | | | EP | 1510207 | A1 | 02 March 2005 |
| | | | | EP | 1510207 | A4 | 31 December 2008 |
| | | | | JP | WO2003103648 | A1 | 06 October 2005 |
| | | | | TW | 200407107 | A | 16 May 2004 |
| | | | | TW | I280876 | B | 11 May 2007 |
| | | | | WO | 03103648 | A1 | 18 December 2003 |
| US | 2006100257 | A1 | 11 May 2006 | AU | 2003242127 | A1 | 22 December 2003 |
| | | | | CA | 2487891 | A1 | 18 December 2003 |
| | | | | CN | 1658849 | A | 24 August 2005 |
| | | | | EA | 200401607 | A1 | 25 August 2005 |
| | | | | EA | 009523 | B1 | 28 February 2008 |
| | | | | EP | 1512396 | A1 | 09 March 2005 |
| | | | | EP | 1512396 | A4 | 31 December 2008 |
| | | | | JP | WO2003103647 | A1 | 06 October 2005 |
| | | | | TW | 200410671 | A | 01 July 2004 |
| | | | | WO | 03103647 | A1 | 18 December 2003 |
| US | 2006019958 | A1 | 26 January 2006 | AU | 2003242131 | A1 | 22 December 2003 |
| | | | | CA | 2487900 | A1 | 18 December 2003 |
| | | | | CN | 1658854 | A | 24 August 2005 |
| | | | | EA | 200401608 | A1 | 25 August 2005 |
| | | | | EA | 008622 | B1 | 29 June 2007 |
| | | | | EP | 1510210 | A1 | 02 March 2005 |
| | | | | EP | 1510210 | A4 | 07 January 2009 |
| | | | | JP | WO2003103658 | A1 | 06 October 2005 |
| | | | | TW | 200407112 | A | 16 May 2004 |
| | | | | WO | 03103658 | A1 | 18 December 2003 |
| WO | 2011069039 | A1 | 09 June 2011 | US | 2012316198 | A1 | 13 December 2012 |
| | | | | US | 8518968 | B2 | 27 August 2013 |
| WO | 2012129562 | A2 | 27 September 2012 | NONE | | | |
| WO | 2020023802 | A1 | 30 January 2020 | US | 2021355103 | A1 | 18 November 2021 |
| EP | 1514544 | A1 | 16 March 2005 | NONE | | | |
| WO | 2012080729 | A2 | 21 June 2012 | NONE | | | |
| CN | 111925357 | A | 13 November 2020 | CN | 111925357 | B | 06 June 2023 |
| CN | 110128343 | A | 16 August 2019 | NONE | | | |

Form PCT/ISA/210 (patent family annex) (July 2022)

**INTERNATIONAL SEARCH REPORT**
**Information on patent family members**

International application No.
**PCT/BR2024/050025**

| Patent document cited in search report | Publication date (day/month/year) | Patent family member(s) | Publication date (day/month/year) |
|---|---|---|---|
| WO 2013123071 A1 | 22 August 2013 | NONE | |
| CN 105481765 A | 13 April 2016 | NONE | |
| US 2010035932 A1 | 11 February 2010 | NONE | |
| WO 2004105488 A1 | 09 December 2004 | AR 045688 A1 | 09 November 2005 |
| | | AU 2004243492 A1 | 09 December 2004 |
| | | BR PI0410626 A | 20 June 2006 |
| | | CL 2004001296 A1 | 08 April 2005 |
| | | CN 1842272 A | 04 October 2006 |
| | | EP 1631143 A1 | 08 March 2006 |
| | | JP 2006528955 A | 28 December 2006 |
| | | KR 20060019558 A | 03 March 2006 |
| | | MX PA05012226 A | 10 February 2006 |
| | | ZA 200510406 B | 31 December 2008 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 10550080 B
- US 9765029 B
- US 10000475 B
- WO 2020261114 A
- WO 2020092667 A
- US 9163042 B
- WO 2014120808 A
- WO 2014120815 A
- WO 2018213426 A
- WO 2019014352 A
- WO 2015006280 A
- US 7928107 B
- WO 2018235851 A
- US 8629149 B
- JP 2017001991 B

### Non-patent literature cited in the description


- **SCHAIBLE.** *Langenbecks Arch. Surg.*, 2004, vol. 389, 237
- **SMITH.** *Pain.*, 2020, vol. 161 (1), 127
- **CAVALLI. INT.** *J. Immunopathol. Pharmacol.*, 2019, vol. 33, 2058738419838383
- **BOUHASSIRA.** *Rev Neurol (Paris).*, 2019, vol. 175 (1-2), 16
- **SCHOLZ.** *Nature Neurosci.*, 2002, vol. 5, 1062
- **COSTIGAN. ANNU.** *Rev. Neurosci.*, 2009, vol. 32, 1
- **DUCREUX.** *Brain*, 2006, vol. 129, 963
- **PAK. CURR.** *Pain Headache Rep.*, 2018, vol. 22 (2), 9
- **KUSHNAREV.** *Expert Opin. Investig. Drugs*, 2020, vol. 29 (3), 259
- **EMERY.** *Expert Opin. Ther. Targets*, 2016, vol. 20 (8), 975
- **CATTERALL.** *Nat. Chem. Biol.*, 2020, vol. 16, 1314
- **WISEDCHAISRI.** *Cell*, 2019, vol. 178 (4), 993
- **CLAIRFEUILLE.** *Science*, 2019, vol. 363, 1302
- **LERA-RUIZ.** *J. Med. Chem.*, 2015, vol. 58 (18), 7093
- **BAGAL.** *J. Med. Chem.*, 2013, vol. 56 (3), 593
- **BAGAL.** *Channels*, 2015, vol. 9 (6), 360
- *Law. Drug Discovery Today*, 2019, vol. 24 (7), 1389
- **BAGAL.** *Channels (Austin)*, 2015, vol. 9 (6), 360
- Vetter.. *Pharmacology & Therapeutics*, 2017, vol. 172, 73
- **AHUJA.** *Science*, 2015, vol. 350 (6267), 1491
- **KINGWELL.** *Nat. Rev. Drug Discov.*, 2019, vol. 18, 321
- **SAFINA.** *J. Med. Chem.*, 2021, vol. 64, 2953
- **LUO.** *J. Med. Chem.*, 2019, vol. 62, 831
- **BANKAR.** *Cell Reports*, 2018, vol. 24, 3133
- *Brown. Bioorg. Med. Chem.*, 2019, vol. 27 (1), 230
- **PAYNE. BR.** *J. Pharmacol.*, 2015, vol. 172 (10), 2654
- **BAGAL.** *Med. Chem. Lett.*, 2015, vol. 6 (6), 650
- **KORT.** *J. Med. Chem.*, 2008, vol. 51, 407
- **ZHANG.** *Neuropharmacology*, 2010, vol. 59, 201-207
- **KORNECOOK.** *J. Pharmacol. Exp. Ther.*, 2017, vol. 362, 146
- *Bagal. Channels (Austin)*, 2015, vol. 9 (6), 360
- **DEUIS.** *Neuropharmacology*, 2017, vol. 127, 87, 108
- **MCKERRALL.** *Bioorganic & Medicinal Chemistry Lett.*, 2018, vol. 28, 3141
- **BAGAL.** *Bioorganic & Medicinal Chemistry Lett.*, 2014, vol. 24, 3690